# EUROPÄISCHE PATENTSCHRIFT

(11) **EP 2 758 041 B1**
(45) Veröffentlichungstag und Bekanntmachung des Hinweises auf die Patenterteilung: **13.01.2021**
(21) Anmeldenummer: 12759485.1
(22) Anmeldetag: 20.09.2012
(51) Int. Cl.: A61K 31/00, A61K 31/122, A61K 31/165, A61K 31/36, A61K 31/366, A61K 31/435, A61K 31/443, A61K 31/4745, A61K 31/4747, A61K 31/5365, A61P 11/00, A61P 11/04, A61P 11/08

(54) **NIEDERMOLEKULARE MODULATOREN DES KÄLTE-MENTHOL-REZEPTORS TRPM8 UND DEREN VERWENDUNG**
LOW-MOLECULAR MODULATORS OF THE COLD-MENTHOL RECEPTOR TRPM8 AND USE OF SAME
MODULATEURS SOUS-MOLÉCULAIRES DU RÉCEPTEUR DE FROID ET DE MENTHOL TRPM8 ET LEUR UTILISATION

(30) Priorität: 20.09.2011 EP 11182033
(43) Veröffentlichungstag der Anmeldung: 30.07.2014
(73) Patentinhaber: BASF SE, 67056 Ludwigshafen am Rhein (DE)
(72) Erfinder: SUBKOWSKI, Thomas, 69198 Schriesheim (DE); BOLLSCHWEILER, Claus, 69118 Heidelberg (DE); WITTENBERG, Jens, 67117 Limburgerhof (DE); SIEGEL, Wolfgang, 67117 Limburgerhof (DE); PELZER, Ralf, 37699 Fürstenberg (DE)
(86) Internationale Anmeldenummer: PCT/EP2012/068538
(87) Internationale Veröffentlichungsnummer: WO 2013/041621

(56) Entgegenhaltungen:
- WO-A1-2008/154243
- WO-A1-2009/070552
- WO-A1-2010/026094
- WO-A2-2011/061330
- SU-A1- 776 048
- ZIEGLER ET AL: "The stereoselective synthesis of nor-marrianolic acids", THE STEREOSELECTIVE SYNTHESIS OF NOR-MARRIANOLIC ACIDS DISSERTATION,, 1. Januar 1966 (1966-01-01), Seite 75pp, XP009165663,
- CSABA SZÁNTAY ET AL: "Azaberbene Derivatives as [alpha]2A-Adrenoceptor Antagonists", ARCHIV DER PHARMAZIE, Bd. 335, Nr. 1, 1. Januar 2002 (2002-01-01), Seiten 22-26, XP055013218, ISSN: 0365-6233, DOI: 10.1002/1521-4184(200201)335:1<22::AID-ARD P22>3.0.CO;2-O
- M RUBIRALTA: "New synthesis of benzo [a]quinolizidin-2-ones via protected 2-aryl-4-piperidones", TETRAHEDRON, Bd. 43, Nr. 13, 1. Januar 1987 (1987-01-01), Seiten 3021-3030, XP55013077, ISSN: 0040-4020, DOI: 10.1016/S0040-4020(01)86842-3
- K. E. FAHRENHOLTZ ET AL: "Octahydrophenanthrene Analogs of Tetrabenazine", JOURNAL OF MEDICINAL CHEMISTRY, Bd. 9, Nr. 3, 1. Mai 1966 (1966-05-01), Seiten 304-310, XP055013079, ISSN: 0022-2623, DOI: 10.1021/jm00321a009
- MENENDEZ J C ET AL: "THE REACTIVITY OF 2 IMINOBENZO-A-QUINOLIZIDINES TOWARDS 2 MERCAPTOACETIC ACID", HETEROCYCLES, ELSEVIER SCIENCE PUBLISHERS B.V. AMSTERDAM, NL, Bd. 31, Nr. 11, 1. Januar 1990 (1990-01-01), Seiten 2065-2071, XP009154398, ISSN: 0385-5414
- DATABASE CHEMABS [Online] CHEMICAL ABSTRACTS SERVICE, COLUMBUS, OHIO, US; 1959, Itoh: "Synthesis of 2-oxo-3-ethyl-9,10-dimethoxy-1,2,3,4,6,8-h exahydro-11bH-benzo[a]quinolizine", XP002664516, Database accession no. 1960:56497
- DATABASE CHEMABS [Online] CHEMICAL ABSTRACTS SERVICE, COLUMBUS, OHIO, US; 1958, King, L.; Abramo, S. V.: "Reaction of pyridine-type bases with iodine and certain quinolines or isoquinolines containing a reactive methyl group", XP002664517, Database accession no. 1959:99863
- DATABASE CHEMABS [Online] CHEMICAL ABSTRACTS SERVICE, COLUMBUS, OHIO, US; 1959, Itoh, N.; Sugasawa, S.: "Bischler-Napiralski reaction. II. Synthesis of ketonic isoquinolines", XP002664518, Database accession no. 1959:99862
- POPP F D ET AL: "Synthesis of potential antineoplastic agents XXVI: 1,3,4,6,7,11b-hexahydro-9,10-dimethoxy-2H- benzo[a]2-quinolizinone derivatives", JOURNAL OF PHARMACEUTICAL SCIENCES, AMERICAN PHARMACEUTICAL ASSOCIATION, WASHINGTON, US, Bd. 67, Nr. 6, 1. Juni 1978 (1978-06-01), Seiten 871-873, XP002513807, ISSN: 0022-3549, DOI: 10.1002/JPS.2600670642
- EMANUELE CAROSATI ET AL: "Discovery of Novel and Cardioselective Diltiazem-like Calcium Channel Blockers via Virtual Screening", JOURNAL OF MEDICINAL CHEMISTRY, Bd. 51, Nr. 18, 25. September 2008 (2008-09-25), Seiten 5552-5565, XP55019769, ISSN: 0022-2623, DOI: 10.1021/jm800151n
- YU Q-S ET AL: "Preparation and characterization of tetrabenazine enantiomers against vesicular monoamine transporter 2", ACS MEDICINAL CHEMISTRY LETTERS, AMERICAN CHEMICAL SOCIETY, US, Bd. 1, Nr. 3, 10. Juni 2010 (2010-06-10), Seiten 105-109, XP009156796, ISSN: 1948-5875, DOI: 10.1021/ML1000189 [gefunden am 2010-03-31]
- CSABA SZANTAY ET AL: "Azaberbene Derivatives as [alpha]2A-Adrenoceptor Antagonists", ARCHIV DER PHARMAZIE, vol. 335, no. 1, 1 January 2002 (2002-01-01), pages 22-26, XP055013218, ISSN: 0365-6233, DOI: 10.1002/1521-4184(200201)335:1<22::AID-ARD P22>3.0.CO;2-O
- Matteo Chiurato ET AL: "New Efficient Route to Fused Aryltetrahydroindolizinones via N -Acyliminium Intermediates", European Journal of Organic Chemistry, vol. 2009, no. 18, 1 June 2009 (2009-06-01), pages 3011-3021, XP055471790, ISSN: 1434-193X, DOI: 10.1002/ejoc.200900195
- COMINS DANIEL L ET AL: "Synthesis of the Benzo-fused Indolizidine Alkaloid Mimics", BEILSTEIN JOURNAL OF ORGANIC CHEMISTRY, BIOMED CENTRAL, LONDON, GB, vol. 3, no. 1, 30 November 2007 (2007-11-30), page 42, XP021041116, ISSN: 1860-5397
- ALSARABI A ET AL: "Short diastereoselective synthesis of cis- and trans-hexahydropyrido[2,1-a]isoindole derivatives", TETRAHEDRON LETTERS, ELSEVIER, AMSTERDAM, NL, vol. 45, no. 49, 29 November 2004 (2004-11-29), pages 9003-9006, XP027297861, ISSN: 0040-4039 [retrieved on 2004-11-05]

## Beschreibung

Die Erfindung betrifft neuartige Modulatoren des Kälte-Menthol-Rezeptors TRPM8, Verfahren zur Modulation des TRPM8-Rezeptors unter Verwendung dieser Modulatoren; insbesondere die Verwendung der Modulatoren zur Induktion von Kältegefühl zu nichttherapeutischen Zwecken; sowie die unter Verwendung dieser Modulatoren hergestellten Gegenstände und Mittel.

### Hintergrund der Erfindung

Der Kälte-Menthol-Rezeptor TRPM8 (auch bezeichnet als Cold-Membrane Receptor (CMR)1) gehört zur Familie der "Transient Receptor Potential Ion Channels", wird spezifisch in einer speziellen Gruppe von Neuronen exprimiert und bildet in der Zellmembran Poren aus (jeweils 4 Einheiten lagern sich dabei zu einem Tetramer zusammen), die selektiv Ca²⁺ Ionen passieren lassen. Das Protein weist 6 Transmembrandomänen auf und einen cytoplasmatischen C- sowie N-Terminus. Durch niedrige Temperaturen (bevorzugt 10-25°C) wird dieser Rezeptor stimuliert, es kommt zu einer Signaltransduktion, die vom Nervensystem als Kältegefühl interpretiert wird. Der Rezeptor ist erstmals 2002 als Kälterezeptor in mehreren Publikationen beschrieben worden (Peier AM et al, .A TRP channel that senses cold stimuli and menthol.Cell. 2002 Mar 8;108(5):705-15; McKemy DD et al. Identification of a cold receptor reveals a general role for TRP channels in thermosensation Nature 2002 Mar 7; 416 (6876): 52-8; Zuker CS. Neurobiology: A cool ion channel Nature 2002 Mar 7; 416 (6876): 27-8)

Kühlende Verbindungen, wie z.B. Menthol, spielen bereits seit langem eine wichtige Rolle in der Geschmacks- und Riechstoffindustrie, um eine Assoziation mit Frische und Sauberkeit zu erzeugen. Für die Verbindung Menthol ist gezeigt worden, dass sie als ein natürlicher Modulator des Rezeptors TRPM8 wirkt (McKemy D.D., Molecular Pain 1, 2005, 16; McKemy D.D., Nature 416, 2002, 52-58; Peier A.M., Cell 108, 2002, 705-715; Dhaka A., Annu. Rev. Neurosci. 29, 2006, 135-161). Durch Applikation von Menthol wird TRPM8 aktiviert, wodurch ein Ca²⁺-Einstrom in die Kälte-sensitiven Neuronen bewirkt wird. Das dadurch erzeugte elektrische Signal wird schließlich als Kältegefühl wahrgenommen. Überhöhte Menthol-Konzentrationen führen zu Irritation und einer anästhetischen Wirkung. Darüber hinaus sind in verschiedenen Publikationen Mentholderivate mit ähnlicher Wirkung beschrieben worden (British Patent 1971 # 1315761 Watson H.R., J. Soc. Cosmet. Chem. 29, 1978, 185-200; Furrer S.M., Chem. Percept. 1, 2008, 119-126). Es gibt auch vereinzelte, strukturell mit Menthol nicht verwandte Verbindungen, die eine signifikante TRPM8-Modulation bewirken, wie z. B. Icilin (Wei E.T., J. Pharm. Pharmacol. 35, 1983, 110-112; WO 2004/026840), WS-23 oder in der Patentanmeldung WO 2007/019719 aufgeführte Verbindungen.

Weitere Wirkungen von Substanzen, die den TRPM8-Rezeptor bzw. seine Insektenanaloga modulieren, sind eine Repellent-Wirkung auf Insekten (WO 2002/015692; WO 2004/000023, US 2004/0028714), sowie Aktivität in der Antitumortherapie (z.B. eine Beeinflussung von Prostatatumoren), Aktivität bei der Behandlung von inflammatorischen Schmerzen / Hyperalgesie und eine Wirkung als TRPM8-Antagonisten in der Therapie von Blasensyndrom bzw. überaktiver Blase (Beck B. Cell Calcium, 41, 2007, 285-294; Levine J.D. Biochim. Biophys. Acta, Mol. Basis Dis. 1772, 2007, 989-1003; Mukerji G., BMC Urology 6, 2006, 6; US 2003/0207904; US 2005/6893626, Dissertation Behrendt H.J. 2004, Universität Bochum; Lashinger E.S.R. Am. J. Physiol. Renal Physiol. Am J Physiol Renal Physiol. 2008 Jun 18. [Epub ahead of print]; PMID: 18562636).

Viele der bisher gefundenen Modulatoren von TRPM8 weisen jedoch Mängel in Bezug auf Wirkstärke, Wirkdauer, Haut-/Schleimhautreizung, Geruch, Geschmack, Löslichkeit und /oder Flüchtigkeit auf.

In der Internationalen Patentanmeldung WO 2010/026094 der Anmelderin werden einzelne spezielle Verbindungen zur Modulation des TRPM8-Rezeptors vorgeschlagen. Diese umfassen die folgenden, konkret offenbarten Verbindungen: 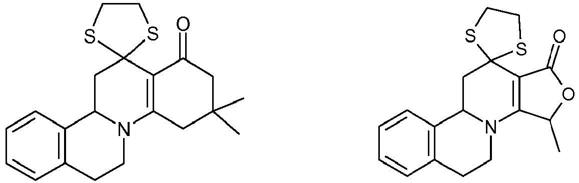 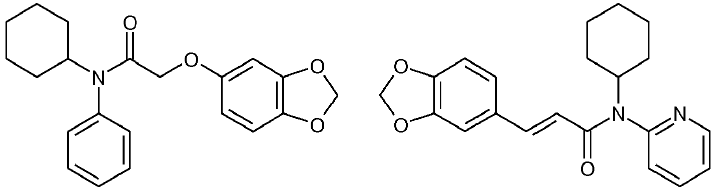 wobei die Verbindung in chemisch reiner oder angereicherter Form, als einzelnes Stereoisomer oder in Form von Stereoisomeren-Gemischen vorliegen kann.

In der WO 2011/061330 der Anmelderin sind zudem drei neuartige Substanzklassen von gattungsgemäßen TRPM-8 Modulatoren beschrieben.

Es besteht nach wie vor Bedarf an weiteren Stoffen zur Modulation des TRPM8-Rezeptors; insbesondere Stoffen zur Induktion von Kältegefühl auf Haut und Schleimhaut.

### Kurzfassung der Erfindung

Aufgabe der vorliegenden Erfindung war es daher, neue Substanzen zu identifizieren, die zu einer Modulation des TRPM8-Rezeptors führen, welche als Alternativen zu den bisher bekannten Modulatoren einsetzbar sind. Solche Verbindungen sollten sich insbesondere auch für Anwendungen im Bereich Kosmetik (z.B. hair care, skin care, oral care), Ernährung (feed/food), Textil, OTC-Produkte (z.B. Brandsalbe), Pharmaka (z.B. Tumorbehandlung, Blasenschwäche) oder Verpackungen, eignen.

### Detaillierte Beschreibung der Erfindung:

### 1. Definitionen

### 1.1 Allgemeine Begriffe

In der Literatur gibt es verschiedene Synonyme für "TRPM8": TRPP8, LTRPC6, CMR1, MGC2849, transient receptor potential cation channel subfamily M member 8. Im Sinne der vorliegenden Erfindung sind alle Bezeichnungen mit umfasst. Mit umfasst sind auch alle funktionalen Modifikationen des Rezeptors, wie insbesondere Splicevarianten, Isoformen, wie z.B. TRPM8 CRA_a, TRPM8 CRA_b und alle analogen Rezeptoren aus verschiedenen Organismen, wie Mensch, Maus, Ratte. Die Nukleotid- bzw. Aminosäuresequenzen der verschiedenen Rezeptoren sind an sich bekannt und in Sequenzdatenbanken hinterlegt. So ist z.B. die Sequenzinformation für hTRPM8 unter der Nummer NM_024080 eingetragen.

Ein "Modulator" im Sinne der Erfindung stellt eine Verbindung dar, die als Agonist und/oder Antagonist des TRPM8-Rezeptors *in vivo* und/oder *in vitro,* insbesondere *in vivo* wirken kann. Das erfindungsgemäße Verfahren betrifft die in-vitro Modulation des TRPM8-Rezepetors.

Geeignete Modulatoren können dabei entweder nur als Antagonist oder Agonist, insbesondere nur als Agonist, oder sowohl als Antagonist als auch als Agonist agieren. Dabei können sich insbesondere eine agonistische oder eine antagonistische Wirkung in Abhängigkeit von der jeweiligen gewählten Modulatorkonzentration einstellen.

Ein "Agonist" ist dabei eine Verbindung, welche eine Aktivierung des TRPM8-Rezeptors vermittelt, also einen Ca²⁺ -Einstrom in die kälte-sensitiven Neuronen induziert und damit ein Kältegefühl vermittelt. Ein "Antagonist" ist dagegen eine Verbindung, welche dieser Aktivierung des TRPM8-Rezeptors entgegenwirken kann.

Die erfindungsgemäß anzuwendenden Mediatoren können ihre Wirkung dadurch ausüben, dass sie reversibel oder irreversibel, spezifisch oder unspezifisch an ein TRPM8-Rezeptormolekül binden. Gewöhnlich erfolgt die Bindung nicht-kovalent über ionische und/oder nichtionische, wie z.B. hydrophobe, Wechselwirkungen mit dem Rezeptormolekül. "Spezifisch" umfasst dabei sowohl ausschließliche Wechselwirkung mit einem oder mehreren verschiedenen TRPM8-Rezeptormolekülen (wie z.B. TRPM8-Molekülen verschiedenen Ursprungs oder verschiedenen Isoformen). "Unspezifisch" ist dagegen eine Wechselwirkung des Modulators mit mehreren verschiedenen Rezeptormolekülen unterschiedlicher Funktion und/oder Sequenz, wobei sich jedoch als Folge eine gewünschte agonistische und/oder antagonistische Modulation (wie oben beschrieben) des TRPM8-Rezeptors feststellen lässt.

Unter "Standardbedingungen" in einem zellularen Aktivitätstest für erfindungsgemäß anzuwendende Modulatoren versteht man in diesem Zusammenhang einen Aktivitätstest, durchgeführt mit HEK293-Zellen, welche transformiert wurden mit humanem TRPM8 und beladen sind mit Calcium-sensitivem Farbstoff (wie z.B. Fluo-4AM, d.h. Fluo-4-Acetoxymethylester), anschließende Zugabe der Testverbindung und Nachweis der Farbänderung, wobei Versuchsdurchführung bei 37 °C erfolgt; wie z.B. beschrieben in Referenzbeispiel 3, unten, oder bei Behrendt et al (2004) a.a.O).

Eine "abgewandelte Form" oder "Derivat" eines erfindungsgemäß anzuwendenden Modulators wird auch als "funktionales Analog" oder "funktional äquivalente Verbindung" bezeichnet, insbesondere wenn sie weiterhin die gewünschte biologische Aktivität (Rezeptor-TRPM8-Modulation) zeigen. "Derivate" im Sinne der Erfindung sind auch Verbindungen die eine Kopplung der konkret offenbarten Substanzen an feste Träger erlauben; eine große Auswahl an entsprechenden Linker/Spacer-Gruppen ist dem Fachmann bekannt. Die Derivatisierung kann dabei vor der Kopplung an eine feste Phase oder erst durch die Kopplung erfolgen.

Ein erfindungsgemäß anzuwendender Modulator dient insbesondere zur Induktion von Kältegefühl, bei Mensch und/oder Tier. Eine "Induktion von Kältegefühl" liegt vor, wenn die Verbindung im oben beschriebenen zellulären Aktivitätstest einen agonistischen Effekt auf hTRPM8 zeigt.

Erfindungsgemäße Mittel enthalten neben den für das jeweilige Mittel üblichen Bestandteilen eine "wirksame Menge" wenigstens eines erfindungsgemäß anzuwendenden Modulators. "Wirksam" bedeutet in diesem Zusammenhang eine Konzentration des Modulators, die ausreicht, um bei Applikation des Mittels (z.B. Auftragung auf die Haut) den gewünschten Effekt, wie z.B. pharmakologischen Effekt, oder sensorischen Effekt, wie z.B. den olfaktorischen Kälteeffekt, zu vermitteln.

Eine "topische" Anwendung umfasst insbesondere, cutane oder orale Anwendungsformen.

Sofern keine anderen Angaben gemacht werden, sind sämtliche hierin erwähnten Verbindungen in allen ihren isomeren oder stereoisomeren Formen, als Reinstoff oder als Gemisch von von isomeren und/oder stereoisomeren Formen mit umfasst.

### 1.2 Chemische Begriffe

Werden keine anderslautenden Angaben gemacht, so gelten im Rahmen der vorliegenden Erfindung die folgenden allgemeinen Bedeutungen:
**Halogen:** F, Cl, Br oder J
**Alkyl sowie alle Alkylteile in davon abgeleiteten Resten, wie z.B. Alkoxy, Alkylthio, Alkoxyalkyl, Alkoxyalkoxy, Alkylamino und Dialkylamino:** gesättigte, geradkettige oder verzweigte Kohlenwasserstoffreste mit 1 bis 4, 1 bis 6, 1 bis 8, 1 bis 10 oder 1 bis 10 Kohlenstoffatomen, z. B.
   - **C₁-C₆-Alkyl** wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.
   - **C₁-C₆-Alkoxy,** umfassend **C₁-C₄-Alkoxy,** wie z.B. Methoxy, Ethoxy, n-Propoxy, 1-Methylethoxy, Butoxy, 1-Methylpropoxy, 2-Methylpropoxy oder 1,1-Dimethylethoxy; sowie z. B. Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
**Alkenyl:** ein- oder mehrfach, insbesondere einfach ungesättigte, geradkettige oder verzweigte Kohlenwasserstoffreste mit 2 bis 4, 2 bis 6, 2 bis 8, 2 bis 10 oder 2 bis 20 Kohlenstoffatomen und einer Doppelbindung in einer beliebigen Position, z. B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
**Alkinyl:** ein- oder mehrfach, insbesondere einfach ungesättigte, geradkettige oder verzweigte Kohlenwasserstoffreste mit 2 bis 4, 2 bis 6, oder 2 bis 8, Kohlenstoffatomen und einer Dreifachbindung in einer beliebigen Position, insbesondere die zu den oben explizit genannten Alkenylresten analogen Reste mit C-C-Dreifachbindung; z. B. C₂-C₆-Alkinyl, wie Ethinyl, 1-Propinyl, 2-Propinyl, 1-Butinyl, 2-Butinyl, 3-Butinyl, 1-Pentinyl, 2-Pentinyl, 3-Pentinyl, 4-Pentinyl, 3-Methyl-1-butinyl, 1-Methyl-2-butinyl, 1-Methyl-2-butinyl, 1-Methyl-3-butinyl, 2-Methyl-3-butinyl, 1,1-Dimethyl-2-propinyl, 1-Ethyl-2-propinyl, 1-Hexinyl und dessen isomere Formen.
**Halo(gen)alkyl:** geradkettige oder verzweigte Alkylgruppen mit 1 bis 4, 1 bis 6, 1 bis 8, 1 bis 10 oder 1 bis 20 Kohlenstoffatomen (wie vorstehend genannt), wobei in diesen Gruppen teilweise oder vollständig die Wasserstoffatome durch Halogenatome wie vorstehend genannt ersetzt sein können, z. B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl und 1,1,1-Trifluorprop-2-yl.
**Halogenalkoxy:** für einen Alkoxyrest mit 1 bis 8, insbesondere 1 bis 6 und speziell 1 bis 4 C-Atomen wie vorstehend genannt, der partiell oder vollständig durch Fluor, Chlor, Brom und/oder lod, vorzugsweise durch Fluor substituiert ist, also z. B. OCH₂F, OCHF₂, OCF₃, OCH₂Cl, OCHCl₂, OCCl₃, Chlorfluormethoxy, Dichlorfluormethoxy, Chlordifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, OC₂F₅, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, OCH₂-C₂F₅, OCF₂-C₂F₅, 1-(CH₂F)-2-fluorethoxy, 1-(CH₂Cl)-2-chlorethoxy, 1-(CH₂Br)-2-bromethoxy, 4-Fluorbutoxy, 4-Chlorbutoxy, 4-Brombutoxy oder Nonafluorbutoxy;
**Cycloalkyl:** carbocyclische Reste mit 3 bis 20 Kohlenstoffatomen, wie z.B. C₃-C₁₂-Cycloalkyl, wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl, Cycloheptyl, sowie Cyclopropyl-methyl, Cyclopropyl-ethyl, Cyclobutyl-methyl, Cyclobutyl-ethyl, Cyclopentyl-methyl, Cyclopentyl-ethyl, Cyclohexyl-methyl oder C₃-C₇-Cycloalkyl, wie Cyclopropyl, Cyclobutyl, Cyclopentyl, Cyclohexyl, Cycloheptyl, Cyclopropyl-methyl, Cyclopropyl-ethyl, Cyclobutyl-methyl, Cyclopentyl-ethyl, Cyclohexyl-methyl, wobei die Anbindung an den Rest des Moleküls über jegliches geeignetes C-Atom erfolgen kann.
**Cycloalkenyl:** monocyclische, einfach ungesättigte Kohlenwasserstoffgruppen mit 5 bis 8, vorzugsweise bis 6 Kohlenstoffringgliedern, wie Cyclopenten-1-yl, Cyclopenten-3-yl, Cyclohexen-1-yl, Cyclohexen-3-yl und Cyclohexen-4-yl;
**Alkylen:** geradkettige oder ein- oder mehrfach verzweigte Kohlenwasserstoff-Brückengruppen mit 1 bis 20 Kohlenstoffatomen, wie z.B. C₁-C₈-Alkylengruppen oder C₁-C₇-Alkylengruppen ausgewählt unter -CH₂-, -(CH₂)₂-, -(CH₂)₃-,-(CH₂)₄-, -(CH₂)₂-CH(CH₃)-, -CH₂-CH(CH₃)-CH₂- , (CH₂)₄-, -(CH₂)₅-, -(CH₂)₆, -(CH₂)₇-, -(CH₂)₈-, -CH(CH₃)-CH₂-CH₂-CH(CH₃)-, -CH(CH₃)-CH₂-CH₂-CH₂-CH(CH₃)- oder -CH(CH₃)-CH₂-CH₂-CH₂-CH₂-CH(CH₃)- oder C₁-C₄-Alkylengruppen ausgewählt unter -CH₂-, -(CH₂)₂-, -(CH₂)₃-,-(CH₂)₄-, -(CH₂)₂-CH(CH₃)-, -CH₂-CH(CH₃)-CH₂-.
**Alkenylen:** die ein- oder mehrfach, insbesondere einfach ungesättigten Analoga obiger Alkylengruppen mit 2 bis 20 Kohlenstoffatomen, insbesondere für C₂-C₇-Alkenylene oder C₂-C₄-Alkenylen, wie -CH=CH-, -CH=CH-CH₂-, - CH₂-CH=CH-, -CH=CH-CH₂-CH₂-, -CH₂-CH=CH-CH₂-, -CH₂-CH₂-CH=CH-, -CH(CH₃)-CH=CH-, -CH₂-C(CH₃)=CH-.
**Aryl:** ein- oder mehrkernige, vorzugsweise ein- oder zweikernige, gegebenenfalls substituierte aromatische Reste mit 6 bis 20, wie z.B. 6 bis 10, Ring-Kohlenstoffatomen, wie z.B. Phenyl, Biphenyl, Naphthyl, wie 1- oder 2-Naphthyl, Tetrahydronaphthyl, Fluorenyl, Indenyl und Phenanthrenyl. Diese Arylreste können gegebenenfalls 1, 2, 3, 4, 5 oder 6 gleiche oder verschiedene Substituenten tragen. Im Falle eines mehrkernigen Arylrestes weist wenigstens einer der Kerne aromatischen Charakter auf; es können aber auch mehrere oder alle Kerne aromatischen Charakter besitzen.
**Arylalkyl:** die Aryl-substituierten Analoga obiger Alkylreste, wobei Aryl ebenfalls die oben angegebenen Bedeutungen besitzt, wie z.B. Phenyl-C₁-C₄-Alkylreste ausgewählt unter Phenyl-methyl oder Phenyl-ethyl.
**Aryloxy:** die Sauerstoff-verknüpften Analoga obiger gegebenenfalls substituierter Arylreste.
**Heterocyclyl:** fünf- bis siebengliedriger gesättigter, partiell ungesättigter oder aromatischer Heterocyclen bzw. Heterocyclylreste, enthaltend ein, zwei, drei oder vier Heteroatome aus der Gruppe O, N oder S. Beispielsweise können folgende Untergruppen genannt werden
   - 5- oder 6-gliedriges gesättigtes oder einfach ungesättigtes Heterocyclyl, enthaltend ein bis zwei Stickstoffatome und/oder ein Sauerstoff- oder Schwefelatom oder ein oder zwei Sauerstoff- und/oder Schwefelatome als Ringglieder, z. B. 2-Tetrahydrofuranyl, 3-Tetrahydrofuranyl, 2-Tetrahydrothienyl, 3-Tetrahydrothienyl, 1-Pyrrolidinyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 2-Pyrrolin-2-yl, 2-Pyrrolin-3-yl, 3-Pyrrolin-2-yl, 3-Pyrrolin-3-yl, 1-Piperidinyl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl und 2-Piperazinyl;
   - 5-gliedriges aromatisches Heterocyclyl (= **Heteroaryl** bzw. **Hetaryl),** enthaltend neben Kohlenstoffatomen ein, zwei oder drei Stickstoffatome oder ein oder zwei Stickstoffatome und ein Schwefel- oder Sauerstoffatom als Ringglieder, z. B. 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, und 1,3,4-Triazol-2-yl;
   - 5-gliedriges aromatisches Heterocyclyl (= **Heteroaryl** bzw. **Hetaryl),** das 1, 2, 3 oder 4 Stickstoffatome als Ringglieder aufweist, wie 1-, 2- oder 3-Pyrrolyl, 1-, 3- oder 4-Pyrazolyl, 1-, 2- oder 4-Imidazolyl, 1,2,3-[1H]-Triazol-1-yl, 1,2,3-[2H]-Triazol-2-yl, 1,2,3-[1H]-Triazol-4-yl, 1,2,3-[1H]-Triazol-5-yl, 1,2,3-[2H]-Triazol-4-yl, 1,2,4-[1H]-Triazol-1-yl, 1,2,4-[1H]-Triazol-3-yl, 1,2,4-[1H]-Triazol-5-yl, 1,2,4-[4H]-Triazol-4-yl, 1,2,4-[4H]-Triazol-3-yl, [1H]-Tetrazol-1-yl, [1H]-Tetrazol-5-yl, [2H]-Tetrazol-2-yl und [2H]-Tetrazol-5-yl;
   - 5-gliedriges aromatisches Heterocyclyl (= **Heteroaryl** bzw. **Hetaryl),** das 1 unter Sauerstoff und Schwefel ausgewähltes Heteroatome und gegebenenfalls 1, 2 oder 3 Stickstoffatome als Ringglieder aufweist, beispielsweise 2-Furyl, 3-Furyl, 2-Thienyl, 3-Thienyl, 3- oder 4-Isoxazolyl, 3- oder 4- Isothiazolyl, 2-, 4- oder 5-Oxazolyl, 2-, 4 oder 5-Thiazolyl, 1,2,4-Thiadiazol-3-yl, 1,2,4-Thiadiazol-5-yl, 1,3,4-Thiadiazol-2-yl, 1,2,4-Oxadiazol-3-yl, 1,2,4-Oxadiazol-5-yl und 1,3,4-Oxadiazol-2-yl;
   - 6-gliedriges Heterocyclyl (= **Heteroaryl** bzw. **Hetaryl),** enthaltend neben Kohlenstoffatomen ein oder zwei bzw. ein, zwei oder drei Stickstoffatome als Ringglieder, z. B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,2,4-Triazin-3-yl; 1,2,4-Triazin-5-yl, 1,2,4-Triazin-6-yl und 1,3,5-Triazin-2-yl;
**"Heterocyclyl" umfasst auch "mehrkernige", wie z.B. zwei-** oder dreikernige, cyclische Ringsysteme, in denen einer der oben genannten einkernigen Heterocyclylreste mit wenigstens einem weiteren, gleichen oder verschiedenen Heterocyclus, wenigstens einem Arylring und/oder wenigstens einem Cycloalkyl- oder Cycloalkenyl-Ring jeweils gemäß obiger Definition kondensiert ist.
**"Heteroaryl" umfasst auch "mehrkernige", wie z.B. zwei-** oder dreikernige, cyclische Ringsysteme, in denen einer der oben genannten einkernigen Heteroarylreste mit wenigstens einem weiteren gleichen oder verschiedenen Heteroarylring, wenigstens einem Arylring und/oder wenigstens einem Cycloalkyl oder Cycloalkenylring jeweils gemäß obiger Definition kondensiert ist.
**Heterocyclyloxy** bzw. **Heteroaryloxy** steht für die Sauerstoff-verknüpften Analoga obigerr Heterocyclyl- bzw. Heteroarylreste.
**Substituenten,** wie insbesondere für obige Reste, sind insbesondere ausgewählt sind unter Ketogruppen, - COOH, -COO-Alkyl, -OH, -SH, -CN, Amino, -NO₂, Alkyl, oder Alkenylgruppen, wobei in den Alkyl- oder Alkenylgruppen ein oder mehrere H-Atome durch Halogen, ersetzt sein können; oder zwei benachbarte Alkyl- oder Alkenylsubstituenten zusammen mit dem C-Atomen an die sie gebunden sind einen 4 bis 7-gliedrigen, insbesondere 5- oder 6-gliedrigen Ring bilden können, und wobei dieser ring auch ein oder mehrere, gleiche oder verschieden Heteroatome, wie O, S, N- oder NH im Ring tragen kann.

Die in diesem Kapitel ausgeführten Definitionen gelten auch für spezielle Aspekte der Erfindung, sofern nicht anders ausgeführt.

### 2. Besondere Ausführungsformen der Erfindung

Der Umfang der Erfindung ist anhand der Ansprüche definiert. Die Erfindung betrifft insbesondere folgende spezielle Ausführungsformen:
1. Verfahren zur in-vitro Modulation des Kälte-Menthol-Rezeptors TRPM8, wobei man den Rezeptor mit wenigstens einem Modulator in Kontakt bringt, der ausgewählt ist unter den folgenden Verbindungen:

| Nr. | Formel | Nr. | Formel |
|---|---|---|---|
| P1 | 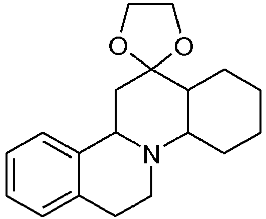 | P2 | 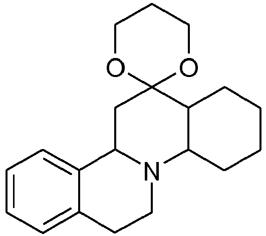 |
| P3 | 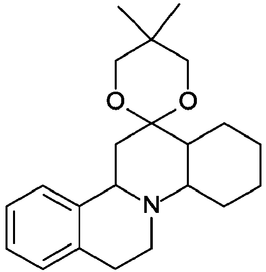 | P4 | 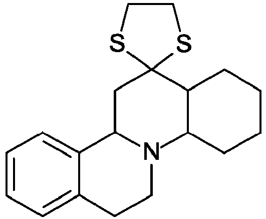 |
| P5 | 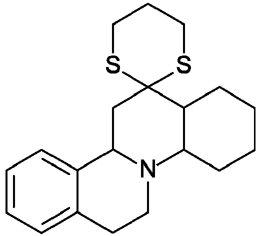 | P6 | 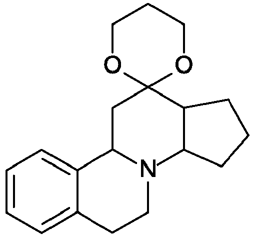 |
| P7 | 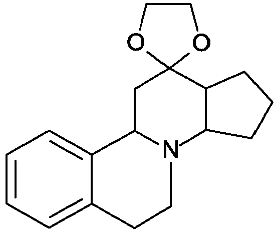 | P8 | 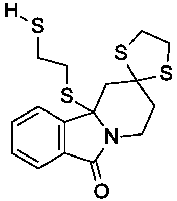 |
| P9 | 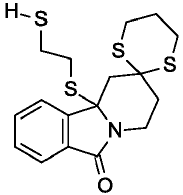 | P10 | 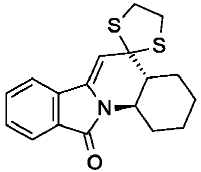 |
| P11 | 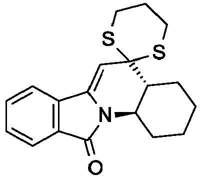 | P12 | 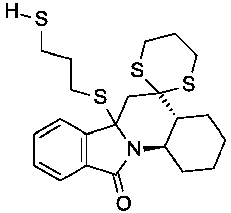 |
| P13 | 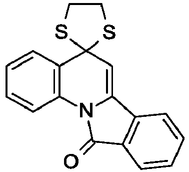 | P14 | 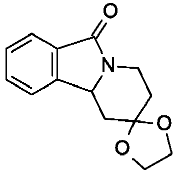 |
| P15 | 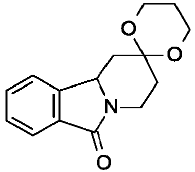 | P16 | 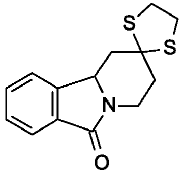 |
| P17 | 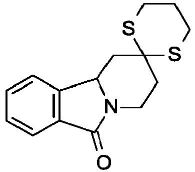 | P18 | 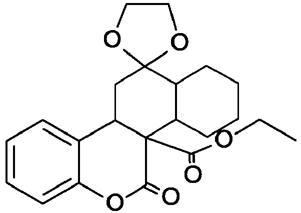 |
| P19 | 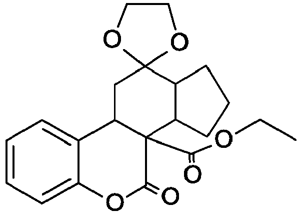 | P20 | 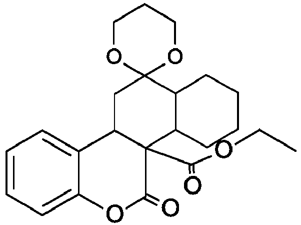 |
| P21 | 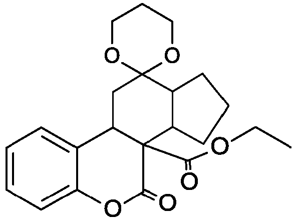 | P22 | 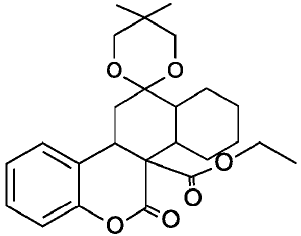 |
| P23 | 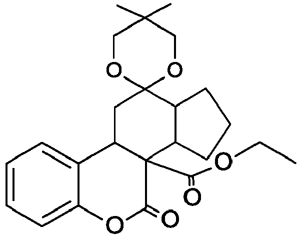 | P24 | 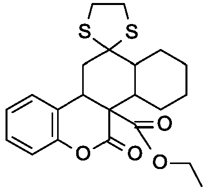 |
| P25 | 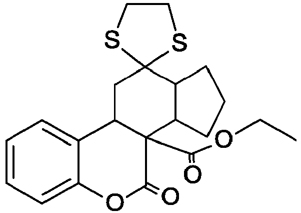 | P26 | 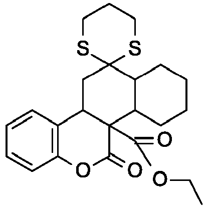 |
| P27 | 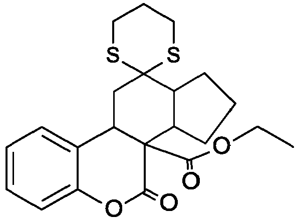 | P28 | 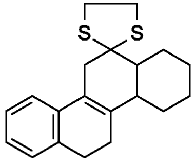 |
| P29 | 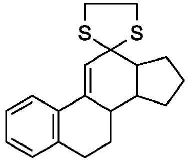 | P30 | 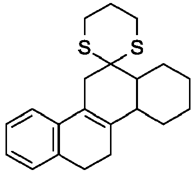 |
| P31 | 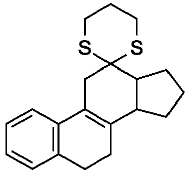 | | |

sowie Salze dieser Verbindungen, insbesondere Säureadditionssalze mit anorganischen oder insbesondere organischen, ein- oder insbesondere mehrwertigen Carbonsäuren;
und gegebenenfalls in Stereoisomeren-reiner Form oder als Gemisch von Stereoisomeren.
2. Verfahren nach Ausführungsform 1, wobei man den Rezeptor mit wenigstens einer Verbindung in Kontakt bringt, welche in einem zellulären Aktivitätstest unter Verwendung von Zellen, welche den humanen TRPM8-Rezeptor rekombinant exprimieren, die Permeabilität dieser Zellen für Ca²⁺ Ionen modulieren.
3. Verfahren nach einem der vorhergehenden Ausführungsformen, wobei die modulierende Verbindung auf die zelluläre Ca²⁺-Ionen-Permeabilität agonistisch oder antagonistisch wirkt.
4. Verfahren nach einem der vorhergehenden Ausführungsformen, wobei die modulierende Verbindung ein TRPM8-Rezeptor-Agonist ist.
5. Verwendung einer Verbindung gemäß der Definition in Ausführungsform 1,
a) zur Induktion von Kältegefühl bei Mensch und/oder Tier zu nichttherapeutischen Zwecken;
d) zur Induktion eines Kältegefühls durch eine Verpackung zu nichttherapeutischen Zwecken; oder
e) zur Induktion eines Kältegefühls durch ein Textil zu nichttherapeutischen Zwecken.

6. Verbindung gemäß der Definition in Ausführungsform 1,
b) zur Verwendung als aktiven Bestandteil eines pharmazeutischen Mittels; oder
c) zur Verwendung bei der Behandlung von Prostatakarzinomen, zur Behandlung von Blasenschwäche oder in der Schmerztherapie.

7. Verwendung nach Ausführungsform 5, oder Verbindung zur Verwendung nach Ausführungsform 6, wobei ein Mittel eingesetzt wird, umfassend eine oder mehrere der Verbindungen gemäß der Definitionen aus einem der Ausführungsformen 1 bis 4 in einer Konzentration von 0,1 ppm bis 10 Gew-% bezogen auf das Gesamtgewicht des Mittels zur Erzielung einer Kühlwirkung auf Haut oder Schleimhaut, die verglichen mit der Kühlwirkung eines Mittels gleicher Zusammensetzung, bei dem lediglich die Verbindung oder die Verbindungen gemäß der Definitionen aus einem der Ausführungsformen 1 bis 4 gegen Menthancarbonsäure-N-ethylamid in gleicher Konzentration ausgetauscht sind, um wenigstens 10 Minuten verlängert ist.
8. Mittel enthaltend wenigstens eine Verbindung nach Ausführungsform 1, wobei das Mittel ausgewählt ist unter
a) pharmazeutischen Mitteln, wie Antitumormittel, Mittel zur Behandlung von Erkrankungen der Blase, Schmerzmittel;
b) Nahrungsmitteln, wie Speiseeis, Mousse, Creme, Getränke, Süßwaren,
c) Mundpflegemitteln, wie Zahnpasta, Mundwasser, Kaugummi,
d) Körperpflegemitteln, wie Haut- oder Haarpflegemitteln, wie Sonnencreme, Sonnenbrandcreme, Lotionen, Shampoos, Pflaster,
e) Schäumen und Gelen,
wobei die Verbindung nach Ausführungsform 1 ausgewählt ist unter den Verbindungen P1 bis P13, P15 und P17 bis P31; sowie Salzen dieser Verbindungen, insbesondere Säureadditionssalzen mit anorganischen oder insbesondere organischen, ein- oder insbesondere mehrwertigen Carbonsäuren; und gegebenenfalls in Stereoisomeren-reiner Form oder als Gemisch von Stereoisomeren.
9. Mittel nach Ausführungsform 8, umfassend
a) einen oder mehrere weitere Stoffe mit physiologischer Kühlwirkung, wobei der weitere Stoff bzw. einer, mehrere oder sämtliche der weiteren Stoffe (i) einen geschmacklichen Effekt verursachen oder (ii) keinen geschmacklichen Effekt verursachen,
   und/oder
b) einen oder mehrere Aromastoffe ohne physiologische Kühlwirkung und/oder
c) einen oder mehrere trigeminal oder mundwässernd wirksame Stoffe ohne physiologische Kühlwirkung
   und/oder
d) (iii) eine oder (iv) mehrere Verbindungen, die im Falle (iv) unabhängig von einander oder gemeinsam zusätzlich einen geschmacksmodulierenden Effekt und/oder einen trigeminalen und/oder einen mundwässernden Reiz verursachen.

10. Produkt enthaltend wenigstens einer Verbindung nach Ausführungsform 1, ausgewählt unter
a) Textilprodukten,
b) Verpackungsmaterialien,
c) Tabakprodukten;
d) Heilmitteln;
e) Hygieneprodukten, oder
f) Erfrischungstüchern,
wobei die Verbindung nach Ausführungsform 1 ausgewählt ist unter den Verbindungen P1 bis P13, P15 und P17 bis P31; sowie Salzen dieser Verbindungen, insbesondere Säureadditionssalzen mit anorganischen oder insbesondere organischen, ein- oder insbesondere mehrwertigen Carbonsäuren; und gegebenenfalls in Stereoisomeren-reiner Form oder als Gemisch von Stereoisomeren.
11. Mittel nach einem der Ausführungsformen 8 oder 9 zur Verwendung bei der Vorbeugung gegen, Bekämpfung oder Linderung von Husten-, Schnupfen-, Entzündungs-, Halsschmerz- oder Heiserkeitssymptomen.
12. Verbindung gemäß der Definition nach Ausführungsform 1, ausgewählt unter den Verbindungen P1 bis P13, P15 und P17 bis P31; sowie Salze dieser Verbindungen, insbesondere Säureadditionssalze mit anorganischen oder insbesondere organischen, ein- oder insbesondere mehrwertigen Carbonsäuren; und gegebenenfalls in Stereoisomeren-reiner Form oder als Gemisch von Stereoisomeren.
13. Verbindung gemäß der Definition nach Ausführungsform 12, ausgewählt unter den Verbindungen P4, P5 und P6; sowie Salze dieser Verbindungen, insbesondere Säureadditionssalze mit anorganischen oder insbesondere organischen, ein- oder insbesondere mehrwertigen Carbonsäuren; und gegebenenfalls in Stereoisomeren-reiner Form oder als Gemisch von Stereoisomeren.

Ferner sind die folgenden Aspekte offenbart (nur teilweise erfindungsgemäß, d.h. nur insofern sie durch die Ansprüche gedeckt sind):
1. Verfahren zur in-vitro oder in-vivo Modulation des Kälte-Menthol-Rezeptors TRPM8, wobei man den Rezeptor mit wenigstens einem Modulator in Kontakt bringt, der ausgewählt ist unter Verbindungen des folgenden Strukturtyps I: 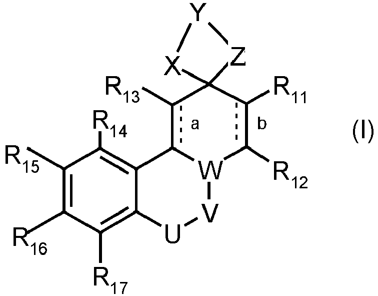 worin
   die Bindungen a und b unabhängig voneinander eine C-C Einfachbindung oder eine C-C-Doppelbindung oder a und b gleichzeitig je eine C-C Doppelbindung oder gleichzeitig je eine C-C Einfachbindung darstellen;
   U -CH₂-, -O- oder eine chemische Einfachbindung steht;
   V für -CH₂- oder Carbonyl steht;
   W für N oder CH steht;
   X und Z unabhängig voneinander ausgewählt sind unter -O-, -S-, -NR₁₈-, -S(=O)-, oder -S(=O)₂- Gruppen;
   Y ausgewählt ist unter
      geradkettigen oder verzweigten C₁-C₈-Alkylengruppen, die gegebenenfalls 1, 2, 3 oder 4 gleichen oder verschiedenen Substituenten tragen, die ausgewählt sind unter NH₂, OH, SH, Halogen oder geradkettigen oder verzweigten C₁-C₆-Alkoxygruppen; oder
   X-Y-Z zusammen mit dem Kohlenstoffatom an das sie gebunden sind, eine Ketogruppe bilden:
   R₁₁ bis R₁₈ unabgängig voneinander ausgewählt sind unter: H; Aryl;
   geradkettigen oder verzweigten gegebenenfalls ein- oder mehrfach, wie z.B. ein- oder zweifach) ungesättigten (durch C-C-Doppel- und/oder Dreifachbindungen, insbesondere Doppelbindungen) C₁-C₆-Alkylgruppen, die gegebenenfalls 1, 2, 3 oder 4 gleichen oder verschiedenen Substituenten tragen, die ausgewählt sind unter Oxogruppen (=O),NH₂, OH, SH, Halogen oder geradkettigen oder verzweigten C₁-C₆-Alkyoxygruppen; und
   geradkettigen oder verzweigten C₁-C₆-Alkyloxygruppen, die gegebenenfalls 1, 2, 3 oder 4 gleichen oder verschiedenen Substituenten tragen, die ausgewählt sind unter NH₂, OH, SH, Halogen oder geradkettigen oder verzweigten C₁-C₆-Alkoxygruppen; oder
   R₁₁ und R₁₂ zusammen mit den Kohlenstoffatomen, an die sie gebunden sind, einen 4-, 5-, 6- oder 7-gliedrigen, gesättigten oder ein- oder mehrfach ungesättigten, carbo- oder heterocyclischen Ring bilden, der gegebenenfalls 1, 2, 3, 4 oder 5 gleiche oder verschiedene Substituenten trägt, die ausgewählt sind unter geradkettigen oder verzweigten C₁-C₆-Alkylgruppen, und Oxogruppen (=O), und die Ring-Heteroatome gleich oder verschieden sind und ausgewählt sind unter O, N und S;
   sowie Salze dieser Verbindungen, insbesondere Säureadditionssalze mit anorganischen oder insbesondere organischen, ein- oder insbesondere mehrwertigen Carbonsäuren;
   und gegebenenfalls in Stereoisomeren-reiner Form oder als Gemisch von Stereoisomeren.
2. Verfahren nach Aspekt 1, worin die Verbindung ausgewählt ist unter:
   a) Verbindungen der allgemeinen Formel IA: 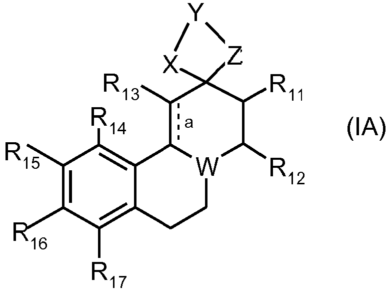 worin:
      a, W, X, Y, Z und R₁₁ bis R₁₈ die in Aspekt 1 angegebenen Bedeutungen besitzen;
      oder
   b) Verbindungen der allgemeinen Formel IB; 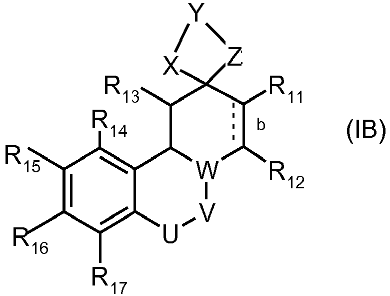 worin:
      b, V, W, X, Y, Z und R₁₁ bis R₁₈ die in Aspekt 1 angegebenen Bedeutungen besitzen;
      U -CH₂-, oder -O- steht; und
      wobei U und V nicht gleichzeitig für -CH₂- stehen ;
      oder
   c) der allgemeinen Formel IC: 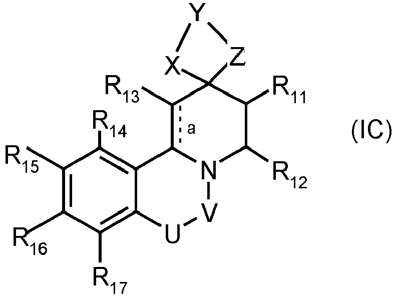
      a, X, Y, Z und R₁₁ bis R₁₈ die in Aspekt 1 angegebenen Bedeutungen besitzen;
      U -CH₂-, oder eine chemische Bindung steht; und
      V für -CH₂- oder Carbonyl steht;
      wobei U und V gegebenenfalls nicht gleichzeitig für -CH₂- stehen.
3. Verfahren zur in-vitro oder in-vivo Modulation des Kälte-Menthol-Rezeptors TRPM8, wobei man den Rezeptor mit wenigstens einem Modulator in Kontakt bringt, der ausgewählt ist unter Verbindungen des folgenden Strukturtyps II: 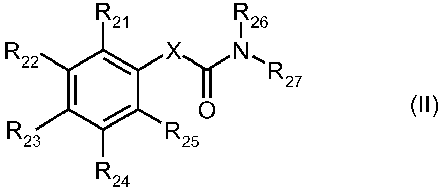 worin
   R₂₁, R₂₂, R₂₃, R₂₄ und R₂₅ gleich oder verschieden sind und ausgewählt sind unter
      H;
      Halogen;
      geradkettigen oder verzweigten C₁-C₆-Alkylgruppen die gegebenenfalls 1, 2, 3 oder 4 gleichen oder verschiedenen Substituenten tragen, die ausgewählt sind unter NH₂, OH, SH, Halogen oder geradkettigen oder verzweigten C₁-C₆-Alkoxygruppen;
      geradkettigen oder verzweigten C₁-C₆-Alkoxygruppen die gegebenenfalls 1, 2, 3 oder 4 gleichen oder verschiedenen Substituenten tragen, die ausgewählt sind unter NH₂, OH, SH, Halogen oder C₁-C₆-Alkoxygruppen;
      ein- oder mehrkernige Aryl-, Arylalkyl- und Heteroarylgruppen, die gegebenenfalls 1, 2, 3 oder 4 gleichen oder verschiedenen Substituenten tragen, die ausgewählt sind unter NH₂, OH, SH, Halogen, geradkettigen oder verzweigten C₁-C₆-Alkylgruppen und geradkettigen oder verzweigten C₁-C₆-Alkyloxygruppen; wobei die Heteroarylgruppen 1, 2, 3 oder 4 Ring-Heteroatome aufweisen, die gleich oder verschieden sind und ausgewählt sind unter O, N und S; oder
   zwei benachbarte Reste R₂₁, R₂₂, R₂₃, R₂₄ und R₂₅ zusammen mit den Kohlenstoffatomen, an die sie gebunden sind, einen 4-, 5-, 6- oder 7-gliedrigen, ein- oder mehrfach ungesättigten heterocyclischen Ring bilden, der gegebenenfalls 1, 2, 3, 4 oder 5 gleiche oder verschiedene Substituenten trägt, die ausgewählt sind unter geradkettigen oder verzweigten C₁-C₆-Alkylgruppen, und der 1, 2 oder 3 Ring-Heteroatome aufweist, die gleich oder verschieden sind und ausgewählt sind unter O, N und S;
   R₂₆ und R₂₇ gleich oder verschieden sind und ausgewählt ist unter:
      geradkettigen oder verzweigten C₁-C₆-Alkylgruppen die gegebenenfalls 1, 2, 3 oder 4 gleichen oder verschiedenen Substituenten tragen, die ausgewählt sind unter NH₂, OH, SH, Halogen oder geradkettigen oder verzweigten C₁-C₆-Alkoxygruppen;
      ein- oder mehrkernige Aryl-, Arylalkyl-, Aryloxy-, Heteroaryl- und Heteroaryloxygruppen, die gegebenenfalls 1, 2, 3 oder 4 gleichen oder verschiedenen Substituenten tragen, die ausgewählt sind unter NH₂, OH, SH, Halogen, geradkettigen oder verzweigten C₁-C₆-Alkylgruppen und geradkettigen oder
      verzweigten C₁-C₆-Alkoxygruppen; wobei die Heteroarylgruppen 1, 2, 3 oder 4 Ring-Heteroatome aufweisen, die gleich oder verschieden sind und
      ausgewählt sind unter O, N und S;
      und C₃-C₇-Cycloalkylgruppen, die gegebenenfalls 1,2, 3 oder 4 gleichen oder verschiedenen Substituenten tragen, die ausgewählt sind unter NH₂, OH, SH, Halogen, geradkettigen oder verzweigten C₁-C₆-Alkylgruppen, oder geradkettigen oder verzweigten C₁-C₆-Alkoxygruppen; wobei die Cycloalkylgruppe gegebenenfalls über eine C₁-C₄-Alkylengruppe gebunden ist;
      und wobei gegebenenfalls 1, 2 oder 3 Ring-Kohlenstoffatome durch gleiche oder verschiedene Heteroatome, ausgewählt unter O, N und S ersetzt sein können;
      wobei insbesondere R₂₆ und R₂₇ gleich oder verschieden sind und ausgewählt ist unter, Cyclohexyl, 2-Pyridyl, und Phenyl;
   X ausgewählt ist unter
      a) bis zu 4 Kohlenstoffatome umfassenden C1-Kohlenstoffbrücken der allgemeinen Formel 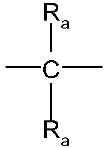 worin
         die Reste Rₐ unabhängig voneinander für H, geradkettiges oder verzweigtes C₁-C₃-Alkyl, geradkettiges oder verzweigtes C₂-C₃-Alkenyl oder C₂-C₃-Alkinyl stehen oder beide Rₐ-Reste zusammen eine Gruppe der Formel 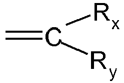 worin Rₓ und R_{y} unabhängig voneinander für H, Methyl oder Ethyl stehen; oder
      b) bis zu 4 Kohlenstoffatome umfassenden C2-Kohlenstoffbrücken der allgemeinen Formeln (i) oder (ii) 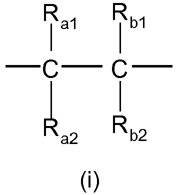 oder 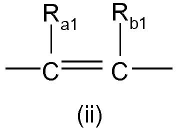 worin
         die Reste Rₐ₁, Rₐ₂, R_{b1} und R_{b2} unabhängig voneinander für H, Methyl, Ethyl, Ethenyl, oder Ethinyl stehen oder ein oder zwei geminale Restepaare Rₐ₁/Rₐ₂ bzw. R_{b1}/R_{b2} unabhängig voneinander für eine Gruppe der Formel =CH₂ stehen; wobei gegebenenfalls in Formel (ii) die Reste Rₐ₁ und R_{b1} nicht gleichzeitig für H stehen
         oder
      c) bis zu 4 Kohlenstoffatome umfassenden C3-Kohlenstoffbrücken, ausgewählt unter 1,3-Propylen- ,1,3 Propenylen- oder 1, 3-Propinylen- Brücken, die gegebenenfalls eine Seitengruppe ausgewählt unter -CH₃ oder =CH₂ tragen;
         oder
      d) lineare 1,4-verknüpfte C4-Kohlenstoffbrücken, die gegebenenfalls eine C-C-Doppelbindung oder 2 konjugierte C-C-Doppelbindungen oder eine oder zwei C-C-Dreifachbindungen aufweist;
      sowie Salze dieser Verbindungen, insbesondere Säureadditionssalze mit anorganischen oder insbesondere organischen, ein- oder insbesondere mehrwertigen Carbonsäuren;
      und gegebenenfalls in Stereoisomeren-reiner Form oder als Gemisch von Stereoisomeren.
4. Verfahren nach Aspekt 3, wobei in den Verbindung der Formel (II)
   X nicht für eine Methylen- oder lineare 1,4-Butylen-Brücke steht.
5. Verfahren nach Aspekt 3 oder 4, worin in den Verbindungen der Formel II
   R₂₁, R₂₄ und R₂₅ für H stehen und
   R₂₂ und R₂₃ zusammen mit den C-Atomen an die sie gebunden sind eine Methylendioxygruppe bilden.
6. Verfahren nach Aspekt 3, 4 oder 5, worin in den Verbindungen der Formel II die Reste R₂₆ und R₂₇ unabhängig voneinander für einen jeweils einkernigen Aryl- oder Heteroarylrest oder einen C₃-C₇-Cycloalkylrest stehen.
7. Verfahren zur in-vitro oder in-vivo Modulation des Kälte-Menthol-Rezeptors TRPM8, wobei man den Rezeptor mit wenigstens einem Modulator in Kontakt bringt, der ausgewählt ist unter Verbindungen des folgenden Strukturtyps III: 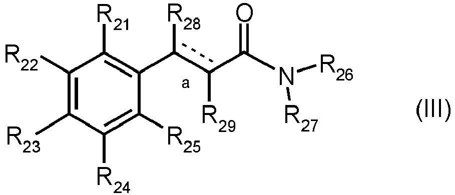 worin
   die Bindung a eine C-C Einfachbindung oder eine C-C-Doppelbindung darstellt; R₂₁, R₂₂, R₂₃, R₂₄ und R₂₅ gleich oder verschieden sind und ausgewählt sind unter
      H;
      Halogen;
      geradkettigen oder verzweigten C₁-C₆-Alkylgruppen die gegebenenfalls 1, 2, 3 oder 4 gleichen oder verschiedenen Substituenten tragen, die ausgewählt sind unter NH₂, OH, SH, Halogen oder geradkettigen oder verzweigten C₁-C₆-Alkoxygruppen;
      geradkettigen oder verzweigten C₁-C₆-Alkoxygruppen die gegebenenfalls 1, 2, 3 oder 4 gleichen oder verschiedenen Substituenten tragen, die ausgewählt sind unter NH₂, OH, SH, Halogen oder C₁-C₆-Alkoxygruppen;
      ein- oder mehrkernige Aryl-, Arylalkyl- und Heteroarylgruppen, die gegebenenfalls 1, 2, 3 oder 4 gleichen oder verschiedenen Substituenten tragen, die ausgewählt sind unter NH₂, OH, SH, Halogen, geradkettigen oder verzweigten C₁-C₆-Alkylgruppen und geradkettigen oder verzweigten C₁-C₆-Alkyloxygruppen; wobei die Heteroarylgruppen 1, 2, 3 oder 4 Ring-Heteroatome aufweisen, die gleich oder verschieden sind und ausgewählt sind unter O, N und S; oder
   zwei benachbarte Reste R₂₁, R₂₂, R₂₃, R₂₄ und R₂₅ zusammen mit den Kohlenstoffatomen, an die sie gebunden sind, einen 4-, 5-, 6- oder 7-gliedrigen, ein- oder mehrfach ungesättigten heterocyclischen Ring bilden, der gegebenenfalls 1, 2, 3, 4 oder 5 gleiche oder verschiedene Substituenten trägt, die ausgewählt sind unter geradkettigen oder verzweigten C₁-C₆-Alkylgruppen, und der 1, 2 oder 3 Ring-Heteroatome aufweist, die gleich oder verschieden sind und ausgewählt sind unter O, N und S;
   R₂₆ und R₂₇ gleich oder verschieden sind und ausgewählt ist unter:
      geradkettigen oder verzweigten C₁-C₆-Alkylgruppen die gegebenenfalls 1, 2, 3 oder 4 gleichen oder verschiedenen Substituenten tragen, die ausgewählt sind unter NH₂, OH, SH, Halogen oder geradkettigen oder verzweigten C₁-C₆-Alkoxygruppen;
      ein- oder mehrkernige Aryl-, Arylalkyl-, Aryloxy-, Heteroaryl- und Heteroaryloxygruppen, die gegebenenfalls 1, 2, 3 oder 4 gleichen oder verschiedenen Substituenten tragen, die ausgewählt sind unter NH₂, OH, SH, Halogen, geradkettigen oder verzweigten C₁-C₆-Alkylgruppen und geradkettigen oder verzweigten C₁-C₆-Alkoxygruppen; wobei die Heteroarylgruppen 1, 2, 3 oder 4 Ring-Heteroatome aufweisen, die gleich oder verschieden sind und ausgewählt sind unter O, N und S;
      und C₃-C₇-Cycloalkylgruppen, die gegebenenfalls 1,2, 3 oder 4 gleichen oder verschiedenen Substituenten tragen, die ausgewählt sind unter NH₂, OH, SH, Halogen, geradkettigen oder verzweigten C₁-C₆-Alkylgruppen, oder geradkettigen oder verzweigten C₁-C₆-Alkoxygruppen; wobei die Cycloalkylgruppe gegebenenfalls über eine C₁-C₄-Alkylengruppe gebunden ist;
      und wobei gegebenenfalls 1, 2 oder 3 Ring-Kohlenstoffatome durch gleiche oder verschiedene Heteroatome, ausgewählt unter O, N und S ersetzt sein können;
      wobei insbesondere R₂₆ und R₂₇ gleich oder verschieden sind und ausgewählt ist unter, Cyclohexyl, 2-Pyridyl, und Phenyl;
      und
   R₂₈ und R₂₉ gleich oder verschieden sind und ausgewählt sind unter H und geradkettiges oder verzweigtes Alkyl, wie C₁-C₈-Alkyl, geradkettiges oder verzweigtes Alkenyl, wie C₂-C₈-Alkenyl, geradkettiges oder verzweigtes Alkinyl, wie C₂-C₈-Alkinyl, Cycloalkyl, wie C₃-C₁₂-Cycloalkyl oder Cycloalkenyl, wie C₅-C₈-Cycloalkenyl, wobei die Kohlensstoffkette, bzw. der Kohlenstoffring dieser Reste gegebenenfalls durch ein oder mehrere, wie z.B. 1, 2, 3 oder 4, insbesondere 1 oder 2, Heteroatome (in der Kette oder im Ring), ausgewählt unter O, S und N (oder -NH-), insbesondere O, unterbrochen ist, oder ein oder mehrere, wie insbesondere 1, 2 oder 3, heteroatomhaltige Substituenten, wie z.B. -COOH, -COO-Alkyl, -OH, -SH, -CN, Amino, Nitro, wie hierein definiert, trägt wobei die Summe der C-Atome in den Resten R₂₈ und R₂₉ zusammen wenigsten 3 beträgt;
   sowie Salze dieser Verbindungen, insbesondere Säureadditionssalze mit anorganischen oder insbesondere organischen, ein- oder insbesondere mehrwertigen Carbonsäuren;
   und gegebenenfalls in Stereoisomeren-reiner Form oder als Gemisch von Stereoisomeren.
8. Verfahren nach einem der vorhergehenden Aspekte, wobei man den Rezeptor mit wenigstens einer Verbindung in Kontakt bringt, welche in einem zellulären Aktivitätstest unter Verwendung von Zellen, welche den humanen TRPM8-Rezeptor rekombinant exprimieren, die Permeabilität dieser Zellen für Ca²⁺ Ionen modulieren.
9. Verfahren nach einem der vorhergehenden Aspekte, wobei die modulierende Verbindung auf die zelluläre Ca²⁺-Ionen-Permeabilität agonistisch oder antagonistisch wirkt.
10. Verfahren nach einem der vorhergehenden Aspekte, wobei die modulierende Verbindung ein TRPM8-Rezeptor-Agonist ist.
11. Verwendung einer Verbindung gemäß der Definition in einem der Aspekte 1 bis 7, zur Induktion von Kältegefühl bei Mensch und/oder Tier, insbesondere zu nichttherapeutischen Zwecken..
12. Verwendung einer Verbindung gemäß der Definition in einem der Aspekte 1 bis 7, als aktiven Bestandteil eines pharmazeutischen Mittels.
13. Verwendung einer Verbindung gemäß der Definition in einem der Aspekte 1 bis 7, zur Behandlung von Prostatakarzinomen, zur Behandlung von Blasenschwäche oder in der Schmerztherapie.
14. Verwendung einer Verbindung gemäß der Definition in einem der Aspekte 1 bis 7 definiert ist, zur Induktion eines Kältegefühls durch eine Verpackung.
15. Verwendung einer Verbindung gemäß der Definition in einem der Aspekte 1 bis 7, zur Induktion eines Kältegefühls durch ein Textil.
16. Verwendung nach Aspekt 11, wobei ein Mittel eingesetzt wird, umfassend eine oder mehre der Verbindungen gemäß der Definitionen aus einem der Aspekte 1 bis 7 in einer Konzentration von 0,1 ppm bis 10 Gew-% bezogen auf das Gesamtgewicht des Mittels zur Erzielung einer Kühlwirkung auf Haut oder Schleimhaut, die verglichen mit der Kühlwirkung eines Mittels gleicher Zusammensetzung, bei dem lediglich die Verbindung oder die Verbindungen gemäß der Definitionen aus einem der Aspekte 1 bis 7 gegen Menthancarbonsäure-N-ethylamid in gleicher Konzentration ausgetauscht sind, um wenigstens 10 Minuten verlängert ist.
17. Stoff gemäß der Definition nach einem der Aspekte 1 bis 7 zur Verwendung als Mediator des TRPM8 Rezeptors.
18. Mittel enthaltend wenigstens eine Verbindung nach einem der Aspekte 1 bis 7.
19. Mittel nach Aspekt 18, ausgewählt unter
   a) pharmazeutischen Mitteln, wie Antitumormittel, Mittel zur Behandlung von Erkrankungen der Blase, Schmerzmittel;
   b) Nahrungsmitteln, wie Speiseeis, Mousse, Creme, Getränke, Süßwaren,
   c) Mundpflegemitteln, wie Zahnpasta, Mundwasser, Kaugummi,
   d) Körperpflegemitteln, wie Haut- oder Haarpflegemitteln, wie Sonnencreme, Sonnenbrandcreme, Lotionen, Shampoos, Pflaster,
   e) Schäumen und Gelen.
20. Mittel nach Aspekt 19, umfassend
   a) einen oder mehrere weitere Stoffe mit physiologischer Kühlwirkung, wobei der weitere Stoff bzw. einer, mehrere oder sämtliche der weiteren Stoffe (i) einen geschmacklichen Effekt verursachen oder (ii) keinen geschmacklichen Effekt verursachen,
      und/oder
   b) einen oder mehrere Aromastoffe ohne physiologische Kühlwirkung
      und/oder
   c) einen oder mehrere trigeminal oder mundwässernd wirksame Stoffe ohne physiologische Kühlwirkung
      und/oder
   d) (iii) eine oder (iv) mehrere Verbindungen, die im Falle (iv) unabhängig von einander oder gemeinsam zusätzlich einen geschmacksmodulierenden Effekt und/oder einen trigeminalen und/oder einen mundwässernden Reiz verursachen.
21. Produkt enthaltend wenigstens eine Verbindung gemäß der Definition nach einem der Aspekte 1 bis 7, ausgewählt unter
   a) Textilprodukten,
   b) Verpackungsmaterialien,
   c) Tabakprodukten;
   d) Heilmitteln;
   e) Hygieneprodukten, oder
   f) Erfrischungstüchern.
22. Stoff gemäß der Definition nach einem der Aspekte 1 bis 7.
23. Stoff nach Aspekt 22, ausgewählt unter Verbindungen gemäß den Tabellen 1 und 2 A bis D.
24. Mittel nach einem der Aspekte 17 bis 20 zur Vorbeugung gegen, Bekämpfung oder Linderung von Husten-, Schnupfen-, Entzündungs-, Halsschmerz- oder Heiserkeitssymptomen.
25. Verfahren, Verwendung, Stoff, Produkt oder Mittel nach einem der Aspekte 2 oder 8 bis 24, wobei anstelle einer Verbindung der Formel IC eine Verbindung der Formel IC' enthalten ist, 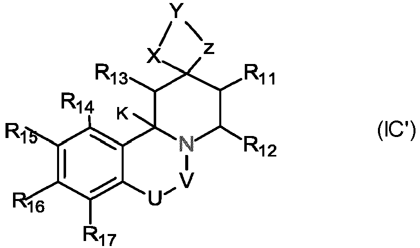 worin
   U, V, X, Y, Z und R₁₁ bis R₁₈ die in Aspekt 2 angegebenen Bedeutungen besitzen; und
   K für einen Rest der Formel

      H-X'-Y'-Z'-
   steht, worin
   X',Y' und Z' unabhängig voneinander die für X, Y und Z angegebenen Bedeutungen besitzen;
   sowie Salze dieser Verbindungen, insbesondere Säureadditionssalze mit anorganischen oder insbesondere organischen, ein- oder insbesondere mehrwertigen Carbonsäuren; und gegebenenfalls Isomere oder Stereoisomere davon jeweils in Reinform oder als Gemisch von Isomeren und/oder Stereoisomeren.
   Beispielsweise steht dabei U für eine chemische Bindung und V für Carbonyl. Beispielsweise stehen Y und Y' für gleiche oder verschiedene, z.B. gleiche C₁-C₈-Alkylengruppen. Beispielsweise sind X, Z, X' und Z' gleich oder verschieden, z.B. gleich, und stehen für -O- oder -S-. R₁₃ bis R₁₇ stehen beispielsweise unabhängig voneinander für H oder - COOH, -COO-Alkyl, -OH, -SH, -CN, Amino, - NO₂, Alkyl, oder Alkenylgruppen, wie insbesondere für H. R₁₁ und R₁₂ bilden beispielsweise zusammen mit den Kohlenstoffatomen, an die sie gebunden sind, einen 4-, 5-, 6- oder 7-gliedrigen, gesättigten oder ein- oder mehrfach ungesättigten, carbocyclischen Ring.
26. Verfahren, Verwendung, Stoff, Produkt oder Mittel nach einem der Aspekte 2 oder 8 bis 24, wobei anstelle einer Verbindung der Formel IB eine Verbindung der Formel IB' enthalten ist, 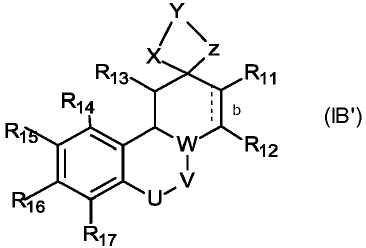 worin:
   b, U, V, X, Y, Z und R₁₁ bis R₁₈ die in Aspekt 2 angegebenen Bedeutungen besitzen; und W für einen Carboxylesterrest steht, insbesondere einen Rest der Formel 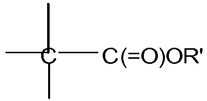 steht,
   wobei R' für Alkyl, wie z.B. C₁-C₆-Alkyl, steht.;
   sowie Salze dieser Verbindungen, insbesondere Säureadditionssalze mit anorganischen oder insbesondere organischen, ein- oder insbesondere mehrwertigen Carbonsäuren; und gegebenenfalls Isomere oder Stereoisomere davon jeweils in Reinform oder als Gemisch von Isomeren und/oder Stereoisomeren.
   U steht dabei z.B. für-O-. V steht beispielsweise für Carbonyl. Z und X sind verschieden oder insbesondere gleich und stehen z.B. für -O- oder -S-. Y steht beispielsweise für C₁-C₈-Alkylen. R₁₃ bis R₁₇ stehen beispielsweise unabhängig voneinander für H oder - COOH, -COO-Alkyl, -OH, -SH, -CN, Amino, -NO₂, Alkyl, oder Alkenylgruppen, wie insbesondere für H. R₁₁ und R₁₂ bilden beispielsweise zusammen mit den Kohlenstoffatomen, an die sie gebunden sind, einen 4-, 5-, 6- oder 7-gliedrigen, gesättigten oder ein- oder mehrfach ungesättigten, carbocyclischen Ring.
27. Verfahren, Verwendung, Stoff, Produkt oder Mittel nach einem der Aspekte 2 oder 8 bis 24, wobei anstelle einer Verbindung der Formel IA eine Verbindung der Formel IA' enthalten ist, 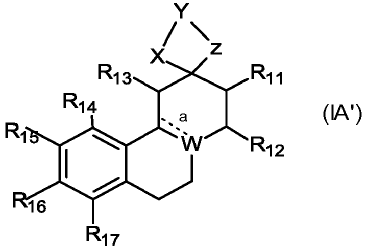 worin X, Y, Z und R₁₁ bis R₁₈ die in Aspekt 1 angegebenen Bedeutungen besitzen; mit der Maßgabe dass W für ein Kohlenstoffatom und a gegebenenfalls für eine Doppelbindung steht;
   sowie Salze dieser Verbindungen, insbesondere Säureadditionssalze mit anorganischen oder insbesondere organischen, ein- oder insbesondere mehrwertigen Carbonsäuren; und gegebenenfalls Isomere oder Stereoisomere davon jeweils in Reinform oder als Gemisch von Isomeren und/oder Stereoisomeren; Z und X sind verschieden oder insbesondere gleich und stehen z.B. für -O- oder -S-. Y steht beispielsweise für C₁-C₈-Alkylen. R₁₃ bis R₁₇ stehen beispielsweise unabhängig voneinander für H oder - COOH, -COO-Alkyl, -OH, -SH, -CN, Amino, -NO₂, Alkyl, oder Alkenylgruppen, wie insbesondere für H. R₁₁ und R₁₂ bilden beispielsweise zusammen mit den Kohlenstoffatomen, an die sie gebunden sind, einen 4-, 5-, 6- oder 7-gliedrigen, gesättigten oder ein- oder mehrfach ungesättigten, carbocyclischen Ring.

### 3. Weitere Ausgestaltungen der weiteren hier beschriebenen Verfahren, Verwendungen und Wirkstoffe (nur teilweise erfindungsgemäß, d.h. nur insofern sie durch die Ansprüche gedeckt sind):

Folgende spezielle Ausgestaltungen der weiteren hier beschriebenen Wirkstoffe gelten sinngemäß sowohl für die Wirkstoffe per se als auch deren hier beschriebenen Anwendungen, wie z.B. in den hier beschriebenen Mitteln, Verfahren sowie Verwendungen.

### 3.1 Verbindungen der Formel I (Strukturtyp I):

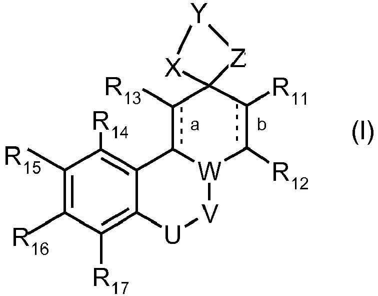

ausgewählt unter Verbindungen der folgenden Gruppen (1) bis (55):
(1) Verbindungen der Formel I, worin R₁₃ bis R₁₇ unabhängig voneinander für H, geradkettige oder verzweigte C₁-C₆-Alkylgruppen, insbesondere für H steht;
(2) Verbindungen der Formel I, worin R₁₁ und R₁₂ zusammen mit den Kohlenstoffatomen, an die sie gebunden sind, einen 5- oder 6-gliedrigen, gesättigten oder einfach ungesättigten carbo- oder heterocyclischen, insbesondere carbocyclischen Ring bilden, der gegebenenfalls 1, 2, oder 3 gleiche oder verschiedene Substituenten trägt, die ausgewählt sind unter geradkettigen oder verzweigten C₁-C₆-Alkylgruppen, und Oxogruppen (=O); und die Ring-Heteroatome O-Atome sind;
(3) Verbindungen der Formel I, worin R₁₁ und R₁₂ zusammen mit den Kohlenstoffatomen, an die sie gebunden sind, einen 5- oder 6-gliedrigen, einfach ungesättigten, carbo- oder heterocyclischen Ring bilden, der gegebenenfalls 1, 2 oder 3 gleiche oder verschiedene Substituenten trägt, die ausgewählt sind unter geradkettigen C₁-C₄-Alkylgruppen, und eine Oxogruppen (=O) trägt; und das Ring-Heteroatom ein O-Atom sind;
(4) Verbindungen der Formel I, worin R₁₁ und R₁₂ zusammen Verbrückungsgruppen bilden, ausgewählt unter

   -C(=O)-O-C*H(CH₃)-

   in beiden stereoisomeren Formen

   -C(=O)-CH₂-C(CH₃)₂-CH₂-

   -C(=O)-CH₂-CH₂-CH₂-

   wobei die Ketogruppe über die R₁₂ oder insbesondere über die R₁₁-Position an das Molekül gebunden ist.
(5) Verbindungen der Formel I, worin X und Z gleich oder verschieden sind und ausgewählt sind unter -NR-, worin R für H oder C₁-C₆ Alkyl steht, -S-, -S(=O)-, oder -S(=O)₂- Gruppen;
(6) Verbindungen der Formel I, worin X und Z gleich sind und jeweils für -S- stehen;
(7) Verbindungen der Formel I, worin X und Z verschieden sind und ausgewählt sind -S(=O)-, oder -S(=O)₂- Gruppen;
(8) Verbindungen der Formel I, worin Y ausgewählt ist unter geradkettigen C₂- oder C₃-Alkylengruppen, insbesondere -CH₂-CH₂- oder -CH₂-CH₂-CH₂- ,
(9) Verbindungen der Formel I, worin X-Y-Z zusammen mit dem Kohlenstoffatom an das sie gebunden sind eine Ketogruppe bilden;
(10) Kombinationen der Ausgestaltungen: (1)+(2), (1)+(3), (1)+(4)
(11) Kombinationen der Ausgestaltungen: (1)+(5), (1)+(6), (1)+(7)
(12) Kombinationen der Ausgestaltungen: (1)+(8),
(13) Kombinationen der Ausgestaltungen: (1)+(9),
(14) Kombinationen der Ausgestaltungen: (10)+(5), (10)+(6), (10)+(7)
(15) Kombinationen der Ausgestaltungen: (10)+(8)
(16) Kombinationen der Ausgestaltungen: (10)+(9)
(17) Kombinationen der Ausgestaltungen: (14)+(8)
(18) Kombinationen der Ausgestaltungen: (11)+(8)
(19) Verbindungen der Formel I, worin a eine C-C-Doppelbindung ist
(20) Verbindungen der Formel I, worin b eine C-C-Einfachbindung ist
(21) Verbindungen der Formel I, worin a und b eine C-C-Doppelbindung sind
(22) Verbindungen der Formel I, worin a und b eine C-C-Einfachbindung sind
(23) Verbindungen der Formel I, worin W für CH steht
(24) Verbindungen der Formel I, worin V für Carbonyl steht
(25) Verbindungen der Formel I, worin U für eine chemische Einfachbindung steht
(26) Verbindungen der Formel I, worin U für -O- steht
(27) Kombinationen der Ausgestaltungen: (1) + (19), (1) + (20), (1) + (21), (1) + (22),
(28) Kombinationen der Ausgestaltungen: (1) + (23), (1) + (24), (1) + (25), (1) + (26)
(29) Kombinationen der Ausgestaltungen: (10) + (19), (10) + (20), (10) + (21), (10) + (22),
(30) Kombinationen der Ausgestaltungen: (10) + (23), (10) + (24), (10) + (25), (10) + (26)
(31) Kombinationen der Ausgestaltungen: (11) + (19), (11) + (20), (11) + (21), (11) + (22),
(32) Kombinationen der Ausgestaltungen: (11) + (23), (11) + (24), (11) + (25), (11) + (26)
(33) Kombinationen der Ausgestaltungen: (12) + (19), (12) + (20), (12) + (21), (12) + (22),
(34) Kombinationen der Ausgestaltungen: (12) + (23), (12) + (24), (12) + (25), (12) + (26)
(35) Kombinationen der Ausgestaltungen: (13) + (19), (13) + (20), (13) + (21), (13) + (22),
(36) Kombinationen der Ausgestaltungen: (13) + (23), (13) + (24), (13) + (25), (13) + (26)
(37) Kombinationen der Ausgestaltungen: (27) + (23), (27) + (24), (37) + (25), (27) + (26)
(38) Kombinationen der Ausgestaltungen: (14) + (19), (14) + (20), (14) + (21), (14) + (22),
(39) Kombinationen der Ausgestaltungen: (14) + (23), (14) + (24), (14) + (25), (14) + (26)
(40) Kombinationen der Ausgestaltungen: (15) + (19), (15) + (20), (15) + (21), (15) + (22),
(41) Kombinationen der Ausgestaltungen: (15) + (23), (15) + (24), (15) + (25), (15) + (26)
(42) Kombinationen der Ausgestaltungen: (16) + (19), (16) + (20), (16) + (21), (16) + (22),
(43) Kombinationen der Ausgestaltungen: (16) + (23), (16) + (24), (16) + (25), (16) + (26)
(44) Kombinationen der Ausgestaltungen: (18) + (19), (18) + (20), (18) + (21), (18) + (22),
(45) Kombinationen der Ausgestaltungen: (18) + (23), (18) + (24), (18) + (25), (18) + (26)
(46) Kombinationen der Ausgestaltungen: (31) + (23), (31) + (24), (31) + (25), (31) + (26)
(47) Kombinationen der Ausgestaltungen: (33) + (23), (33) + (24), (33) + (25), (33) + (26)
(48) Kombinationen der Ausgestaltungen: (35) + (23), (35) + (24), (35) + (25), (35) + (26)
(49) Kombinationen der Ausgestaltungen: (17) + (19), (17) + (20), (17) + (21), (17) + (22),
(50) Kombinationen der Ausgestaltungen: (17) + (23), (17) + (24), (17) + (25), (17) + (26)
(51) Kombinationen der Ausgestaltungen: (38) + (23), (38) + (24), (38) + (25), (38) + (26)
(52) Kombinationen der Ausgestaltungen: (40) + (23), (40) + (24), (40) + (25), (40) + (26)
(53) Kombinationen der Ausgestaltungen: (42) + (23), (42) + (24), (42) + (25), (42) + (26)
(54) Kombinationen der Ausgestaltungen: (44) + (23), (44) + (24), (44) + (25), (44) + (26)
(55) Kombinationen der Ausgestaltungen: (29) + (23), (29) + (24), (29) + (25), (29) + (26)

### 3.2 Verbindungen der Formel IA:

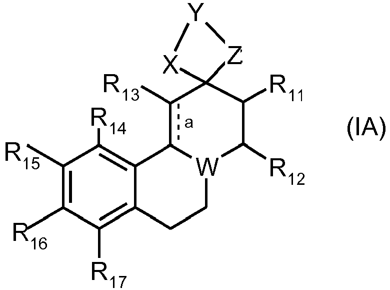

Diese sind ausgewählt unter Verbindungen entsprechend den oben für Formel I abgegebenen Gruppen (1) bis (55), soweit durch die spezielleren Bedeutungen der Formel IA zulässig.

### 3.3 Verbindungen der allgemeinen Formel IB:

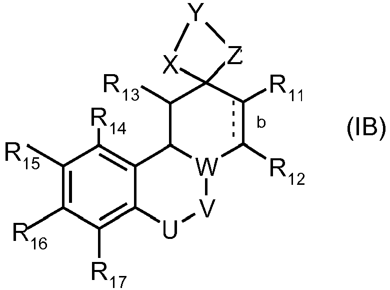

Diese sind ausgewählt unter Verbindungen entsprechend den oben für Formel I angegebenen Gruppen (1) bis (55), soweit durch die spezielleren Bedeutungen der Formel IB zulässig.

### 3.4 Verbindungen der allgemeinen Formel IC:

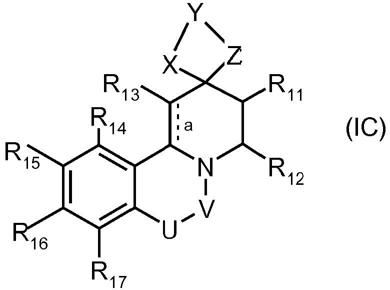

ausgewählt unter Verbindungen entsprechend den oben für Formel I angegebenen Gruppen (1) bis (55), soweit durch die spezielleren Bedeutungen der Formel IC zulässig.

### 3.4 Verbindungen der allgemeinen Formeln IA', IB' und IC':

Diese sind ausgewählt unter speziellen Verbindungen entsprechend den oben für Formel I angegebenen Gruppen (1) bis (55), soweit durch die spezielleren Bedeutungen der obigen Formeln IA', IB' oder IC' zulässig.

Beispiele für Verbindungen der Formel I (bzw. der Formeln IA, IB, IC, IA', IB' und IC') sind in folgender Tabelle 1 zusammengefasst.

**Tabelle 1: Beispiele für Verbindungen der Formel I**

| Nr. | Formel | Nr. | Formel |
|---|---|---|---|
| 1 | 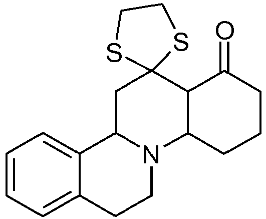 | 2 | 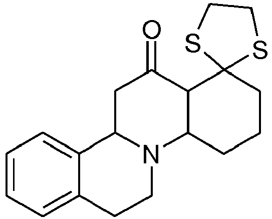 |
| 3 | 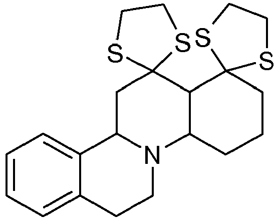 | 4 | 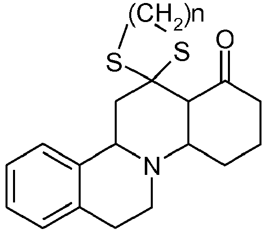 |
| 5 | 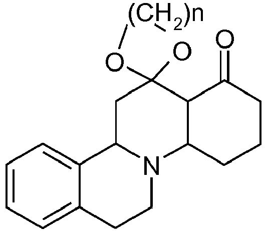 | 6 | 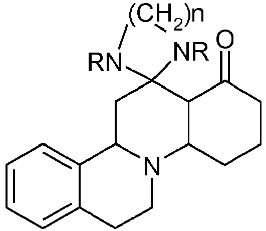 |
| 7 | 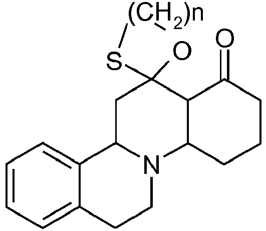 | 8 | 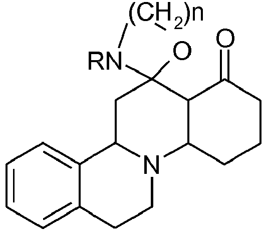 |
| 9 | 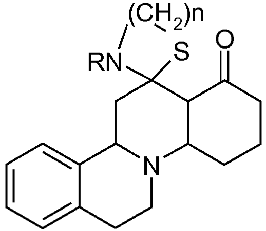 | | |
| | | | |
| 10 | 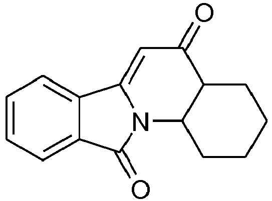 | 11 | 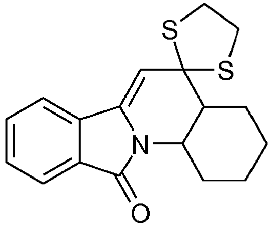 |
| 12 | 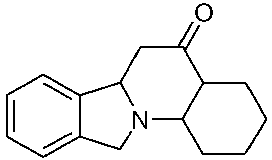 | 13 | 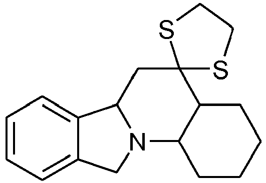 |
| | | | |
| 14 | 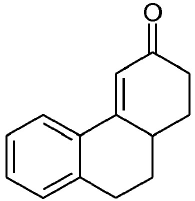 | 15 | 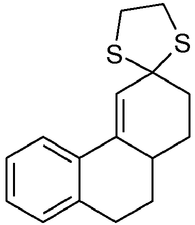 |
| 16 | 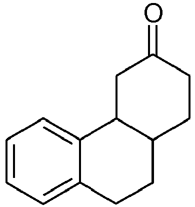 | 17 | 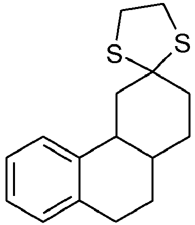 |
| 18 | 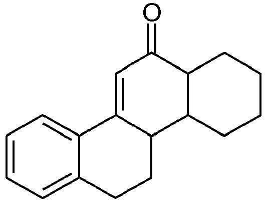 | 19 | 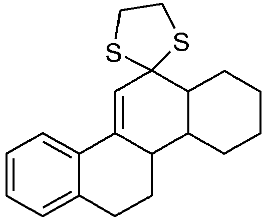 |
| 20 | 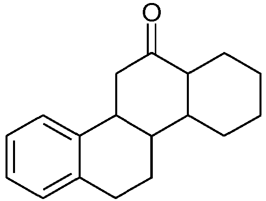 | 21 | 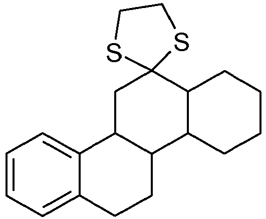 |
| 22 | 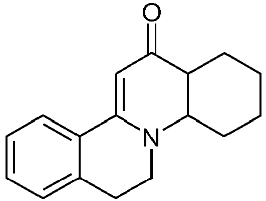 | 23 | 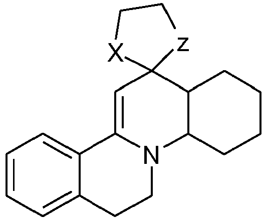 |
| 24 | 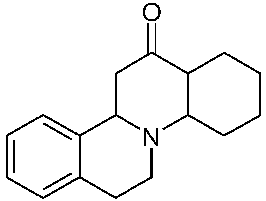 | 25 | 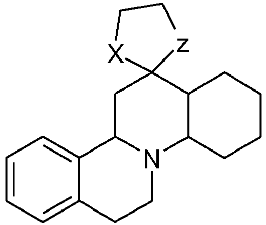 |
| | | | |
| 26 | 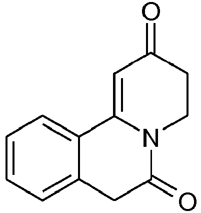 | | |
| 27 | 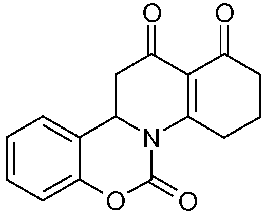 | 28 | 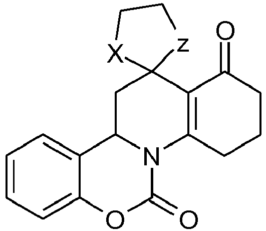 |
| 29 | 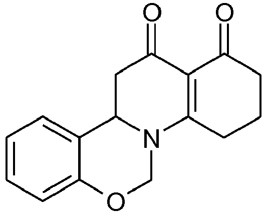 | 30 | 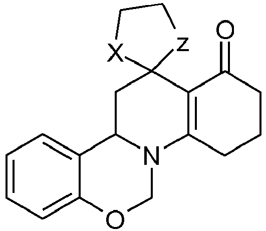 |
| 31 | 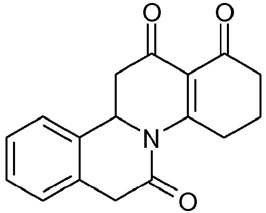 | 32 | 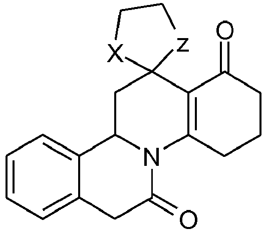 |
| 33 | 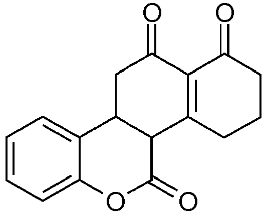 | 34 | 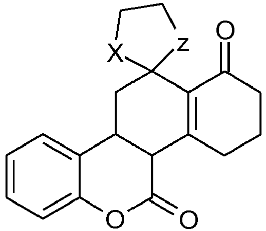 |
| 35 | 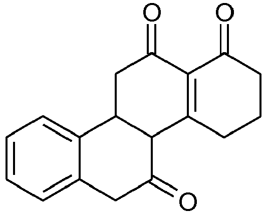 | 36 | 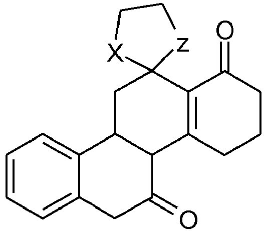 |

X und Z stehen dabei insbesondere für: O, S, NMe, NEt, SO, SO₂, NPh, NiPr, NPr, NC₄H₉, NC₅H₁₁, NC₆H₁₃ ; n steht dabei insbesondere für 2, 3 oder 4; R steht insbesondere für C₁ - C₆-Alkyl oder Phenyl.

Weitere Besipiele sind:

**Tabelle 1 (Fortsetzung)**

| Nr. | Formel | Nr. | Formel |
|---|---|---|---|
| P1 | 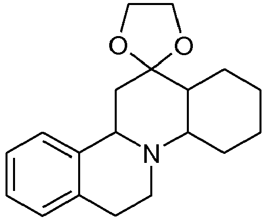 | P2 | 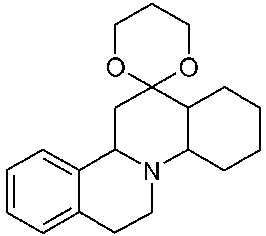 |
| P3 | 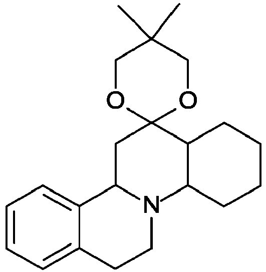 | P4 | 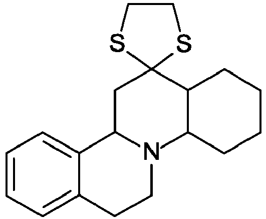 |
| P5 | 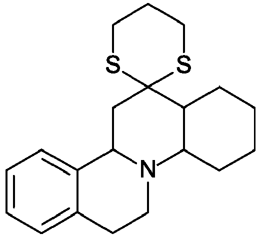 | P6 | 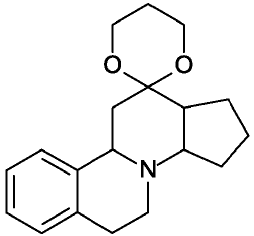 |
| P7 | 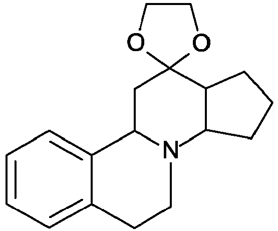 | P8 | 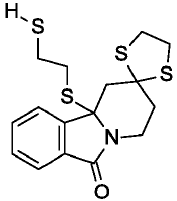 |
| P9 | 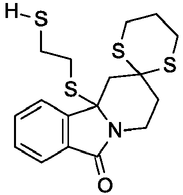 | P10 | 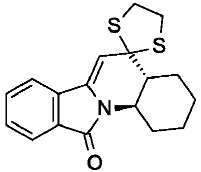 |
| P11 | 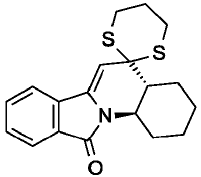 | P12 | 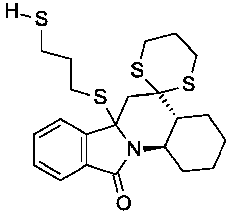 |
| P13 | 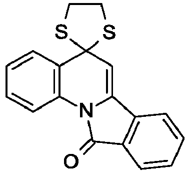 | P14 | 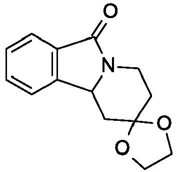 |
| P15 | 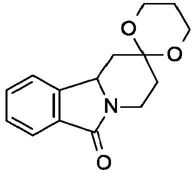 | P16 | 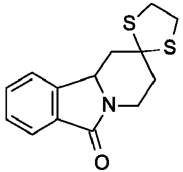 |
| P17 | 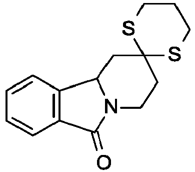 | P18 | 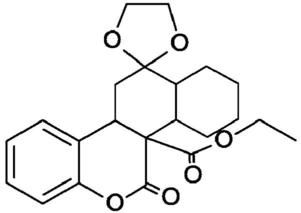 |
| P19 | 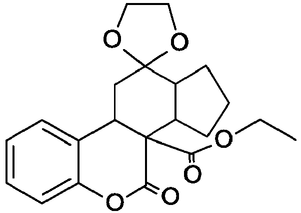 | P20 | 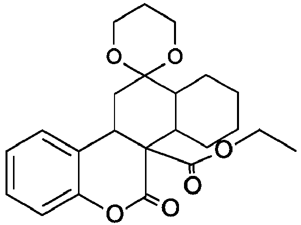 |
| P21 | 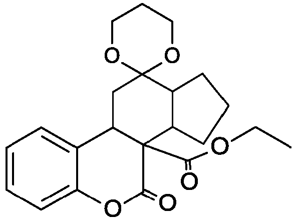 | P22 | 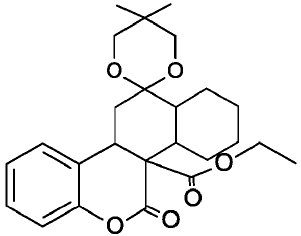 |
| P23 | 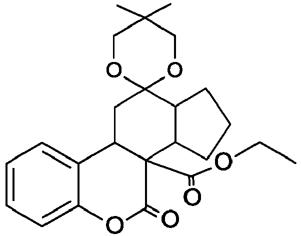 | P24 | 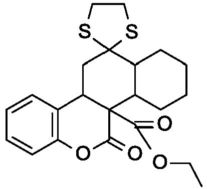 |
| P25 | 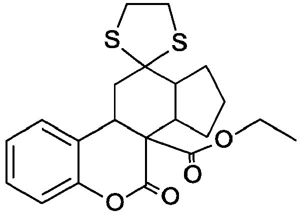 | P26 | 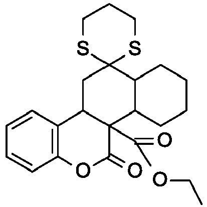 |
| P27 | 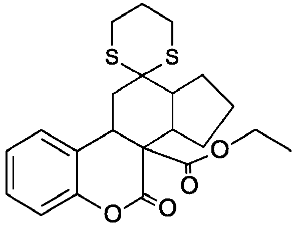 | P28 | 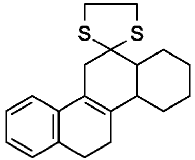 |
| P29 | 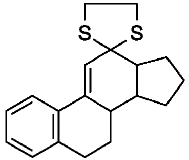 | P30 | 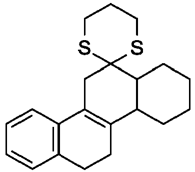 |
| P31 | 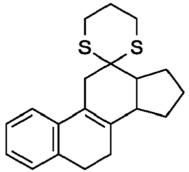 | | |

Diese Beispiele stellen die im erfindungsgemäßen Verfahren zu verwendenden Modulatoren dar. Gegenstand sind auch Salze dieser Verbindungen in obiger Tabelle 1, insbesondere Säureadditionssalze mit anorganischen oder insbesondere organischen, ein- oder insbesondere mehrwertigen Carbonsäuren; und alle Stereoisomeren in Reinform oder als Gemisch von Stereoisomeren.

### 3.6 Verbindungen der allgemeinen Formel II:

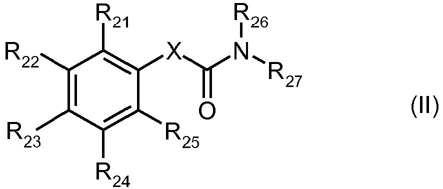

ausgewählt unter Verbindungen der folgenden Gruppen (1) bis (63):
(1) Verbindungen der Formel II, worin R₂₄ für H steht;
(2) Verbindungen der Formel II, worin R₂₅ für H oder Halogen steht;
(3) Verbindungen der Formel II, worin R₂₁ für H, Halogen, eine geradkettige oder verzweigte C₁-C₆-Alkylgruppe, die gegebenenfalls 1, 2, 3 oder 4 gleiche oder verschiedene Substituenten trägt, die ausgewählt sind unter NH₂, OH, SH , Halogen oder geradkettigen oder verzweigten C₁-C₆-Alkoxygruppen; oder eine geradkettige oder verzweigte C₁-C₆-Alkoxygruppe steht, die gegebenenfalls 1, 2, 3 oder 4 gleiche oder verschiedene Substituenten trägt, die ausgewählt sind unter NH₂, OH, SH , Halogen oder C₁-C₆-Alkoxygruppen;
(4) Verbindungen der Formel II, worin R₂₁ für H, Halogen, eine geradkettige oder verzweigte C₁-C₆-Alkylgruppe; oder eine geradkettige oder verzweigte C₁-C₆-Alkoxygruppe steht;
(5) Verbindungen der Formel II, worin R₂₁ für H, Methyl, Ethyl, Methoxy, Ethoxy, oder Halogen, insbesondere H, Fluor, Chlor, Brom, Methyl oder Methoxy steht;
(6) Verbindungen der Formel II, worin die Reste R₂₂ und R₂₃ gleich oder verschieden sind und ausgewählt sind unter H; Halogen, geradkettigen oder verzweigten C₁-C₆-Alkylgruppen, die gegebenenfalls 1, 2, 3 oder 4 gleiche oder verschiedene Substituenten tragen, die ausgewählt sind unter NH₂, OH, SH, Halogen oder geradkettigen oder verzweigten C₁-C₆-Alkoxygruppen; oder geradkettigen oder verzweigten C₁-C₆-Alkoxygruppen steht, die gegebenenfalls 1, 2, 3 oder 4 gleiche oder verschiedene Substituenten tragen, die ausgewählt sind unter NH₂, OH, SH, Halogen oder C₁-C₆-Alkoxygruppen;
(7) Verbindungen der Formel II, worin die Reste R₂₂ und R₂₃ gleich oder verschieden sind und ausgewählt sind unter H, Halogen, geradkettigen oder verzweigten C₁-C₆-Alkylgruppen; oder geradkettigen oder verzweigten C₁-C₆-Alkoxygruppen;
(8) Verbindungen der Formel II, worin die Reste R₂₂ und R₂₃ gleich oder verschieden sind und ausgewählt sind unter H, Halogen, Methyl oder Methoxy
(9) Verbindungen der Formel II, worin die benachbarten Reste R₂₂ und R₂₃, zusammen mit den Kohlenstoffatomen, an die sie gebunden sind, einen 4-, 5-, 6- oder 7-gliedrigen, ein- oder mehrfach ungesättigten heterocyclischen Ring bilden, der gegebenenfalls 1, 2, 3, 4 oder 5 gleiche oder verschiedene Substituenten trägt, die ausgewählt sind unter geradkettigen oder verzweigten C₁-C₆-Alkylgruppen, und der 1, 2 oder 3 Ring-Heteroatome aufweist, die gleich oder verschieden sind und ausgewählt sind unter O, N und S;
(10) Verbindungen der Formel II, worin die benachbarten Reste R₂₂ und R₂₃, zusammen mit den Kohlenstoffatomen, an die sie gebunden sind, einen 5-oder 6-gliedrigen, einfach ungesättigten heterocyclischen Ring bilden, der 1 oder 2 Ring-Heteroatome aufweist, die gleich oder verschieden sind und ausgewählt sind unter O, N und S;
(11) Verbindungen der Formel II, worin die benachbarten Reste R₂₂ und R₂₃, zusammen eine der Gruppen -O-CH₂-O- oder -O-CH₂-CH₂-O-, bilden;
(12) Verbindungen der Formel II, worin die Reste R₂₆ und R₂₇ gleich oder verschieden sind und ausgewählt sind unter geradkettigen oder verzweigten C₁-C₆-Alkylgruppen die gegebenenfalls 1 oder 2 gleiche oder verschiedene Substituenten tragen, die ausgewählt sind unter NH₂, OH, SH , Halogen oder geradkettigen oder verzweigten C₁-C₆-Alkoxygruppen; einkernigen Aryl-, Arylalkyl- und Heteroarylgruppen, die gegebenenfalls 1 oder 2 gleiche oder verschiedene Substituenten tragen, die ausgewählt sind unter NH₂, OH, SH, Halogen geradkettigen oder verzweigten C₁-C₆-Alkylgruppen und geradkettigen oder verzweigten C₁-C₆-Alkyloxygruppen; wobei die Heteroarylgruppen 1, 2 oder 3 Ring-Heteroatome aufweisen, die gleich oder verschieden sind und ausgewählt sind unter O, N und S; und C₃-C₇-Cycloalkylgruppen, die gegebenenfalls 1 oder 2 gleiche oder verschiedene Substituenten tragen, die ausgewählt sind unter NH₂, OH, SH, Halogen geradkettigen oder verzweigten C₁-C₆-Alkylgruppen, oder geradkettigen oder verzweigten C₁-C₆-Alkoxygruppen; wobei die Cycloalkylgruppe gegebenenfalls über eine C₁-C₄-Alkylengruppe gebunden ist und wobei gegebenenfalls 1, 2 oder 3 Ring-Kohlenstoffatome durch gleiche oder verschiedene Heteroatome, ausgewählt unter O, N und S ersetzt sein können;
(13) Verbindungen der Formel II, worin die Reste R₂₆ und R₂₇ gleich oder verschieden sind und ausgewählt sind unter geradkettigen oder verzweigten C₁-C₆-Alkylgruppen; einkernigen Aryl-, Arylalkyl- und Heteroarylgruppen, die gegebenenfalls einen Substituenten tragen, der ausgewählt ist unter NH₂, OH, SH, Halogen, geradkettigen C₁-C₆-Alkylgruppen und geradkettigen C₁-C₆-Alkyloxygruppen; wobei die Heteroarylgruppen 1, 2 oder 3 Ring-Heteroatome aufweisen, die gleich oder verschieden sind und ausgewählt sind unter O, N und S; und C₃-C₇-Cycloalkylgruppen, die gegebenenfalls 1 oder 2 gleiche oder verschiedene Substituenten tragen, die ausgewählt sind unter NH₂, OH, SH, Halogen geradkettigen oder verzweigten C₁-C₆-Alkylgruppen, oder geradkettigen oder verzweigten C₁-C₆-Alkoxygruppen; wobei die Cycloalkylgruppe gegebenenfalls über eine C₁-C₄-Alkylengruppe gebunden ist; und wobei gegebenenfalls 1 oder 2 Ring-Kohlenstoffatome durch gleiche oder verschiedene Heteroatome, ausgewählt unter O und N ersetzt sein können;
(14) Verbindungen der Formel II, worin die Reste R₂₆ und R₂₇ gleich oder verschieden sind und ausgewählt sind unter
   Methyl, Ethyl, n-Prop-1-yl, n-Prop-2-yl, n-Butyl, sec.-Butyl, i-Butyl, tert-Butyln-Pentyl (Amyl), 2-Pentyl (sec-Pentyl), 3-Pentyl, 2-Methylbutyl, 3-Methylbutyl (isoPentyl oder iso-Amyl), 3-Methylbut-2-yl, 2-Methylbut-2-yl; 2,2-Dimethylpropyl (Neopentyl);
   Cyclopropyl, Cyclopropyl-methyl, Cyclopropyl-ethyl, Cyclobutyl, Cyclobutyl-methyl, Cyclopentyl, Cyclopentyl-methyl, Cyclohexyl, Cyclohexyl-methyl; Cycloheptyl; Benzyl; Phenyl; 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl; 2-Fluorbenzyl, 3-Fluorbenzyl, 4-Fluorbenzyl, 2-Chlorbenzyl, 3-Chlorbenzyl, 4-Chlorbenzyl, 2-Brombenzyl, 3-Brombenzyl, 4-Brombenzyl; 2-Methylphenyl, 3-Methylphenyl, 4-Methylphenyl, 2-Methylbenzyl, 3-Methylbenzyl, 4-Methylbenzyl;
   2-Pyridyl, 3-Pyridyl, 4-Pyridyl, Thiazolyl, Oxazolyl, Pyrazolyl, Furanyl, Morpholinyl, Pyranyl,
   insbesondere Cyclohexyl, Cyclopropylmethyl, Phenyl, Benzyl, 4-Chlorphenyl, 2-Methylphenyl, 2-Pyridyl, 2-Thiazolyl, Methyl, Ethyl, n-Propyl, i-Propyl, n-Butyl, i-Butyl;
(15) Verbindungen der Formel II, worin X ausgewählt ist unter;
   a) 1, 2, 3 oder 4 Kohlenstoffatome umfassenden C1-Kohlenstoffbrücken der allgemeinen Formel 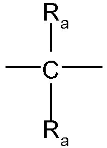 worin
      die einer der Reste Rₐ für H oder Methyl steht und der andere für geradkettiges oder verzweigtes C₁-C₃-Alkyl, oder geradkettiges oder verzweigtes C₂-C₃-Alkenyl stehen oder beide Rₐ-Reste eine Gruppe der Formel 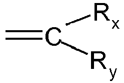 worin Rₓ und R_{y} unabhängig voneinander für H, Methyl oder Ethyl stehen; oder
   b) 2, 3 oder 4 Kohlenstoffatome umfassenden C2-Kohlenstoffbrücken der allgemeinen Formeln 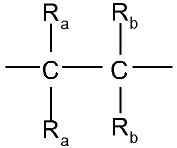 oder 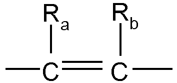 worin
      die Reste Rₐ und R_{b} unabhängig voneinander für H, Methyl, Ethyl, Ethenyl, stehen oder zwei geminale Reste Rₐ bzw R_{b} für eine Gruppe der Formel =CH₂ stehen;
      oder
   c) 3 oder 4 Kohlenstoffatome umfassenden C3-Kohlenstoffbrücken, ausgewählt unter 1,3-Propylen- und 1,3 Propenylen-Brücken, die gegebenenfalls eine Methyl-Seitengruppe tragen;
      oder
   d) lineare 1,4-verknüpfte C4-Kohlenstoffbrücken, die gegebenenfalls 1 Doppelbindung aufweist;
(16) Verbindungen der Formel II, worin X ausgewählt ist unter den in den folgenden Tabellen 2A, 2B, 2C und 2D aufgeführen C1- C2-, C3- und C4-Brücken.
(17) Kombinationen der Ausgestaltungen: (1)+(2);
(18) Kombinationen der Ausgestaltungen: (1)+(3), (1)+(4), (1)+5);
(19) Kombinationen der Ausgestaltungen: (1)+(6), (1)+(7), (1)+(8);
(20) Kombinationen der Ausgestaltungen:(1)+(9), (1)+(10), (1)+(11);
(21) Kombinationen der Ausgestaltungen: (1)+(12), (1)+(13), (1)+(14);
(22) Kombinationen der Ausgestaltungen: (1)+(15), (1)+(16);
(23) Kombinationen der Ausgestaltungen: (17)+(3), (17)+(4), (17)+(5)
(24) Kombinationen der Ausgestaltungen: (17)+(6), (17)+(7), (17)+(8);
(25) Kombinationen der Ausgestaltungen: (17)+(9), (17)+(10), (17)+(11);
(26) Kombinationen der Ausgestaltungen: (17)+(12), (17)+(13), (17)+(14);
(27) Kombinationen der Ausgestaltungen: (17)+(15), (17)+(16);
(28) Kombinationen der Ausgestaltungen: (23)+(6), (23)+(7), (23)+(8);
(29) Kombinationen der Ausgestaltungen: (23)+(9), (23)+(10), (23)+(11);
(30) Kombinationen der Ausgestaltungen: (23)+(12), (23)+(13), (23)+(14);
(31) Kombinationen der Ausgestaltungen: (23)+(15), (23)+(16);
(32) Kombinationen der Ausgestaltungen: (28)+(12), (28)+(13), (28)+(14);
(33) Kombinationen der Ausgestaltungen: (28)+(15), (28)+(16);
(34) Kombinationen der Ausgestaltungen: (32)+(15), (32)+(16);
(35) Kombinationen der Ausgestaltungen: (29)+(12), (29)+(13), (29)+(14);
(36) Kombinationen der Ausgestaltungen: (29)+(15), (29)+(16);
(37) Kombinationen der Ausgestaltungen: (35)+(15), (35)+(16);
(38) Kombinationen der Ausgestaltungen: (30)+(15), (30)+(16);
(39) Kombinationen der Ausgestaltungen: (24)+(12), (24)+(13), (24)+(14);
(40) Kombinationen der Ausgestaltungen: (24)+(15), (24)+(16);
(41) Kombinationen der Ausgestaltungen: (39)+(15), (39)+(16);
(42) Kombinationen der Ausgestaltungen: (25)+(12), (25)+(13), (25)+(14);
(43) Kombinationen der Ausgestaltungen: (25)+(15), (25)+(16);
(44) Kombinationen der Ausgestaltungen: (42)+(15), (42)+(16);
(45) Kombinationen der Ausgestaltungen: (26)+(15), (26)+(16);
(46) Kombinationen der Ausgestaltungen: (18)+(6), (18)+(7), (18)+(8);
(47) Kombinationen der Ausgestaltungen: (18)+(9), (18)+(10), (18)+(11);
(48) Kombinationen der Ausgestaltungen: (18)+(12), (18)+(13), (18)+(14);
(49) Kombinationen der Ausgestaltungen: (18)+(15), (18)+(16);
(50) Kombinationen der Ausgestaltungen: (46)+(12), (46)+(13), (46)+(14);
(51) Kombinationen der Ausgestaltungen: (46)+(15), (46)+(16);
(52) Kombinationen der Ausgestaltungen:; (50)+(15), (50)+(16)
(53) Kombinationen der Ausgestaltungen: (47)+(12), (47)+(13), (47)+(14);
(54) Kombinationen der Ausgestaltungen: (47)+(15), (47)+(16);
(55) Kombinationen der Ausgestaltungen: (53)+(15), (53)+(16);
(56) Kombinationen der Ausgestaltungen: (48)+(15), (48)+(16);
(57) Kombinationen der Ausgestaltungen: (19)+(12), (19)+(13), (19)+(14);
(58) Kombinationen der Ausgestaltungen: (19)+(15), (19)+(16);
(59) Kombinationen der Ausgestaltungen: (57)+(15), (57)+(16);
(60) Kombinationen der Ausgestaltungen: (20)+(12), (20)+(13), (20)+(14);
(61) Kombinationen der Ausgestaltungen: (20)+(15), (20)+(16);
(62) Kombinationen der Ausgestaltungen: (60)+(15), (60)+(16);
(63) Kombinationen der Ausgestaltungen: (21)+(15), (21)+(16);
(64) Verbindungen der Formel II, worin die Reste R₂₆ und R₂₇ gleich oder verschieden sind und ausgewählt ist unter, Cyclohexyl, 2-Pyridyl, und Phenyl;
(65) Verbindungen der Formel II, worin die Reste R₂₆ und R₂₇ zusammen mit dem N-Atom an das sie gebunden sind für -N(Cyclohexyl)(2-Pyridin-2yl) oder - N(Phenyl)₂ steht.
(66) Kombination der Ausführungsform (64) mit einer der Ausgestaltungen (1) bis (63)
(67) Kombination der Ausführungsform (65) mit einer der Ausgestaltungen (1) bis (63)

Spezielle Beispiele für Verbindungen der Formel 2 sind in folgender Tabelle 2 (A, B, C und B) zusammengefasst

**Tabelle 2A: Verbindungen mit C1-Brücken**

| ein C-Atom | | | |
|---|---|---|---|
| 1 | 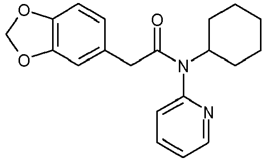 | | |

| zwei C-Atome | | | |
|---|---|---|---|
| 2 | 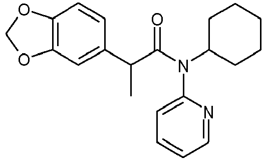 | 3 | 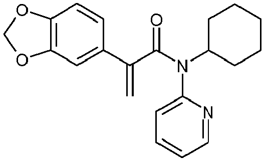 |

| drei C-Atome | | | |
|---|---|---|---|
| 4 | 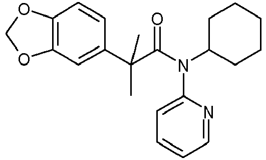 | 5 | 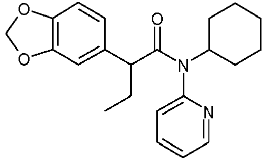 |
| 6 | 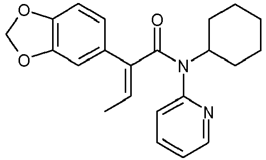 | 7 | 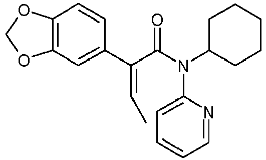 |
| 8 | 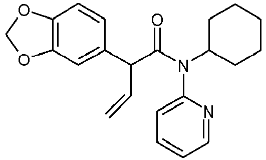 | 9 | 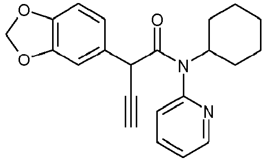 |

| vier C-Atome | | | |
|---|---|---|---|
| 10 | 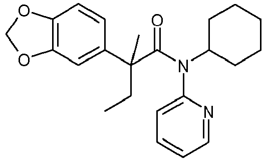 | 11 | 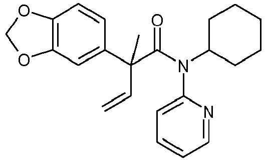 |
| 12 | 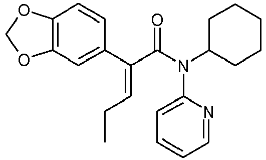 | 13 | 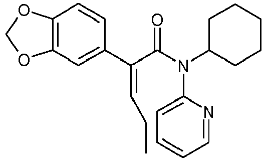 |
| 14 | 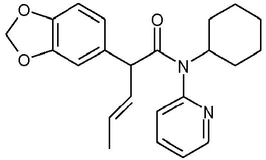 | 15 | 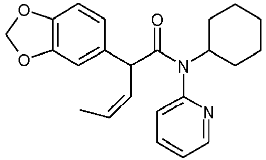 |
| 16 | 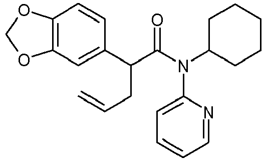 | 17 | 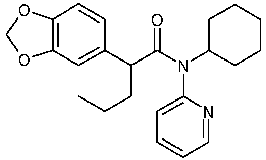 |
| 18 | 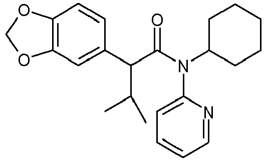 | 19 | 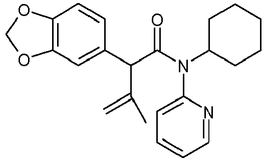 |
| 20 | 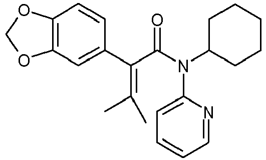 | 21 | 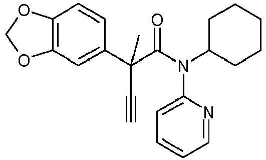 |
| 22 | 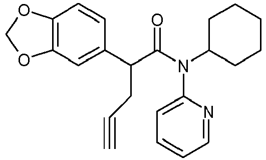 | 23 | 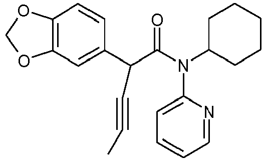 |

Offenbart sind auch Salze dieser Verbindungen in obiger Tabelle, insbesondere Säureadditionssalze mit anorganischen oder insbesondere organischen, ein- oder insbesondere mehrwertigen Carbonsäuren; und alle Isomeren oder Stereoisomeren in Reinform oder als Gemisch von Isomeren und/ oder Stereoisomeren.

**Tabelle 2B: Verbindungen mit C2-Brücken**

| zwei C-Atome | | | |
|---|---|---|---|
| 24 | 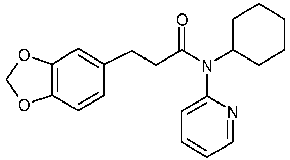 | 21a | 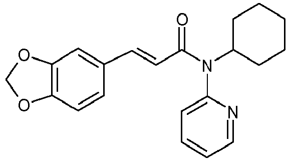 |
| 26 | 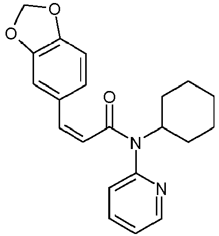 | | |

| drei C-Atome | | | |
|---|---|---|---|
| 27 | 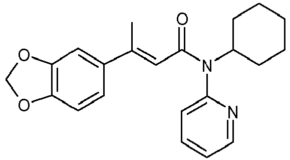 | 28 | 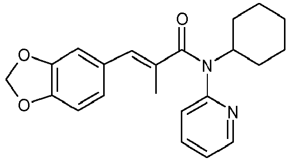 |
| 29 | 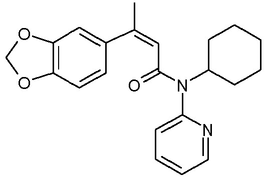 | 30 | 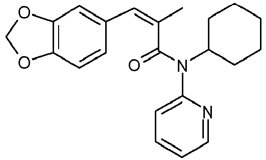 |
| 31 | 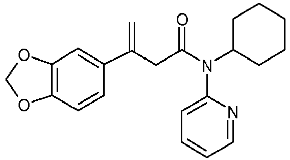 | 32 | 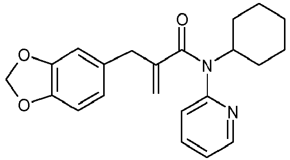 |
| 33 | 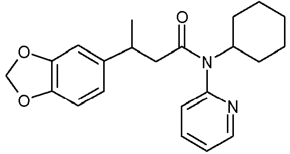 | 34 | 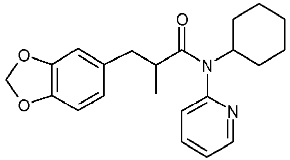 |

| vier C-Atome | | | |
|---|---|---|---|
| 35 | 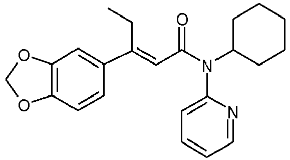 | 36 | 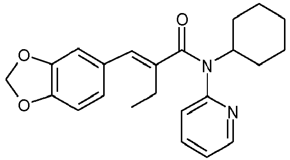 |
| 37 | 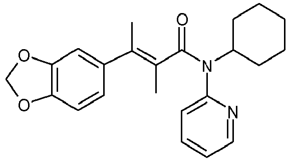 | 38 | 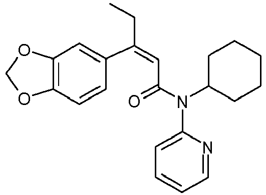 |
| 39 | 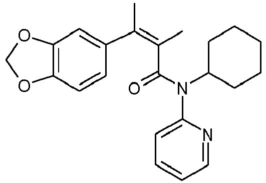 | 40 | 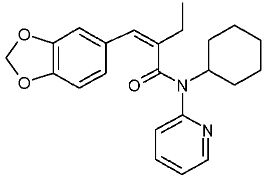 |
| 41 | 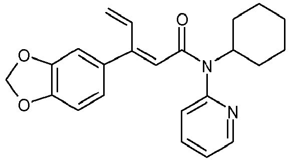 | 42 | 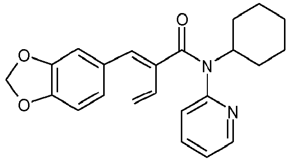 |
| 43 | 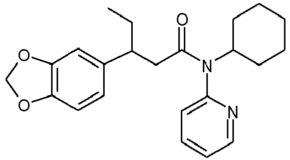 | 44 | 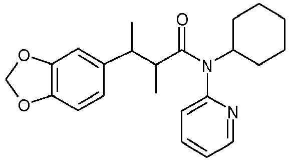 |
| 45 | 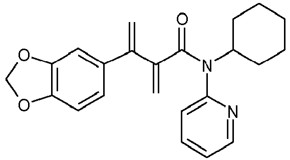 | 46 | 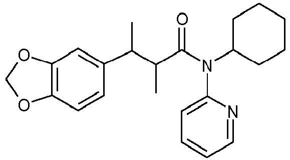 |
| 47 | 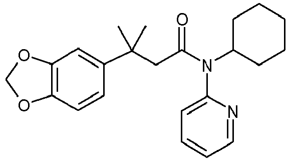 | 48 | 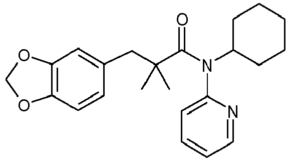 |
| 49 | 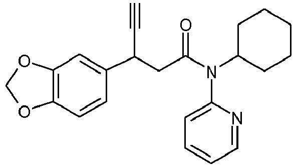 | 50 | 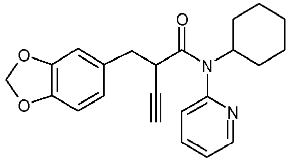 |
| 51 | 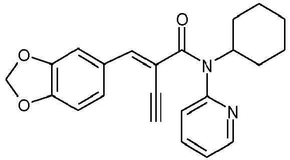 | 52 | 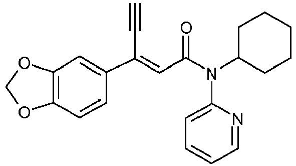 |
| 53 | 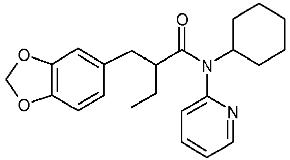 | | |

Offenbart sind auch Salze dieser Verbindungen in obiger Tabelle, insbesondere Säureadditionssalze mit anorganischen oder insbesondere organischen, ein- oder insbesondere mehrwertigen Carbonsäuren; und alle Isomeren oder Stereoisomeren in Reinform oder als Gemisch von Isomeren und/ oder Stereoisomeren.

**Tabelle 2C: Verbindungen mit C3-Brücken**

| drei C-Atome | | | |
|---|---|---|---|
| 54 | 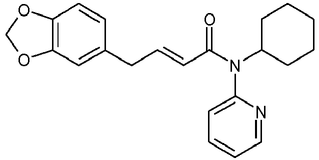 | 55 | 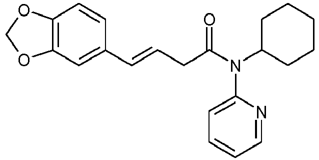 |
| 56 | 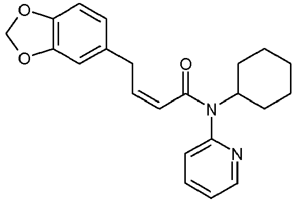 | 57 | 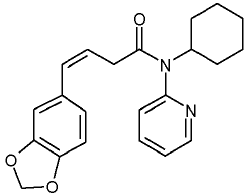 |
| 58 | 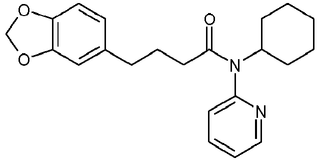 | | |

| vier C-Atome | | | |
|---|---|---|---|
| 59 | 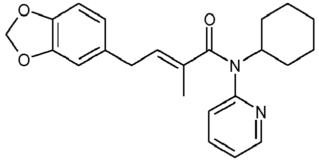 | 60 | 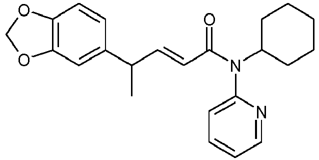 |
| 61 | 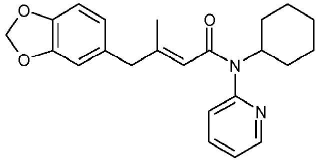 | 62 | 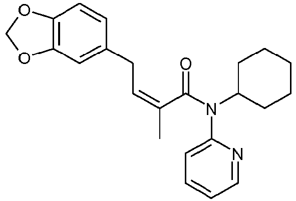 |
| 63 | 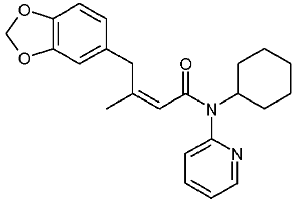 | 64 | 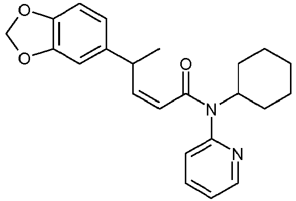 |
| 65 | 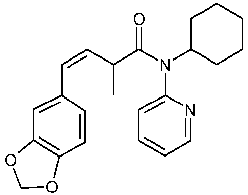 | 66 | 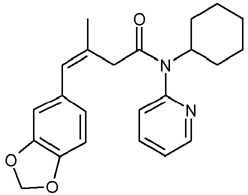 |
| 67 | 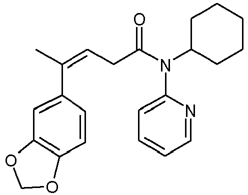 | 68 | 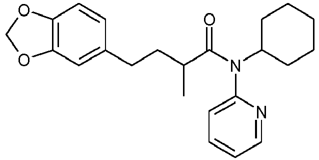 |
| 69 | 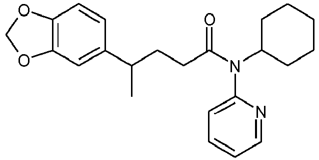 | 70 | 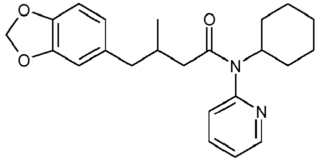 |
| 71 | 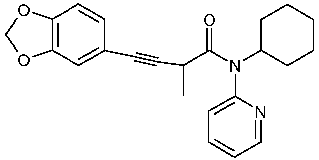 | 72 | 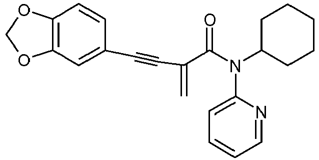 |
| 73 | 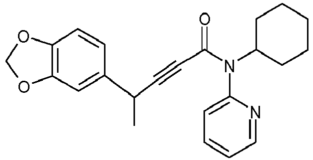 | 74 | 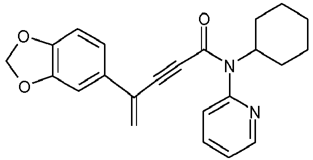 |

Offenbart sind auch Salze dieser Verbindungen in obiger Tabelle, insbesondere Säureadditionssalze mit anorganischen oder insbesondere organischen, ein- oder insbesondere mehrwertigen Carbonsäuren; und alle Isomeren oder Stereoisomeren in Reinform oder als Gemisch von Isomeren und/ oder Stereoisomeren.

**Tabelle 2D: Verbindungen mit C-4 Brücken**

| vier C-Atome | | | |
|---|---|---|---|
| 75 | 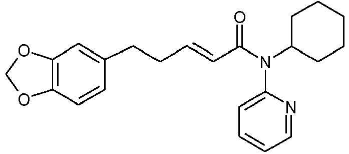 | 76 | 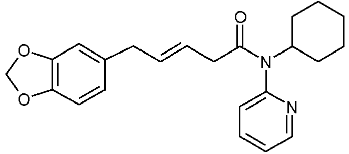 |
| 77 | 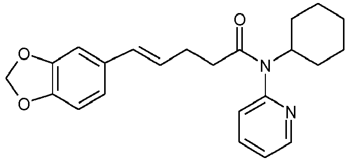 | 78 | 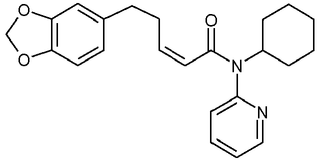 |
| 79 | 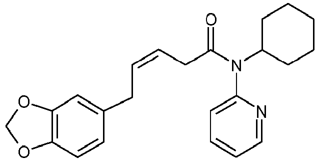 | 80 | 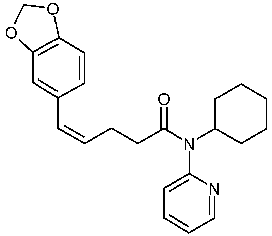 |
| 81 | 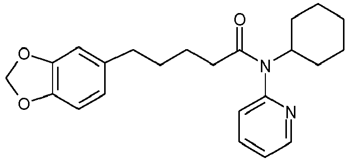 | 82 | 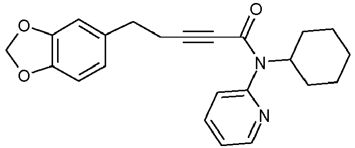 |
| 83 | 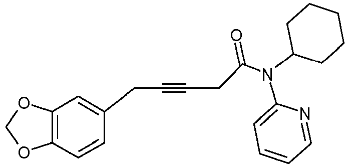 | 84 | 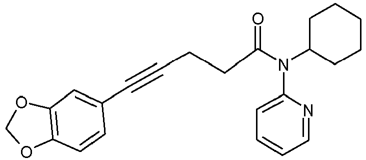 |

Offenbart sind auch Salze dieser Verbindungen in obiger Tabelle, insbesondere Säureadditionssalze mit anorganischen oder insbesondere organischen, ein- oder insbesondere mehrwertigen Carbonsäuren; und alle Isomeren oder Stereoisomeren in Reinform oder als Gemisch von Isomeren und/ oder Stereoisomeren.

### 3.7 Verbindungen der allgemeinen Formel III

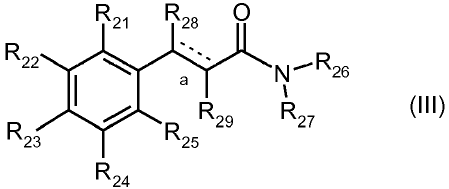

ausgewählt unter Verbindungen entsprechend den obigen für Verbindungen der Formel II angegeben Gruppen (1) bis (67), mit der Maßgabe dass X durch die Brückengruppe 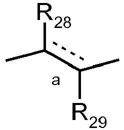 ersetzt ist und wobei die Gruppen (15) und (16) folgenden Bedeutungen besitzen
(15) Verbindungen der Formel III worin R₂₈ und R₂₉ gleich oder verschieden sind und ausgewählt sind unter H und geradkettiges oder verzweigtes C₁-C₈-Alkyl oder geradkettiges oder verzweigtes C₂-C₈-Alkenyl, C₃-C₁₂-Cycloalkyl oder C₅-C₈-Cycloalkenyl, wobei die Kohlensstoffkette, bzw. der Kohlenstoffring dieser Reste gegebenenfalls durch ein oder zwei Ring-Heteroatome, ausgewählt unter O, S und N (oder -NH-), insbesondere O, unterbrochen ist, wobei die Summe der C-Atome in den Resten R₂₈ und R₂₉ zusammen wenigsten 3 beträgt;
(16) Verbindungen der Formel III worin R₂₈ und R₂₉ gleich oder verschieden sind und ausgewählt sind unter H und geradkettiges oder verzweigtes C₁-C₄-Alkyl oder geradkettiges oder verzweigtes C₂-C₄-Alkenyl, C₃-C₆-Cycloalkyl oder C₅-C₆-Cycloalkenyl, wobei die Kohlensstoffkette, bzw. der Kohlenstoffring dieser Reste gegebenenfalls durch ein oder zwei Ring-Heteroatome, ausgewählt unter O und S unterbrochen ist, wobei die Summe der C-Atome in den Resten R₂₈ und R₂₉ zusammen wenigsten 3 beträgt;

Konkrete Beispiele für Verbindungen der Formel III sind Verbindungen gemäß obiger Tabelle 2B, wobei jedoch die dortige C2-Brücke durch eine Brücke der Formel -R₂₈C=CR₂₉- gemäß obiger Definition für Formel III, insbesondere gemäß obigen Gruppen (15) und (16) ersetzt ist.

### 4. Weitere Ausgestaltungen der erfindungsgemäß anzuwendenden Mittel:

### 4.1 Allgemeine Angaben zu Anwendungsgebieten und Formulierungen erfindungsgemäß anzuwendender Wirkstoffe

Die erfindungsgemäß anzuwendenden Wirkstoffe besitzen ein breites Anwendungsgebiet in der Humankosmetik- und -pflege, insbesondere der Haut- und Haarpflege, sind aber auch pharmakologisch einsetzbar, sowie in Nahrungsmitten und Textilprodukten, aber auch als Repellents und als Bestandteil von insektizid wirkenden Zusammensetzungen verwendbar.

Bei den erfindungsgemäßen Mitteln kann es sich insbesondere um hautkosmetische, haarkosmetische, dermatologische, hygienische oder pharmazeutische Mittel handeln. Insbesondere werden die erfindungsgemäß anzuwendenden, insbesondere kühlend wirkenden Wirkstoffe für die Haut-, und/oder Haarkosmetik oder als Mundpflegemittel angewendet.

Die erfindungsgemäßen haar- oder hautpflegenden Zubereitungen liegen insbesondere in Form einer Emulsion, einer Dispersion, einer Suspension, in Form einer wässrigen Tensidzubereitung, einer Milch, einer Lotion, einer Creme, eines Balsams, einer Salbe, eines Gels, eines Granulats, eines Puders, eines Stiftpräparates, wie z. B. eines Lippenstifts, eines Schaums, eines Aerosols oder eines Sprays vor. Solche Formulierungen sind gut geeignet für topische Zubereitungen. Als Emulsionen kommen Öl-in-Wasser-Emulsionen und Wasser-in-ÖI-Emulsionen oder Mikroemulsionen in Frage.

Im Regelfall wird die haar- oder hautkosmetische Zubereitung zur Applikation auf der Haut (topisch) oder dem Haar verwendet. Unter "topischen Zubereitungen" sind dabei solche Zubereitungen zu verstehen, die dazu geeignet sind, die Wirkstoffe in feiner Verteilung, wie z.B. in einer durch die Haut resorbierbaren Form auf die Haut aufzubringen. Hierfür eignen sich z. B. wässrige und wässrig-alkoholische Lösungen, Sprays, Schäume, Schaumaerosole, Salben, wässrige Gele, Emulsionen vom O/W- oder W/O-Typ, Mikroemulsionen oder kosmetische Stiftpräparate.

Nach einer Ausführungsform des erfindungsgemäßen kosmetischen Mittels enthält dieses einen Träger. Bevorzugt als Träger ist Wasser, ein Gas, eine Wasser-basierte Flüssigkeit, ein Öl, ein Gel, eine Emulsion oder Mikroemulsion, eine Dispersion oder eine Mischung davon. Die genannten Träger zeigen eine gute Hautverträglichkeit. Besonders vorteilhaft für topische Zubereitungen sind wässrige Gele, Emulsionen oder Mikroemulsionen.

Die erfindungsgemäße Lehre umfasst auch die erfindungsgemäß anzuwendenden Wirkstoffe zur Verwendung in pharmazeutischen Mitteln zur Behandlung eines Individuums, vorzugsweise eines Säugers, insbesondere eines Menschen, Nutz- oder Haustieres. Die Wirkstoffe werden dazu in Form von pharmazeutischen Zusammensetzungen verabreicht, die einen pharmazeutisch verträglichen Exzipienten mit wenigstens einem erfindungsgemäß anzuwendenden Wirkstoff und gegebenenfalls weiteren Wirkstoffen umfassen. Diese Zusammensetzungen können beispielsweise auf oralem, rektalem, transdermalem, subkutanem, intravenösem, intramuskulärem oder intranasalem Weg verabreicht werden.

Beispiele geeigneter pharmazeutischer Formulierungen sind feste Arzneiformen, wie Pulver, Puder, Granulate, Tabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäß anzuwendender Inhibitoren verwendet werden. Ferner können auch Liposomen, Mikrosphären oder Polymermatrizes zur Anwendung kommen.

Bei der Herstellung erfindungsgemäßer Zusammensetzungen werden erfindungsgemäß anzuwendende Wirkstoffe gewöhnlich mit einem Exzipienten vermischt oder verdünnt. Exzipienten können feste, halbfeste oder flüssige Materialien sein, die als Vehikel, Träger oder Medium für den Wirkstoff dienen. Der Wirkstoffgehalt (eines oder mehrerer gleichzeitig enthaltener erfindungsgemäßanzuwendender Wirkstoffe) kann dabei in einem weiten Bereich variieren und liegt, jeweils bezogen auf das Gesamtgewicht der Zusammensetzung, im ppm-Bereich von 0,05 ppm - < 0,1 ppm und 0,1 bis 1000 ppm (d.h. 0,00001 bis 0,1 Gew.-%), wie z.B. 1 bis 800 ppm oder 100 bis 500 ppm oder im Bereich von 0,1 bis 50, 1 bis 30 oder 2 bis 10 Gew.-%.

Zu geeigneten Exzipienten gehören beispielsweise Lactose, Dextrose, Sucrose, Sorbitol, Mannitol, Stärken, Akaziengummi, Calciumphosphat, Alginate, Traganth, Gelatine, Calciumsilikat, mikrokristalline Cellulose, Polyvinylpyrrolidon, Cellulose, Wasser, Sirup und Methylcellulose. Ferner können die Formulierungen pharmazeutisch akzeptable Träger oder übliche Hilfsstoffe, wie Gleitmittel, beispielsweise Talg, Magnesiumstearat und Mineralöl; Netzmittel; emulgierende und suspendierende Mittel; konservierende Mittel, wie Methyl- und Propylhydroxybenzoate; Antioxidantien; Antireizstoffe; Chelatbildner; Dragierhilfsmittel; Emulsionsstabilisatoren Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, oder Hager's Handbuch der Pharmazeutischen Praxis, Springer Verlag, Heidelberg dargestellt ist.

Die erfindungsgemäßen Mittel können neben üblichen Zusatz- oder Hilfsstoffen zusätzlich kosmetisch und/oder dermatologisch und/oder pharmakologisch aktive Wirkstoffe enthalten.

Als Beispiele geeigneter weiterer Wirkstoffe sind zu nennen:
Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, keratinhärtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder - feuchthaltende Stoffe, rückfettende Wirkstoffe, antierythimatös oder antiallergisch aktive Wirkstoffe, verzweigte Fettsäuren, wie 18-Methyleicosansäure, und Mischungen davon.

Künstlich hautbräunende Wirkstoffe, die geeignet sind, die Haut ohne natürliche oder künstliche Bestrahlung mit UV-Strahlen zu bräunen; dies sind z. B. Dihydroxyaceton, Alloxan und Walnussschalenextrakt. Geeignete keratinhärtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat etc.

Antimikrobielle Wirkstoffe, die eingesetzt werden, um Mikroorganismen zu zerstören bzw. ihr Wachstum zu hemmen. Sie dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure etc. Derartige desodorierend wirkende Stoffe sind z. B. Zinkricinoleat, Triclosan, Undecylensäurealkylolamide, Citronensäuretriethylester, Chlorhexidin etc.

Geeignete Hilfs- und Zusatzstoffe für die Herstellung von haarkosmetischen oder hautkosmetischen Zubereitungen sind dem Fachmann geläufig und können aus Handbüchern der Kosmetik, beispielsweise Schrader, Grundlagen und Rezepturen der Kosmetika, Hüthig Verlag, Heidelberg, 1989, ISBN 3-7785-1491-1, entnommen werden. Die Hilfs- und Zusatzstoffe sind bevorzugt kosmetisch und/oder pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB, Ph. Eur., BP, NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidanzien, Antireizstoffe, Chelatbildner, Emulsionsstabilisatoren, Filmbildner, Gelbildner, Geruchsmaskierungsmittel, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilanzien, Treibmittel, Trocknungsmittel, Trübungsmittel, Verdickungsmittel, Wachse, Weichmacher, Weißöl. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Weitere geeignete Zusatzstoffe sind ausgewählt unter Parfümölen, Haarpolymeren, Haar- und Hautkonditionern, Pfropfpolymeren, wasserlöslichen oder dispergierbaren silikonhaltigen Polymeren, Lichtschutzmitteln, Bleichmitteln, Pflegemitteln, Färbemitteln, Tönungsmitteln, Bräunungsmitteln, Farbstoffen, Konsistenzgebern, Feuchthaltemitteln, Rückfettern, Collagen, Eiweißhydrolysaten, Lipiden, Antioxidanzien, Entschäumern, Antistatika, Emollienzien, Weichmachern, Peroxidzersetzern.

Als Beispiele geeigneter Hilfs- und Zusatzstoffe sind zu nennen:
(1) Antioxidanzien, ausgewählt unter Aminosäuren (z.B. Glycin, Histidin, Tyrosin, Tryptophan) und deren Derivaten, Imidazolen (z. B. Urocaninsäure) und deren Derivaten, Peptiden wie D,L-Carnosin, D-Carnosin, L-Carnosin und deren Derivaten (z. B. Anserin), Carotinoiden, Carotinen (z. B. β-Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thiorodoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z. B. Buthioninsulfoximine, Homocysteinsulfoximine, Buthioninsulfone, Penta-, Hexa-, Heptathioninsulfoximin), insbesondere in sehr geringen, verträglichen Dosierungen (z. B. pmol bis µmol/kg Bereich), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA und deren Derivaten, ungesättigten Fettsäuren und deren Derivaten (z. B. γ-Linolensäure, Linolsäure, Ölsäure), Folsäure und deren Derivaten, Ubichinon und Ubichinol und deren Derivaten, Vitamin C und dessen Derivaten (z. B. Natriumascorbat, Ascorbylpalmitat, Mg-Ascorbylphosphat, Ascorbylacetat), Tocopherol und Derivate (z. B. Vitamin-E-Acetat, Tocotrienol), Vitamin A und Derivate (Vitamin-A-Palmitat) sowie Koniferylbenzoat des Benzoeharzes, Rutinsäure und deren Derivaten, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z. B. ZnO, ZnSO₄), Selen und dessen Derivaten (z. B. Selenmethionin), Stilbenen und deren Derivaten (z. B. Stilbenoxid, Trans-Stilbenoxid).
(2) Peroxidzersetzer, d. h. Verbindungen, die in der Lage sind Peroxide, besonders bevorzugt Lipidperoxide, zu zersetzen. Darunter sind organische Substanzen zu verstehen, wie z. B. Pyridin-2-thiol-3-carbonsäure, 2-Methoxy-pyrimidinolcarbonsäuren, 2-Methoxy-pyridincarbonsäuren, 2-Dimethylamino-pyrimidinolcarbonsäuren, 2-Dimethylamino-pyridincarbonsäuren.
(3) Verdickungsmittel, wie vernetzte Polyacrylsäuren und deren Derivate, Polysaccharide und deren Derivate, wie Xanthangummi, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon. Insbesondere werden nichtionische Verdicker eingesetzt.
(4) Konservierungsmittel, die mit ihrer E-Nummer nachfolgend aufgeführt sind

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner sind erfindungsgemäß in der Kosmetik gebräuchliche Konservierungsmittel oder Konservierungshilfsstoffe geeignet, wie Dibromdicyanobutan (2-Brom-2-brommethylglutarodinitril), 3-Jod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, Formaldehydabspalter.
Ferner sind Phenylhydroxyalkylether, insbesondere die unter der Bezeichnung Phenoxyethanol bekannte Verbindung, aufgrund ihrer bakteriziden und fungiziden Wirkungen auf eine Anzahl von Mikroorganismen als Konservierungsmittel geeignet.
Auch andere keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden. Ebenso können auch antimikrobielle Polypeptide eingesetzt werden.
(5) Lichtfilterwirkstoffe, die UV-Strahlen im UV-B- und/oder UV-A-Bereich absorbieren. Geeignete UV-Filter sind z. B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl, und Mischungen davon. Geeignet sind weiterhin p-Aminobenzoesäureester, Zimtsäureester, Benzophenone, Kampferderivate sowie UV-Strahlen abhaltende Pigmente, wie Titandioxid, Talkum und Zinkoxid.
Als UV-Filtersubstanzen kommen beliebige UV-A- und UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'-Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natriumu. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfobenzyliden)-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoat oder: 5-Methyl-2-(1-methylethyl)-2-aminobenzoat | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexenon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfobenzyliden)-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | 2,2'-Methylen-bis-[6-(2H-benzotriazol-2-yl)-4-(1,1,3,3,-tetramethylbutyl)phenol] | 103597-45-1 |
| 30 | 2,2-(1,4-Phenylen)-bis-1H-benzimidazol-4,6-disulfonsäure, Na-Salz | 180898-37-7 |
| 31 | 2,4-bis-[4-(2-Ethylhexyloxy)-2-hydroxy]phenyl-6-(4-methoxyphenyl)-(1,3,5)-triazin | 187393-00-6 |
| 32 | 3-(4-Methylbenzyliden)-campher | 36861-47-9 |
| 33 | 4-Bis(polyethoxy)paraaminobenzoesäurepolyethoxyethyl-ester | 113010-52-9 |
| 34 | 2,4-Dihydroxybenzophenon | 131-56-6 |
| 35 | 2,2'-Dihydroxy-4,4'-dimethoxybenzophenon-5,5'-dinatriumsulfonat | 3121-60-6 |
| 36 | Benzoesäure, 2-[4-(diethylamino)-2-hydroxybenzoyl]-, hexylester | 302776-68-7 |
| 37 | 2-(2H-Benzotriazol-2-yl)-4-methyl-6-[2-methyl-3-[1,3,3,3-tetramethyl-1-[(trimethylsilyl)oxy]disiloxanyl]propyl]phenol | 155633-54-8 |
| 38 | 1,1-[(2,2'-Dimethylpropoxy)carbonyl]-4,4-diphenyl-1,3-butadien | 363602-15-7 |

Die erfindungsgemäßen kosmetischen und dermatologischen Zubereitungen können vorteilhafterweise außerdem UV-Strahlen abhaltende anorganische Pigmente auf Basis von Metalloxiden und/oder anderen in Wasser schwer löslichen oder unlöslichen Metallverbindungen, ausgewählt aus der Gruppe der Oxide des Zinks (ZnO), Titan (TiO₂), Eisens (z. B. Fe₂O₃), Zirkoniums (ZrO₂), Siliciums (SiO₂), Mangans (z.B. MnO), Aluminiums (Al₂O₃), Cers (z. B. Ce₂O₃), Mischoxiden der entsprechenden Metalle sowie Abmischungen aus solchen Oxiden enthalten.
Die anorganischen Pigmente können dabei in gecoateter Form vorliegen, d. h. dass sie oberflächlich behandelt sind. Diese Oberflächenbehandlung kann beispielsweise darin bestehen, dass die Pigmente nach an sich bekannter Weise, wie in DE-A-33 14 742 beschrieben, mit einer dünnen hydrophoben Schicht versehen sind.
(6) Repellentwirkstoffe, d. h. Verbindungen, die in der Lage sind, bestimmte Tiere, insbesondere Insekten, vom Menschen abzuhalten oder zu vertreiben. Dazu gehört z. B. 2-Ethyl-1, 3-hexandiol, N, N-Diethyl-m-toluamid etc.
(7) Geeignete hyperemisierend wirkende Stoffe, welche die Durchblutung der Haut anregen, sind z. B. ätherische Öle, wie Latschenkieferextrakt, Lavendelextrakt, Rosmarinextrakt, Wacholderbeerextrakt, Rosskastanienextrakt, Birkenblätterextrakt, Heublumenextrakt, Ethylacetat, Kampfer, Menthol, Pfefferminzöl, Rosmarinextrakt, Eukalyptusöl etc.
(8) Geeignete keratolytisch und keratoplastisch wirkende Stoffe sind z. B. Salicylsäure, Kalziumthioglykolat, Thioglykolsäure und ihre Salze, Schwefel etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion etc.
(9) Geeignete Antiphlogistika, die Hautreizungen entgegenwirken, sind z. B. Allantoin, Bisabolol, Dragosantol, Kamillenextrakt, Panthenol etc.
(10) Kosmetisch oder pharmazeutisch akzeptable Polymere, wie kationische, amphotere und neutrale Polymere.
Geeignete Polymere sind z. B. kationische Polymere mit der Bezeichnung Polyquaternium nach INCI, z. B. Copolymere aus Vinylpyrrolidon/N-Vinylimidazoliumsalzen (Luviquat FC, Luviquat HM, Luviquat MS, Luviquat Care), Copolymere aus N-Vinylpyrrolidon/Dimethylaminoethylmethacrylat, quaternisiert mit Diethylsulfat (Luviquat PQ 11), Copolymere aus N-Vinylcaprolactam/N-Vinylpyrrolidon/N-Vinylimidazoliumsalzen (Luviquat E Hold), kationische Cellulosederivate (Polyquaternium-4 und -10), Acrylamidocopolymere (Polyquaternium-7) und Chitosan.
Geeignete kationische (quaternisierte) Polymere sind auch Merquat (Polymer auf Basis von Dimethyldiallylammoniumchlorid), Gafquat (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar-Marken der Firma Rhodia.
Weitere geeignete Polymere sind auch neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und deren Derivate. Dazu zählt beispielsweise Luviflex ® Swing (teilverseiftes Copolymerisat von Polyvinylacetat und Polyethylenglykol, Firma BASF).
Geeignete Polymere sind auch nichtionische, wasserlösliche bzw. wasserdispergierbare Polymere oder Oligomere, wie Polyvinylcaprolactam, z. B. Luviskol ® Plus (BASF), oder Polyvinylpyrrolidon und deren Copolymere, insbesondere mit Vinylestern, wie Vinylacetat, z. B. Luviskol ® VA 37 (BASF), Polyamide, z. B. auf Basis von Itaconsäure und aliphatischen Diaminen, wie sie z. B. in der DE-A-43 33 238 beschrieben sind.
Geeignete Polymere sind auch amphotere oder zwitterionische Polymere, wie die unter den Bezeichnungen Amphomer (National Starch) erhältlichen Octylacrylamid / Methylmethacrylat / tert.-Butylaminoethylmethacrylat-Hydroxypropylmethacrylat-Copolymere sowie zwitterionische Polymere, wie sie beispielsweise in den deutschen Patentanmeldungen DE39 29 973, DE 21 50 557, DE28 17 369 und DE 3708 451 offenbart sind. Acrylamidopropyltrimethylammoniumchlorid/Acrylsäure- bzw. Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette (AMERCHOL) im Handel erhältlich sind, und Copolymere aus Hydroxyethylmethacrylat, Methylmethacrylat, N, N-Dimethylaminoethylmethacrylat und Acrylsäure (Jordapon (D)).
Geeignete Polymere sind auch nichtionische, siloxanhaltige, wasserlösliche oder dispergierbare Polymere, z. B. Polyethersiloxane, wie Tegopren ® (Firma Goldschmidt) oder Besi (Firma Wacker).

Im Folgenden werden einzelne besondere Anwendungsformen erfindungsgemäß anzuwendender Wirkstoffe beispielhaft näher erläutert.

### 4.2 Kühlende Haut- und Haarpflegemittel

Nach einer bevorzugten Ausführungsform handelt es sich bei den erfindungsgemäßen Mitteln um ein kühlendes Haut- oder Haarpflege- oder-reinigungsmittel.

Bevorzugte Haut- oder Haarreinigungsmittel sind Seifen von flüssiger bis gelförmiger Konsistenz, wie Transparentseifen, Luxusseifen, Deoseifen, Cremeseifen, Babyseifen, Hautschutzseifen, abrasive Seifen und Syndets, pastose Seifen, Schmierseifen und Waschpasten, Peelingseifen, Feuchtigkeitstücher, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Nach einer weiteren bevorzugten Ausführungsform handelt es sich bei den erfindungsgemäßen Mitteln um ein Duschgel, eine Shampoo-Formulierung oder ein Badepräparat. Solche Formulierungen enthalten wenigstens einen erfindungsgemäß anzuwendenden Wirkstoff sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Grundsätzlich kann der Wirkstoffgehalt über einen weiten Bereich, wie z.B. 0,00001 bis 50 Gew.-%, insbesondere 0,001 bis 10 Gew.-% oder 0,005 bis 1 Gew.-% variieren.

### i) Spezielle Ausgestaltungen für Mittel zur Auftragung auf die Haut:

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyeliner, Rouges, Puder und Augenbrauenstifte.

Außerdem können die erfindungsgemäßen dermatologischen Mittel verwendet werden in Nose-Strips zur Porenreinigung, in Antiaknemitteln, Repellents, Rasiermitteln, After- und Pre-Shave-Pflegemitteln, After-Sun-Pflegemitteln, Haarentfernungsmitteln, Haarfärbemitteln, Intimpflegemitteln, Fußpflegemitteln sowie in der Babypflege.

Bei den erfindungsgemäßen Hautpflegemitteln handelt es sich insbesondere um W/O- oder O/W-Hautcremes, Tag- und Nachtcremes, Augencremes, Gesichtscremes, Antifa-Itencremes, Sonnenschutzcremes, Feuchthaltecremes, Bleichcremes, Selbstbräunungscremes, Vitamincremes, Hautlotionen, Pflegelotionen und Feuchthaltelotionen.

Hautkosmetische und dermatologische Mittel enthalten insbesondere wenigstens einen erfindungsgemäß anzuwendenden Wirkstoff in einem Anteil von 0,0001 bis 50 Gew.-%, wie z. B. 0,001 bis 10 Gew.-%, insbesondere 0,005 bis 0,1 Gew.-%, bezogen auf das Gesamtgewicht des Mittels.

Je nach Anwendungsgebiet können die erfindungsgemäßen Hautkosmetikmittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den erfindungsgemäß anzuwendenden Wirkstoffen und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Enzyme, Eiweißhydrolysate, Stabilisatoren, pH-Wert-Regulatoren, Farbstoffe, Salze, Verdicker, Gelbildner, Konsistenzgeber, Silikone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Silikonöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und acetyliertes Lanolin sowie Mischungen davon.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane oder Silikonharze.

Die Herstellung der kosmetischen oder dermatologischen Zubereitungen erfolgt nach üblichen, dem Fachmann bekannten Verfahren.

Zur Herstellung der erfindungsgemäßen dermatologischen Mittel können die Wirkstoffe mit einem geeigneten Hilfsstoff (Exzipient) vermischt oder verdünnt werden. Exzipienten können feste, halbfeste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Weiterhin sind die Polymere und Dispersionen geeignet, als Hilfsmittel in der Pharmazie, bevorzugt als oder in Beschichtungsmittel(n) oder Bindemittel(n) für feste Arzneiformen. Sie können auch in Cremes und als Tablettenüberzugsmittel und Tablettenbindemittel verwendet werden.

Bevorzugt liegen die kosmetischen und dermatologischen Mittel in Form von Emulsionen insbesondere als Wasser-in-ÖI (W/O)- oder Öl-in-Wasser (O/W)-Emulsionen vor. Es ist aber auch möglich, andere Formulierungsarten zu wählen, beispielsweise, Gele, Öle, Oleogele, multiple Emulsionen, beispielsweise in Form von W/O/W- oder O/W/O-Emulsionen, wasserfreie Salben bzw. Salbengrundlagen usw. Auch emulgatorfreie Formulierungen wie Hydrodispersionen, Hydrogele oder eine Pickering-Emulsion sind vorteilhafte Ausführungsformen.

Die Herstellung von Emulsionen erfolgt nach bekannten Methoden. Die Emulsionen enthalten neben wenigstens einem erfindungsgemäß anzuwendenden Wirkstoff in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der emulsionstypspezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil.

Eine geeignete Emulsion als W/O-Emulsion, z. B. für eine Hautcreme etc., enthält im Allgemeinen eine wässrige Phase, die mittels eines geeigneten Emulgatorsystems in einer Öl- oder Fettphase emulgiert ist. Zur Bereitstellung der wässrigen Phase kann ein Polyelektrolytkomplex eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Rizinusöl, Sesamöl, Olivenöl, Jojobaöl, Karite-Öl, Hoplostethus-Öl, mineralische Öle, deren Destillationsbeginn unter Atmosphärendruck bei ca. 250°C und deren Destillationsendpunkt bei 410°C liegt, wie z. B. Vaselinöl, Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Silikonöle, wie Dimethylpolysiloxan, Methylphenylpolysiloxan und das Silikonglykol-Copolymer, Fettsäuren und Fettalkohole enthalten.

Neben den erfindungsgemäß anzuwendenden Wirkstoffen können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und - Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Duschgel, eine Shampooformulierung oder ein Badepräparat.

Solche Formulierungen enthalten wenigstens einen erfindungsgemäß anzuwendenden Wirkstoff sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidanzien sowie Verdickern/Gelbildnern, Hautkonditioniermitteln und Feuchthaltemitteln.

Diese Formulierungen enthalten insbesondere 2 bis 50 Gew.-%, wie 5 bis 40 Gew.-%, oder 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten, im Molekül aufweisen.

Dazu zählen z. B. Natriumlaurylsulfat, Ammoniumtaurytsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder - propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid geeignet. Die Menge Alkylenoxid beträgt 6 bis 60 Mole auf ein Mol Alkohol. Ferner sind Alkylaminoxide, Mono-oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z. B. quaternäre Ammoniumverbindungen, beispielsweise Cetyltrimethylammoniumchlorid.

Weiterhin können die Duschgel-/Shampooformulierungen Verdicker, wie z. B. Kochsalz, PEG-55, Propylenglykol-Oleat, PEG-120-Methylglucosedioleat und andere sowie Konservierungsmittel, weitere Wirk- und Hilfsstoffe und Wasser enthalten.

### ii) Spezielle Ausgestaltungen für Mittel zur Auftragung auf das Haar

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten insbesondere wenigstens einen erfindungsgemäß anzuwendenden Wirkstoff in einer Menge im Bereich von 0,0001 bis 50 Gew.-%, wie z.B. 0,001 bis 10 Gew.-%, insbesondere 0,005 bis 0,1 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen, Haarfärbe- und -bleichmittels oder "Hot-Oil-Treatments" vor. Je nach Anwendungsgebiet können die haarkosmetischen Zubereitungen als (Aerosol-) Spray, (Aerosol-) Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von 0,5 bis 20 Gew.-%. Diese Mikrodispersionen benötigen in der Regel keine Emulgatoren oder Tenside zu ihrer Stabilisierung.

Die erfindungsgemäßen haarkosmetischen Formulierungen enthalten in einer speziellen Ausführungsform a) 0,0001 bis 50 Gew.-% oder 0,001 bis 10, oder 0,005 bis 1 Gew.-% wenigstens eines erfindungsgemäß anzuwendenden Wirkstoffs b) 20 bis 99,95 Gew.-% Wasser und/oder Alkohol, c) 0 bis 50 Gew.-% wenigstens eines Treibgases, d) 0 bis 5 Gew.-% wenigstens eines Emulgators, e) 0 bis 3 Gew.-% wenigstens eines Verdickers, sowie bis zu 25 Gew.-% weitere Bestandteile.

Unter Alkohol sind alle in der Kosmetik üblichen Alkohole zu verstehen, z. B. Ethanol, Isopropanol, n-Propanol.

Weiterhin zählen hierzu alle in der Kosmetik bekannten Styling- und Conditioner-Polymere, die in Kombination mit den erfindungsgemäß anzuwendenden Wirkstoffen eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon-Copolyole (CTFA) und aminofunktionelle Silikonverbindungen wie Amodimethicone (CTFA).

Die erfindungsgemäßen Polymerisate eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

In einer bevorzugten Ausführungsform enthalten Sprayzubereitungen a) 0,0001 bis 50 Gew.-% oder 0,001 bis 10, oder 0,005 bis 1 Gew.-% wenigstens eines erfindungsgemäß anzuwendenden Wirkstoffs, b) 20 bis 99,9 Gew.-% Wasser und/oder Alkohol, c) 0 bis 70 Gew.-% wenigstens eines Treibmittel, d) 0 bis 20 Gew.-% weitere Bestandteile.

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält a) 0,0001 bis 50 Gew.-% oder 0,001 bis 10, oder 0,005 bis 1 Gew.-% wenigstens eines erfindungsgemäß anzuwendenden Wirkstoffs, b) 55 bis 99,8 Gew.-% Wasser und/oder Alkohol, c) 5 bis 20 Gew.-% eines Treibmittel, d) 0,1 bis 5 Gew.-% eines Emulgators, e) 0 bis 10 Gew.-% weitere Bestandteile.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z. B. Laureth-4 ; Cetethe, z. B. Cetheth-1, Polyethylenglycolcetylether, Cetearethe, z. B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammonium-dihydrogenphosphat, Cetyltrimoniumchlorid, Cetyltrimmoniumbromid, Cocotrimoniummethylsulfat, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein: a) 0,0001 bis 50 Gew.-% oder 0,001 bis 10, oder 0,005 bis 1 Gew.-% wenigstens eines erfindungsgemäß anzuwendenden Wirkstoffs b) 80 bis 99,85 Gew.-% Wasser und/oder Alkohol, c) 0 bis 3 Gew.-%, bevorzugt 0,05 bis 2 Gew.-%, eines Gelbildners, d) 0 bis 20 Gew.-% weitere Bestandteile.

Der Einsatz von Gelbildnern kann von Vorteil sein, um spezielle rheologische oder andere anwendungstechnische Eigenschaften der Gele einzustellen. Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquaternium-32 (und) Paraffinum Liquidum (INCI), Natriumacrylat-Copolymere (und) Paraffinum Liquidum (und) PPG-1 Trideceth-6, Acrylamidopropyltrimoniumchlorid / Acrylamid-Copolymere, Steareth-10-Allylether, Acrylat-Copolymere, Polyquaternium-37 (und) Paraffinum Liquidum (und) PPG-1 Trideceth-6, Polyquaternium 37 (und) Propylenglycoldicapratdicaprylat (und) PPG-1 Trideceth-6, Polyquaternium-7, Polyquaternium-44.

Spezielle Shampooformulierungen enthalten a) 0,0001 bis 50 Gew.-% oder 0,001 bis 10, oder 0,005 bis 1 Gew.-% wenigstens eines erfindungsgemäß anzuwendenden Wirkstoffs, b) 25 bis 94,95 Gew.-% Wasser, c) 5 bis 50 Gew.-% Tenside, c) 0 bis 5 Gew.-% eines weiteren Konditioniermittels, d) 0 bis 10 Gew.-% weitere kosmetische Bestandteile.

In den Shampooformulierungen können alle in Shampoos üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxid-Einheiten, bevorzugt 1 bis 3 Ethylenoxid-Einheiten, im Molekül aufweisen.

Geeignet sind zum Beispiel Natriumlaurylsulfat, Ammoniumlaurysulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder - propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid geeignet. Die Menge Alkylenoxid beträgt 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den erfindungsgemäß anzuwendenden Wirkstoffen eingesetzt werden.

Hierzu zählen beispielsweise die zuvor genannten kationischen Polymere mit der Bezeichnung Polyquaternium nach INCI, insbesondere Copolymere aus Vinylpyrrolidon/ N-Vinylimidazoliumsalzen (Luviquat FC, Luviquat HM, Luviquat MS, Luviquat Care), Copolymere aus N-Vinylpyrrolidon/Dimethylaminoethylmethacrylat, quaternisiert mit Diethylsulfat (Luviquat D PQ 11), Copolymere aus N-Vinylcaprolactam/N-Vinylpyrrolidon/N-Vinylimidazoliumsalzen (Luviquat D Hold), kationische Cellulosederivate (Polyquaternium-4 und -10), Acrylamidcopolymere (Polyquaternium-7). Ferner können Eiweißhydrolysate verwendet werden sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon-Kopolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

### 4.3 Kühlende Mundpflegemittel

Erfindungsgemäße Mundpflegemittel können in an sich bekannter Weise, z.B. als Zahnpasta, Zahngel, oder wässrige oder wässrig-alkoholische Mundpflegemittel (Mundwasser) formuliert sein.

Erfindungsgemäße Mundpflegezusammensetzungen enthalten, bezogen auf das Gewicht der Zusammensetzung, 0,00001 bis 50 Gew.-%, 0,0001 bis 10 Gew.-%, 0,001 bis 5 Gew.-%, 0,005 bis 1 Gew.-% oder 0,1 bis 20 Gew.-%, 0,5 bis 15 Gew.-% oder 1 bis 5 Gew.-% der Gesamtmenge wenigstens eines erfindungsgemäß anzuwendenden Wirkstoffs

Darüber hinaus können die Mundpflegemittel, insbesondere Zahnpasten, auch Abrasiva enthalten, wie Siliziumoxidhydrat, Dicalciumphosphatdihydrat, Calciumcarbonat, Natriumhydrogencarbonat, Calciumpyrophosphat und Aluminiumoxid. Beispielsweise kann auch eine Mischung aus zur Zähflüssigkeit neigendem gefällten Silizium und scheuernden gefällten Silizium [Handbook of Pharmaceutical Excipients, The Pharmaceutical Society of Great Britain, 1 Lambeth High Street, London SE 1 7JN, England, pages 253-256] verwendet werden. Das zuerst genannte wird auf Grund seiner thixotropen Eigenschaften, das zweite wegen seiner besseren Wirksamkeit beim Beseitigen der an den Zahnflächen anhaftenden Substanzen verwendet. Der Einsatz dieser Produkte gewährleistet eine geringe Scheuerwirkung, denn es handelt sich um amorphe Feststoffe mittlerer Härte, die gleichzeitig voll und ganz mit dem als Mineralisierungsmittel benutzten Fluorid kompatibel sind, da sie keine Kalksalze enthalten, welche ihre Unlöslichkeit bewirken und ihre Bioverfügbarkeit verringern würden.

Die Rezeptur der erfindungsgemäßen Mundpflegemittel, wie z.B. Zahnpasta, kann auch geeignete Zusätze und Vehikel enthalten, um deren Eigenschaften zu verbessern und die Herstellung zu erleichtern. Diese sind z.B. unter Bindemitteln, Verdickungsmittel, Duftstoffen, Farbstoffen, Konservierungsmitteln, Benetzungsmitteln oder Feuchthaltemittel, Tensiden, Schmiermitteln, Trübungsmitteln, Remineralisierungsstoffen, Tensiden, Puffern, Alkoholen, Vitaminen, Wasser, zusätzlichen Wirkstoffen und deren Mischungen ausgewählt.

Als Bindemittel kann jedes normalerweise bei der Herstellung dieser Art von Rezepturen verwendete Mittel eingesetzt werden, z. B. Tragantgummi. Das Bindemittel kann in der Rezeptur in einer Menge von 0,5-1,5 Gew.-% der Gesamtmenge enthalten sein.

In dem Mundpflegemittel können auch organische Verdickungsmittel eingearbeitet werden, wie Natriumcarboxymethylcellulose, Celluloseether, Xanthangummi, Karageenane, Natriumalginat und Carbopole. Es können auch anorganische Verdickungsmittel, wie Siliziumoxid-Verdickungsmittel, Natriumaluminiumsilikate und Tone, zur Bereitstellung der entsprechenden Rheologie verwendet werden. Das Verdickungsmittel kann in der Rezeptur in einer Menge von 0,5-5 Gew.-% der Gesamtmenge enthalten sein.

Die Zahnpasta kann durch Zusatz eines geeigneten üblichen Duftstoffes, z. B. eines Pfefferminzaromas, aromatisiert werden. Ätherische Öle einschließlich u.a. Nelkenöl, Zimtöl, Pfefferminzöl und Krauseminzöl sind ebenfalls geeignet. Der Duftstoff kann in der Rezeptur in einer Menge von 0,5-15 Gew.-% der Gesamtmenge enthalten sein.

Als Farbstoff kann jeder der üblicherweise bei der Herstellung von Zahnpasta verwendete Farbstoff eingesetzt werden zum Beispiel Brillantblau FCF, C.42090 [KIRSCH PHARMA]. Der Farbstoff kann in der Rezeptur in einer Menge von 0,001 - 0,005 Gew.-% der Gesamtmenge enthalten sein.

Bei dem Konservierungsmittel kann es sich um jedes übliche Mittel, wie etwa ein Derivat der Benzoesäure, z. B. p-Hydroxy-Methylbenzoat handeln. Das Konservierungsmittel kann in der Rezeptur in einer Menge von 0,1- 0,3 Gew.-% der Gesamtmenge enthalten sein.

Als Süßstoff kann man z. B. Natriumsaccharin oder Zyklaminsäure und deren Derivate, z. B. Natriumcyclamat, verwenden. Der Süßstoff kann in der Rezeptur in einer Menge von 0,08-0,15 Gew.-% der Gesamtmenge enthalten sein.

Das zur Verhinderung des Austrocknens und der Härtung der Zahnpasta verwendete Benetzungs- oder Feuchthaltemittel ist insbesondere ausgewählt unter Glycerin, Sorbit, Propylenglykol, Xylit und flüssige Polyethylenglykolen, insbesondere eine Mischung aus Sorbit, Glycerin und Xylit, z.B in einem Anteil von 1-60 Gew.-% der Gesamtmenge.

Als Schmiermittel kann jedes der üblicherweise in der Rezeptur einer Zahnpasta verwendeten Mittel sein, z. B. Dimethikon (Polymer des Dimethylpolysiloxans), bei dem es sich um ein oberflächenaktives Mittel handelt, das dazu beiträgt, der erfindungsgemäßen Zahnpasta gute rheologische Eigenschaften zu verleihen. Das Schmiermittel kann in der Rezeptur in einer Menge von 0,25 bis 0,75 Gew.-% der Gesamtmenge enthalten sein.

Als Trübungsmittel kann jedes der üblicherweise eingesetzten Mittel verwendet werden, z. B. Titandioxyd. Das Trübungsmittel kann in der Rezeptur in einer Menge von 0,05 bis 1 Gew.-% der Gesamtmenge enthalten sein.

Als Reminalisierungsmittel verwendet man eine Fluoridquelle, z. B. Natriumfluorid, Zinn(II)-fluorid und Natriummonofluorophosphat, da man auf diese Weise 100% eines aktiven Fluorids als Mittel zur Remineralisierung der weißen Läsionen, verursacht durch die organischen Säuren, die eine Folge des bakteriellen Stoffwechsels sind. Das Remineralisierungsmittel kann in der Rezeptur in einer Menge von 0,2 bis 0,4 Gew.-% der Gesamtmenge enthalten sein.

Typischerweise können des Weiteren übliche Bestandteile enthalten sein, wie anionische Tenside, wie z.B. Natriumlaurylsulphat, Natrium-N-Iaurylsarcosinat, Natriumlaurylsulphoacetat und Natriumalkylglycerylethersulphonat. Das Tensid kann in der Rezeptur in einer Menge von 0,05 bis 5 Gew.-% der Gesamtmenge enthalten sein

Wenn gewünscht, kann die durch die Erfindung vorgeschlagene Zahnpasta auch Vitamin enthalten, das aus der von Vitamin A, Vitamin B5, Vitamin C und Vitamin E gebildeten Gruppe und deren Mischungen ausgewählt wird. Im Fall einer Verwendung kann jedes Vitamin in der Rezeptur in einer Menge von 0,1 bis 5 Gew.-% der Gesamtmenge enthalten sein. Diese Vitamine können als solche, in Form von Provitaminen oder in Form akzeptierbarer pharmazeutischer Salze eingesetzt werden. Das Vitamin A, das in der Regel in Form seines Palmitatsalzes verwendet wird, fördert die Epithelisierung der Mundschleimhaut und schützt das Zahnfleisch. Das Vitamin B5, genauer gesagt das D- Panthenol, wirkt schmerzstillend, heilend und entzündungshemmend, schützt die Epithelschleimhaut, fördert die Epithelisierung der Verletzungen und glättet die Narben; es ist für die Behandlung von Verletzungen geeignet, welche infolge von Zahnextraktionen, Gingivitis, Stomatitis, Schmerzen nach dem Einsetzen von Zahnprothesen, Geschwüren, traumatischen Lädierungen der Schleimhaut, chronischen und rückläufigen Aphthen entstehen. Das Vitamin C regeneriert das Epithel der Mundschleimhaut, fördert die Kollagensynthese und das Immunsystem (Entzündungsmechanismus) und erhöht die Schutzfähigkeit der Phagozyten gegen Bakterien. Das Vitamin E, das normalerweise in Form seines Azetatsalzes eingesetzt wird, wirkt schmerzstillend und entzündungshemmend, schützt die Mundschleimhaut gegen die Fettüberoxidierung infolge der Bildung freier Radikale und gegen kontaminisierende Substanzen der Umwelt (Ozon, Zigarettenrauch usw.) und begünstigt die Heilung der Verletzungen. Durch die Zugabe eines oder einiger dieser Vitamine bietet die Erfindung eine Zahnpasta, die neben den zuvor genannten Eigenschaften auch entzündungshemmende Merkmale und schmerzstillende Auswirkungen besitzt, welche die Schutzfähigkeit der Membranen der Mundschleimhaut erhöhen und den Index der Zahnbelags- und Zahnsteinbildung sowie den der bakteriellen Kontamination verringern.

Zusätzliche Wirkstoffe sind z.B. antimikrobielle und Plaque-penetrierende Mittel, wie beta-Naphthol, Thymol, Chlorthymol und Hexylresorcin; oder keimtötende Verbindungen, wie quaternäre Ammoniumverbindungen.; Zahnsteinbekämpfungsmittel, wie Tetranatriumpyrophosphat, GANTREZ-Polymer® S-70, Natriumtripolyphosphat und Zinkcitrat; Peroxidverbindungen, wie Wasserstoffperoxid und anorganischen Peroxide.

Gegebenenfalls kann auch ein Puffer verwendet werden, der in für die Aufrechterhaltung eines pH-Werts von 6-8 geeigneten Konzentrationen enthalten ist, wie z.B Alkalimetallphosphatpuffer. Die Anwesenheit von Kaliumionen übt zudem eine die Überempflindichkeit lindernde Wirkung aus.

Wasser oder Alkohol können in einem Anteil von 1 bis 20 Gew.-% der Gesamtmenge des Mittels enthalten sein.

In Kombination mit dem Alkohol oder anstelle des Alkohols können auch Glykolverbindungen verwendet werden, wie Glycerin, Sorbit oder Propylenglykol.

Das erfindungsgemäße Mundpflegemittel kann leicht durch Mischung geeigneter Mengen der verschiedenen Bestandteile in einem z.B. mit Rührschaufeln ausgestatteten Reaktor hergestellt werden.

### 4.4. Kühlende Pflaster

Grundsätzlich kann der Wirkstoffgehalt über einen weiten Bereich, wie z.B. 0,00001 bis 50 Gew.-%, insbesondere 0,001 bis 10 Gew.-% oder 0,005 bis 1 Gew.-% variieren.

Erfindungsgemäße Pflaster können in beliebiger Weise aufgebaut sein, beispielsweise nach dem Matrixsystem, dem Membransystem oder dem Vliessystem (Drug Dev. Ind. Pharm. 14 (1988), 183-209; Drug Dev. Ind. Pharm. 13 (1987), 589-651; Drugs of Today 23 (1987), 625-646.

Das Matrixsystem besteht in einfachster Weise aus 3 Teilen: der flexiblen Stützfolie, der den Wirkstoff enthaltenden Klebematrix und einer Abziehfolie. Falls eine nichtklebende Matrix verwendet wird, muss zur Haftung auf der Haut eine Randzone der Stützfolie mit Klebstoff versehen werden.

Ein Membransystem weist hingegen mindestens 5 Teile auf: eine flexible Stützfolie, ein Reservoir mit gelöstem oder suspendiertem Wirkstoff, eine Membran zur Steuerung der Wirkstofffreigabe, eine auf die Membran aufgezogene Klebeschicht und eine Abziehfolie.

Beim Vliessystem besteht die den Wirkstoff enthaltende Schicht aus einem saugfähigen Vlies oder porösen Polymer, das mit einer Wirkstofflösung oder -suspension getränkt ist. Diese Schicht, die fest mit der Stützfolie verbunden ist, wird durch eine Abziehfolie abgedeckt. Der Rand der Stützfolie ist, zur Applikation auf die Haut, mit Klebstoff versehen.

Grundsätzlich sind alle erfindungsgemäß anzuwendenden Wirkstoffe in dieser Weise formulierbar.

Die zu verwendenden Hilfsstoffe sind die für die Herstellung von Pflastern üblichen. Neben dem klebenden Agens, in der Regel ein Polymer mit einer Glastemperatur zwischen -70 und -10, insbesondere -55 und -25 °C, sowie einer Trägerfolie, die mit diesem klebenden Agens beschichtet wird, und dem Wirkstoff werden häufig Emulgatoren, Verdickungsmittel sowie Stoffe, die die Wirkstofffreigabe beeinflussen sollen, und andere Hilfsmittel zugesetzt.

Die klebrigen Polymeren mit den oben genannten niedrigen Glastemperaturen sind bekannt, beispielsweise aus den US-Patenten 2 973 282 und 3 307 544. Die selbstklebenden Bänder und Folien sollen auf bloßen Kontakt auf der menschlichen Haut kleben, doch soll die Kohäsion der Klebschicht und deren Adhäsion an der Trägerfolie größer sein als die Adhäsion an der Haut, so dass sie sich weitgehend rückstandslos wieder abziehen lassen. Es handelt sich in der Regel um Copolymerisate auf der Basis von Acryl- und Methacrylsäureestern von Alkoholen mit 2 bis 12, insbesondere 4 bis 8 Kohlenstoffatomen, die zahlreiche andere Comonomere einpolymerisiert enthalten können, beispielsweise (Meth)acrylsäure, (Meth)acrylnitril, (Meth)acrylamid, N-tert.-Butyl-(meth-)acrylamid, Vinylester wie Vinylacetat, -propionat oder -butyrat, andere Vinylverbindungen wie Styrol, ferner Butadien. Besonders hervorgehoben seien Butylacrylat und 2-Ethylhexylacrylat. Die Polymeren können durch Zusatz geringer Mengen Comonomerer mit 2 oder mehr copolymerisierbaren Doppelbindungen, also beispielsweise von Diacrylaten, wie Butandioldiacrylat, oder Divinylverbindungen, wie Divinylbenzol, oder durch Zusatz anderer Vernetzungsmittel, z.B. Melamin-Formaldehydharze, vernetzt sein. Als klebrige Polymere können ferner Polyisobutylene und Polyvinylether unterschiedlichen Molekulargewichts verwendet werden.

Die Teilchengröße der Dispersionen soll zwischen 50 und 500 nm, insbesondere zwischen 50 und 200 nm liegen. Die Teilchengröße und der Vernetzungsgrad können in bekannter Weise in Abhängigkeit von den Polymerisationsbedingungen und den Comonomeren eingestellt werden. Kleinere Teilchengrößen und ein erhöhter Vernetzungsgrad können eine Steigerung der Wirkstofffreisetzung bewirken.

Matrix-Pflaster können in üblicher Weise hergestellt werden, indem man den Wirkstoff in einer geeigneten Polymerlösung löst oder fein dispergiert und anschließend diese wirkstoffhaltige Selbstklebemasse mittels Walzen- oder Rakelauftragsverfahren zum Film auszieht. In einigen Fällen ist es zweckmäßig, den Wirkstoff vor der Zugabe zu der Polymerlösung in einem organischen Lösungsmittel, z.B. Ethanol oder Aceton, aufzulösen oder feinst zu dispergieren. Dadurch kann eine bessere Verteilung des Wirkstoffes im Polymer erzielt werden.

Die Pflaster können auch nach der deutschen Patentanmeldung P 38 07 283.1 hergestellt werden, indem man den Wirkstoff in feinpulvriger Form (Teilchengröße unter 200, insbesondere unter 50 µm) in die wässrige Latexdispersion einarbeitet, oder in einer wässrigen Emulgatorlösung dispergiert oder löst und diese Mischung der wässrigen Latexdispersion bei einer Temperatur von 10 bis 80, insbesondere 30 bis 70 °C zumischt. Daneben kann auch das Salz eines Wirkstoffs in wässriger Lösung mit der Polymerdispersion bei einem pH-Wert gemischt werden, bei dem der Wirkstoff vorwiegend in der wasserlöslichen ionisierten Form vorliegt. Durch pH-Verschiebung wird dann der Wirkstoff in die ungeladene wasserunlösliche Form gebracht und simultan in die Dispersion einemulgiert.

Zweckmäßig legt man den Wirkstoff vor, gibt den Emulgator und Wasser zu und mischt dann mit der Polymerdispersion. Die so erhaltene wirkstoffhaltige Dispersion wird gegebenenfalls mit weiteren Hilfsstoffen versehen und, wie erwähnt, in an sich bekannter Weise auf einer Stützfolie zu einem Film ausgezogen und getrocknet. Die Trocknungstemperatur kann hierbei zwischen Raumtemperatur und 100°C liegen, wobei ein Optimum zwischen angestrebter schneller Trocknung und zu vermeidender Blasenbildung im Film sowie thermischer Belastung des Wirkstoffs im Allgemeinen bei 35 bis 45°C liegt.

Dieses Verfahren hat den großen Vorteil der Vermeidung von organischen Lösungsmitteln. Jedoch kommen im Prinzip auch alle anderen üblichen Herstellverfahren für Matrix-Pflaster in Betracht.

Die resultierenden Filme haben Dicken von 10 bis 800, bevorzugt 50 bis 300 µm. Die Filmherstellung kann kontinuierlich oder diskontinuierlich erfolgen. Das Auftrageverfahren kann mehrmals wiederholt werden, bis der Film die gewünschte Dicke erreicht hat. Die klebrige Polymerschicht enthält den Wirkstoff in einer Konzentration im Bereich von 1 bis 40, insbesondere 5 bis 25 Gew.-%. Die gleiche Konzentration gilt auch für die Reservoirflüssigkeit beim Membransystem und für die Wirkstofflösung oder - Dispersion, mit der beim Vliessystem das Vlies oder das poröse Polymere getränkt wird.

Als Emulgatoren sowohl für die Wirkstoffe wie auch die Polymeren werden die hierfür üblichen Tenside verwendet, wie das Natriumsalz von längerkettigen Fettsäuren und des Schwefelsäurehalbesters eines (gegebenenfalls oxethylierten) Fettalkohols als Beispiele für anionische Tenside sowie polyoxethylierte Alkylphenole und längerkettige Fettalkohole (z.B. Hexadecan-(l)-ol) und Glycerin-Fettsäurepartialester als Beispiele für nichtionische Tenside und Coemulgatoren.

Die gewünschte Viskosität der ausziehfertigen Masse kann z.B. mit Polyacrylsäuren oder Cellulosederivaten eingestellt werden.

Als zusätzliche Vernetzungsmittel die die Kohäsion und damit die Klebeeigenschaften der Filme verbessern, können z.B. Melamin-Formaldehydharze verwendet werden.

Im Sinne einer Verbesserung der Wirkstofffreisetzung wirken Quellstoffe wie Polyvinylpyrrolidon, Cellulosederivate oder Polyacrylate, da der Film vermehrt Wasser aufnehmen kann und dadurch der Diffusionswiderstand sinkt. Die Freisetzung der Wirkstoffe kann ferner verbessert werden durch den Zusatz von hydrophilen Weichmachern wie Glycerin, 1, 2-Propandiol der Polyethylenglykolen und lipophilen Weichmachern wie Triacetin, Dibutylphthalat oder Isopropylmyristat.

Matrixpflaster ergeben üblicherweise eine Wirkstofffreisetzung 1. Ordnung. Durch die Verwendung von Füllstoffen, die den Wirkstoff adsorbieren, wie Aerosil, mikrokristalline Cellulose oder Lactose, resultiert annähernd eine Freisetzung 0. Ordnung.

Die Stützfolie, auf die die wirkstoffhaltige Selbstklebemasse aufgetrocknet wird, ist zweckmäßig sowohl für den Wirkstoff wie für Wasserdampf praktisch undurchlässig. Sie kann z.B. aus einer Aluminium-Kunststoff-Verbundfolie, einer metallisierten Kunststofffolie, einer Kunststofffolie, die zur Wirkstoffseite hin mit einer Sperrschicht aus z.B. Polyvinylidenchlorid versehen ist, oder aus einer einfachen Kunststofffolie, z.B. Polyesterfolie, bestehen.

Die erfindungsgemäßen Pflaster, die nach dem Membransystem aufgebaut sind, werden ebenfalls in üblicher Weise hergestellt (z.B. EP 0 186 071 A2, US 4 262 003).

Die Herstellung der nach dem Vliessystem aufgebauten Pflaster erfolgt dadurch, dass auf der Stützfolie befestigte Vliese oder poröse Polymere mit einer Lösung oder Dispersion des Wirkstoffes in einem hydrophilen oder lipophilen Lösungsmittel oder Lösungsmittelgemisch getränkt werden. Anschließend wird die undurchlässige Abziehfolie aufgebracht.

### 4.5 Kühlende Nahrungsmittel

Erfindungsgemäße kühlende Nahrungsmittel können in (bei Umgebungstemperatur) fester, flüssiger, halbfester, pastöser, cremiger order geschäumter Form vorliegen. Sie enthalten neben herkömmlichen Nahrungsbestandteilen wenigstens eine wirksame (d.h. als kühlend wirkende) Menge wenigstens eines erfindungsgemäß anzuwendenden Wirkstoffs.

Typische Bestandteile sind dabei Fette, Kohlenhydrate, Proteine, Ballaststoffe, Wasser, Alkohol und dergleichen.

Der Proteinanteil kann z.B. 0 bis 50 Gew.-%, bezogen auf das Gesamtgewicht des Nahrungsmittels betragen;
der Fettanteil kann z.B. der 0 bis 50 Gew.-%, bezogen auf das Gesamtgewicht des Nahrungsmittels betragen;
der Kohlenhydratanteil kann z.B. 0 bis 90 Gew.-%, bezogen auf das Gesamtgewicht des Nahrungsmittels betragen;
der Ballaststoffanteil kann z.B. 0 bis 90 Gew.-%, bezogen auf das Gesamtgewicht des Nahrungsmittels betragen;
der Wasseranteil kann z.B. 0 bis 95 Gew.-%, bezogen auf das Gesamtgewicht des Nahrungsmittels betragen;
der Alkoholanteil kann z.B. 0 bis 15 Gew.-%, bezogen auf das Gesamtgewicht des Nahrungsmittels betragen;
der Anteil erfindungsgemäß anzuwendender Wirkstoffe kann z.B. im Bereich von 0,0001 bis 50, 0,001 bis 20, 0,005 bis 1, oder 0,01 bis 10, insbesondere 0,1 bis 10 oder 1 bis 5 Gew.-%, bezogen auf das Gesamtgewicht des Nahrungsmittels liegen.

Beispiele für Kohlehydrate sind z.B. Mono- und Disaccharide, Glukose, Galaktose, Mannose, Laktose, Maltose, und Saccharose; Fruktose und Mannose; Polysaccharide wie z. B. Stärken. Maltodextrine, Mehl.

Der Begriff "Ballaststoff" bezieht sich auf lösliche, unlösliche, fermentierbare, nicht fermentierbare oder eine beliebige Kombination solcher Ballaststoffe. Bei dem Ballaststoff kann es sich z. B. um Sojafasern, Pektin, bestimmte resistente Stärken, Oligofruktose, Inuline, Haferfasern, Erbsenfasern, Guargummi, Gum acazia, modifizierte Cellulose handeln.

Bei dem Fettbestandteil kann es sich um ein beliebiges Lipid oder Fett handeln, dessen Eignung zur Verwendung in Nährmitteln bekannt ist. Typische Fette sind unter anderem Milchfett, Distelöl, Canolaöl, Eidotterlipid, Olivenöl, Baumwollsamenöl, Kokosnussöl, Palmöl, Palmkernöl, Sojaöl, Sonnenblumenöl, Fischöl und Fraktionen aller vorstehenden Öle, die sich davon ableiten, wie Palmolein, mittelkettige Triglyceride (MCT), und Fettsäureester, wobei es sich bei den Fettsäuren z. B. um Arachidonsäure, Linolsäure, Palmitinsäure, Stearinsäure, Docosahexaensäure, Eicosapentaensäure, Linolensäure, Ölsäure, Laurinsäure, Caprinsäure, Caprylsäure, Capronsäure handelt. Hochölsäurehaltige Formen verschiedener Öle werden auch als geeignet für die vorliegende Verwendung erachtet, wie hochölsäurehaltiges Sonnenblumenöl und hochölsäurehaltiges Distelöl.

Bei dem Protein kann es sich um ein beliebiges Protein und/oder Aminosäuregemisch handeln, dessen Eignung zur Verwendung in Nährmitteln bekannt ist. Typische Proteine sind tierische Proteine, pflanzliche Proteine wie Sojaprotein, Milchprotein wie Magermilchprotein, Molkenprotein und Casein, und Aminosäuren (oder Salze davon) wie Isoleucin, Phenylalanin, Leucin, Lysin, Methionin, Threonin, Tryptophan, Arginin, Glutamin, Taurin, Valin. Bevorzugte Proteinquellen sind Molkenprotein, Natriumcaseinat oder Calciumcaseinat, das gegebenenfalls mit Aminosäuren versetzt ist. Für einige Anwendungen ist eine bevorzugte Proteinquelle hydrolysiertes Protein (Proteinhydrolysat) das gegebenenfalls mit Aminosäuren versetzt ist.

Bei dem Proteinhydrolysat kann es sich um ein beliebiges geeignetes Proteinhydrolysat handeln, das in einem Nährmittel verwendet wird, wie Sojaproteinhydrolysat, Caseinhydrolysat, Molkenproteinhydrolysat, andere tierische und pflanzliche Proteinhydrolysate und Gemische davon. Das Proteinhydrolysat der erfindungsgemäßen Zusammensetzung ist vorzugsweise ein Sojaprotein-, Molkenprotein- oder ein Caseinproteinhydrolysat, das kurze Peptide und Aminosäuren umfasst und gegebenenfalls mit zusätzlichen Aminosäuren versetzt ist. In einer bevorzugten Ausführungsform enthält das erfindungsgemäß geeignete Proteinhydrolysat einen hohen Anteil freier Aminosäuren (z. B. mehr als 40%) und niedermolekularer Peptidfragmente.

Das hydrolysierte Protein der erfindungsgemäßen Zusammensetzung ist auch vorzugsweise versetzt mit verschiedenen freien Aminosäuren, um einen ernährungsmäßig ausgewogenen Aminosäuregehalt bereitzustellen. Beispiele für solche freien Aminosäuren sind unter anderem L-Tryptophan, L-Methionin, L-Cystin, L-Tyrosin und L-Arginin.

Die erfindungsgemäßen Nährmittel enthalten gegebenenfalls auch Vitamine und Mineralien. Dem Fachmann ist geläufig, dass Minimalerfordernisse für bestimmte Vitamine und Mineralien aufgestellt worden sind, die für die normale physiologische Funktion notwendig sind. Der Fachmann weiß außerdem, dass den Nährmitteln angemessene zusätzliche Mengen von Vitamin- und Mineralbestandteilen zugesetzt werden müssen, um gewisse Verluste bei der Verarbeitung und Lagerung derartiger Zusammensetzungen zu kompensieren. Die erfindungsgemäße Zusammensetzung enthält gegebenenfalls ernährungsmäßig signifikante Mengen Vitamine und Mineralien.

Beispiele für Mineralien, Vitamine und andere Nährstoffe, die gegebenenfalls in der erfindungsgemäßen Zusammensetzung vorliegen, sind unter anderem Vitamin A, Vitamin B₆, Vitamin B₁₂, Vitamin E, Vitamin K, Vitamin C, Vitamin D, Inositol, Taurin, Folsäure, Thiamin, Riboflavin, Niacin, Biotin, Pantothensäure, Cholin, Calcium, Phosphor, lod, Eisen, Magnesium, Kupfer, Zink, Mangan, Chlorid, Kalium, Natrium, Beta-Carotin, Nucleotide, Selen, Chrom, Molybdän und L-Carnitin. Mineralien werden üblicherweise in Salzform zugesetzt.

Die erfindungsgemäße Zusammensetzung enthält gegebenenfalls auch üblicherweise Emulgatoren und/oder Stabilisatoren wie Lecithin (z. B. aus Ei oder Soja), modifiziertes Lecithin (z. B. enzymatisch oder acetyliert), Carrageenan, Xanthangummi, Mono- und Diglyceride, Guargummi, Carboxymethylcellulose, Stearoyllactylate, succinylierte Monoglyceride, Saccharoseester von Fettsäuren, Diacetylweinsäureester von Monoglyceriden, Polyglycerolester von Fettsäuren oder beliebige Gemische davon.

Die erfindungsgemäße Zusammensetzung enthält wahlweise ein oder mehrere natürliche oder künstliche Geschmacksträger zur Verbesserung der Schmackhaftigkeit. Man kann jeden auf dem Sektor verwendeten Geschmacksträger verwenden, wie Erdbeer, Kirsch, Schokolade, Orange, Kokosnuss, Vanille; Gewürze wie Muskat und Zimt; oder Citronensäure. In einigen Fällen, in denen natürliche Geschmacksträger, wie Kokosnussstücke, verwendet werden, trägt der Bestandteil zum Gesamtnährwertprofil der Zusammensetzung bei, d. h. er trägt zur Qualität und Quantität des Fett-, Protein- und/oder Kohlenhydratbestandteils bei.

Die erfindungsgemäße Zusammensetzung enthält wahlweise auch verschiedene andere Bestandteile, die zum Nährwertprofil der Zusammensetzung beitragen und/oder erwünschte Geschmackseigenschaften, wie Geschmacksverstärkung oder Mundgefühl, verleihen können. Derartige Bestandteile sind unter anderem Erdnüsse, Rosinen, Käsepulver, Essig, Salz, Natriumbicarbonat. Bei Riegeln wird die Zusammensetzung üblicherweise mit einem Schokoladen- oder einem aromatisierten Überzug (z. B. Schokolade, Vanille, Erdbeer usw.) versehen.

Die erfindungsgemäße Zusammensetzung enthält wahlweise auch natürliche oder künstliche Farbstoffe, um den ästhetischen Anreiz zu verbessern.

Die erfindungsgemäßen Zusammensetzungen können in mehreren physikalischen Erscheinungsformen vorliegen, z. B. als flüssige enterale Nährmittel oder Getränke für Erwachsene oder Kinder, in einer halbfesten Form wie Pudding, Creme, Mousse, oder einer festen Form, wie einem Nährmittelriegel oder Keks.

Die erfindungsgemäße Zusammensetzung kann nach bekannten Standardverfahren der Lebensmitteltechnik hergestellt werden, zum Beispiel nach analogen Verfahren zu den in folgenden Druckschriften beschriebenen: US-Patente 4,670,268; 4,497,800; 4,900,566; 5,104,677; 5,389,395; und 5,223,285; Chocolate, Cocoa and Confectionery: Science and Technology, 3. Auflage, Bernard W. Minifie, Van Nostrand Reinhold, New York, 1989, S. 502-506.

Bei Nährmittelriegeln und Keksen ist es üblicherweise angestrebt, die Zusammensetzung nach der physikalischen Formung zu backen.

Die erfindungsgemäße Zusammensetzung kann gewünschtenfalls nach bekannten Verfahren sterilisiert werden, zum Beispiel durch Wärmebehandlung wie Autoklavieren oder Sterilisieren oder Bestrahlung, oder mit sterilen Verfahren hergestellt und verpackt werden.

Die erfindungsgemäße Zusammensetzung kann in eine beliebige Art von Behälter oder Verpackung verpackt werden, deren Eignung zur Aufbewahrung von Lebensmitteln bekannt ist, wie Papier, Glas, beschichteter Karton, Kunststoff oder beschichtete Metalldosen.

Die erfindungsgemäße Zusammensetzung kann ernährungsmäßig ausgewogen sein. Unter dem Begriff "ernährungsmäßig ausgewogen" versteht man, dass die Zusammensetzung angemessene Nährstoffe enthält, um ein gesundes menschliches Leben über ausgedehnte Zeitspannen zu erhalten.

### 4.6. Textilprodukte, ausgerüstet mit erfindungsgemäß anzuwendenden Wirkstoffen.

Grundsätzlich kann der Wirkstoffgehalt über einen weiten Bereich, wie z.B. 0,00001 bis 50 Gew.-%, insbesondere 0,001 bis 10 Gew.-% oder 0,005 bis 1 Gew.-% variieren.

Die Ausrüstung von Textilien mit erfindungsgemäß anzuwendenden Wirkstoffen, ist in vielerlei Hinsicht von Interesse.

So findet die Ausrüstung von Textilien mit kühlend wirkenden Verbindungen insbesondere dort Verwendung wo Kleidungsstücke in direkten Kontakt mit der Haut gelangen können, so dass der Wirkstoff durch transdermale Übertragung seine Wirkungen, z.B. lokal oder systemisch, entfalten kann. Kürzlich wurde über Textilien berichtet, die mit sogenannten Wellness-Additiven, d.h. Substanzen, welche das Wohlbefinden fördern, ausgerüstet sind (R. Breier "Megatrend Wellness - Innovative Ideen für die Textilausrüstung", 31. Aachener Textiltage November 2004).

Eine insektizide Ausrüstung wiederum ist im Hinblick auf den Materialschutz, z.B. Ausrüstung des Textils gegen Mottenfraß etc., aber insbesondere auch zur Abwehr von parasitären Insekten, wie Mücken, von Interesse.

Grundlegendes Problem bei der Ausrüstung von Textilien mit Wirkstoffen ist die Anbindung des Wirkstoffs an den textilen Träger, die einerseits eine Permanenz der Ausrüstung gewährleisten muss und andererseits so gewählt werden muss, dass der Wirkstoff seine Wirkung nicht verliert. Hierzu werden im Stand der Technik verschiedene Ansätze vorgeschlagen.

So wurden z.B. Cyclodextrine zur Anbindung von Wirkstoffen an Textilien vorgeschlagen (siehe beispielsweise DE-A-19810951 und EP-A-0 392 608). Cyclodextrine sind cyclische Oligosaccharide, die durch enzymatischen Abbau von Stärke gebildet werden. Die häufigsten Cyclodextrine sind α-, β- und γ-Cyclodextrine, die aus sechs, sieben bzw. acht α-1,4-verknüpften Glucose-Einheiten bestehen. Eine charakteristische Eigenschaft der Cyclodextrin-Moleküle ist ihre Ringstruktur mit weitgehend unveränderlichen Abmessungen. Der Innendurchmesser der Ringe beträgt etwa 570 pm für α-Cyclodextrin, etwa 780 pm für β-Cyclodextrin und etwa 950 pm für γ-Cyclodextrin. Aufgrund ihrer Struktur sind Cyclodextrine in der Lage, Gastmoleküle, insbesondere hydrophobe Gastmoleküle, in wechselnden Mengen bis zur Sättigung einschließen zu können.

Die EP-A-1710345 beschreibt die Ausrüstung von Textilien mit Duftstoffen und anderen niedermolekularen organischen Wirkstoffen über eine amylosehaltige Substanz mit einem Amylosegehalt von wenigstens 30 % an das Textil gebunden sind.

Durch die Amyloseanteile der amylosehaltigen Substanz wird der Wirkstoff auf dem Textil gebunden und kontrolliert abgegeben, so dass die Wirkung über einen langen Zeitraum erhalten bleibt. Man nimmt an, dass der Wirkstoff ähnlich wie bei Cyclodextrinen in den durch die helikale Konformation der Amylose gebildeten Hohlräumen im Sinne einer Einschlussverbindung reversibel gebunden wird, wodurch einerseits eine Fixierung des Wirkstoffs auf der Oberfläche des textilen Trägers erreicht wird und andererseits eine kontrollierte Freisetzung möglich ist.

Für die erfindungsgemäße Ausrüstung von Textilien sind neben Amylose grundsätzlich alle Substanzen, insbesondere amylosehaltigen Stärken, d.h. native Stärken, modifizierte Stärken und Stärkederivate, geeignet, deren Amylosegehalt wenigstens 30 Gew.-% und insbesondere wenigstens 40 Gew.-% beträgt. Die Stärke kann nativ sein, z.B. Maisstärke, Weizenstärke, Kartoffelstärke, Sorghumstärke, Reisstärke oder Marantastärke, durch Teilaufschluss nativer Stärke gewonnen oder chemisch modifiziert sein. Geeignet ist auch reine Amylose als solche, z.B. enzymatisch gewonnene Amylose, z. B. aus Sucrose gewonnne Amylose. Geeignet sind auch Mischungen von Amylose und Stärke, sofern der Gesamtgehalt an Amylose wenigstens 30 Gew.-%, bezogen auf das Gesamtgewicht der Mischung beträgt. Es versteht sich, dass hier und im Folgenden alle Angaben in Gew.-%, welche sich auf Amylose oder amylosehaltige Substanzen beziehen, bei Mischungen aus Amylose und Stärke stets auf das Gesamtgewicht von Amylose + Stärke bezogen sind, sofern nicht ausdrücklich anderes angegeben ist.

Erfindungsgemäß besonders geeignet sind amylosehaltige Substanzen, insbesondere Amylose und amylosehaltige Stärken sowie Amylose/Stärkegemische, deren Amylosegehalt wenigstens 40 Gew.-% und insbesondere wenigstens 45 Gew.-%, bezogen auf das Gesamtgewicht der Substanz beträgt. In der Regel wird der Amylosegehalt 90 Gew.-% und insbesondere 80 Gew.-% nicht überschreiten. Derartige Substanzen sind bekannt und kommerziell erhältlich. Beispielsweise werden amylosehaltige Stärken von den Firmen Cerestar unter der Handelsmarke Amylogel® und National Starch unter den Handelsbezeichnungen HYLON® V und VII vertrieben.

Zur Erreichung der Anbindung des/der Wirkstoff(e) and das Textil, kann man das Textil mit der amylosehaltigen Substanz in der Regel in einer Menge von wenigstens 0,5 Gew.-%, vorzugsweise wenigstens 1 Gew.-% und insbesondere wenigstens 2 Gew.-%, jeweils bezogen auf das Gewicht des Textils, ausrüsten. In der Regel wird man die amylosehaltige Substanz in einer Menge von nicht mehr als 25 Gew.-%, häufig nicht mehr als 20 Gew.-% und insbesondere nicht mehr als 15 Gew.-%, bezogen auf das Gewicht des Textils, einsetzen um die taktilen Eigenschaften des Textils nicht nachteilig zu beeinflussen.

Zunächst wird das textile Material mit der amylosehaltigen Substanz als solcher ausgerüstet und anschließend das so ausgerüstete Textil mit einer geeigneten Aufbereitung des Wirkstoffs behandelt. Hierdurch wird die auf dem textilen Material befindliche amylosehaltige Substanz mit dem Wirkstoff beladen.

Man kann aber auch die amylosehaltige Substanz gemeinsam mit einem Wirkstoff einsetzen, um die Textilie auszurüsten. Hierbei können der Wirkstoff und die amylosehaltige Substanz sowohl als Mischung separater Komponenten als auch in der bereits vorgefertigten Form des Amylose-Wirkstoff-Komplexes angewandt werden.

In der Regel wird man den Wirkstoff in einer Menge einsetzen, die für den gewünschten Effekt ausreicht. Die Obergrenze wird durch die maximale Aufnahmekapazität der Amyloseeinheiten der eingesetzten amylosehaltigen Substanz bestimmt und wird in der Regel 20 Gew.-% und häufig 10 Gew.-%, bezogen auf den Amyloseanteil der Substanz, nicht überschreiten. Sofern erwünscht, setzt man den Wirkstoff in der Regel in einer Menge von 0,00001 bis 15 Gew.-%, 0,0001 bis 10 Gew.-%, 0,001 bis 5 Gew.-%, 0,005 bis 1 Gew.-% oder 0,1 bis 10 Gew.-% oder 0,5 bis 5 Gew.-%, bezogen auf den Amyloseanteil der amylosehaltigen Substanz ein.

Zur Textilausrüstung können auch Kombinationen erfindungsgemäß anzuwendender Wirkstoffen mit anderen an sich bekannten und zur Textilausrüstung geeigneten Wirkstoffen verwendet werden.

Als weitere Wirkstoffe sind grundsätzlich alle organischen Verbindungen und Mischungen organischer Verbindungen geeignet, die als Wirkstoffe bekannt sind und die in Lebewesen wie Menschen und Tieren, einschließlich Mikroorganismen, eine physiologische Wirkung induzieren. Zu nennen sind solche Wirkstoffe, die bekanntermaßen mit Cyclodextrinen Einschlussverbindungen bilden können. Besonders geeignet sind Wirkstoffe, die Kohlenwasserstoffgruppen und insbesondere aliphatische, cycloaliphatische und/oder aromatische Strukturen aufweisen. Das Molekulargewicht der Wirkstoffe liegt typischerweise unterhalb 1000 Dalton und häufig im Bereich von 100 bis 600 Dalton. Geeignet sind außerdem anorganische Verbindungen wie Wasserstoffperoxid, die bekanntermaßen in Cyclodextrinen gebunden werden können (siehe hierzu F. Vögtle, Supramolekulare Chemie, 2. Auflage, B. G. Teubner, Stuttgart 1992, Cyclodextrine und dort zitierte Literatur)

Zu den weiteren Wirkstoffen zählen insbesondere pharmazeutische Wirkstoffe und Wirkstoffe, die das Wohlbefinden von Lebewesen, insbesondere von Menschen fördern und die gemeinhin auch als "Wellness-Additive" bezeichnet werden. Anders als bei pharmazeutischen Wirkstoffen muss bei den Wellness-Additiven nicht zwingend eine therapeutische Wirkung gegeben sein. Vielmehr kann der das Wohlbefinden fördernde Effekt auf einer Vielzahl von Faktoren wie pflegenden, anregenden, kosmetischen oder sonstigen Wirkungen beruhen. Gleichermaßen geeignet sind organische Wirkstoffe, die gegen parasitäre Organismen wirken. Hierzu zählen beispielsweise Wirkstoffe, die gegen Pilze und/oder Mikroorganismen wirken, z.B. Fungizide und Bakterizide, oder die gegen tierische Schädlinge wie Schnecken, Würmer, Milben, Insekten und/oder Nager wirken, z.B. Nematizide, Molluskizide, Insektizide, Akarizide, Rodentizide und Repellent-Wirkstoffe, sowie weiterhin Wirkstoffe gegen Ungräser, d.h. Herbizide, oder Duftstoffe.

Bevorzugte pharmazeutische Wirkstoffe sind solche, die bekanntermaßen über die Haut resorbiert werden können. Hierzu zählen beispielsweise Ibuprofen, Flurbiprofen, Acetylsalicylsäure, Acetamidophen, Apomorphin, butyliertes Hydroxytoluol, Chamzulen, Gujazulen, Chlorthalidon, Cholecalciferol, Dicumarol, Digoxin, Diphenylhydantoin, Furosemid, Hydroflumethiazid, Indomethacin, Iproniazid-Phosphat, Nitroglycerin, Nicotin, Nicotinsäureamid, Oubain, Oxprenolol, Papaverin-Alkaloide wie Papaverin, Laudanosin, Ethaverin und Narcotin sowie Berberin, weiterhin Retionol, trans-Retinolsäure, Pretinol, Spironolacton, Sulpirid, Theophyllin, Theobromin, Corticosteroide und Derivate wie Testosteron, 17-Methyltestosteron, Cortison, Corticosteron, Dexamethason, Triamcinolon, Methylprednisolon, Fludrocortison, Fluocortolon, Prednison, Prednisolon, Progesteron, u.a. Estrogene und Gestagene wie Estradiol, Estriol, Ethinylestradiol-3-methylether, Norethisteron und Ethisteron, sowie Phenethylamin und Derivate wie Tyramin, Adrenalin, Noradrenalin und Dopamin.

Beispiele für erfindungsgemäß geeignete Wirkstoffe mit einer Wirkung gegen parasitäre Organismen sind die unter www.reith-pfister.de/w.list.html sowie unter www.hclrss.demon.co.uk/class pesticides.html genannten Nematizide, Bakterizide, Fungizide, Insektizide, Insekten-Repellents, Akarizide und Molluskizide.

Beispiele für bakterizide und fungizide Substanzen umfassen:
- Antibiotioka, z.B. Cycloheximid, Griseofulvin, Kasugamycin, Natamycin, Polyoxin, Streptomycin, Penizillin oder Gentamycin;
- Organische Verbindungen und Komplexe biozider Metalle, z.B. Komplexe des Silbers, Kupfers, Zinns und/oder Zinks wie Bis-(tributyllzinn)oxid, Kupfer-, Zink- und Zinn-Naphthenate, Oxine-Kupfer wie Cu-8, Tris-N-(cyclohexyldiazeniumdioxy)-aluminium, N-(Cyclohexyldiazeniumdioxy)-tributylzinn, Bis-N-(cyclohexyldiazeniumdioxy)-kupfer;;
- Quarternäre Ammoniumsalze, z.B. Benzyl-C₈-C₁₈-alkyldimethylammonium-halogenide, insbesondere Chloride (Benzalkoniumchloride);
- aliphatische Stickstofffungizide und Bactericide wie Cymoxanil, Dodin, Dodicin, Guazidine, Iminoctadin, Dodemorph, Fenpropimorph, Fenpropidin, Tridemorph,
- Substanzen mit Peroxidgruppen wie Wasserstoffperoxid, und organische Peroxide wie Dibenzoylperodid;
- Organische Chlorverbindungen wie z. B. Chlorhexidin;
- Triazolfungizide wie Azaconazol, Cyproconazol, Diclobutrazol, Difenoconazol, Diniconazol, Epoxiconazo, Fenbuconazol, Fluquinconazol, Flusilazol, Flutriafol, Hexaconazol, Metconazol, Propiconazol, Tetraconazol, Tebuconazol und Triticonazol;
- Strobilurine wie Dimoxystrobin, Fluoxastrobin, Kresoxim-methyl, Metominostrobin, Orysastrobin, Picoxystrobin, Pyraclostrobin und Trifloxystrobin
- Sulfonamide wie Tolylfluanid und Diclofluanid;
- lodverbindungen wie Diiodmethyl-p-tolylsulfon, Napcocide 3-lod-2-propinylalkohol, 4-Chlorphenyl-3-iodpropargylformal, 3-Brom-2,3-diiod-3-propenylethylcarbonat, 2,3,3-Triiodallylalkohol, 3-Iod-2-propinyl-n-hexylcarbamat, 3-Brom-2,3-diiod-2-propenylalkohol, 3-Iod-2-propinylphenyl-carbamat, 3-Iod-2-propinyl-n-butylcarbamat, O-1-(6-Iod-3-oxohex-5-inyl)phenylcarbamat, O-1-(6-Iod-3-oxohex-5-inyl)butylcarbamat;
- Isothiazolinone wie N-Methylisothiazolin-3-on, 5-Chloro-N-methylisothiazolin-3-on, 4,5-Dichloror-N-octylisothiazolin-3-on, 1,2-Benzisothiazol-3(2H)on, 4,5-Trimethylisothiazol-3-on und N-Octyl-isothiazolin-3-on.

Beispiele für Insektizide und Acarizide sind
- Organophosphate wie Acephate, Azamethiphos, Azinphos-methyl, Chlorpyrifos, Chlorpyriphos-methyl, Chlorfenvinphos, Diazinon, Dichlorvos, Dicrotophos, Dimethoate, Disulfoton, Ethion, Fenitrothion, Fenthion, Isoxathion, Malathion, Methamidophos, Methidathion, Methyl-Parathion, Mevinphos, Monocrotophos, Oxydemeton-methyl, Paraoxon, Parathion, Phenthoate, Phosalone, Phosmet, Phosphamidon, Phorate, Phoxim, Pirimiphos-methyl, Profenofos, Prothiofos, Sulprophos, Triazophos, Trichlorfon;
- insbesondere Pyrethroide wie Acrinatrin, Allethrin, Bioallethrin, Barthrin, Bifenthrin, Bioethanomethrin, Cyclethrin, Cycloprothrin, Cyfluthrin, beta-Cyfluthrin, Cyhalothrin, gamma-Cyhalothrin, lambda-Cyhalothrin, Cypermethrin, α-Cypermethrin, β-Cypermethrin, λ-Cypermethrin, zeta-Cypermethrin, Cyphenothrin, Deltamethrin, Dimefluthrin, Dimethrin, Empenthrin, Fenfluthrin, Fenprithrin, Fenpropathrin, Fenvalerat, Esfenvalerat, Flucythrinat, Fluvinate, tau-Fluvinate, Furethrin, Permethrin, Biopermethrin, Trans-Permethrin, Phenothrin, Prallethrin, Profluthrin, Pyresmethrin, Resmethrin, Bioresmethrin, Cismethrin, Tefluthrin, Terallethrin, Tetramethrin, Tralomethrin, Transfluthrin, Etofenprox, Flufenprox, Halfenprox, Protrifenbute und Silafulfen.
- Pyrrol- und Pyrazol-Insektizide wie Acetoprole, Ethiprole, Fipronil, Tebufenpyrad, Tolfenpyrad, Chlorfenapyr und Vaniliprole.

Beispiele für Repellent-Wirkstoffe sind insbesondere Anthrachinon, Acridinbasen, Kupfernaphthenat, Butopyronoxyl, Dibutylphthalat, Dimethylphthalat, Dimethylcarbat, Ethohexadiol, Hexamide, Methoquin-Butyl, N-Methylneodecanamid, Kampfer, Bergamotte-Öl, Pyrethrum, Nelkenöl, Geraniumöl, Thymianöl und insbesondere Diethyl-m-toluamid und 1-Piperidincarboxylsäure-2-(2-hydroxyethyl)-1-methylpropylester (Picardin).

Beispiele für Wellness-Additive sind insbesondere die nachfolgend aufgeführten Substanzen und Substanzgemische, z.B.
- Fette, vorzugsweise pflanzlichen Ursprungs, z.B. Lecithine,
- Pflanzenöle wie Jojoba-Öl, Teebaumöl, Nelkenöl, Nachtkerzenöl, Mandelöl, Kokosöl, Avocadoöl, Sojaöl und dergleichen,
- Fettsäuren, z.B. ω-6-Fettsäuren, Linolensäure, Linolsäure,
- Wachse tierischen oder planzlichen Ursprungs wie Bienenwachs, Candelillawachs, Sheabutter, Shoreabutter, Mangokernbutter, Japanwachs und dergleichen,
- Vitamine, insbesondere fettlösliche Vitamine, z. B. Tocopherole, Vitamin E, Vitamin A und dergleichen,
- Cortico-Steroide wie Cortison, Corticosteron, Dexamethason, Triamcinolon, Methylprednisolon, Fludrocortison, Fluocortolon, Prednison, Prednisolon, Progesteron,
- Aminosäuren, z.B. Arginin, Methionin,
- Pflanzenextrakte wie Algenextrakt, Rosskastanienextrakt, Mangoextrakt und dergleichen.

Zur Verbesserung der Waschpermanenz der erfindungsgemäßen Ausrüstung hat es sich bewährt, wenn man die amylosehaltige Substanz mit einem Bindemittel auf dem Textil fixiert. Als Bindemittel kommen zum einen filmbildende, wasserunlösliche Polymere und zum anderen niedermolekulare reaktive Substanzen, die unter Erwärmen polymerisieren, in Betracht. In der Regel wird man das Bindemittel in einer Menge einsetzten, dass das Gewichtsverhältnis von amylosehaltiger Substanz zu wasserunlöslichem Polymer im Bereich von 1:1 bis 100:1, vorzugsweise im Bereich von 1,5:1 bis 50:1 und insbesondere im Bereich von 2:1 bis 20:1 liegt.

In der Regel werden die filmbildenden Polymere in Form einer wässrigen Dispersion von feinteiligen Polymerteilchen eingesetzt. Die Teilchengröße ist für den erfindungsgemäßen Erfolg von untergeordneter Bedeutung. Sie liegt jedoch in der Regel unterhalb 5 µm (Gewichtsmittel) und beträgt in der Regel 50 nm bis 2 µm.

Das filmbildende Polymer kann insbesondere eine Glasübergangstemperatur T_{G} im Bereich von -40 bis 100 °C, vorzugsweise -30 bis +60 °C, insbesondere -20 bis +40 °C, aufweist. Sofern das polymere Bindemittel mehrere Polymerkomponenten umfasst sollte zumindest der Hauptbestandteil eine Glasübergangstemperatur in diesem Bereich aufweisen. Insbesondere liegt die Glasübergangstemperatur des Hauptbestandteils im Bereich von -30 °C bis +60 °C und besonders bevorzugt im Bereich von -20 °C bis +40 °C. Vorzugsweise weisen alle polymeren Bestandteile eine Glasübergangstemperatur in diesen Bereichen auf. Die angegebenen Glasübergangstemperaturen beziehen sich dabei auf die gemäß ASTM-D 3418-82 mittels DSC bestimmte "midpoint temperature". Im Falle vernetzbare Bindemittel bezieht sich die Glasübergangstemperatur auf den unvernetzten Zustand.

Beispiele für geeignete filmbildende Polymere basieren auf den folgenden Polymerklassen:
(1) Polyurethan-Harze
(2) Acrylatharze (Reinacrylate: Copolymere aus Alkylacrylaten und Alkylmethacrylaten);
(3) Styrolacrylate (Copolymere aus Styrol und Alkylacrylaten);
(4) Styrol/Butadien-Copolymerisate;
(5) Polyvinylester, insbesondere Polyvinylacetate und Copolymere des Vinylacetats mit Vinylpropionat;
(6) Vinylester-Olefin-Copolymere, z. B. Vinylacetat/Ethylen-Copolymere;
(7) Vinylester-Acrylat-Copolymere, z. B. Vinylacetat/Alkylacrylat-Copolymere sowie Vinylacetat/Alkylacrylat/Ethylen-Terpolymere;

Derartige Polymere sind bekannt und im Handel erhältlich, z. B. Polymere der Klassen (2) bis (7) in Form wässriger Dispersionen unter den Bezeichnungen ACRONAL, STY-ROFAN, BUTOFAN (BASF-AG), MOWILITH, MOWIPLUS, APPRETAN (Clariant), VINNAPAS, VINNOL (WACKER). Wässrige, für das erfindungsgemäße Verfahren geeignete Polyurethan-Dispersionen (1) sind insbesondere solche, die für die Beschichtung von Textilien verwendet werden (siehe z. B. J. Hemmrich, Int. Text. Bull. 39, 1993, Nr.2, S. 53-56; "Wässrige Polyurethan-Beschichtungssysteme" Chemiefasern/Textilind. 39 91 (1989) T149, T150; W. Schröer, Textilveredelung 22, 1987, S. 459-467). Wässrige Polyurethandispersionen sind im Handel erhältlich, z. B. unter den Handelsbezeichnungen Alberdingk® der Fa. Alberdingk, Impranil® der Fa. BAYER AG, Permutex® der Fa. Stahl, Waalwijk, Niederlande, der Fa. BASF SE oder können nach bekannten Verfahren hergestellt werden, wie sie beispielsweise in "Herstellverfahren für Polyurethane" in Houben-Weyl, "Methoden der organischen Chemie", Band E 20/Makromolekulare Stoffe, S. 1587, D. Dietrich et al., Angew. Chem. 82 (1970), S. 53 ff., Angew. Makrom. Chem. 76, 1972, 85 ff. und Angew. Makrom. Chem. 98, 1981, 133-165, Progress in Organic Coatings, 9, 1981, S. 281-240, bzw. Römpp Chemielexikon, 9. Auflage, Band 5, S. 3575 beschrieben werden.

Die filmbildenden Polymere können selbstvernetzend sein, d. h. die Polymere weisen funktionelle Gruppen (vernetzbare Gruppen) auf, die beim Trocknen der Zusammensetzung, gegebenenfalls beim Erwärmen, miteinander, mit den funktionellen Gruppen der Amylose oder mit einem niedermolekularen Vernetzer unter Bindungsbildung reagieren.

Beispiele für vernetzbare funktionelle Gruppen umfassen aliphatisch gebundene OH-Gruppen, NH-CH₂-OH-Gruppen, Carboxylat-Gruppen, Anhydrid-Gruppen, verkappte Isocyanatgruppen und Aminogruppen. Häufig wird man ein Polymer verwenden, dass noch freie OH-Gruppen als reaktive Gruppen aufweist. In der Regel beträgt der Anteil der reaktiven funktionellen Gruppen 0,1 bis 3 mol/kg Polymer. Die Vernetzung kann innerhalb des Polymers durch Reaktion komplementär-reaktiver funktioneller Gruppen bewirkt werden. Vorzugsweise bewirkt man die Vernetzung des Polymers durch Zugabe eines Vernetzers, der reaktive Gruppen aufweist, welche hinsichtlich ihrer Reaktivität zu den funktionellen Gruppen des Vernetzers komplementär sind. Geeignete Paare funktioneller Gruppen, die eine komplementäre Reaktivität aufweisen sind dem Fachmann bekannt. Beispiele für solche Paare sind OH/COOH, OH/NCO, NH₂/COOH, NH₂/NCO sowie M²⁺/COOH, wobei M²⁺ für ein zweiwertiges Metallion wie Zn²⁺, Ca²⁺, oder Mg²⁺ steht. Beispiele für geeignete Vernetzer sind die nachstehend bei den Polyurethanen genannten Di- oder Polyole; primäre oder sekundäre Diamine, vorzugsweise primäre Diamine, z. B. Alkylendiamine wie Hexamethylendiamin, Diethylentriamin, Triethylentetramin, Tetraethylenpentamin, N,N-Bis[(aminopropyl)amino]-ethan, 3,6-Dioxaoctandiamin, 3,7-Dioxanonandiamin, 3,6,9-Trioxaundecandiamin oder Jeffamine, (4,4'-Diaminodicyclohexyl)methan, (4,4'-Diamino-3,3-dimethyldicyclohexyl)methan; Aminoalkohole wie Ethanolamin, Hydroxypropylamin; ethoxylierte Di- und Oligoamine; Dihydrazide von aliphatischen oder aromatischen Dicarbonsäuren wie Adipinsäuredihydrazid; Dialdehyde wie Glyoxal; teilweise oder vollständig O-methylierte Melamine, sowie Verbindungen oder Oligomere, die im Mittel zwei oder mehr, vorzugsweise drei oder mehr Isocyanatgruppen oder reversibel z. B. Hydrogensulfit blockierte Isocyanatgruppen aufweisen. In diesem Fall wird das Mengenverhältnis von Vernetzer zu polymerem Bindemittel so bemessen, dass das Molverhältnis der reaktiven Gruppen im polymeren Bindemittel (Gesamtmenge der reaktiven Gruppen in den Polymeren) zu den reaktiven Gruppen im Vernetzer in der Regel im Bereich von 1:10 bis 10:1 und vorzugsweise im Bereich von 3:1 bis 1:3 liegt. Üblicherweise liegt das Gewichtsverhältnis von polymerem Bindemittel (gerechnet als Feststoff) zu Vernetzer im Bereich von 100:1 bis 1:1 und insbesondere im Bereich von 50:1 bis 5:1.

Alternativ zur Fixierung der amylosehaltigen Substanz mit wasserunlöslichen Polymeren kann man die Amylose bzw. die amylosehaltigen Substanz auch mit reaktiven Verbindungen, die wenigstens eine, gegenüber den OH-Gruppen der Amylose reaktive Gruppe und wenigstens eine weitere funktionelle Gruppe aufweisen, die gegenüber den funktionellen Gruppen auf den Fasern des textilen Materials, z.B. OH-Gruppen, NH₂-Gruppen oder COOH-Gruppen, reaktiv ist, auf dem textilen Material fixieren. Zu den reaktiven Verbindungen zählen die oben genannten Vernetzer sowie die in DE-A 40 35 378 für die Fixierung von Cyclodextrinen vorgeschlagenen Substanzen, z.B. N-Hydroxmethyl- und N-Alkoxymethylderivate von Harnstoff oder harnstoffartigen Verbindungen wie Dimethylolharnstoff (Bis(hydroxymethyl)harnstoff), Di(methoxymethyl)-harnstoff, Dimethylolalkandioldiurethane wie N,N-Dimethylolethylenharnstoff (N,N-Bis(hydroxymethyl)imidazolin-2-on), N,N-Dimethylol-dihydroxyethylenharnstoff (N,N-Bis(hydroxymethyl)-4,5-dihydroxyimidazolin-2-on), Dimethylolpropylenharnstoff und dergleichen. Derartige Materialien sind in Form wässrige Formulierungen für die Ausrüstung von Textilien im Handel, z.B. unter den Handelsbezeichnungen Fixapret® und Fixapret®-eco der BASF SE. Zu den reaktiven Materialien, die zur Fixierung der amylosehaltigen Substanz auf dem textilen Material genutzt werden können, zählen insbesondere auch Verbindungen mit 2, 3, 4 oder mehr (gegebenenfalls reversibel blockierten) Isocyanat-Gruppen, speziell die mit Bisulfit oder CH-aciden Verbindungen oder Oximene, z.B. Butanonoxim reversibel blockierten Polyisocyanat-Präpolymere auf Basis von Polyether- und Polyesterurethanen, die in DE 2837851, DE 19919816 und der älteren Europäischen Patentanmeldung 03015121 beschrieben werden. Derartige Produkte sind auch kommerziell erhältlich, beispielsweise unter den Handelsbezeichnungen PROTOLAN®367 und PROTOLAN®357 der Rotta GmbH, Mannheim.

Zur Fixierung der amylosehaltigen Substanz kann auch die für die Fixierung von Cyclodextrinen bekannte Vorgehensweise in analoger Weise genutzt werden, bei der man das Cyclodextrin bzw. im vorliegenden Fall die amylosehaltige Substanz mit Reaktivankern versieht, beispielsweise indem man sie mit Dicarbonsäuren oder Dicarbonsäureanhydriden wie Maleinsäure, Fumarsäure, Maleinsäureanhydrid, Bernsteinsäure, Bernsteinsäureanhydrid oder Adipinsäure, mit Diisocyanaten, z. B. Toluoldiisocyanat, Isophorondiisocyanat, Tetramethylendiisocyanat oder Hexamethylendiisocyanat, oder mit Aminocarbonsäuren in an sich bekannter Weise dergestalt umsetzt, dass nur eine der in diesen Verbindungen vorhandenen Funktionalitäten mit den OH-Gruppen der amylosehaltigen Substanz reagiert und die andere zur Anbindung an die reaktiven Gruppen des Fasermaterials erhalten bleibt. Reaktivanker lassen sich auf der amylosehaltigen Substanz auch durch Umsetzung mit 1,3,5-Trichlortriazin, 2,3-Dichlorchinoxalin-5,6-carbonsäurechlorid sowie mit Chlordifluorpyrimidin erzeugen.

Ferner können zur Fixierung der Amylose auch Alkoxysilane, wie Diethoxydimethylsilan, Dimethoxydimethylsilan, Triethoxyphenylsilan, Tetraethoxysilan sowie dimere, trimere und höhere Kondensationsprodukte dieser Verbindungen eingesetzt werden.

Auf diese Weise können grundsätzlich alle textilen Materialien ausgerüstet werden, d.h. nicht konfektionierte Ware als auch konfektionierte Ware. Textile Materialien umfassen hier und im Folgenden Gewebe, Gestricke, Gewirke und Vliese. Die textilen Materialen können aus Naturfasergarnen, Synthesefasergarnen und/oder Mischgarnen aufgebaut sein. Als Fasermaterialien kommen grundsätzlich alle für die Herstellung von Textilien üblicherweise eingesetzten Fasermaterialien in Betracht. Hierzu zählen Baumwolle, Wolle, Hanffaser, Sisalfasern, Flachs, Ramie, Polyacrylnitrilfasern, Polyesterfasern, Polyamidfasern, Viskosefasern, Seide, Acetatfasern, Triacetatfasern, Aramidfasern und dergleichen sowie Mischungen dieser Fasermaterialien.

Das Ausrüsten bzw. Behandeln der textilen Materialien mit der amylosehaltigen Substanz kann in an sich bekannter Weise, z.B. mittels der in DE-A 4035378 für die Ausrüstung von Textilien mit Cyclodextrinen beschriebenen Verfahren erfolgen.

Zu nennen sind beispielsweise Verfahren, bei denen die amylosehaltige Substanz, gegebenenfalls als Komplex mit dem Wirkstoff bereits in die Faser, das Filament und/oder das Garn eingesponnen ist, aus dem das Gewebe hergestellt ist.

Häufig wird man jedoch das textile Material vor oder nach Konfektionierung mit der amylosehaltigen Substanz oder einem Komplex aus amylosehaltiger Substanz und Wirkstoff behandeln. In der Regel wird man hierzu das Textil mit einer wässrigen Flotte behandeln, welche die amylosehaltige Substanz und gegebenenfalls den Wirkstoff in ausreichender Menge enthält. Je nach Art des Auftrags und der gewünschten Menge, in der die amylosehaltiger Substanz aufgebracht werden soll, liegt die Konzentration an amylosehaltiger Substanz in der Flotte im Bereich von 1 bis 40 Gew.-%, insbesondere im Bereich von 2 bis 20 Gew.-% und speziell im Bereich von 4 bis 15 Gew.-%.

Die Art der Behandlung ist von untergeordneter Bedeutung und kann beispielsweise als Minimalauftrag, z.B. durch Sprühauftrag, als Normalauftrag im Foulard oder als Hochfeuchteauftrag erfolgen. Hierbei wird das textile Material mit der wässrigen Flotte getränkt. Gegebenenfalls kann man danach überschüssige Flotte entfernen, z.B. durch Abquetschen auf eine Flottenaufnahme von 30 bis 120 %.

Eine andere Möglichkeit zur Behandlung des Textils mit amylosehaltiger Substanz bzw. Komplex aus amylosehaltiger Substanz und Wirkstoff, ist eine Flotte mit Wasser anzusetzen, in der die gewünschten Menge an amylosehaltiger Substanz und gegebenenfalls Wirkstoff enthalten ist, z.B. 0,5 bis 20 Gew.-% (bezogen auf die Masse der auszurüstenden Textilie). Das textile Material wird über einen gewissen Zeitraum, z.B. 10-60 min mit der Behandlungsflotte in hierfür geeigneten Ausrüstungsaggregaten (z.B. Haspelkufe; Rollenkufe; Paddel; etc.) durchtränkt und danach wie oben angegeben abgequetscht und/oder abgeschleudert. Das Flottenverhältnis liegt hierbei in der Regel im Bereich von 1:2 bis 1:50 und insbesondere im Bereich von 1:3 bis 1:20.

Derartige Verfahren sind dem Fachmann bekannt, beispielsweise aus H.K Rouette, Lexikon der Textilveredlung, Laumann-Verlag, Dülmen 1995, S. 669 ff.

In der Regel schließt sich an die Behandlung mit der Flotte ein Trocknungsvorgang an. Die Temperaturen liegen dabei in der Regel im Bereich von 100 bis 200°C und vorzugsweise im Bereich von 120 bis 180°C. Das Trocknen kann in den hierfür üblichen Vorrichtungen durchführen, bei konfektionierter Ware beispielsweise durch trockentumblen bei den oben angegebenen Temperaturen. Bei nicht-konfektionierter Ware wird man in der Regel im Anschluss an den Auftrag das textile Material über ein oder mehrere Spannrahmen führen.

Sofern die amylosehaltige Substanz zusammen mit einem filmbildenden Polymer eingesetzt wird, führt das Trocknen zu einer Fixierung der amylosehaltigen Substanz auf den Textilfasern. In der Regel wird dann die Trocknungstemperatur 100 nicht unterschreiten und liegt vorzugsweise im Bereich von 120 bis 200°C und insbesondere im Bereich von 140 bis 180°C. Im Allgemeinen erfolgt das Trocknen während einer Zeitdauer von 1 bis 10 min, insbesondere 1 bis 2 min, wobei längere Trockenzeiten ebenfalls geeignet sind.

Für das Behandeln mit einer wässrigen Flotte hat es sich als vorteilhaft erwiesen, wenn die wässrige Flotte neben der amylosehaltigen Substanz und gegebenenfalls dem Wirkstoff wenigstens eine oberflächenaktive Substanz (bzw. grenzflächenaktive Substanz) enthält, welche zur Dispergierung der amylosehaltigen Substanz und dem Wirkstoff in der wässrigen Flotte geeignet ist. Vorzugsweise handelt es sich bei der oberflächenaktiven Substanz um ein oligomeres oder polymeres Dispergiermittel. Der Begriff oligomeres oder polymeres Dispergiermittel umfasst im Unterschied zu niedermolekularen oberflächenaktiven Substanzen solche Dispergiermittel, deren zahlenmittleres Molekulargewicht in der Regel wenigstens 2000 Dalton beträgt, z.B. 2000 bis 100000 Dalton und insbesondere im Bereich von 3000 bis 70000 Dalton liegt.

In der Regel enthält die wässrige Flotte das polymere oder oligomere Dispergiermittel in einer Menge von 0,5 bis 20 Gew.-%, vorzugsweise 1 bis 18 Gew.-% und insbesondere 5 bis 15 Gew.-%, bezogen auf die amylosehaltige Substanz.

Geeignete oligomeres oder polymeres Dispergiermittel sind in Wasser löslich und umfassen sowohl neutrale und amphotere wasserlösliche Polymere als auch kationische und anionische Polymere, wobei letztere bevorzugt sind.

Beispiele für neutrale polymere Dispergiermittel sind Polyethylenoxid, Ethylenoxid/ Propylenoxid-Copolymere, bevorzugt Blockcopolymere, Polyvinylpyrrolidon sowie Copolymere von Vinylacetat mit Vinylpyrrolidon.

Die bevorzugten anionischen oligomeren bzw. polymeren Dispergiermittel zeichnen sich dadurch aus, dass sie Carboxylgruppen und/oder Sulfonsäuregruppen aufweisen und üblicherweise als Salze, z.B. als Alkalimetallsalze oder Ammoniumsalze eingesetzt werden.

Bevorzugte anionische Dispergiermittel sind beispielsweise carboxylierte Derivate der Zellulose wie Carboxymethylcellulose, Homopolymere ethylenisch ungesättigter C₃-C₈-Mono- und C₄-C₈-Dicarbonsäuren, z.B. der Acrylsäure, der Methacrylsäure, der Maleinsäure, der Itaconsäure, Copolymere wenigstens zweier verschiedener ethylenisch ungesättigter C₃-C₈-Mono- und C₄-C₈-Dicarbonsäuren wie vorstehend genannt, und Copolymere wenigstens einer der vorgenannten ethylenisch ungesättigten C₃-C₈-Mono- oder C₄-C₈-Dicarbonsäure mit wenigstens einem neutralen Comonomeren. Beispiele für neutrale Comonomere sind N-Vinyllactame wie N-Vinylpyrrolidon, Vinylester aliphatischer C₂-C₁₆-Carbonsäuren wie Vinylacetat, Vinylpropionat, Amide der vorgenannten ethylenisch ungesättigten Carbonsäuren, wie Acrylamid, Methacrylamid und dergleichen, Hydroxy-C₁-C₄-alkyl(meth)acrylate wie Hydroxyethylacrylat und -methacrylat, Ester ethylenisch ungesättigter C₃-C₈-Mono- oder C₄-C₈-Dicarbonsäuren mit Polyethern, z.B. Ester der Acrylsäure oder der Methacrylsäure mit Polyethylenoxiden oder Ethylenoxid/Propylenoxid-Blockcopolymeren, Vinylaromaten wie Styrol und C₂-C₁₆-Olefine wie Ethylen, Propen, 1-Hexen, 1-Octen, 1-Decen, 1-Dodecen und dergleichen. Weiterhin bevorzugt sind Homopolymere ethylenisch ungesättigter Sulfonsäuren wie Styrolsulfonsäure und Acrylamidopropansulfonsäure und deren Copolymer mit den vorgenannten Comonomeren. In den Copolymeren wird der Anteil der ethylenisch ungesättigten Säure in der Regel wenigstens 20 Gew.-% betragen und einen Wert von 90 Gew.-% und insbesondere 80 Gew.-%, jeweils bezogen auf das Gesamtgewicht aller das Polymer konstituierenden Monomere, nicht überschreiten.

Copolymerisate aus mindestens einer der oben genannten Säuren und mindestens einem Comonomer sind für diesen Zweck bekannt und im Handel erhältlich, beispielsweise die Copolymere von Acrylsäure und Maleinsäure als Sokalan-Marken der BASF-AG.

Ebenfalls bevorzugte anionische Dispergiermittel sind Phenolsulfonsäure-Formaldehyd-Kondensate und Naphthalinsulfonsäure-Formaldehyd-Kondensate (z.B. die Tamol- und Setamol-Marken der BASF) und Ligninsulfonate.

Brauchbare Dispergiermittel sind außerdem niedermolekulare anionische, nicht-ionische, kationische, ampholytische und zwitterionische Tenside. Geeignete Tenside sind z.B. die Alkalimetall-, Ammonium- oder Aminsalze von C₈-C₁₈-Alkylsulfaten, wie Natriumlaurylsulfat; C₈-C₁₈-Alkylsulfonaten, wie Dodecylsulfonat; C₈-C₁₈-Alkylethersulfaten; sowie C₈-C₁₈-Alkylethoxylate; Polyoxyethylensorbitanester; C₈-C₁₈-Alkylglycinate; C₈-C₁₈-Alkyldimethylaminoxide; Betaine etc. Bevorzugt sind die Alkylsulfate und Alkylsulfonate.

Sofern die amylosehaltige Substanz nicht gemeinsam mit einem filmbildenden, wasserunlöslichen Polymer eingesetzt wird, kann das Textil in einem separaten Arbeitsschritt mit dem Polymer behandelt werden. Insbesondere erfolgt die Behandlung gemeinsam mit der amylosehaltigen Substanz. Dementsprechend betrifft eine besondere Ausführungsform ein Verfahren, bei dem die wässrige Flotte zusätzlich ein dispergiertes, filmbildendes, wasserunlösliches Polymer der oben bezeichneten Art umfasst. Die Menge an filmbildenden Polymer ist dabei so gewählt, dass das Gewichtsverhältnis von amylosehaltiger Substanz zu wasserunlöslichem Polymer im Bereich von 1:1 bis 100:1, vorzugsweise im Bereich von 1,5:1 bis 50:1 und insbesondere im Bereich von 2:1 bis 20:1 liegt.

Die Ausrüstung des Textils mit dem Wirkstoff kann in einem separaten Vorgang oder in einem Arbeitsgang zusammen mit der Ausrüstung mit der amylosehaltigen Substanz erfolgen.

Sofern man das Textil in einem separaten Arbeitsgang mit dem Wirkstoff ausrüstet, wird man das Textil zweckmäßigerweise ebenfalls mit einer wässrigen Flotte des Wirkstoffs behandeln. Hierzu wird man in der Regel den Wirkstoff, der üblicherweise in Wasser nicht löslich ist, in Wasser emulgieren oder dispergieren, gegebenenfalls unter Verwendung geeigneter oberflächenaktiver Substanzen. Geeignete oberflächenaktive Substanzen sind insbesondere die zuvor erwähnten niedermolekularen Tenside und hierunter bevorzugt die nichtionischen Tenside, insbesondere Polyoxyethylensorbitanester, Ester von Mono- oder Oligosacchariden mit C₆-C₁₈-Fettsäuren und besondere bevorzugt C₈-C₁₈-Alkylethoxylate, insbesondere solche mit einem Ethoxylierungsgrad im Bereich von 6 bis 50. In der Regel enthält die wässrige Flotte den Wirkstoff in einer Menge von 0,1 bis 10 Gew.-% und insbesondere in einer Menge von 0,2 bis 5 Gew.-%. Die Menge an oberflächenaktiver Substanz liegt in der Regel im Bereich von 0,5 bis 50 Gew.-% und insbesondere im Bereich von 3 bis 30 Gew.-%, bezogen auf den Wirkstoff. Das Aufbringen des Wirkstoffs aus wässriger Flotte kann mit den hierfür üblichen Methoden erfolgen, z.B. mittels eines Foulards.

Man kann aber auch die Ausrüstung mit Wirkstoff und amylosehaltiger Substanz in einem Arbeitsgang durchführen. Dabei kann man grundsätzlich wie für die Ausrüstung mit der amylosehaltigen Substanz beschrieben vorgehen, wobei die wässrige Flotte der amylosehaltigen Substanz nunmehr zusätzlich den wenigstens einen Wirkstoff enthält. Der Wirkstoff kann dabei separat der Flotte zugegeben werden oder in Form einer Einschlussverbindung, d.h. in Form eines Wirt-Gast-Komplexes mit der amylosehaltigen Substanz.

Das erfindungsgemäße Verfahren kann zur Ausrüstung beliebiger Textilien, einschließlich Gewebe, Gewirke, Vliese und dergleichen eingesetzt werden. Die Art des textilen Materials richtet sich in erster Linie nach dem gewünschten Anwendungszweck.

Bei den auszurüstenden Textilien kann es sich um fertig konfektionierte Produkte wie Bekleidung, einschließlich Unterwäsche und Oberbekleidung, wie z.B. Hemden, Hosen, Jacken, Outdoor-, Trecking- und Militärausrüstungen, Dächer, Zelte, Netze, z.B. Insektenschutznetze und Vorhänge, Hand- und Badetücher, Bettwäsche und dergleichen handeln.

In gleicher Weise kann die Ausrüstung auf der Rohware in Ballen- oder Rollenform erfolgen.

Die mit Wirkstoffen gegen parasitäre Organismen wie Insekten und Acariden ausgerüsteten Textilien sind neben dem Schutz des Menschen auch besonders im Tierschutz zum Schutz gegen Zecken, Milben, Flöhe und dergleichen geeignet.

Die textilen Materialen können aus Naturfasergarnen, Synthesefasergarnen und/oder Mischgarnen aufgebaut sein, wobei die Gewebe üblicherweise ein Flächengewicht im Bereich von 10 bis 500 g/m² vorzugsweise 20 bis 250 g/m² aufweisen. Als Fasermaterialien kommen grundsätzlich alle für die Herstellung von Textilien üblicherweise eingesetzten Fasermaterialien in Betracht. Hierzu zählen Baumwolle, Wolle, Hanffaser, Sisalfasern, Flachs, Ramie, Polyacrylnitrilfasern, Polyesterfasern, Polyamidfasern, Viskosefasern, Seide, Acetatfasern, Triacetatfasern, Aramidfasern und dergleichen sowie Mischungen dieser Fasermaterialien. Geeignet sind auch Glasfasern sowie Mischungen der vorgenannten Fasermaterialien mit Glasfasern z. B. Glasfaser/Kevlar-Mischungen.

Mit einer oben beschriebenen amylose-basierten Wirkstoff-Ausrüstung verbleiben die Wirkstoffe auch nach mehreren Wäschen in den damit ausgerüsteten Textilien. Zudem zeichnen sich die so ausgerüsteten Textilien durch einen angenehmen Griff aus, was insbesondere für den Tragekomfort von aus diesen Textilien hergestellter Bekleidung von Vorteil ist.

### 4.7 Kühlende Tabakprodukte

Grundsätzlich kann der Wirkstoffgehalt über einen weiten Bereich, wie z.B. 0,00001 bis 50 Gew.-%, insbesondere 0,001 bis 10 Gew.-% oder 0,005 bis 1 Gew.-% variieren.

Die erfindungsgemäß anzuwendenden Wirkstoffe können in vorteilhafter Weise auch zur Herstellung von Tabakprodukten verwendet werden. Beispiele für derartige Tabakprodukte umfassen, Zigarren, Zigaretten, Pfeifentabak, Kautabak, und Schnupftabak.

Die Herstellung von Tabakprodukten, welche mit kühlend wirkende Zusätzen ergänzt sind, ist an sich bekannt und z.B. beschrieben in US 3,111,127, US 5,752,529 und US 2005/0000529.

### 4.8 Kühlende Verpackungsmaterialien

Die erfindungsgemäß anzuwendenden Wirkstoffe sind auch in vorteilhafter Weise zur Herstellung von Verpackungsmaterialien geeignet.

Die Herstellung erfolgt dabei ebenfalls in an sich bekannter Weise. Die Wirkstoffe können dabei in das Verpackungsmaterial, in freier oder z.B. verkapselter Form, eingearbeitet oder auf das Verpackungsmaterial, in freier oder verkapselter Form aufgebracht werden.

So sind entsprechend ausgerüstete Kunststoffverpackungsmaterialien entsprechend den Angaben in der Literatur zur Herstellung von Polymerfilmen herstellbar (z.B. Ullmann, 6th Edn, 2003. Vol. 36, S. 567). Die Herstellung in geeigneter Weise beschichteter Papiere ist ebenfalls bekannt und z.B. beschrieben in Ullmann, Vol. 25, S. 106 ff, 6th Edn, 2003.

### 5. Wirkstoffkombinationen

Gegebenenfalls können die erfindungsgemäß anzuwendenden Verbindungen (Kühlwirkstoffe) der Formeln I (IA, IB, IC), II und/oder III mit weiteren bekannten Wirkstoffen, insbesondere auch solchen mit vergleichbarer Wirkung, kombiniert werden. Beispielsweise können diese mit bekannten kühlenden Verbindungen, wie z.B. Menthol, Menthon, N-Ethyl-p-menthancarboxamid (WS-3, auch Menthan-3-carbonsäure-N-ethylamid genannt), N-2,3-Trimethyl-2-isopropylbutanamid (WS-23), Menthyllactat (Frescolat® ML), Menthonglycerinacetal (Frescolat® MGA), Mono-menthylsuccinat (Physcool ®), Mono-menthylglutarat, O-Menthyl-glycerin, Menthyl-N,N-dimethylsuccinamat kombiniert werden.

Die erfindungsgemäß anzuwendenden Kühlwirkstoffe können bevorzugt mit den folgenden Kühlwirkstoffen kombiniert werden:
Menthol und Mentholderivate (z.B. L-Menthol, D-Menthol, racemisches Menthol, Isomenthol, Neoisomenthol, Neomenthol) Menthylether (z.B. (L-Menthoxy)-1,2-propandiol, (L-Menthoxy)-2-methyl-1,2-propandiol, L-Menthyl-methylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat oder deren Mischungen), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), Menthancarbonsäureamide (z.B. Menthancarbonsäure-N-ethylamid [WS3], Nα-(Menthancarbonyl)glycinethylester [WS5], Menthancarbonsäure-N-(4-cyanophenyl)amid, Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (I-(-)-Isopulegol, I-(-)-Isopulegolacetat), Menthanderivate (z.B. p-Menthan-3,8-diol), Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, wie in WO 2004/026840 beschrieben).

Die erfindungsgemäß anzuwendenden Kühlwirkstoffe können besonders bevorzugt mit den folgenden Kühlwirkstoffen kombiniert werden: Menthylether (z.B. (L-Menthoxy)-1,2-propandiol, (L-Menthoxy)-2-methyl-1,2-propandiol), polarere Menthylester (z.B. Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), nicht erfindungsgemäße Menthancarbonsäureamide (z.B. Menthancarbonsäure-N-ethylamid [WS3], Nα-(Menthancarbonyl)glycinethylester [WS5], Menthancarbonsäure-N-(4-cyanophenyl)amid, Menthancarbonsäure-N-(alkoxyalkyl)amide), Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid), Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, die in WO 2004/026840 beschrieben sind).

Die im gesamten Kapitel 4 und 5 genannten Ausführungen gelten auch für die speziellen Anwendungen im folgenden Kapitel 6 sofern nicht anders angegeben.

### 6. Spezielle Anwendungen

Folgender spezielle Teil der Erfindung betrifft die Verwendung von erfindungsgemäß anzuwendenden Verbindungen (TRPM8-Rezeptor-Modulatoren) zur Erzeugung einer langanhaltenden physiologischen Kühlwirkung auf Haut oder Schleimhaut.

Physiologische Kühlwirkstoffe werden regelmäßig eingesetzt, um einen kühlen sensorischen Eindruck auf der Haut bzw. Schleimhaut, zum Beispiel auf der Schleimhaut im Mund-, Nasen- und/oder Rachenraum hervorzurufen, wobei allerdings tatsächlich keine physikalische Abkühlung wie zum Beispiel bei der Verdunstung von Lösungsmitteln stattfindet. Als physiologische Kühlwirkstoffe können sowohl Einzelkomponenten als auch Mischungen verwendet werden. Dabei ist zu berücksichtigen, dass nicht alle Verbindungen, die *in vitro* Rezeptoren beeinflussen, die (auch) an der Vermittlung einer physiologischen Kühlwirkung beteiligt sind, tatsächlich einen solchen Effekt *in vivo* auf der Haut oder auf Schleimhäuten erzeugen. Insbesondere wird ein solcher Effekt nicht immer identisch verlaufen. Das bedeutet zum Beispiel, dass die Stärke der vermittelten physiologischen Kühlwirkung sowie der Verlauf der Stärke der Kühlwirkung gegen Zeitdauer nicht alleine aus der Tatsache geschlossen werden können, dass eine bestimmte Verbindung ein Argonist eines an der Vermittlung eines Kühleindruckes beteiligten Rezeptors ist.

Der bekannteste physiologisch wirksame Kühlwirkstoff ist L-Menthol, der aber einige Nachteile besitzt, z. B. starker Geruchseindruck, eine hohe Flüchtigkeit und in höheren Konzentrationen einen bitteren und/oder scharfen Eigengeschmack, bzw. eine Haut reizende Wirkung.

Es wurde daher schon früher nach starken Kühlwirkstoffen gesucht, die nicht die nachteiligen Eigenschaften des L-Menthols aufweisen. So wurden z.B. Milchsäureester von Menthol(en) gemäß DE 2 608 226 und gemischte Carbonate mit Menthol(en) und Polyolen gemäß DE 4 226 043 und Menthonketale gemäß EP 0 507 190 beschrieben.

Menthylmonoester von Disäuren nach US 5,725,865 und US 5,843,466 sind zwar interessante natürlich vorkommende Alternativen, können aber in sensorischen Tests nicht die Stärke der vorher beschriebenen Kühlwirkstoffe erreichen.

In J. Soc. Cosmet. Chem. 1978, 29, 185-200 wurden die Ergebnisse einer Studie an ca. 1200 Verbindungen präsentiert, bei der die Verbindungen L-Menthancarbonsäure-*N*-ethylamid ("WS3") und insbesondere *N*^{α}-(L-Menthancarbonyl)-glycinethylester ("WS5") als die stärksten Kühlwirkstoffe gefunden wurden. Letzterer hat bei starker Wirkung aber den Nachteil, hydrolyseempfindlich zu sein und dabei die entsprechende freie Säure *N*^{α}-(L-Menthancarbonyl)-glycin zu bilden, die selbst nur noch eine sehr schwache Kühlwirkung zeigt. Trotz der beschriebenen ausführlichen Untersuchungen ist eine systematische Vorhersage zu den Eigenschaften von potentiellen Kühlwirkstoffen, insbesondere zu deren Bitterkeit und/oder deren anderen trigeminalen Effekten nicht möglich und auch nicht beschrieben. So sind auch viele unter die Klasse der Menthancarbonsäureamide fallende Moleküle zwar stark kühlend, zeigen jedoch häufig gleichzeitig ausgeprägt bittere Noten (z.B. die Menthancarbonsäure-N-(alkyloxyalkyl)amide nach JP 2004059474) oder sind zusätzlich stark reizend (WS5: N-[[5-Methyl-2-(1-methylethyl)cyclohexyl]carbonyl]glycinethylester, US 2005/0222256).

*N*^{α}-(Menthancarbonyl)alkyloxyalkylamide wurden in JP 2004059474 beschrieben. Diese haben bei starker Kühlwirkung und hoher Hydrolysestabilität jedoch den Nachteil, stark bitter zu sein, und sie sind somit in Nahrungsmitteln und auch in der Gesichtspflege dienenden kosmetischen Produkten nicht einsetzbar.

Des Weiteren sind Menthylglyoxylate und ihre Hydrate in JP 2005343795 als Kühlsubstanzen beschrieben worden.

Übersichten zu den bisher hergestellten und verwendeten Kühlwirkstoffen findet man bei M. Erman, Perfumer & Flavorist 32(10), 20-35 (2007**)** und M. L. Dewis in D. J. Rowe, Chemistry and Technology of Flavors and Fragrances, Blackwell Publishing Ltd, Oxford 2005, p. 212 - 222.

Im Folgenden werden neue Mittel beschrieben, die eine besondere physiologische Kühlwirkung besitzen und dabei in Nahrungs- und/oder Genussmitteln und/oder Mundpflegeprodukten und/oder (oralen) pharmazeutischen Zubereitungen und/oder kosmetischen Zubereitungen als Kühlsubstanzen (Kühlwirkstoffe) eingesetzt werden können. Die anzugebenden Verbindungen bzw. Mischungen von Verbindungen sollten vorzugsweise einen möglichst schwachen Eigengeschmack zeigen, insbesondere wenig oder gar nicht bitter schmecken sowie möglichst nicht reizend sein.

Die Erfindung betrifft Mittel, wie z.B. eine Aromamischung oder Nahrungsmittel, Mundhygienemittel oder dem Genuss dienende pharmazeutische oder kosmetische Zubereitung, umfassend eine, zwei, drei oder mehr der Verbindungen ausgewählt unter Verbindungen P1 bis P13, P15 und P17 bis P31 gemäß obiger Tabelle 1, wobei die Verbindung oder die Verbindungen in einer Konzentration von 0,05 ppm - < 0,1 ppm oder 0,1 ppm bis 50 Gew-% bezogen auf das Gesamtgewicht der Zubereitung enthalten ist.

Insbesondere sind in den für diesen speziellen Aspekt der Erfindung beschriebenen Mitteln die erfindungsgemäß einzusetzenden Verbindungen in einer (Gesamt-) Konzentration von 0,05 ppm bis 50 Gew.-% bezogen auf das Gesamtgewicht der Zubereitung bzw. des Mittels enthalten. Dabei setzt sich dieser Bereich insbesondere aus folgenden Unterbereichen zusammen: 0,05 ppm - < 0,1 ppm. 0,1 ppm - 1.000 ppm und 0,1 - 50 Gew.-%. Bevorzugte Konzentrationsbereiche, bezogen auf das Gesamtgewicht der Zubereitung bzw. des Mittels sind:
0,05 ppm - 10 Gew.-%,. 0,5 ppm-5 Gew.-%, 1 ppm, - 2,5 Gew.-%.

Bevorzugt ist ein erfindungsgemäßes Mittel, insbesondere eines gemäß des speziellen Aspektes der Erfindung, umfassend
(1) einen oder mehrere weitere Stoffe mit physiologischer Kühlwirkung, wobei der weitere Stoff bzw. einer, mehrere oder sämtliche der weiteren Stoffe (i) einen geschmacklichen Effekt verursachen oder (ii) keinen geschmacklichen Effekt verursachen,
   und/oder
(2) einen oder mehrere Aromastoffe ohne physiologische Kühlwirkung und/oder
(3) einen oder mehrere trigeminal oder mundwässernd wirksame Stoffe ohne physiologische Kühlwirkung
   und/oder
(4) (iii) eine oder
   (iv) mehrere Verbindungen, die im Falle (iv) unabhängig von einander oder gemeinsam zusätzlich einen geschmacksmodulierenden Effekt und/oder einen trigeminalen und/oder einen mundwässernden Reiz verursachen.

Weiter bevorzugt umfasst ein solches Mittel einen oder mehrere weitere Stoffe mit physiologischer Kühlwirkung ohne geschmacklichen Effekt. Hierdurch wird vermieden, dass mit dem erfindungsgemäßen Mittel beispielsweise nur Aromen mit einem minzigen Aroma-Charakter erhalten werden können.

Ganz besonders bevorzugt ist ein erfindungsgemäßes Mittel umfassend als Bestandteil (2) einen oder mehrere Aromastoffe ohne physiologische Kühlwirkung und/oder als Bestandteil (3) eine oder mehrere Verbindungen, der oder die unabhängig voneinander oder gemeinsam zusätzlich einen geschmacksmodulierenden Effekt und/oder einen trigiminalen und/oder einen mundwässernden Reiz verursachen, wobei der trigiminale Reiz bevorzugt keine physiologische Kühlwirkung darstellt. Insbesondere besitzen solche erfindungsgemäßen Mittel, die die letztgenannten Bestandteile (2) und (3) gleichzeitig enthalten eine angenehme Kühlwirkung und ein ausgeglichenes sensorisches Profil bei gleichzeitig hohem Impact, d.h. hohem geschmacklichen Ersteindruck.

Der spezielle Aspekt der Erfindung betrifft bevorzugt auch Mittel insbesondere als der Ernährung, der Mundhygiene oder dem Genuss dienende oder pharmazeutische oder kosmetische Zubereitungen, die eine zum Erreichen einer physiologischen Kühlwirkung auf der Haut und/oder Schleimhaut ausreichende Menge einer erfindungsgemäß einzusetzenden Verbindung oder einer erfindungsgemäß einzusetzenden Mischung von solchen Verbindungen umfassen. Insbesondere soll die eingesetzte Menge dieser Verbindung oder dieser Mischung zum Erreichen einer physiologischen Kühlwirkung auf der Schleimhaut im Mund-, Nasen- und/oder Rachenraum ausreichen.

In diesem Zusammenhang sei darauf hingewiesen, dass die Begriffe "Mittel" und "Zubereitung" synonym verwendet werden können. Bevorzugt ist jedoch, dass eine Zubereitung mittels eines Arbeitsschrittes hergestellt werden muss, der über das ledigliche Mischen der Einzelverbindungen hinaus geht. Ein solcher Arbeitsschritt kann zum Beispiel zum Erzeugen einer Suspension oder einer Emulgierung dienen.

Bevorzugte erfindungsgemäße Mittel umfassen übliche Grund-, Hilfs- und Zusatzstoffe für der Ernährung, der Mundhygiene oder dem Genuss dienende oder pharmazeutische oder kosmetische Zubereitungen. Bevorzugte erfindungsgemäße Zubereitungen enthalten 0,000005 Gew.-% bis 20 Gew.-%, bevorzugt 0,00001 bis 10 Gew.-%, besonders bevorzugt 0,0001 Gew.-% bis 0,5 Gew.-% an erfindungsgemäßen einzusetzenden Verbindungen, bezogen auf das Gesamtgewicht der Zubereitung. Weitere Bestandteile, insbesondere Bestandteile (1) (weitere Stoffe mit physiologischer Kühlwirkung), (2) (Aromastoffe ohne physiologische Kühlwirkung) und/oder (3) (trigeminal oder mundwässernd wirksame Stoffe ohne physiologische Kühlwirkung) (wie oben beschrieben) sowie weitere übliche Grund-, Hilfs- und Zusatzstoffe können in Mengen von 0,0000001 bis 99,99 Gew.-%, vorzugsweise 10 bis 80 Gew.-%, bezogen auf das Gesamtgewicht der Zubereitung, enthalten sein. Ferner können die erfindungsgemäßen Zubereitungen Wasser in einer Menge bis zu 99,99 Gew.-%, vorzugsweise 5 bis 80 Gew.-%, bezogen auf das Gesamtgewicht der Zubereitung, enthalten.

Bevorzugt werden die erfindungsgemäß einzusetzenden Verbindungen oder deren Mischungen zum Herstellen eines Arzneimittels, das der Bekämpfung oder der Linderung von Husten-Schnupfen-, Mund-, Nasen-, Hals- oder Rachenentzündungs-, Halsschmerz- oder Heiserkeitssymptomen dient, verwendet.

Ein weiterer Aspekt des speziellen Aspektes der vorliegenden Erfindung betrifft ein nicht-therapeutisches Verfahren zum Erreichen einer physiologischen Kühlwirkung auf der Haut und/oder einer Schleimhaut, mit folgendem Schritt:
- Applizieren einer zum Erreichen einer physiologischen Kühlwirkung ausreichenden Menge eines erfindungsgemäßen Mittelsauf die Haut und/oder eine Schleimhaut.

Bevorzugt ist im Sinne des speziellen Aspektes der Erfindung, dass das erfindungsgemäße Mittel eine Aromamischung ist, welche einen oder mehrere Aromastoffe und/oder einen oder mehrere weitere Kühlwirkstoffe umfasst, zur Aromatisierung von unter Verwendung der Aromamischung gefertigter Fertigwaren.

In Zubereitungen (Aromamischungen), die zu Aromatisierungen von Zahnpasten und - cremes eingesetzt werden, beträgt der Gehalt der erfindungsgemäß zu verwendenden Stoffe 0,001 bis 50 Gew.-%; bevorzugt ist ein Bereich von 0,005 bis 5 Gew.-% und besonders bevorzugt ein Bereich von 0,01 bis 2 Gew.-%. Bei üblichen Dosierungen der Aromen zwischen 0,5 und 1,5 Gew.-%, bezogen auf die gebrauchsfertigen Zahnpasten und -cremes beträgt dann der Gehalt der erfindungsgemäß zu verwendenden Stoffe 0,000005 bis 0,75 Gew-% bezogen auf das Fertigprodukt; bevorzugt ist entsprechend ein Bereich von 0,000025 bis 0,075 Gew.-% und besonders bevorzugt entsprechend ein Gehalt von 0,00005 bis 0,03 Gew.-%.

In Zubereitungen (Aromamischungen), die zu Aromatisierungen von Kaugummis eingesetzt werden, beträgt der Gehalt der erfindungsgemäß zu verwendenden Stoffe 0,005 bis 10 Gew.-%; bevorzugt ist ein Bereich von 0,01 bis 5 Gew.-% und besonders bevorzugt ein Bereich von 0,05 bis 2,5 Gew.-%. Bei einer üblichen Dosierung der Aromen von 1 - 2 Gew.-%, bezogen auf das gebrauchsfertige Kaugummi beträgt dann der Gehalt der erfindungsgemäß zu verwendenden Stoffe 0,00005 bis 0,2 Gew-% bezogen auf das Fertigprodukt; bevorzugt ist entsprechend ein Bereich von 0,0001 bis 0,1 Gew.-% und besonders bevorzugt entsprechend ein Gehalt von 0,0005 bis 0,05 Gew.-%.

In Zubereitungen (Aromamischungen), die zu Aromatisierungen von Mundwässern und -spülungen eingesetzt werden, beträgt der Gehalt der erfindungsgemäß zu verwendenden Stoffe 0,01 bis 10 Gew.-%; bevorzugt ist ein Bereich von 0,05 bis 5 Gew.-% und besonders bevorzugt ein Bereich von 0,1 bis 2,5 Gew.-%. Bei einer üblichen Dosierung des Aromas von 2 - 4 Gew.-%, bezogen auf ein gebrauchsfertiges Mundwasserkonzentrat beträgt dann der Gehalt der erfindungsgemäß zu verwendenden Stoffe 0,0002 bis 0,4 Gew-% bezogen auf das Fertigprodukt; bevorzugt ist entsprechend ein Bereich von 0,001 bis 0,2 Gew.-% und besonders bevorzugt entsprechend ein Gehalt von 0,002 bis 0,1 Gew.-%. In gebrauchsfertigen Mundwässern und -spülungen beträgt dann bei einer üblichen Dosierung der Aromamischung von 0,1 - 0,3 Gew.-% der Gehalt der erfindungsgemäß zu verwendenden Stoffe 0,00001 bis 0,03 Gew-% bezogen auf das Fertigprodukt; bevorzugt ist entsprechend ein Bereich von 0,00005 bis 0,015 Gew.-% und besonders bevorzugt entsprechend ein Gehalt von 0,0001 bis 0,0075 Gew.-%.

Als Aromastoffe eignen sich sowohl komplexe natürliche Rohstoffe wie aus Pflanzen gewonnene Extrakte und etherische Öle, bzw. daraus gewonnene Fraktionen und einheitliche Stoffe, als auch einheitliche synthetisch oder biotechnologisch gewonnene Aromastoffe.

Beispiele für natürliche Rohstoffe sind z.B.:
Pfefferminzöle, Krauseminzöle, Mentha-Arvensis-Öle, Anisöle, Nelkenöle, Citrusöle, Zimtrindenöle, Wintergrünöle, Cassiaöle, Davanaöle, Fichtennadelöle, Eucalyptusöle, Fenchelöle, Galbanumöle, Ingweröle, Kamillenöle, Kümmelöle, Rosenöle, Geraniumöle, Salbeiöle, Scharfgarbenöle, Sternanisöle, Thymianöle, Wacholderbeeröle, Rosmarinöle, Angelikawurzelöle, und die Fraktionen dieser Öle.

Beispiele für einheitliche Aromastoffe sind z.B.:
Anethol, Menthol, Menthon, Isomenthon, Menthylacetat, Menthofuran, Menthylmethylether, Mintlacton, Eucalyptol, Limonen, Eugenol, Pinen, Sabinenhydrat, 3-Octanol, Carvon, gamma-Octalacton, gamma-Nonalacton, Germacren-D, Viridiflorol, 1,3E,5Z-Undecatrien, Isopulegol, Piperiton, 2-Butanon, Ethylformiat, 3-Octylacetat, Isoamylisovalerianat, Hexanol, Hexanal, cis-3-Hexenol, Linalool, alpha-Terpineol, cis und trans Carvylacetat, p-Cymol, Thymol, 4,8-Dimethyl-3,7-nonadien-2-on, Damascenon, Damascone, Rosenoxid, Dimethylsulfid, Fenchol, Acetaldehyddiethylacetal, cis-4-Heptenal, Isobutyraldehyd, Isovaleraldehyd, cis-Jasmon, Anisaldehyd, Methylsalicylat, Myrtenylacetat, 8-Ocimenylacetat, 2-Phenylethylalkohol, 2-Phenylethylisobutyrat, 2-Phenylethylisovalerat, Zimtaldehyd, Geraniol, Nerol. Bei chiralen Verbindungen können die genannten Aromastoffe als Racemat, als einzelnes Enantiomer oder als enantiomerenangereicherte Mischungen vorliegen.

In der Ernährung oder dem Genuss dienenden Zubereitungen beträgt der Gehalt der erfindungsgemäß zu verwendenden Stoffe 0,000005 bis 0,1 Gew.-%; bevorzugt ist ein Bereich von 0,00005 bis 0,05 Gew.-% und besonders bevorzugt ein Bereich von 0,0001 bis 0,02 Gew.-%.

In kosmetischen Zubereitungen beträgt der Gehalt der erfindungsgemäß zu verwendenden Stoffe 0,001 bis 10 Gew.-%; bevorzugt ist ein Bereich von 0,005 bis 5 Gew.-% und besonders bevorzugt ein Bereich von 0,01 bis 2 Gew.-%.

Erfindungsgemäß besonders bevorzugt ist, dass das gemäß dem speziellen Aspekt der Erfindung erfindungsgemäße Mittel eine Zahnpasta ist.

### 7. Mögliche Herstellungswege erfindungsgemäß anzuwendender Verbindungen

Im folgenden Abschnitt werden verschiedene Synthesemethoden für einen repräsentativen Querschnitt erfindungsgemäß anzuwendender Wirkstoffe beschrieben.

### a) Allgemeine Herstellungsbeispiele für Verbindungen gemäß Formel I

Die Herstellung repräsentativer Verbindungen der Formel I ist in folgendem Abschnitt beschrieben.

Grundsätzlich sind die Verbindungen der Formel I aus der Umsetzung von Keto-Vorstufen der Formel V-I mit ketoreaktiven Verbindungen der Formel Y-I zugänglich (siehe Akhrem et al.,Kimiya Geteotsiklicheskikh Soedinenii, 1995, 187-194, Akhrem et al., Journal of Organic Chemistry of the USSR, 1985, 21 (6), 1227-1232)

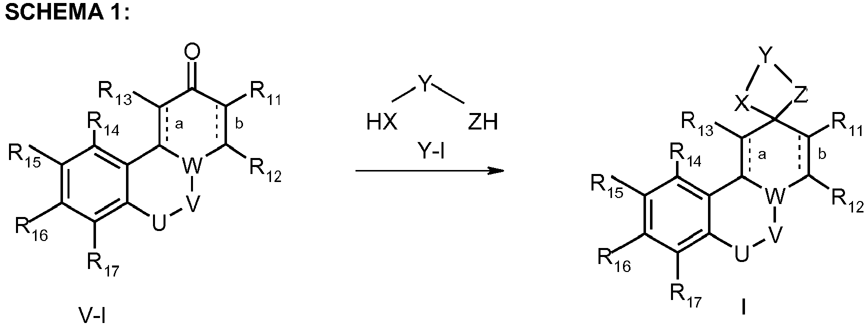

Die verschiedenen dazu erforderlichen Ausgangsstoffe sind ebenfalls bekannt oder nach bekannten Verfahren zugänglich.

Zur Herstellung von Verbindungen vom Typ V-I siehe allgemein: Akhrem et al. in Journal of Organic Chemistry of the USSR, 1979, 1247-1252, Akhrem et al., Izvestia Akademii Nauk SSSR Seria Himiceskaa, 1969, (10), 2338-2339, Akhrem et al., Doklady Akademii Nauk SSSR, 1972, 203 (1), 95-98.

In den folgenden Abschnitten werden die Synthesewege einiger spezieller Verbindungsklassen schematisch veranschaulicht. Die Umsetzungen erfolgen dabei unter Anwendung üblicher Methoden der organisch-chemischen Synthese.

### Herstellungsweg 1-1: Allgemeine Herstellung von Verbindungen der Formel I (Gruppe 1)

Verbindungen der Formel IA 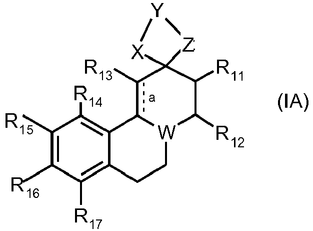 mit
W = N,
a = Einfachbindung,
R₁₁, R₁₂ = Cyclus;
R₁₃ bis R₁₇ = H;

Die Herstellung erfolgt dabei nach folgendem Schema :

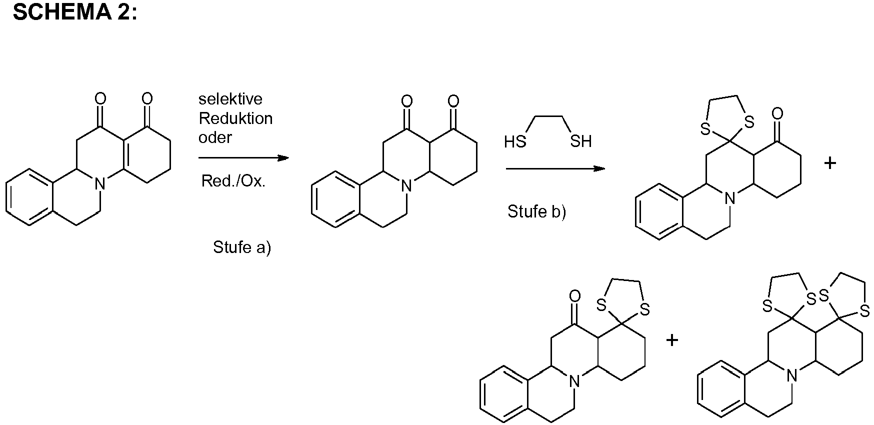

Die Reduktion gemäß Stufe a) erfolgt unter Anwendung üblicher Syntheseorschriften (A.Guarna et al., J. Org. Chem, 63 (12), 4111-4115, 1998, H. Takahata et al., Chem. Pharm. Bull., 34(11), 4523, 1986)Stufe b) kann in Anlehnung an die Synthesen korrespondierender Verbindungen in der WO 2011/61330 durchgeführt werden.

Durch Variation des Reaktanden in Stufe b) lassen sich in analoger Weise folgende Verbindungen herstellen (für einen Überblick dazu siehe "T. W. Greene, P. G. M. Wuts, Protective Groups in Organic Synthesis, John Wiley and Sons, Inc., 1991)

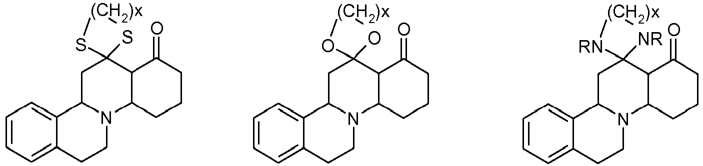

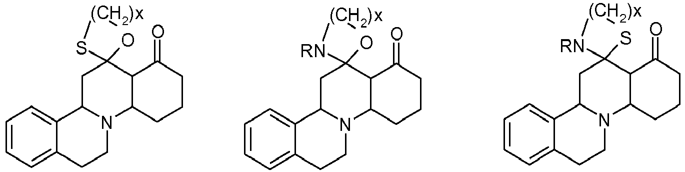

### Herstellungsweg H1-2: Allgemeine Herstellung von Verbindungen der Formel I(Gruppe 2)

### Verbindungen der Formel IC

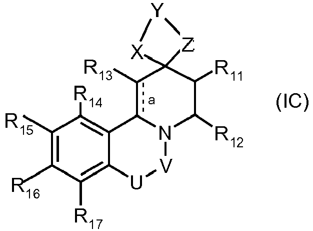

mit
U = chemische Bindung
a = Einfach- oder Doppelbindung,
R₁₁, R₁₂ = Cyclus;
R₁₃ bis R₁₇ = H;

Die Herstellung erfolgt dabei nach folgendem Schema:

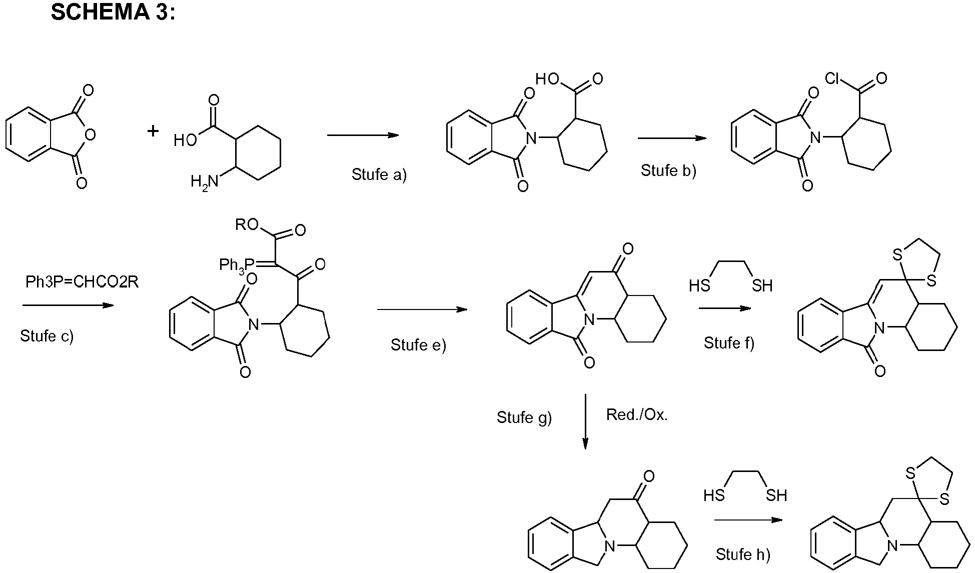

(Stufe a vgl. E. Guernin et al., Eur J. Org. Chem, 3380-3391, 2007, Stufen b, c, e, g: vergl. W. Flitsch, Liebigs Ann. Chem., 649-654, 1987) Alternative Reaktanden für Stufe a):

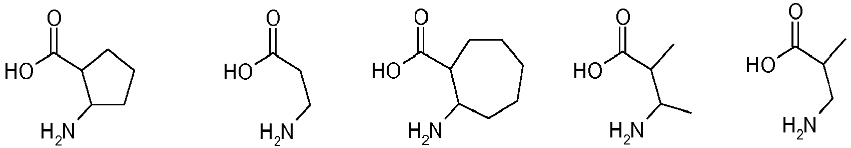

Alternative Reaktanden zu Stufe f) und h) analog Schema 2.

### Herstellungsweg H1-3: Allgemeine Herstellung von Verbindungen der Formel I (Gruppe 3)

Verbindungen der Formel IA 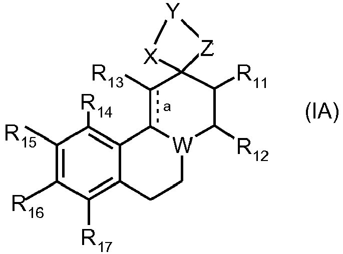 mit
W=CH
a = Einfach- oder Doppelbindung,
R₁₁, R₁₂ = Cyclus;
R₁₃ bis R₁₇ = H;

Die Herstellung erfolgt dabei nach folgendem Schema:

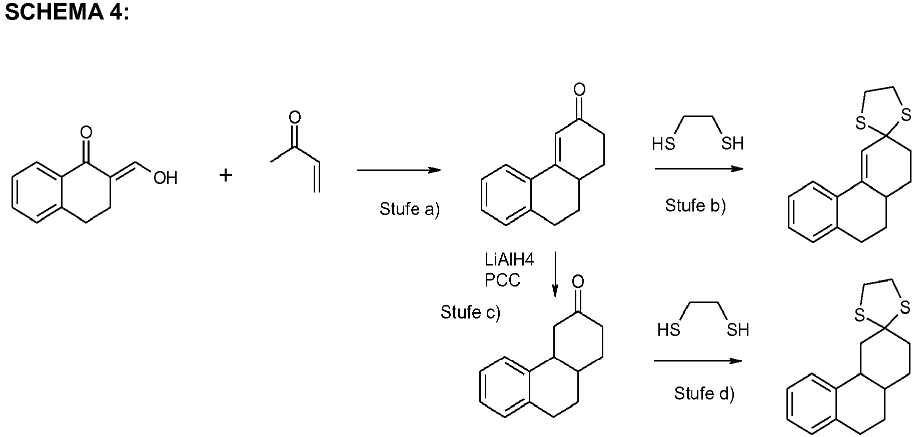

Stufen a,c: siehe E. J. Eisenbraun et al., J. Org. Chem, 452-455, 1988 . Durch Variation der Reaktanden der Stufe a) z.B. durch Einsatz von 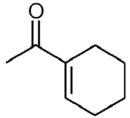 erhält man die Strukturen:

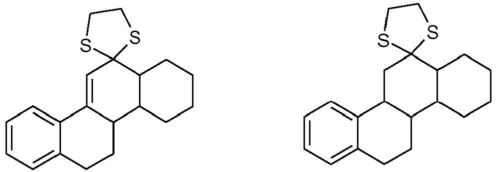

Durch Variation der Reaktanden der Stufen b) und d) analog Schema 2 erhält man weitere Strukturvarianten.

### Herstellungsweg H1-4: Allgemeine Herstellung von Verbindungen der Formel I (Gruppe 4a)

Verbindungen der Formel IA, 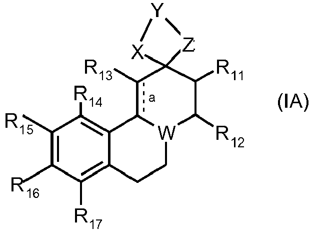 mit
W=N
a = Doppel- oder Einfachbindung
R₁₁, R₁₂ = Cyclus;
R₁₃ bis R₁₇ = H;

Die Herstellung erfolgt dabei nach folgendem Schema:

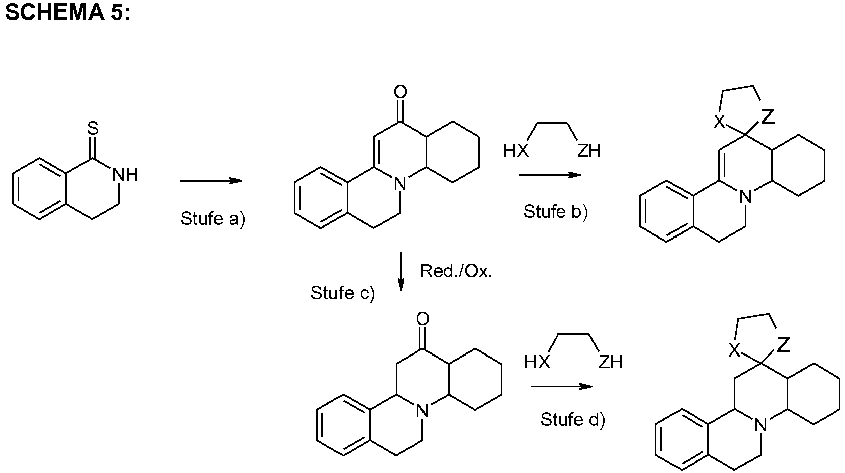

Vergl. S. J. Danishefsky et al.,Tetrahedron Lett., 3635-3628, 1989, Durch Variation der Reaktanden der Stufen b) und d) analog Schema 2 erhält man weitere Strukturvarianten.

### Herstellungsweg H1-5: Allgemeine Herstellung von Verbindungen der Formel I (Gruppe 4b)

Verbindungen der Formel IC, 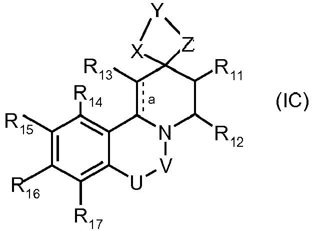
mit U = CH₂
V = Carboxyl
a = Doppel- oder Einfachbindung
R₁₁, R₁₂ = H;
R₁₃ bis R₁₇ = H;

Die Herstellung erfolgt dabei nach folgendem Schema:

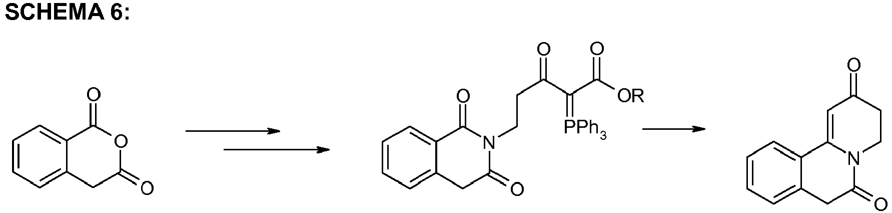

### Herstellungsweg H1-6: Allgemeine Herstellung von Verbindungen der Formel I (Gruppe 5)

Verbindungen der Formel IB, 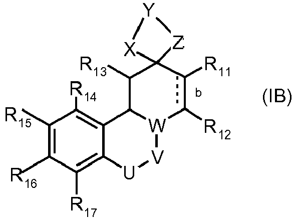 mit
W=N
b = Doppelbindung
R₁₁, R₁₂ = Cyclus;
R₁₃ bis R₁₇ = H;

Die Herstellung erfolgt dabei nach folgendem Schema:

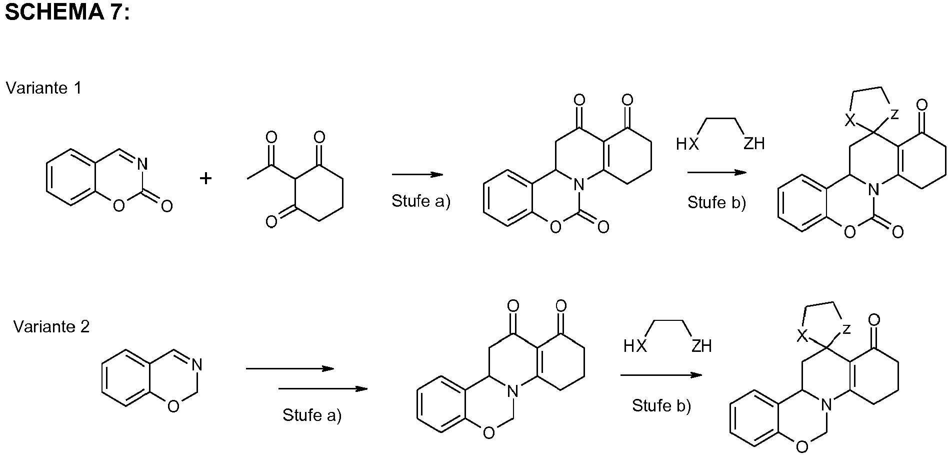

Durch Variation der Reaktanden der Stufen b) analog Schema 2 erhält man weitere Strukturvarianten.

### Herstellungsweg H1-7: Allgemeine Herstellung von Verbindungen der Formel I (Gruppe 6)

Verbindungen der Formel IB, 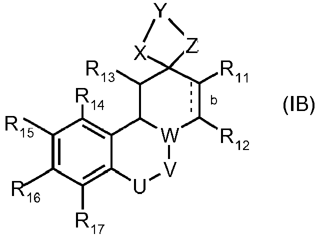 mit
W=CH
b = Doppelbindung
R₁₁, R₁₂ = Cyclus;
R₁₃ bis R₁₇ = H;

Die Herstellung erfolgt dabei nach folgendem Schema:

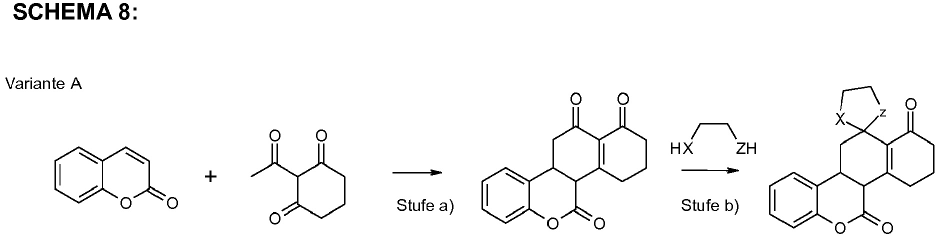

Durch Variation der Reaktanden der Stufen b) analog Schema 2 erhält man weitere Strukturvarianten.

Weitere Beispiele für mögliche Herstellungswege von Verbindungen der Formeln IA, IB, IC, IA', IB' und IC' sind in folgenden Schemata zusammengefasst 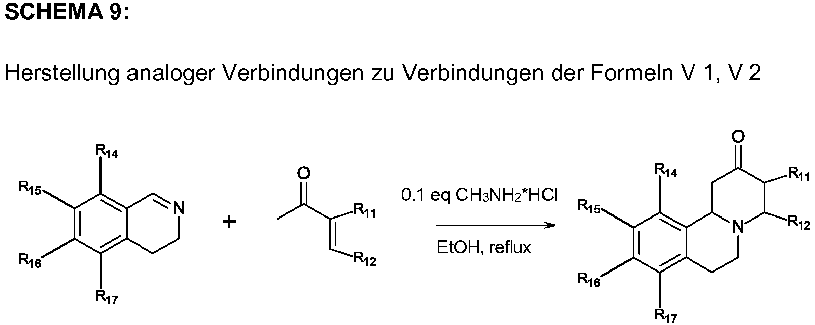 worin R₁₁ bis R₁₇ die oben angegebenen Bedeutungen besitzen können.

Dabei wird das Isoquinolin, das Acetylalken (ggf in molarem Überschuss) und ein geeigneter Katalysator (z.B. Methylammoniumchlorid) in einem geeigneten Lösungsmittel (z.B. Ethanol) vorgelegt und bei einer geeigneten Temperatur (z.B. Rückfluss) gerührt. Nach Reaktionsende erfolgt die Abtrennung des Katalysators in der Regel durch Kristallisation aus einem geeigneten Lösungsmittel/Lösungsmittelgemisch (z. B. iPrOH/Wasser). Gegebenenfalls ist dazu vorab ein destillativer Lösungsmittelwechsel notwendig. 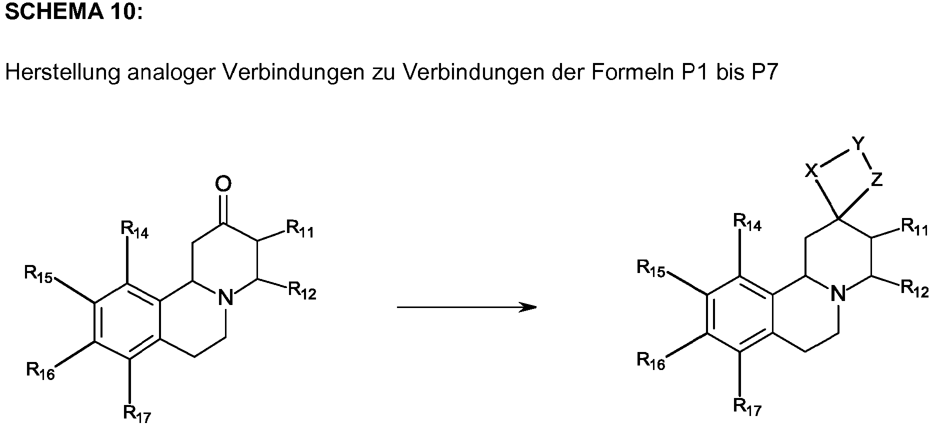 worin R₁₁ bis R₁₇, X, Y und Z die oben angegebenen Bedeutungen besitzen können, insbesondere worin X, Z = O, S und Y = -CH2-CH2-, -CH2-CH2-CH2-, CH2-C(CH3)2-CH2- ist

Das Aminoketon wird zunächst in einem geeigneten Lösemittel (z.B. MTBE) oder Lösungsmittelgemisch und einem Überschuss einer geeigneten Säure (z.B. Chlorwasserstoff) in das korrespondierende Salz überführt und anschließend mit dem entsprechenden Dialkohol, Dithiol oder Thioalkohol umgesetzt. Anschließend wird in der Regel wässrig extraktiv aufgearbeitet und das Produkt durch destillative Entfernung des Lösungsmittels isoliert. Sofern eine weitere Reinigung erforderlich ist, kann diese durch Kristallisation aus einem geeigneten Lösungsmittel oder chromatographisch erfolgen. 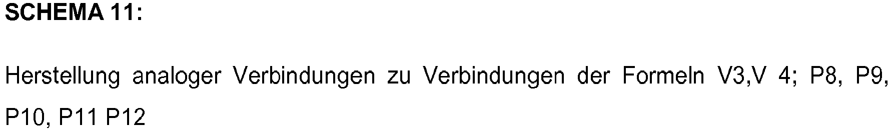 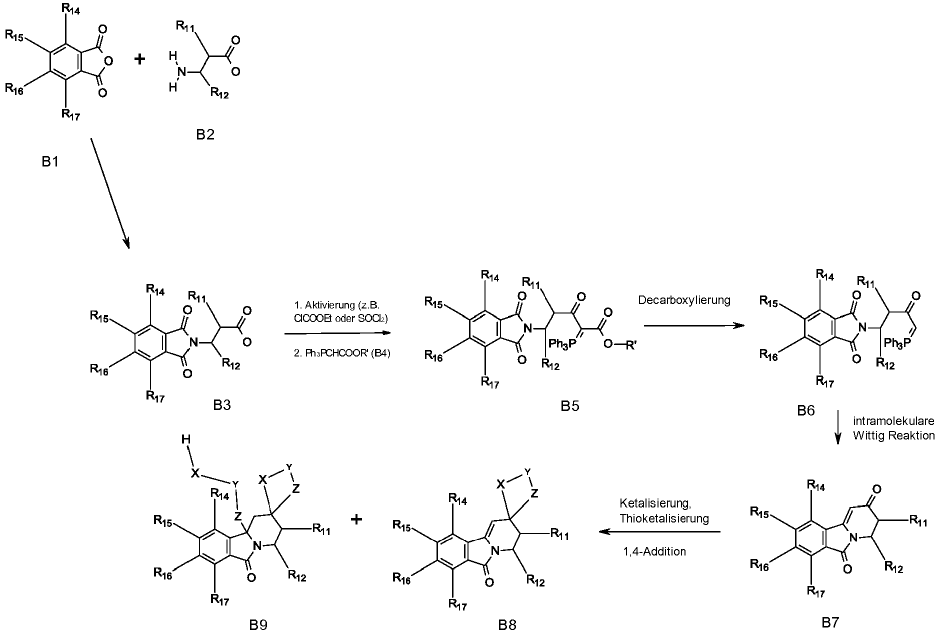 worin R₁₁ bis R₁₇, X, Y und Z die oben angegebenen Bedeutungen besitzen können, und z.B. R₁₁, R ₁₂ = H ist oder diese beiden Reste zusammen mit den C-Atomen an die sie gebunden sind einen carbocyclischen Ring mit 5, 6 oder 7 C-Atomen bilden.

Zur Synthese der tri- bzw. tetracyclischen Analoga der Ketone V3 bzw. V4 startet man idealerweise von einem Anhydrid B1 und einer β-Aminosäure B2, die sich, wie in dem sterisch anspruchsvollen Referenz-Herstellungsbeispiel H1-20 gezeigt, durch Rühren der beiden Substanzen bei einer geeigneten Temperatur zu Verbindungen des Typs B3 umsetzen lassen. Zur Darstellung eines Phosphorans B5 wird die Säuregruppe einer Verbindung B3 durch Umsetzung zum Säurechlorid oder einem Anhydrid aktiviert und anschließend mit einem Phosporan B4 zu einer Verbindung B5 umgesetzt. Die entstandene Esterfunktion wird im Folgeschritt decarboxyliert und das freigesetzte Phosphoran in einer Wittigreaktion zu einem ungesättigten Keton B6 zyklisiert. Zum Schluss wird unter Säurekatalyse mit einem Dithiol, Diol oder Thioalkohol zu einer Verbindungen B8 ketalisiert, wobei, je nach Reaktionsbedingungen, auch ein Additionsprodukt B9 entstehen kann. Sofern nach extraktiver Aufarbeitung eine weitere Reinigung erforderlich ist, kann diese durch Kristallisation aus einem geeigneten Lösungsmittel oder chromatographisch erfolgen 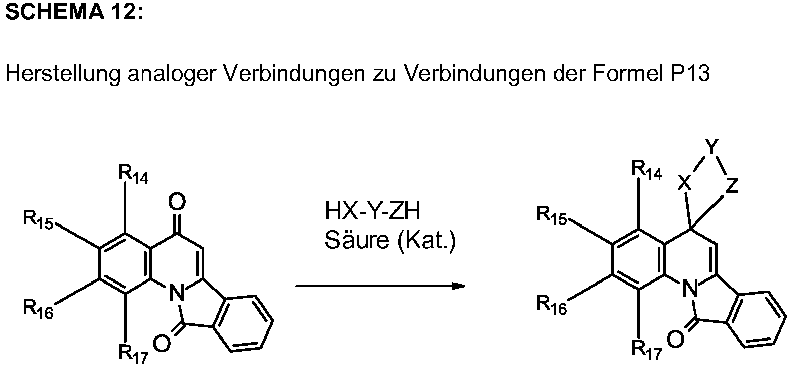 worin R₁₄ bis R₁₇, X, Y und Z die oben angegebenen Bedeutungen besitzen können.

Das Keton wird unter Zugabe eines wasserziehenden Mittels (Beispielsweise 4 Å Molekularsieb, Magnesiumsulfat) mit einem Überschuss Dithiol, Diol oder Thioalkohol und einer Säure oder Lewis-Säure (z.B. Zinkchlorid) versetzt Die Reaktionsmischung wird bei einer geeigneten Reaktionstemperatur gerührt und anschließend in der Regel wässrig extraktiv aufgearbeitet. Sofern eine weitere Reinigung erforderlich ist, kann diese durch Kristallisation aus einem geeigneten Lösungsmittel oder chromatographisch erfolgen. 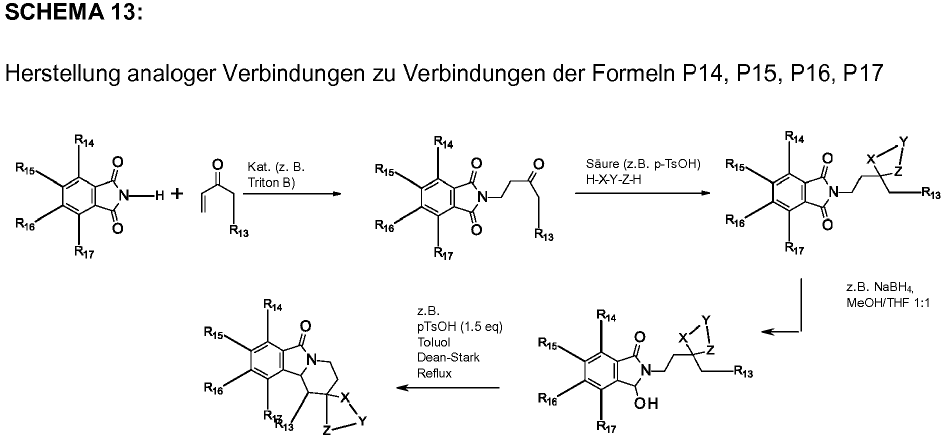 worin R₁₄ bis R₁₇, X, Y und Z die oben angegebenen Bedeutungen besitzen können.

Die Herstellung von Analogen zu P14 und P15 verläuft nach den folgenden Literaturvorschriften, sowie den im experimentellen Teil angegebenen Vorgehensweisen. (hier nicht mehr explizit aufgeführt):
A. M. Islam, R. A. Raphael, J. Chem. Soc. 1955, 3151-3154. Bosch, Joan; Rubiralta, Mario; Moral, Montserrat; Arino, Joaquin. Journal of the Chemical Society, Perkin Transactions 1: Organic and Bio-Organic Chemistry (1972-1999) (1986), (8), 1533-9. M. Chiurato, S. Routier, Y. Troin, G. Guillaumet, Eur. J. Org. Chem 2009, 3011-3021

Die O,O-Acetal-Analoga zu P14 und P15 können in die entsprechenden Thioketale wie folgt überführt werden: Das Sauerstoffketal wird in einem Lösungsmittel (z.B. Dichlormethan) gelöst und mit einem Überschuss des Dithiols oder Thioalkohols, sowie einer Lewis-Säure (z.B. Bortrifluorid-diethyletherat) versetzt. Die Reaktionsmischung wird bei geeigneter Reaktionstemperatur gerührt und anschließend in der Regel unter neutralisierenden Bedingungen wässrig aufgearbeitet. Sofern nach Entfernen des organischen Lösungsmittels eine weitere Aufreinigung der Produkte erforderlich ist, kann diese durch Kristallisation aus einem geeigneten Lösungsmittel oder säulenchromatographisch erfolgen. 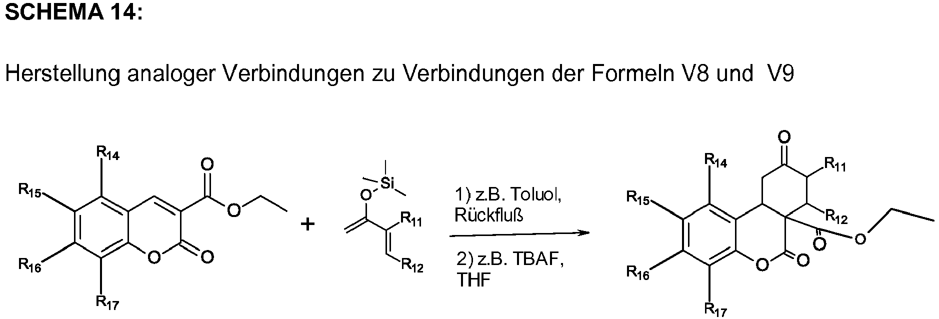 worin R₁₁ bis R₁₇ die oben angegebenen Bedeutungen besitzen können und z.B. R₁₁, R ₁₂ = H ist oder diese beiden Reste zusammen mit den C-Atomen an die sie gebunden sind einen carbocyclischen Ring mit 5, 6 oder 7 C-Atomen bilden.

Das Cumarincarboxylat und ein Überschuss 1-Cycloalk-1-enyl-vinyloxy-trialkyl-silan (z.B die Trimethylsilan-Verbindung) werden in Toluol oder einem anderen geeigneten organischen Lösungsmittel gelöst und bei einer geeigneten Reaktionstemperatur (z.B. Rückfluss) gerührt. Zur Abspaltung der Trialkylsilylgruppe wird ein Überschuss eines Fluorids z.B. Tetrabutyl-ammoniumfluorid zugesetzt und bei einer geeigneten Temperatur (z.B. Raumtemperatur) weitergerührt. (Alternativ ist auch die säurekatalysierte Spaltung des Silylenolethers zum Keton möglich.) Dabei kann es sich als notwendig erweisen, das bisher eingesetzte Lösungsmittel vorab gegen ein geeigneteres Lösungsmittel wie THF destillativ auszutauschen. Alternativ ist auch die säurekatalysierte Spaltung des Silylenolethers zum Keton möglich. Nach Beendigung der Reaktion wird in der Regel wässrig extraktiv aufgearbeitet. Sofern nach Entfernen des organischen Lösungsmittels eine weitere Aufreinigung der Produkte erforderlich ist, kann diese durch Kristallisation aus einem geeigneten Lösungsmittel oder säulenchromatographisch erfolgen. 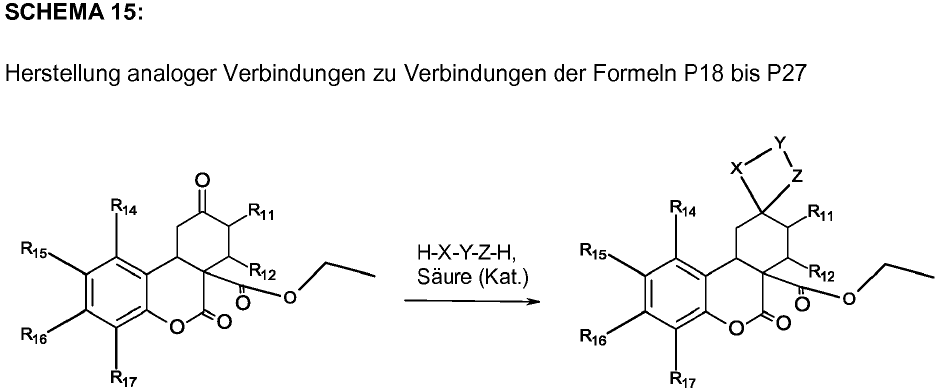 worin R₁₁ bis R₁₇ die oben angegebenen Bedeutungen besitzen können und z.B. R₁₁, R ₁₂ = H ist oder diese beiden Reste zusammen mit den C-Atomen an die sie gebunden sind einen carbocyclischen Ring mit 5, 6 oder 7 C-Atomen bilden.

Der Ketoester wird mit einem Überschuss Dialkohol, Dithiol oder Thioalkohol , einem sauren Katalysator (z.B. Amberlyst 15) und einem geeigneten Lösungsmittel (z.B. Toluol) versetzt und das Gemisch bei einer geeigneten Temperatur (z. B. Rückfluss) gerührt. Gegebenenfalls erweist es sich als notwendig Wasser durch Zugabe eines wasserentziehenden Mittels (z.B. 4 Å Molekularsieb) oder azeotrope Destillation dem Reaktionsgemisch zu entziehen. Nach Beendigung der Reaktion wird in der Regel wässrig extraktiv aufgearbeitet. Sofern nach destillativem Entfernen des organischen Lösungsmittels eine weitere Aufreinigung der Produkte erforderlich ist, kann diese durch Kristallisation aus einem geeigneten Lösungsmittel oder säulenchromatographisch erfolgen. 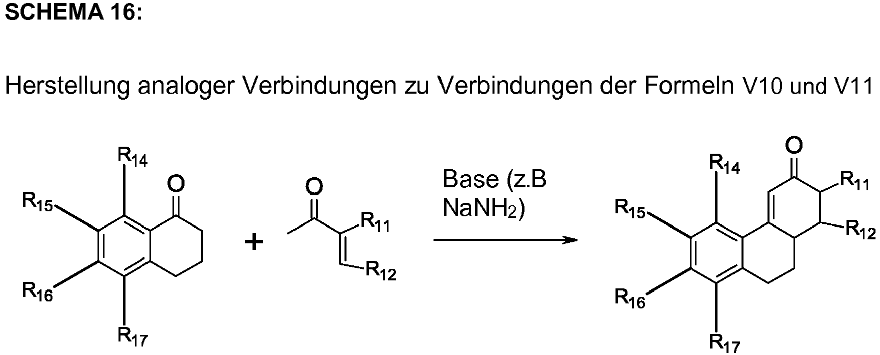 worin R₁₁ bis R₁₇ die oben angegebenen Bedeutungen besitzen können und z.B. R₁₁, R ₁₂ = H ist oder diese beiden Reste zusammen mit den C-Atomen an die sie gebunden sind einen carbocyclischen Ring mit 5, 6 oder 7 C-Atomen bilden.

Die Durchführung analog Peak, Robinson J. Chem. Soc., 1936, 759-762 bzw. J. Chem. Soc., 1937, 1581-1583. 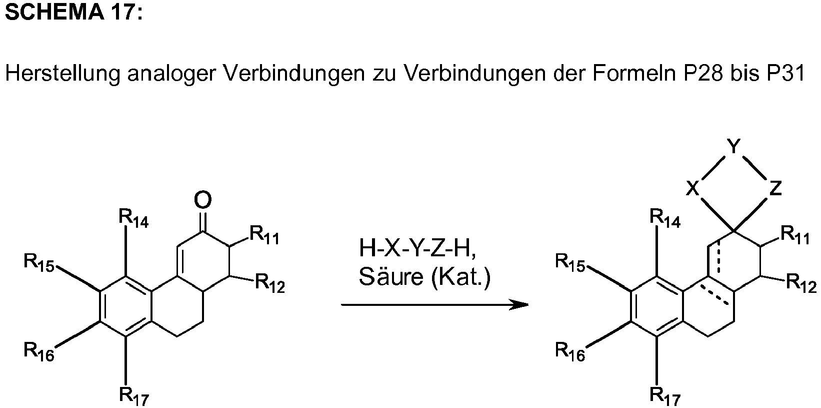 worin R₁₁ bis R₁₇ die oben angegebenen Bedeutungen besitzen können und z.B. R₁₁, R ₁₂ = H ist oder diese beiden Reste zusammen mit den C-Atomen an die sie gebunden sind einen carbocyclischen Ring mit 5, 6 oder 7 C-Atomen bilden.

Der Ketoester wird mit einem Überschuss Diol, Dithiol oder Thioalkohol, einem sauren Katalysator (z.B. Amberlyst 15) und einem geeigneten Lösungsmittel (z.B. Toluol) versetzt und das Gemisch bei einer geeigneten Temperatur (z.B. Rückfluss) gerührt. Gegebenenfalls erweist es sich als notwendig Wasser durch Zugabe eines wasserentziehenden Mittels (z.B. 4 Å Molekularsieb) oder azeotrope Destillation dem Reaktionsgemisch zu entziehen. Nach Beendigung der Reaktion wird in der Regel wässrig extraktiv aufgearbeitet. Sofern nach destillativem Entfernen des organischen Lösungsmittels eine weitere Aufreinigung der Produkte erforderlich ist, kann diese durch Kristallisation aus einem geeigneten Lösungsmittel oder säulenchromatographisch erfolgen.

### b) Allgemeine Herstellungswege für Verbindungen gemäß Formel II

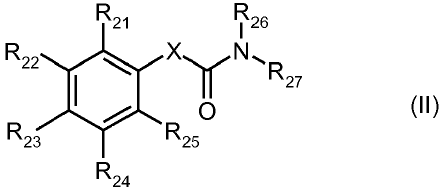

Die Herstellung repräsentativer Verbindungen des der Formel II ist in folgendem Abschnitt beschrieben. Die verschiedenen dazu erforderlichen Ausgangsstoffe der allgemeinen Formel C-II und B-II sind ebenfalls bekannt oder nach bekannten Verfahren zugänglich.

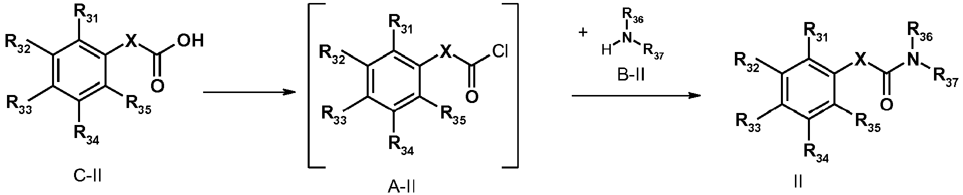

Die Reaktion wird beispielsweise in einem apolaren organischen Lösungsmittel in Gegenwart eines Säurefängers durchgeführt.

### c) Allgemeine Herstellungswege für Verbindungen gemäß Formel III

Im folgenden Abschnitt werden mehrere Synthesewege für Verbindungen der Formel III unter Verweis auf Literatur-bekannte Verfahren beschrieben:

### Syntheseweg 1:

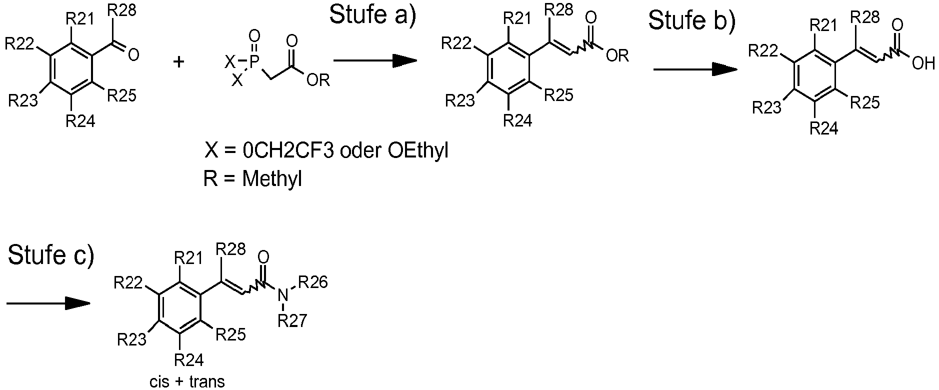

Stufe a) wird nach dem Prinzip einer Horner-Emmons Reaktion durchgeführt, indem man die aromatische Ketoverbindung mit einer geeigneten Phosphonoverbindung (z.B. Phosphonoessigsäure-P,P-bis(2,2,2-trifluorethyl)methylester, oder Dimethylmethoxycarbonylmethanphosphonat) umsetzt (vgl. z.B. Sano, S. et al Chemical and Pharmaceutical Bulletin, 2002, 50, Nr. 5, 706-709; Duan, Z.-C., et al Journal of organic Chemistry, 2010, 75,Nr 23, 8319-8321; Eguchit. et al; Tetrahedron Letters, 1992,33, 38, 5545-5546; Sano, S. et al., Chemical and Pharmaceutical Bulletin, 2002, 50, Nr. 9, 1300-1302; Chintareddy et al., Journal of Organic Chemistry, 2010, 75,21, 7166-7174; WO2004/31116; Miller, D.J. et al., Organic and Biiomoelcular Chemistry, 2007, 5, 20, 3287-3298; WO2009/ 53443; Strehlke et al., Eur. J. of Med. Chem., 1979, 14, 238-242; Tashchuk et al ., Sov. Prog. Chem., 1968, 34, 11,1148-1150; WO2005/40104; US 6,110,922; US 6,878,700; US2004/116518, WO2006/41404; WO2006/41405, WO2008/99415; EP 2189443; EP 2189440; WO2010/80537; US2005/26917; Luci, D. et al., Heterocycles 2004, 62, 1, 543-558; Berardi, f., et al., Bioorganic and Medical Chemistry, 2001, 9, 5, 1325 - 1336; Singh, C. et al., J. Med. Chem., 2008, 51, 23, 7561-7592. Gangjee, A. et al., Bioorganic and Medical Chemistry, 2006, 14, 24, 8341-8351; Sauerberg, P. et al.,J. Med. Chem., 2003, 46, 23, 4883- 4894) Das Reaktionsprodukt aus Stufe a) wird verseift, in das Säurechlorid überführt und anschließend amidiert, wie in der WO 2011/61330 für den dortigen Strukturtyp 3 beschrieben.

### Syntheseweg 2:

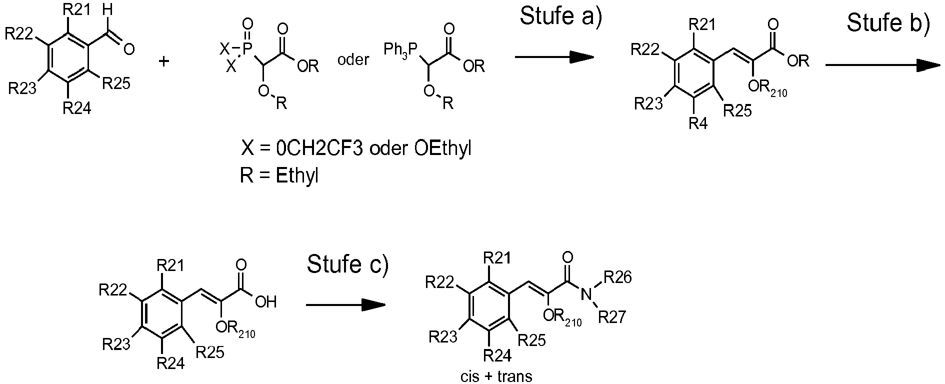

Stufe a) wird nach dem Prinzip einer Horner-Emmons Reaktion durchgeführt, indem man den aromatische Aldehyd mit einer geeigneten Phosphonoverbindung umsetzt (vgl. z.B. Ebdrup, S. et al, Journal of Medical Chemistry, 2003,46, Nr.8, 1306-1317; US2010/240636; WO 2008/108602; WO2004/20420; WO2004/31162; WO2004/41275; WO2009/46606; US6,258,850; US 2003/18207; WO2008/10238; US2010/144884; Siu, T. et al., Angewandte Chemie 2001, 113, 4849-4852). Das Reaktionsprodukt aus Stufe a) wird verseift, in das Säurechlorid überführt und anschließend amidiert (wie z.B. beschrieben in: Fürstner, A., et al., 2006, JACS, 128, 24, 8087-8094; Fürstner, A., et al., 2005, JACS, 127, 33, 11620-11621, US 6,362,210 ; Takada, K. et al., Bioorganic and Medical Chemistry Letters, 2010, 20, 4, 1330-1333; US 3,072,649).

### Syntheseweg 3:

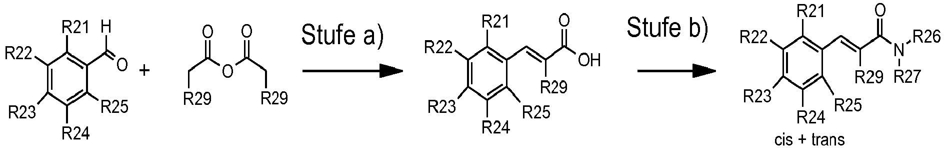

In Stufe a) wird nach dem Prinzip einer Perkin-Reaktion der aromatische Aldehyd mit einem Dicarbonsäureanhydrid in Gegenwart einer Base zur Zimtsäurezwischenstufe umgesetzt (vgl. z.B. J.R. Johnson, Org. React. 1942, 1, 210-266; Hagishita, S. et al., Journal of medical Chemistry 1996, 39, 19, 3636-3658; Müller et al., 1944, Chemische Berichte, 77/79, 766-774; Verma, et al., Medical Chemistry Research, 2004, 13, 8-9, 660-676; US 2003/232862; EP-A-1577290; Chiriac, C. et al., Revue Romaine de Chimie, 2005, 50, 7-8, 627-631; v. Miller, Chemische Berichte, 1890, 23, 1899, Hamer, D. et ;al, J. Chem. Soc., 1964, 1847-1848;). Das Reaktionsprodukt aus Stufe a) wird verseift, in das Säurechlorid überführt und anschließend amidiert, wie in der WO 2011/61330 für den dortigen Strukturtyp 3 beschrieben.

### Syntheseweg 4:

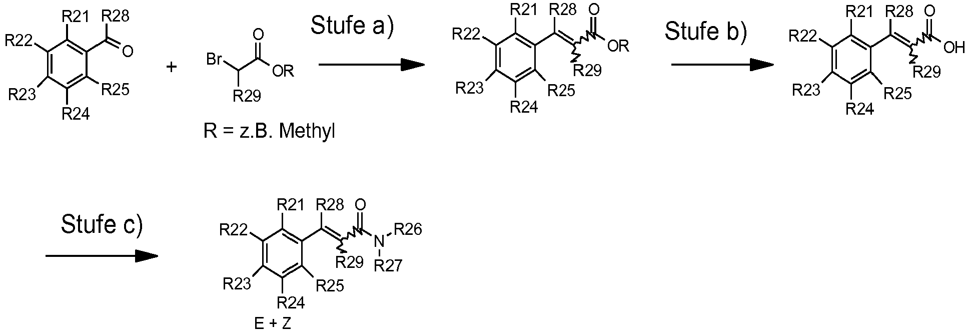

In Stufe a) wird nach dem Prinzip einer Reformatsky-Reaktion das aromatische Keton mit Brom-substituierten Carbonsäureester in Gegenwart von Zink in die ungesättigten aromatischen Esterzwischenstufe überführt (vgl. z.B. S. Reformatsky, 1887, Ber. Dtsch. Chem. Ges. 20, 1210-1211, R.L. Shriner, 1946, Org Reaction, 1, 434-460). Das Reaktionsprodukt aus Stufe a) wird verseift, in das Säurechlorid überführt und anschließend amidiert, wie in der WO 2011/61330 für den dortigen Strukturtyp 3 beschrieben.

### Syntheseweg 5:

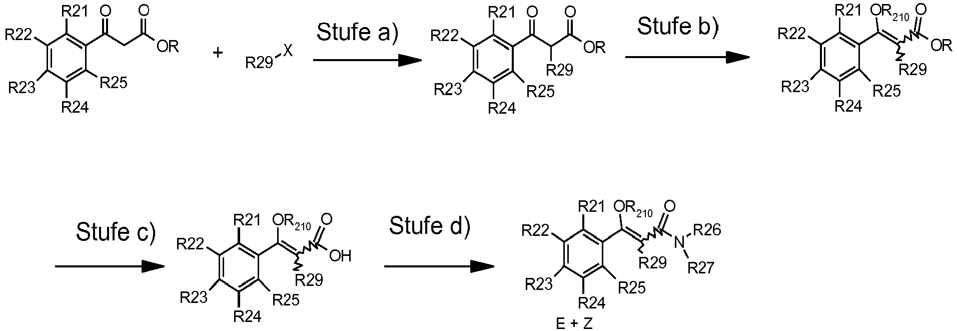

Die Umsetzung der aromatischen β-Keto-Carbonsäure gemäß Stufe a) erfolgt in an sich bekannter Weise. Sie ist für verschiedene Reaktanden im Stand der Technik beschrieben (vgl. z.B. Xiao, Y., 2009 chemical communications, 3594-3596; Li, H. et al Organic Letters 2009, 4176 - 4179;Chen Y.-F. et al., 2010 Advanced Synthesis and Catalysis 352, 7, 1163-1168; Wang, H. et al., Tetrahedron letters 2005, 46, 6, 963 - 966; Bunce, R. A., et al.; Journal of Heterocyclic Chemistry; 2007, 44; 5; 1051 - 1057; Gomez, V. et al., Journal of Organic Chemistry; 1994, 59, 5, 1219 - 1221; Okimoto, M., et al., Synlett; 2005, 16, 2507 - 2509 ;Lou, S. et al., Journal of Organic Chemistry; 2007, 72, 26; 9998 - 10008; Emelina, E. E. et al., Zhurnal Organicheskoi Khimii 1987, 23, 12, 2565 - 2570,2263- 2268; Gompper, R. et al., Chemische Berichte; 1981, 114, 8, 2866 - 2883; Bunce, R. et al., Journal of Organic Chemistry, 1993, 58, 25, 7143 - 7148; Yang, T. et al.,Journal of the American Chemical Society, 2009, 131, 26, 9140 - 9141;Tanikaga, R. et al., Chemistry Letters, 1985),1583 - 1586; Renaud, J.-L. et al., Tetrahedron, 1999, 55, 16, 5113 - 5128 ; Verhe, R. et al., Tetrahedron, 1982, 38, 24, 3649 - 3660 ; Pfleger, et al., Chemische Berichte, 1957, 90, 2404-2409; Temnikova,T.I. et al., Zhurnal Organicheskoi Khimii, 1966, 2, 7, 1168 - 1171,1160 - 1162; Ershov,B.A. et al.; Zhurnal Organicheskoi Khimii;1969, 5, 1378 - 1383,1346 - 1350). Verschiedene Methoden zur Durchführung der Stfe b) sind ebenfalls aus dem Stand der techik bekannt;(vgl. z.B. Gompper, R, et al., Chemische Berichte, 1981, 114, 8, 2866 - 2883; Arndt et al, Chemische berichte 1938, 71, 1631 - 1639; Claisen, Justus Liebigs Annalen der Chemie, 1917, 413, 274). Nach Verseifung und Überführung in das Saurechloriderfolg anschließend eine Amidierung zum Endprodukt (wie z.B. beschrieben in: Fürstner, A., et al., 2006, JACS, 128, 24, 8087-8094; Fürstner, A., et al., 2005, JACS, 127, 33, 11620-11621, US6,362,210 ; Takada, K. et al., Bioorganic and Medical Chemistry Letters, 2010, 20, 4, 1330-1333; US 3,072,649).

### Syntheseweg 6:

Die Herstellung von α,β-gesättigten Formen von Verbindungen der allgemeine Formel III erfolgt beispielsweise durch gezielte Reduktion der entspechenden ungesättigten, nach einem der Synthesewege 1 bis 5 erhaltenen Vorstufe (Variante a)) (vgl. Desai et al., Tetrahdron Lett., 5963-5965, 2001) oder durch Amidierung von an sich bekannten α,β-gesättigten Carbonsäure-Vorstufen wie z.B. in der WO 2011/61330 für den dortigen Strukturtyp 3 beschrieben und ggf in der Anwesenheit von Basen, wie NEt₃, (Variante b)).

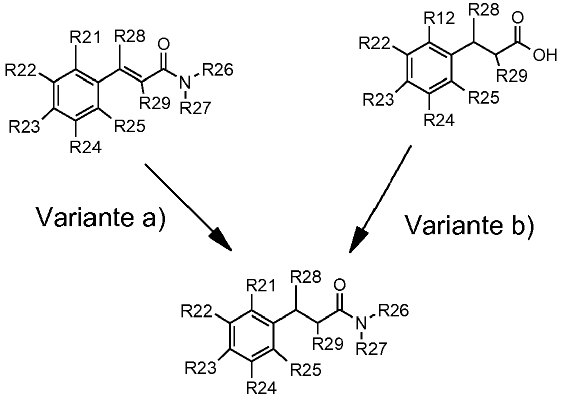

Weitere Aspekte der vorliegenden Erfindung ergeben sich aus den nachfolgenden Beispielen sowie den beigefügten Patentansprüchen.

### Experimenteller Teil

Die folgenden Beispiele dienen zur Verdeutlichung der Erfindung. Sofern nicht anders angegeben, sind alle Gehaltsangaben auf das Gewichts bezogen. Soweit keine anderen Angaben gemacht werden, werden zur Durchführung der folgenden Experimente übliche, dem jeweiliegn Fachmannn auf dem Gebiet der organischen Synthese, der Formulierung und der Biochemie geläufige Methoden eingesetzt.

Die Wirkstoffe der Formeln I, II und III sind entweder an sich bekannte Verbindungen oder können in Anlehnung an bekannte Synthesemethoden vom Fachmann auf dem Gebiet der organischen Synthese hergestellt werden.

Die Erfindung wird nun anhand folgender Ausführungsbeispiele beschrieben.

### 1. Referenzbeispiele

### Referenzbeispiel 1 - Klonierung von humanem TRPM8

Ausgangspunkt für die Klonierung des humanen TRPM8 Rezeptors ist eine LnCaP cDNA-Bank. Diese ist beispielsweise kommerziell erhältlich (z. B. Firma BioChain, Hayward, USA) oder aus der androgensensitiven humanen Prostata-Adenokarzinomzelllinie LnCaP (z.B. ATCC, CRL1740 oder ECACC, 89110211) unter Verwendung von Standardkits herstellbar.

Die kodierende TRPM8 Sequenz (WO 2010/026094; sowie **http://www.ncbi.nlm.nih.gov/entrez/viewer.fcgi?db=nuccore&id=109689694)** kann unter Verwendung von Standardmethoden PCR-amplifiziert und kloniert werden. Das so isolierte humane TRPM8 Gen wurde zur Herstellung des Plasmids plnd_M8 verwendet, dessen Konstruktion durch die Plasmidkarte gemäß Figur 2 der WO 2010/026094 veranschaulicht wird.

Alternativ dazu kann das TRPM8 Gen auch synthetisch hergestellt werden.

### Referenzbeispiel 2 - Generierung der HEK293-Testzellen

Als Testzellsystem wurde mit der humanen TRPM8 DNA (vgl obiges Plasmid plnd-M8) stabil transfizierte HEK293 Zelllinie hergestellt. Bevorzugt wird dabei HEK293 die über das eingebrachte Plasmid die Möglichkeit zur Induktion der TRPM8-Expression mittels Tetrazyklin bietet.

Methoden zur Herstellung geeigneter Testzellsysteme sind dem Fachmann bekannt. So kann man die Herstellung der erfindungsgemäß verwendeten Zellen den Angaben in Behrendt H.J. et al.,, Br. J. Pharmacol. 141, 2004, 737-745 oder der Dissertation von Behrendt "Vergleichende funktionale Untersuchungen des Hitze-Capsaicin-Rezeptors (TRPV1) und des Kälte-Menthol-Rezeptors (TRPM8) in rekombinanten und nativen Zellssystemen", zugänglich unter http://www-brs.ub.ruhr-uni-bochum.de/netahtml/HSS/Diss/BehrendtHansJoerg/diss.pdf entnehmen.

### Referenzbeispiel 3- Assay auf TRPM8 Modulatoren

Es wird ein Test, vergleichbar mit dem bereits in der Literatur beschrieben Test von Behrendt H.J. et al., Br. J. Pharmacol. 141, 2004, 737-745, durchgeführt. Die Agonisierung bzw. Antagonisierung des Rezeptors kann mittels eines Ca²⁺-sensitiven Farbstoffs (z.B. FURA, Fluo-4 etc.) quantifiziert werden. Agonisten bewirken alleine eine Zunahme des Ca²⁺-Signals; Antagonisten bewirken in Gegenwart von z.B. Menthol eine Reduzierung des Ca²⁺-Signals (jeweils detektiert über den Farbstoff Fluo-4, der durch Ca²⁺ andere Fluoreszenzeigenschaften hat).

Zunächst wird in an sich bekannter Weise in Zellkulturflaschen eine frische Kultur transformierter HEK-Zellen hergestellt. Die Testzellen HEK293-TRPM8 werden mittels Trypsin von den Zellkulturflaschen abgelöst und 40.000 Zellen/well werden mit 100 µl Medium in 96-Lochplatten (Greiner # 655948 Poly-D-Lysin-beschichtet) ausgesät. Zur Induktion des Rezeptors TRPM8 wird dem Wachstumsmedium Tetrazyklin beigemischt (DMEM/HG, 10% FCS tetrazyklinfrei, 4 mM L-Glutamin, 15 µg/ml Blasticidin, 100 µg/ml Hygromycin B, 1 µg/ml Tetrazyklin). Am darauffolgenden Tag werden die Zellen mit Fluo-4AM Farbstoff beladen und der Test wird durchgeführt. Dazu wird wie folgt vorgegangen:
- Zugabe von je 100 µl/well Färbelösung Ca-4 Kit (RB 141, Molecular Devices) zu je 100 µl Medium (DMEM/HG, 10% FCS tetrazyklinfrei, 4 mM L-Glutamin, 15 µg/ml Blasticidin, 100 µg/ml Hygromycin B, 1 µg/ml Tetrazyklin)
- Inkubation im Brutschrank, 30 Minuten / 37°C / 5% CO₂, 30 Minuten / RT
- Vorbereitung der Testsubstanzen (unterschiedliche Konzentrationen in 200 µl HBSS-Puffer), sowie von Positivkontrollen (unterschiedliche Konzentrationen von Menthol, Icilin bzw. lonomycin in 200 µl HBSS-Puffer) und Negativkontrollen (nur 200 µl HBSS-Puffer)
- Zugabe der Testsubstanzen in Mengen von 50 µl/well und Messung der Fluoreszenzänderung (z.B. im Assaygerät FLIPR, Molecular Devices oder NovoStar, BMG) bei 485 nm Anregung, 520 nm Emission, und Auswertung der Wirkstärke der verschiedenen Substanzen/Konzentrationen und Bestimmung der EC50-Werte

Die Testsubstanzen werden in Triplikaten in Konzentrationen von 0,1 - 200 µM im Assay eingesetzt. Normalerweise werden die Verbindungen in DMSO-Lösungen bereitgehalten und für den Assay auf eine maximale DMSO-Konzentration von 2% herunterverdünnt.

Die Auswertung zeigt überraschenderweise, dass erfindungsgemäß erstmalig Agomisten von TRPM8 bereitgestellt werden konnten, welche sich strukturell von bisher bekannten Agonisten, wie (-) Menthol, Icilin und anderen von Behrendt H.J. et al.,in Br. J. Pharmacol. 141, 2004, 737-745 (vgl dortige Tabelle 1) beschriebenen Modulatoren, signifikant unterscheiden und zudem teilweise bessere Aktivitäten als (-) Menthol zeigen, bzw. vergleichbar stark wirken wie Icilin.

Es folgen einige konkrete Herstellungsbeispiele für Verbindungen der obigen allgemeinen Formel I:

### 2. Herstellungsbeispiele

### Herstellungsbeispiel H1-8: Herstellung der Vorstufe V1

analog Szantay et al., Heterocycles, 1977, 1793

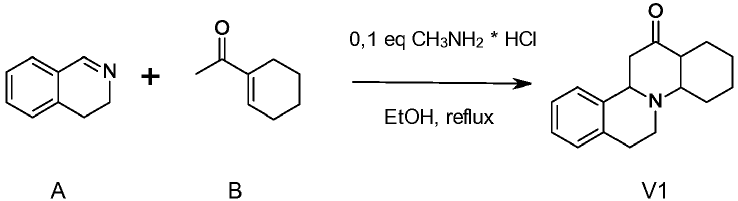

15 g (0,11 mol, 1,0 eq) 3,4-Dihydroisochinolin (A), 28,4 g (0,23 mol, 2,0 eq) of 1-Acetylcyclohexen (B), 0,77 g (0,011 mol, 0,1 eq) of Methylammoniumchlorid und 150 ml Ethanol werden zusammengegeben und für 64 h auf Rückfluss erhitzt. Die Reaktionsmischung wird aufkonzentriert und der Rückstand mit 100 ml iPrOH versetzt. Die warme Lösung wird angeimpft und es werden über 15 min 8 ml Wasser zugegeben. Die Mischung wird auf 0-5°C abgekühlt und filtriert. Der Feststoff wird mit 20 ml iPrOH gewaschen und über Nacht im Stickstoffstrom getrocknet.

Ausbeute (V1): 15,7 g (54 %) mit einer HPLC-Reinheit von 99 Fl-%
CAS-Nummer Produkt: 1424-91-5

### Herstellungsbeispiel H1-9: Herstellung der Vorstufe V2

analog Szantay et al., Heterocycles, 1977, 1793

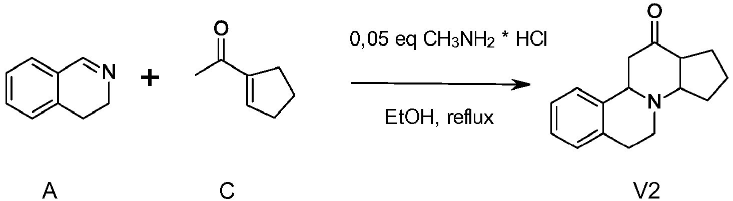

5 g (38 mmol, 1,0 eq) 3,4-Dihydroisochinolin (A), 4,6 g (42 mmol, 1,1 eq) 1-Acetylcyclopenten (C), 0,13 g (1,9 mmol, 0,05 eq) Methylammoniumchlorid und 63 ml Ethanol werden zusammengegeben und für 14 Tage unter Rückfluss erhitzt. Die Reaktionsmischung wird aufkonzentriert und mit 60 ml 1 N wässriger HCl und 50 ml EtOAc versetzt. Die Phasen werden getrennt, die organische Phase verworfen und die wässrige Phase tropfenweise mit 25 % wässriger Natriumhydroxid-Lösung versetzt bis ein pH-Wert von 9-10 erreicht wird. Der auskristallisierte Feststoff wird abfiltriert und mit 100 ml Wasser gewaschen. Er wird im Stickstoffstrom über Nacht getrocknet und aus EtOH umkristallisiert.

Ausbeute (V2): 2,6 g Feststoff mit einer HPLC-Reinheit von 100 Fl-%.

CAS-Nr. Produkt: 1424-86-5

### Herstellungsbeispiel H1-10: Herstellung von Verbindung P1

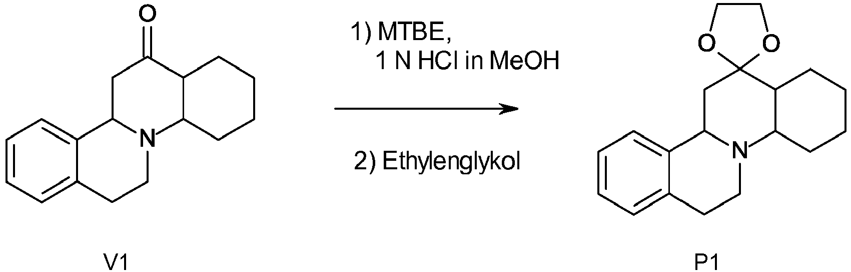

1 g (3,9 mmol, 1 eq) Keton V1 werden mit 5 ml MTBE und anschließend mit 4,8 ml 1 N HCl in Methanol versetzt. Die entstehende Lösung wird in der Kälte aufkonzentriert und anschließend mit 5 ml Ethylenglykol versetzt. Es wird nochmals andestilliert und die Lösung anschließend 5 h bei 75°C gerührt. Es wird auf Raumtemperatur abgekühlt und die Lösung mit 5 ml gesättigter Natriumcarbonatlösung und 10 ml Wasser versetzt. Die organische Phase wird abgetrennt und die wässrige Phase mit 5 ml EtOAc nachextrahiert. Die vereinigten organischen Phasen werden 3 mal mit je 25 ml Wasser gewaschen und anschließend über Natriumsulfat getrocknet. Das Lösungsmittel wird unter vermindertem Druck entfernt und der Rückstand aus Petrolether kristallisiert. Die Kristalle werden abgesaugt und im Vakuum getrocknet.

Ausbeute (P1): 710 mg (61 %) mit einer HPLC-Reinheit von 92,4 Fl-%
¹H-NMR (CDCl₃): 7,2-7,0 (m, 4 H); 4,13-3,90 (m, 4 H); 3,69 (d, 1 H); 3,42-3,33 (m, 1 H); 3,08-2,94 (m, 1 H); 2,83-2,70 (m, 1 H); 2,43-2,15 (m, 4 H); 1,90-1,59 (m, 5 H); 1,40-1,13 (m, 4 H) [ppm]

### Herstellungsbeispiel H1-11: Herstellung von Verbindung P2

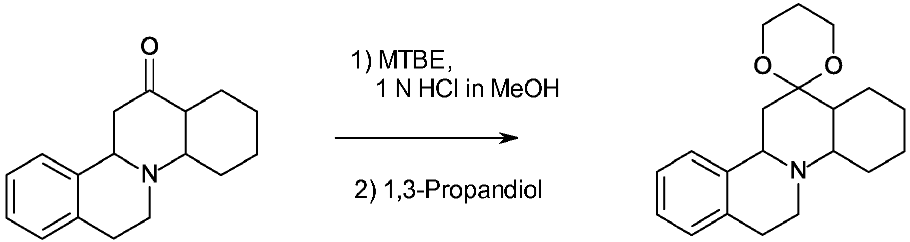

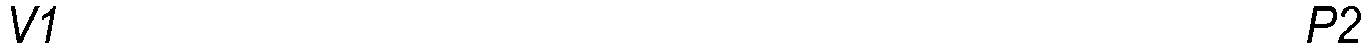

1 g (3,9 mmol, 1 eq) Keton V1 werden mit 5 ml MTBE und anschließend mit 4,8 ml 1 N HCl in Methanol versetzt. Die entstehende Lösung wird in der Kälte aufkonzentriert und anschließend mit 7,5 ml 1,3-Propandiol versetzt. Es wird nochmals andestilliert und die Lösung anschließend 45 h bei 75°C gerührt. Es wird auf Raumtemperatur abgekühlt und die Lösung mit 5 ml gesättigter Natriumcarbonatlösung und 10 ml Wasser versetzt. Die organische Phase wird abgetrennt und die wässrige Phase mit 5 ml EtOAc nachextrahiert. Die vereinigten organischen Phasen werden 3 mal mit je 25 ml Wasser gewaschen und anschließend über Natriumsulfat getrocknet. Das Lösungsmittel wird unter vermindertem Druck entfernt und der Rückstand säulenchromatographisch gereinigt.

Ausbeute (P2): 890 mg (73 %) mit einer HPLC-Reinheit von 82,9 Fl-% (enthält ca. 15 % Startmaterial)
¹H-NMR (CDCl₃): 7,25-6,95 (m, 4 H); 4,16-4,05 (m, 2 H); 3,92-3,72 (m, 2 H); 3,63 (d, 1 H); 3,36-3,18 (m, 2 H); 3,05-2,75 (m, 2 H); 2,44-2,34 (m, 1 H); 2,20-1,97 (m, 3 H); 1,83-1,68 (m, 2 H); 1,54-1,08 (m, 8 H) [ppm]

### Herstellungsbeispiel H1-12: Herstellung von Verbindung P3

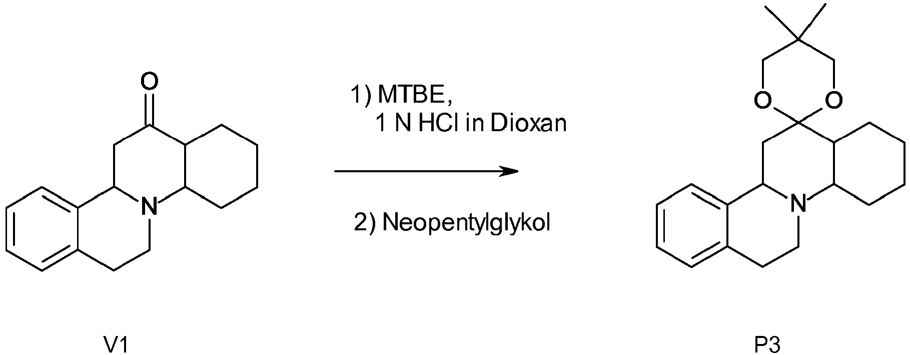

1 g (3,9 mmol, 1 eq) Keton V1 werden mit 2 ml MTBE und anschließend mit 1,5 ml 1,25 N HCl in Dioxan versetzt. Die entstehende Lösung wird am Rotationsverdampfer aufkonzentriert und anschließend mit 1,2 g (3 eq) Neopentylglykol und 3 ml DMSO in einen Reaktionskolben überführt. Es wird eine Woche bei 70-75°C gerührt. Zur Aufarbeitung werden 10 ml gesättigte Natriumcarbonatlösung, 20 ml Wasser und 10 ml EtOAc zugesetzt. Die organische Phase wird 2 mal mit je 10 ml Wasser gewaschen, über Natriumsulfat getrocknet und am Rotationsverdampfer eingeengt. Der Rückstand wird säulenchromatographisch gereinigt.

Ausbeute (P3): 450 mg (34 %) mit einer HPLC-Reinheit von 90,8 FI-%.
¹H-NMR (CDCl₃): 7,20-7,03 (m, 4 H); 3,83 (tr, 2 H), 3,62 (d, 1 H); 3,44 (d, 1 H); 3,234 (d, 2 H); 3,17 (d, 1 H); 3,05 (m, 1 H); 2,83-2,75 (m, 1 H); 2,46-2,35 (m, 2 H); 2,21-2,11 (m, 2 H); 1,85-1,71 (m, 2 H); 1,58-1,08 (m, 6 H); 1,22 (s, 3 H), 0,78 (s, 3 H) [ppm]

### Herstellungsbeispiel H1-13: Herstellung von Verbindung P4

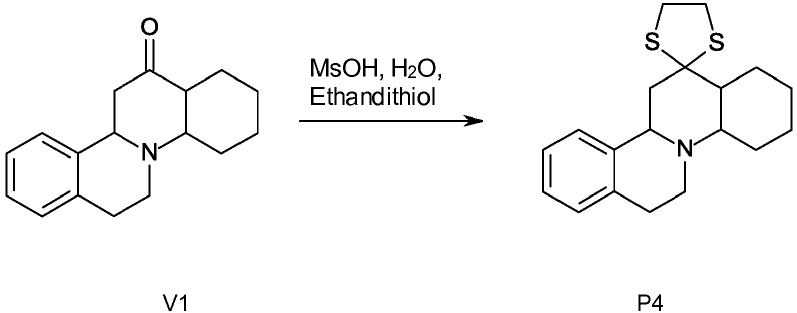

1,3 g Wasser werden vorgelegt und langsam mit 2,3 g (6 eq) Methansulfonsäure versetzt. Es werden 0,55 g (1,5 eq) Ethandithiol zugegeben und anschließend 1 g (3,9 mmol) Keton V1. Die Reaktionsmischung wird über Nacht bei Raumtemperatur gerührt und langsam zu einer Mischung aus 12 ml Toluol und 5 ml 25 % NaOH gegeben. Es wird 5 min gerührt und anschließend werden die Phasen getrennt. Die organische Phase wird dreimal mit je 5 ml Wasser gewaschen, eingeengt und der Rückstand aus Heptan/MTBE unter Zusatz von wenigen Tropfen iPrOH kristallisiert.

Ausbeute (P4): 580 mg (45 %) mit einer HPLC-Reinheit von 93,4 FI-%
¹H-NMR (CDCl₃): 7,24-6,99 (m, 4 H); 3,70 (d, 1 H); 3,43-3,15 (m, 5 H); 3,05-2,93 (m, 1 H); 2,76 (d, 2 H); 2,40-2,10 (m, 5 H); 1,88-1,62 (m, 3 H); 1,52-1,37 (m, 1 H); 1,37-1,12 (m, 3 H) [ppm]

### Herstellungsbeispiel H1-14: Herstellung von Verbindung P5

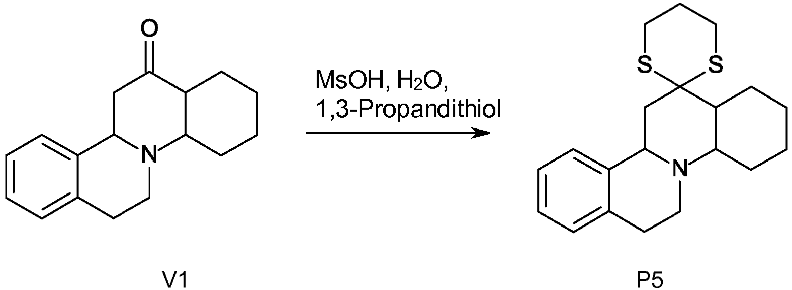

1,3 g Wasser werden vorgelegt und langsam mit 2,3 g (6 eq) Methansulfonsäure versetzt. Es werden 0,64 g (1,5 eq) 1,3-Propandithiol zugegeben und anschließend 1 g (3,9 mmol) Keton V 1. Die Reaktionsmischung wird über Nacht bei Raumtemperatur gerührt und danach langsam zu einer Mischung aus 12 ml Toluol und 5 ml 25 % NaOH gegeben. Es wird 5 min gerührt und anschließend werden die Phasen getrennt. Die organische Phase wird eingeengt und der Rückstand säulenchromatographisch gereinigt.

Ausbeute (P5): 605 mg (45 %) mit einer HPLC-Reinheit von 93,8 FI-%.

¹H-NMR (CDCl₃): 7,20-7,00 (m, 4 H); 4,06 (d, 1 H); 3,40-3,29 (m, 1 H); 3,29-3,18 (m, 1 H); 3,18-3,04 (m, 2 H); 3,04-2,92 (m, 1 H); 2,84-2,55 (m, 4 H); 2,49-2,37 (m, 1 H); 2,35 (d, 1 H); 2,26-2,00 (m, 3 H); 1,93-1,72 (m, 3 H); 1,72-1,61 (m , 1 H); 1,57-1,42 (m, 1 H); 1,38-1,02 (m, 3 H) [ppm]

### Herstellungsbeispiel H1-15: Herstellung von Verbindung P6

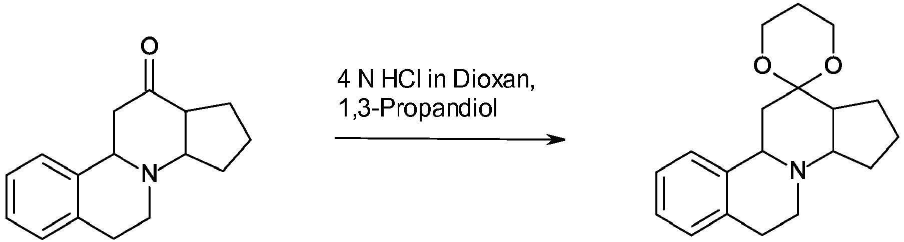

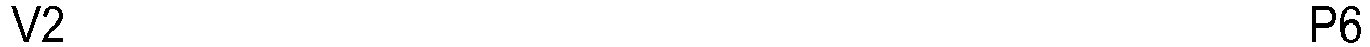

0,8 g Keton V2 werden mit 40 ml 1,3-Propandiol und 3 ml 4 N HCl in Dioxan versetzt und 4 Tage bei RT gerührt. Es werden 20 ml MTBE, 20 ml ges. Natriumhydrogencarbonat-Lösung und 80 ml Wasser zugegeben und 5 min gerührt. Die Phasen werden getrennt, die wässrige Phase 2 x mit je 20 ml MTBE nachextrahiert und die vereinigten organischen Phasen 2 mal mit je 40 ml Wasser gewaschen. Die MTBE-Phase wird über Natriumsulfat getrocknet und einrotiert.

Ausbeute (P6): 1,1 g mit einer HPLC-Reinheit von 95,9 FI-% (85:15-Isomerengemisch).
¹H-NMR (CDCl₃): 7,30-7,00 (m, 4 H); 4,11-3,38 (m, 5 H); 3,27-3,14 (m, 1 H); 3,10-2,97 (m, 1 H); 2,79-2,45 (m, 4 H); 2,26-1,53 (m, 10 H) [ppm]

### Herstellungsbeispiel H1-16: Herstellung von Verbindung P7

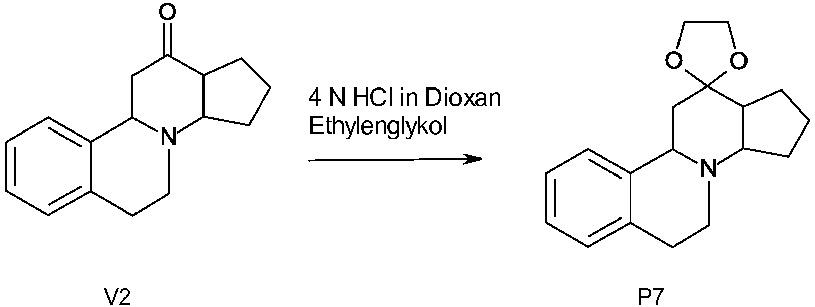

0,8 g Keton V2 werden mit 40 ml Ethylenglykol und 3 ml 4 N HCl in Dioxan versetzt und 4 Tage bei RT gerührt. Es werden 20 ml MTBE, 20 ml ges. Natriumhydrogencarbonat-Lösung und 80 ml Wasser zugegeben und 5 min gerührt. Die Phasen werden getrennt, die wässrige Phase 2 x mit je 20 ml MTBE nachextrahiert und die vereinigten organischen Phasen 2 mal mit je 40 ml Wasser gewaschen. Die MTBE-Phase wird über Natriumsulfat getrocknet und einrotiert.

Ausbeute (P7): 0,97 g mit einer HPLC-Reinheit von 97,2 FI-%.
¹H-NMR (CDCl₃): 7,26-7,00 (m, 4 H); 4,11-3,92 (m, 4 H); 3,58 (d, 1H); 3,28-3,18 (m, 1 H); 3,10-2,95 (m, 1 H); 2,78-2,72 (m, 1 H); 2,68 d, 1 H); 2,24 (d, 1 H); 2,13 (tr, 1 H); 2,07-1,52 (m, 8 H) [ppm]

### Herstellungsbeispiel H1-17: Herstellung der Vorstufe V3

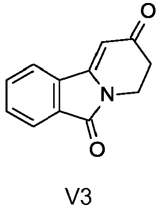

Die Herstellung erfolgt analog zu Flitsch und Pandl, Liebigs Ann. Chem., 1987, 649.
CAS-Nr (Produkt): 108292-84-8

### Herstellungsbeispiel H1-18: Herstellung von Verbindung P8

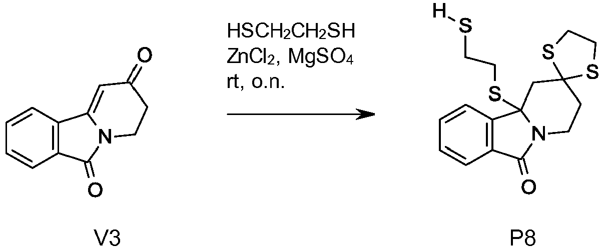

254 mg (1,28 mmol) Keton V3 und 256 mg Magnesiumsulfat werden in 1,3 ml (12 eq) Ethandithiol suspendiert und unter Rühren mit 256 mg (1,5 eq) ZnCl₂ versetzt. Es wird für zwei Tage bei Raumtemperatur gerührt und die Reaktionsmischung anschließend mit 40 ml Dichlormethan und 40 ml Wasser versetzt. Die wässrige Phase wird abgetrennt und noch zweimal mit je 40 ml Dichlormethan extrahiert. Die vereinigten organischen Phasen werden über Natriumsulfat getrocknet und am Rotationsverdampfer aufkonzentriert. Das gewonnene Rohprodukt wird säulenchromatographisch über Kieselgel mit Heptan/EtOAc als Laufmittel gereinigt.

Ausbeute (P8): 334 g (71 %) zähes farbloses Öl mit einer HPLC-Reinheit von 100 FI-%
¹H-NMR (CDCl₃): 7,82 (d, 1 H); 7,68-7,55 (m, 1 H); 7,50 (tr, 1 H); 4,38 (d, 1 H); 3,56-3,24 (m, 5 H); 2,94 (d, 1 H); 2,32 (d, 1 H); 2,29-1,85 (m, 6 H); 1,47 (tr, 1 H), [ppm]

### Herstellungsbeispiel H1-19: Herstellung von Verbindung P9

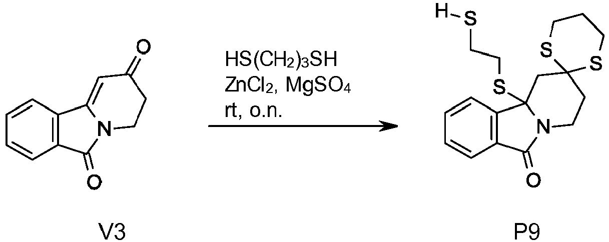

210 mg (1,05 mmol) Keton V3 und 210 mg Magnesiumsulfat werden in 1,3 ml (12 eq) 1,3-Propandithiol suspendiert und unter Rühren mit 210 mg (1,5 eq) ZnCl₂ versetzt. Es wird 64 h bei Raumtemperatur gerührt und die Reaktionsmischung anschließend mit 40 ml Dichlormethan und 40 ml Wasser versetzt. Die wässrige Phase wird abgetrennt und noch zweimal mit je 40 ml Dichlormethan extrahiert. Die vereinigten organischen Phasen werden über Natriumsulfat getrocknet und am Rotationsverdampfer aufkonzentriert. Das gewonnene Rohprodukt wird säulenchromatographisch über Kieselgel mit Heptan/EtOAc als Laufmittel gereinigt.

Ausbeute (P9): 229 mg (55 %) farbloses Harz mit einer HPLC-Reinheit von 100 FI-%
¹H-NMR (CDCl₃): 7,83 (d, 1 H); 7,68-7,53 (m, 2 H); 7,48 (tr, 1 H); 4,32 (d, 1 H); 3,59 (tr, 1 H); 3,05-2,91 (m, 3 H); 2,91-2,77 (m, 2 H); 2,58 (d, 1 H); 2,40-2,28 (m, 2 H); 2,13-1,88 (m, 4 H); 1,85-1,72 (m, 2 H); 1,53-1,37 (m, 2 H); 1,13 (tr, 1 H) [ppm]

### Herstellungsbeispiel H1-20: Herstellung der Vorstufe Z1

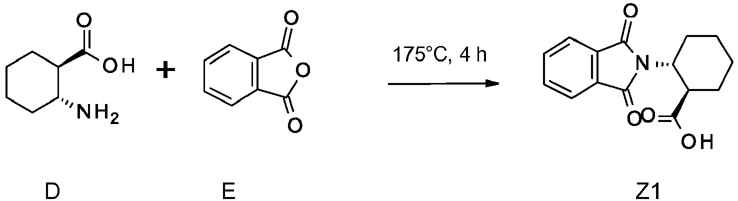

Eine Mischung aus 14,8 g (100 mmol) Phthalsäureanhydrid (E) und 14,3 g (100 mmol) (1R, 2R)-2-Aminocyclohexancarbonsäure (D) wird 4 h unter Rühren auf 175°C erhitzt. Es wird auf Raumtemperatur abgekühlt und das zähe Öl in 400 ml Dichlormethan aufgenommen. Die organische Phase wird dreimal mit je 60 ml 0,1 N HCl gewaschen, über Natriumsulfat getrocknet und am Rotationsverdampfer eingeengt. Das resultierende zähe, hellgelbe Öl wird mit 50 ml Heptan verrührt. Der entstehende weiße Feststoff wird abfiltriert, 2 mal mit je 5 ml Heptan gewaschen und im Vakuum getrocknet.

Ausbeute (Z1): 27,3 g (100 %) mit einer HPLC-Reinheit von 100 %.
CAS-Nr (Produkt): 755025-03-7
¹H-NMR (CDCl₃): 11,2 -9,3 (br s, 1 H); 7,82-7,73 (m, 2 H); 7,73-7,57 (m, 2 H); 4,30 (tr, 1 H); 3,44 (tr, 1 H); 2,20-1,99 (m, 2 H); 1,99-1,28 (m, 6 H) [ppm]

### Herstellungsbeispiel H1-20: Herstellung der Vorstufe Z2

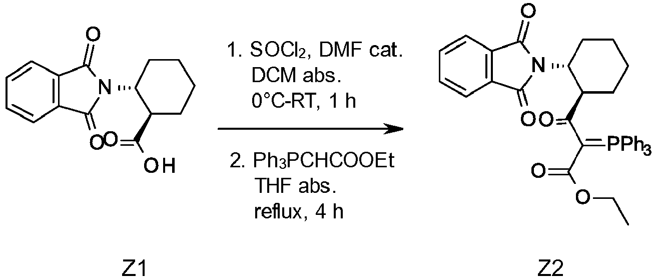

6,83 g (25 mmol) 2-Phthalimidocyclohexancarbonsäure (Z1) werden in 250 ml trockenem Dichlormethan gelöst und mit mit 0,19 ml DMF versetzt. Die Lösung wird auf 0°C bis -10°C abgekühlt und bei dieser Temperatur innerhalb von 20 min mit 29,7 g (250 mmol, 10 eq) Thionylchlorid versetzt. Es wird auf RT erwärmt, die Reaktionsmischung eine Stunde bei RT nachgerührt und am Rotationsverdampfer eingeengt. Das Säurechlorid wird in THF aufgenommen und zu einer Lösung von 17,4 g (50 mmol, 2,0 eq) Triphenylphosphoraniliden-essigsäureethylester in 150 ml THF bei 19-27°C innerhalb von 90 min zugetropft. Anschließend wird die Reaktionslösung für 8 h refluxiert, wobei ein weißer Feststoff ausfällt und sich die Reaktionslösung tiefrot färbt. Es wird auf RT abgekühlt, der ausgefallene Feststoff über eine Fritte abfiltriert und der Filterkuchen mit THF nachgewaschen. Die vereinigten Mutter- und Waschlaugen werden am Rotationsverdampfer eingeengt, um das Produkt als roten Schaum zu isolieren.

Ausbeute (Z2): 18,8 g mit einer HPLC-Reinheit von 55 FI-%.

### Herstellungsbeispiel H1-22: Herstellung der Vorstufe Z3

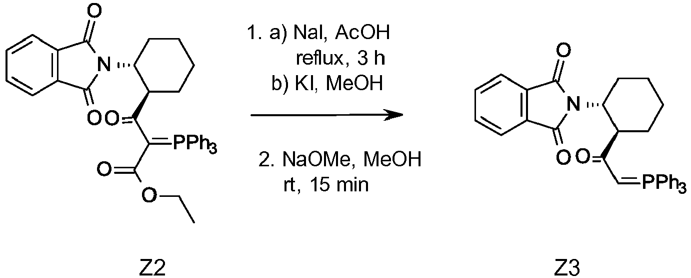

15 g (∼20 mmol) Phosphoran Z2 werden in 60 ml Eisessig gelöst und in Gegenwart von 6 g (40 mmol, 2,0 eq) Natriumiodid für 3 h unter Rückfluss erhitzt. Die dunkelrote Reaktionslösung wird am Rotationsverdampfer eingeengt, der rotbraune Rückstand in 40 ml Methanol aufgenommen und zu einer Lösung von 12 g (72 mmol, 3,6 eq) Kaliumiodid in 120 ml Wasser gegeben. Die wässrige Phase wird mit Dichlormethan (3 x 60 ml) extrahiert und die vereinten org. Phasen mit Wasser (2 x 100 ml) gewaschen und über Natriumsulfat getrocknet. Die org. Lösung wird am Rotationsverdampfer bis auf ein Volumen von 20-40 ml eingeengt und mit der gleichen Menge Heptan versetzt. Es wird weiter im Vakuum aufkonzentriert, wobei ein braungelber Schaum zurückbleibt.

Der Schaum wird in 40 ml Methanol gelöst und unter Rühren bei RT über einen Tropftrichter mit einer Mischung aus 16 ml MeOH und 3,9 ml NaOMe-Lösung (30% in Me-OH) versetzt. Anschließend wird 15 min bei RT gerührt. Die dunkelbraune MeOH-Lösung wird auf 200 ml Eis/Wasser-Gemisch gegossen, wodurch nach kräftigem Rühren ein brauner Schleim ausfällt. Die wässrige Lösung wird abdekantiert und der Rest filtriert. Der dabei anfallende braune Rückstand wird in Dichlormethan (ca. 150 ml) gelöst und etwaige Wasserreste werden über einen Scheidetrichter abgetrennt. Die Dichlormethan-Phase wird über Natriumsulfat getrocknet und einrotiert.

Ausbeute (Z3): 10,02 g (94 %) braunweißer Schaum

### Herstellungsbeispiel H1-23: Herstellung der Vorstufe V4

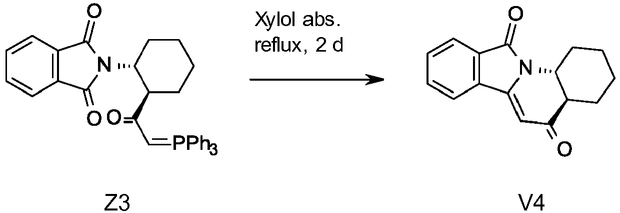

8 g (15 mmol) Phosporan Z3 werden mit 500 m Xylol 2 Tage unter Rückfluss gerührt. Das Lösungsmittel wird am Rotationsverdampfer abgezogen und das verbliebene braune Öl über Kieselgel gesäult. Es werden 1,75 g Produkt isoliert, die aus Ethanol umkristallisiert werden.

Ausbeute (V4): 1,0 g (26 %) mit einer HPLC-Reinheit von 92,3 % und 391 mg mit einer HPLC-Reinheit von 63 %.

¹H-NMR (CDCl₃): 7,88 (d, 1 H); 7,77 (d, 1 H); 7,74-7,60 (m, 2 H); 6,07-5,98 (m, 1 H); 4,70-4,59 (m, 1 H); 2,95 (s, 1 H); 2,68-2,55 (m, 1 H); 1,98-1,84 (m, 1 H); 1,84-1,68 (m, 1 H); 1,58 (d, 1 H); 1,50-1,32 (m, 3 H); 1,32-1,17 (m, 1 H) [ppm]

### Herstellungsbeispiel H1-24: Herstellung von Verbindung P10

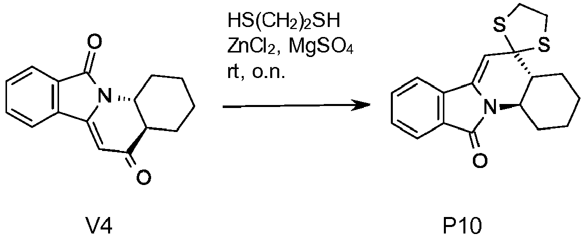

500 mg (1,97 mmol) Tetracyclus V4 und 400 mg (1,7 eq) Magnesiumsulfat werden in 2 ml (12 eq) Ethandithiol suspendiert und unter Rühren bei RT mit 400 mg (1,5 eq) Zinkchlorid versetzt. Es wird 42 h bei RT gerührt und anschließend zur Aufarbeitung mit 50 ml Dichlormethan und 50 ml Wasser verdünnt. Die wässrige Phase wird abgetrennt und noch zweimal mit je 50 ml Dichlormethan (2 x 50 ml) extrahiert. Die vereinigten organischen Phasen werden über Natriumsulfat getrocknet und am Rotationsverdampfer aufkonzentriert. Das resultierende zähflüssige gelbe Öl wird säulenchromatographisch gereinigt.

Ausbeute (P10): 683 mg hellgelbes zähes Öl mit einer HPLC-Reinheit von 82 FI-%
¹H-NMR (CDCl₃): 7,80-7,73 (m, 1 H); 7,62-7,39 (m, 3 H), 5,90 (s, 1 H); 4,39 (s, 1 H); 3,51-3,35 (m, 3 H); 3,35-3,21 (m, 1 H); 2,20-1,05 (m, 9 H) [ppm]

### Herstellungsbeispiel H1-25: Herstellung von Verbindung P11 und P12

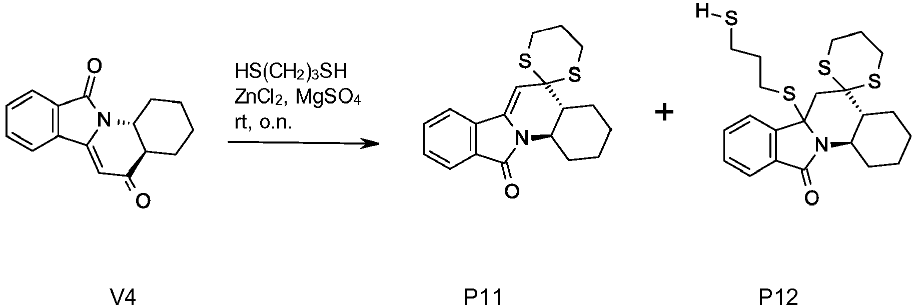

269 mg (1,06 mmol) Tetracyclus V4 und 212 mg (1,7 eq) Magnesiumsulfat werden in 1,3 ml (12 eq) 1,3-Propandithiol suspendiert und unter Rühren bei RT mit 212 mg (1,5 eq) Zinkchlorid versetzt. Es wird für 64 h bei RT gerührt und anschließend zur Aufarbeitung mit 50 ml Dichlormethan und 50 ml Wasser verdünnt. Die wässrige Phase wird abgetrennt und noch zweimal mit je 50 ml Dichlormethan extrahiert. Die vereinigten organischen Phasen werden über Natriumsulfat getrocknet und am Rotationsverdampfer aufkonzentriert. Das resultierende gelbe Öl wird säulenchromatographisch gereinigt.

Ausbeute (P11): 63 mg (17 %) Diastereomer 1
73 mg (20 %) Diastereomer 2
Ausbeute (P12): 151 mg (31 %)

Diastereomer 1 von P11
¹H-NMR (CDCl₃): 7,78 (d, 1 H); 7,61 (d, 1 H); 7,54 (tr, 1 H); 7,47 (tr, 1 H); 5,79 (s, 1 H); 4,64 (s, 1 H); 3,06 (tr, 1 H), 2,97-2,70 (m, 3 H); 2,30 (d, 1 H); 2,23 -1,04 (m, 10 H) [ppm]

Diastereomer 2 von P11
¹H-NMR (CDCl₃): 7,80 (d, 1 H); 7,66 (d, 1 H); 7,55 (tr, 1 H); 7,47 (tr, 1 H); 6,49 (s, 1 H); 3,97 (tr, 1 H); 3,79 (d, 1 H); 3,40 (tr, 1 H); 3,11 (tr, 1 H); 2,97-2,89 (m, 1 H); 2,84-2,74 (m, 1 H); 2,44 (d, 1 H); 2,22-2,15 (m, 1 H); 2,03-1,17 (m, 8 H) [ppm]

P12:
¹H-NMR (CDCl₃): 7,78 (d, 1 H); 7,67-7,51 (m, 2 H); 7,44 (tr, 1 H); 4,02 (tr, 1 H); 3,38 (d, 1 H); 3,02 (tr, 1 H); 2,93-2,70 (m, 3 H); 2,70-2,57 (m, 4 H); 2,57-2,46 (m, 1 H); 2,44-1,18 (m, 15 H); 1,12 m, 1H) [ppm]

### Herstellungsbeispiel H1-26: Herstellung der Vorstufe V5

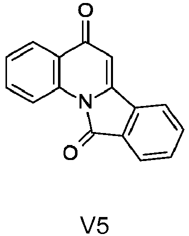

Die Herstellung erfolgt analog zu Helv. Chim Acta, 2011, 94,1703-1717.

### Herstellungsbeispiel H1-27: Herstellung von Verbindung P13

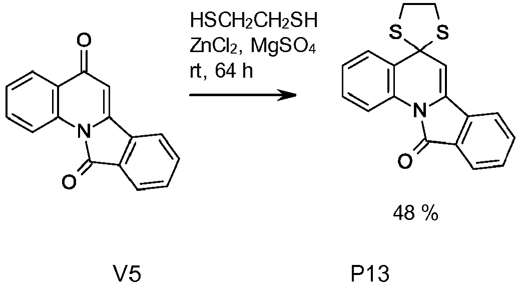

990 mg (4,0 mmol) Tetracyclus V5 und 800 mg (1,7 eq) Magnesiumsulfat werden in 4,0 ml (12 eq) Ethandithiol suspendiert und unter Rühren bei RT mit 800 mg (1,5 eq) Zinkchlorid versetzt. Es wird für 64 h bei RT gerührt und anschließend zur Aufarbeitung mit 80 ml Dichlormethan und 80 ml Wasser verdünnt (Produkt teilweise unlöslich). Die wässrige Phase wird abgetrennt und die organische Phase aufkonzentriert. Der Rückstand wird in iPrOH/Wasser digeriert, abgesaugt und mit wenig iPrOH gewaschen. Es wird im Stickstoffstrom getrocknet und der Rückstand nochmals in MeOH digeriert, abgesaugt und getrocknet.

Ausbeute (P13): 880 mg (68 %) gelber Feststoff mit einem Gehalt von ca. 70 % (enthält nach NMR noch ca. 30 % Startmaterial)
¹H-NMR (DMSO-d₆): 8,30-8,16 (m, 1 H); 8,04 (d, 1 H); 8,01 (d, 1 H); 7,91-7,78 (m, 1 H); 7,75-7,68 (m, 1 H); 7,60 (tr, 1 H); 7,53-7,44 (m, 1 H); 6,92 (s, 1 H); 4,20-4,02 (m, 4 H) [ppm]

### Herstellungsbeispiel H1-28: Herstellung der Vorstufe Z4

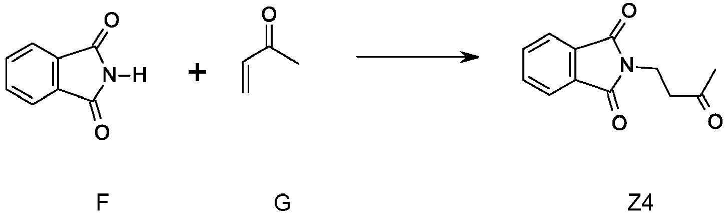

CAS: 3783-77-5

Die Herstellung erfolgt nach: A. M. Islam, R. A. Raphael, J. Chem. Soc. 1955, 3151-3154.

### Herstellungsbeispiel H1-29: Herstellung der Vorstufe Z5

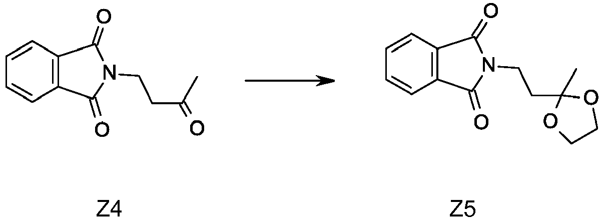

CAS: 87764-41-0

Die Herstellung erfolgt in Anlehnung an:
Bosch, Joan; Rubiralta, Mario; Moral, Montserrat; Arino, Joaquin. Journal of the Chemical Society, Perkin Transactions 1: Organic and Bio-Organic Chemistry (1972-1999), 1986, (8), 1533-9.

### Herstellungsbeispiel H1-30: Herstellung von Verbindung P14

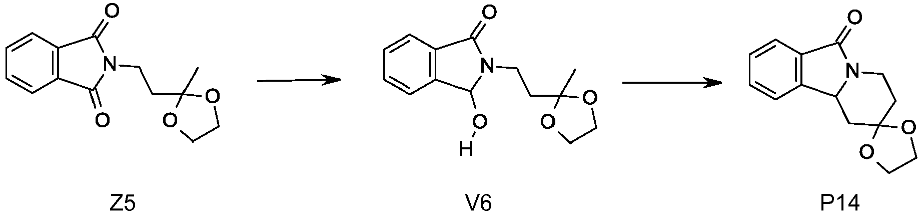

CAS (Intermediat; V6): 1174751-64-4
CAS (Produkt, P14): 817554-52-2
Hergestellt analog: M. Chiurato, S. Routier, Y. Troin, G. Guillaumet, Eur. J. Org. Chem 2009, 3011-3021

### Herstellungsbeispiel H1-31: Herstellung der Vorstufe Z6

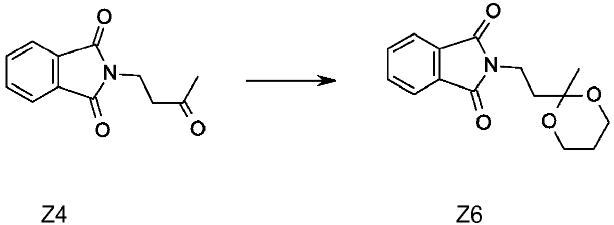

5,43 g (25 mmol) 4-Phthalimidobutan-2-on Z4 werden in 150 ml Toluol gelöst, mit 4,28 g (56,25 mmol, 2,25 eq.) 1,3-Propandiol und 0,24 g (1,25 mmol, 0,05 eq) para-Toluolsulfonsäure Monohydrat versetzt und 24 h am Wasserabscheider unter Rückfluss erhitzt. Es wird auf RT abgekühlt und 3 mal mit je 30 ml ges. Natriumhydrogencarbonatlösung gewaschen. Die vereinigten wässrigen Phasen werden einmal mit 90 ml EtOAc extrahiert und die vereinigten organischen Phasen über Natriumsulfat getrocknet und am Rotationsverdampfer aufkonzentriert. Das erhaltene farblose Öl wird aus Heptan kristallisiert.

Ausbeute (Z6): 7,09 g mit einer HPLC-Reinheit von 88 FI-%
¹H-NMR (CDCl₃): 7,89-7,80 (m, 2 H); 7,74-7,66 (m, 2H); 3,97-3,80 (m, 6 H); 2,11 (tr, 2 H); 1,84-1,72 (m, 1 H); 1,66-1,56 (m, 1 H); 1,48 (s, 3 H) [ppm]

### Herstellungsbeispiel H1-32: Herstellung der Vorstufe V7

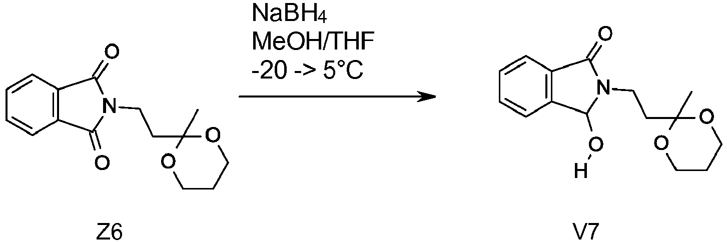

Unter Stickstoffatmosphäre werden 3,64 g (12,5 mmol) Verbindung Z6 vorgelegt und in einer Mischung aus 100 ml MeOH und 125 ml THF gelöst. Es wird auf -15°C abgekühlt und es werden portionsweise 0,71 g (18,75 mmol, 1,5 eq) Natriumborhydrid zugegeben. Es wird 30 min bei -15°C nachgerührt und anschließend innerhalb von 1 h auf 0-5°C erwärmt.

Zur Aufarbeitung werden 30 ml Wasser und 75 ml Natriumhydrogencarbonat-Lösung zugegeben und das Gemisch 3 mal mit je 150 ml Dichlormethan und einmal mit 250 ml EtOAc extrahiert. Die vereinigten organischen Phasen werden über Natriumsulfat getrocknet und anschließend am Rotationsverdampfer aufkonzentriert. Das resultierende hellrote Öl wird säulenchromatographisch gereinigt.

Ausbeute (V7): 3,20 g (87 %, farbloses Öl);
¹H-NMR (CDCl₃): 7,66-7,55 (m, 3 H); 7,52 (tr, 1 H); 6,45 (d, 1 H); 5,83 (d, 1 H); 3,91-3,77 (m, 4 H); 3,69-3,59 (m, 1 H); 3,43-3,34 (m, 1H); 2,08-1,87 (m, 2 H); 1,70-1,47 (m, 2 H); 1,37 (s, 3 H) [ppm]

### Herstellungsbeispiel H1-33: Herstellung von Verbindung P15

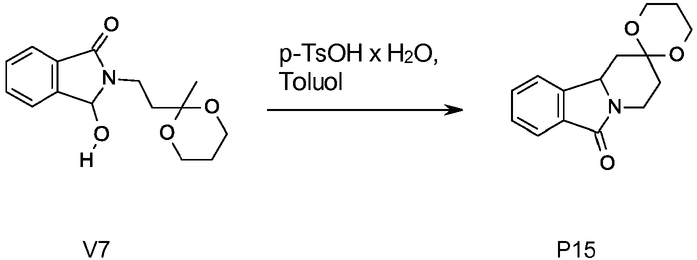

2,77 g (10 mmol) Verbindung V7 werden mit 200 ml Toluol und 3,57 g (15 mmol, 1,5 eq.) para-Toluolsulfonsäure Monohydrat versetzt und 24 h am Wasserabscheider refluxiert. Die Reaktionsmischung wird auf RT abgekühlt und mit gesättigter NaHCO₃-Lösung gewaschen. Die wässrige Phase wird 2 mal mit je 200 ml Dichlormethan extrahiert. Die vereinigten organischen Phasen werden über Natriumsulfat getrocknet, filtriert und am Rotationsverdampfer aufkonzentriert. Das Rohprodukt wird säulenchromatographisch gereinigt.

Ausbeute (P15): 321 mg (12 %, hellgelbes Öl, Produktgehalt nach ¹³C-NMR: 55-60 %)

### Herstellungsbeispiel H1-34: Herstellung von Verbindung P16

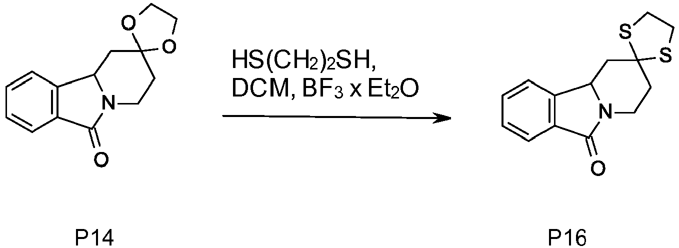

470 mg (1,92 mmol) Verbindung P14 werden in 25 ml Dichlormethan gelöst und bei RT langsam mit 0,9 g (10 mmol, 5 eq.) Ethandithiol und 1,36 g (10 mmol, 5 eq.) Bortrifluorid-Diethyletherat versetzt. Es wird 18 h bei RT gerührt und anschließend die Reaktionsmischung auf 25 ml 2 N NaOH gegeben. Die organische Phase wird abgetrennt und die wässrige Phase 3 mal mit je 50 ml Dichlormethan extrahiert. Die vereinigten organischen Phasen werden über Magnesiumsulfat getrocknet, filtriert und das Lösemittel unter vermindertem Druck entfernt.

Ausbeute (P16): 532 mg (100 %)
¹H-NMR (CDCl₃): 7,89-7,24 (m, 4 H); 4,59-4,44 (m, 2 H); 3,51-3,32 (m, 4 H); 3,25 tr, 1 H); 2,70 d, 1 H); 2,20 (d, 1 H); 2,07-1,96 (m, 1 H); 1,62 (tr, 1 H) [ppm]

### Herstellungsbeispiel H1-35: Herstellung von Verbindung P17

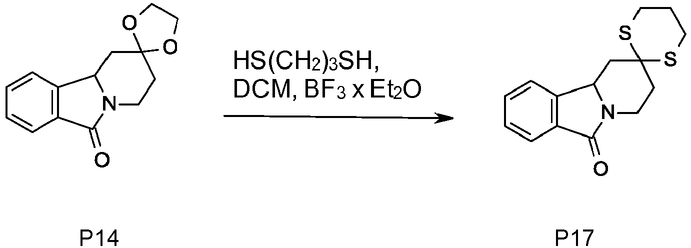

470 mg (1,92 mmol) Verbindung P14 werden in 25 ml DCM gelöst und bei RT langsam mit 1,04 g (9,6 mmol, 5 eq) 1,3-Propandithiol und 1,36 g (9,6 mmol, 5 eq.) Bortrifluorid-Diethyletherat versetzt. Es wird 18 h bei RT gerührt und anschließend die Reaktionsmischung auf 25 ml 2 N NaOH gegeben. Die organische Phase wird abgetrennt und anschließend die wässrige Phase 3 mal mit je 50 ml Dichlormethan extrahiert. Die vereinigten organischen Phasen werden über Magnesiumsulfat getrocknet, filtriert und das Lösemittel unter vermindertem Druck entfernt.

Ausbeute (P17): 578 mg (104 %)
¹H-NMR (CDCl₃): 7,89-7,25 (m, 4 H); 4,76 (d, 1 H); 4,41-4,30 (m, 1 H); 3,40 (tr, 1 H); 3,01-2,81 (m, 5 H); 2,50-2,37 (m, 1 H); 2,07 (br s, 2 H); 1,77 (m, 1 H); 1,45 (tr, 1 H) [ppm]

### Herstellungsbeispiel H1-36: Herstellung der Vorstufe V8

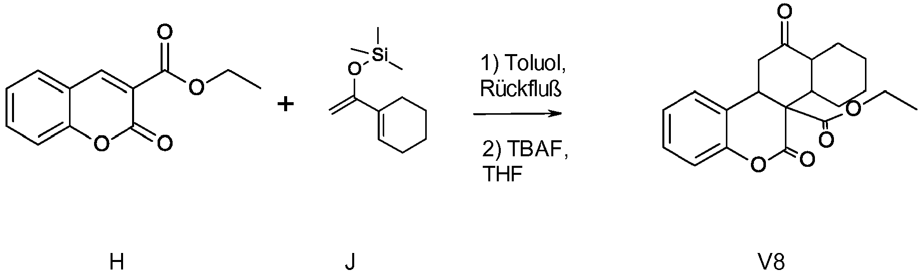

23 g (105 mmol) Ethyl-3-cumarincarboxylat (H) und 34,9 g (141 mmol, 1,34 eq) 1-Cyclohex-1-enyl-vinyloxy-trimethyl-silan (J) werden in 438 ml Toluol gelöst und 22 h am Rückfluss gekocht. Die Reaktionsmischung wird eingeengt, mit 231 ml THF und anschließend bei 16°C mit 43,2 g (1,30 eq) Tetrabutylammoniumfluorid versetzt. Es wird 15 Minuten bei RT nachgerührt. Es werden 500 ml Wasser und 250 ml MTBE zugegeben, die Mischung wird gut gerührt und die Phasen getrennt Die wässrige Phase wird mit 100 ml MTBE nachextrahiert. Die vereinigten organischen Phasen werden mit 500 ml Wasser gewaschen und anschließend am Rotationsverdampfer eingeengt. Das resultierende Öl wird mit n-Heptan versetzt, die erhaltene Kristallmaische 15 min gerührt und abgesaugt. Der Filterrückstand wird mit 50 ml iPrOH nachgewaschen und anschließend im Stickstoffstrom getrocknet.

Ausbeute (V8): 25,5 g (71 %) mit einer HPLC-Reinheit 97,9 FI-% (zwei Diastereo-mere 78:18)
¹H-NMR (CDCl₃, Hauptisomer): 7,35-7,04 (m, 4 H); 4,14-3,93 (m, 2 H); 3,56 (dd, 1 H); 2,75-2,55 (m, 2H); 2,50-2,36 (m, 2 H); 2,17-2,03 (m, 2 H); 1,88-0,93 (tr, 3 H) [ppm]

### Herstellungsbeispiel H1-37: Herstellung der Vorstufe V9

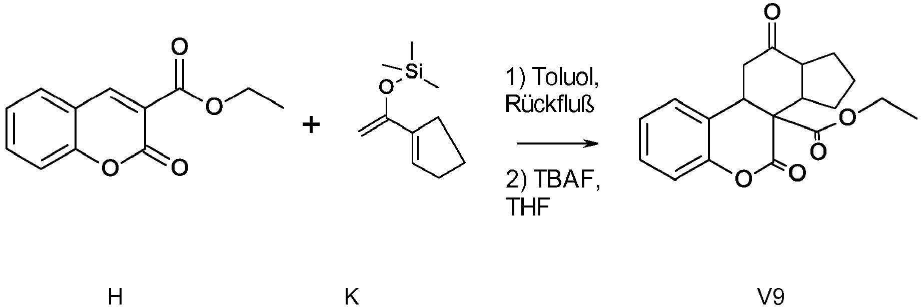

14 g (64,2 mmol) Ethyl-3-cumarincarboxylat (H) und 22,8 g (89,8 mmol, 1,40 eq) 1-Cyclopent-1-enyl-vinyloxy)-trimethyl-silan (K) werden in 266 ml Toluol gelöst und 24 h am Rückfluss gekocht. Die Reaktionsmischung wird einrotiert, mit 140 ml THF und bei 16°C mit 26,3 g (83,4 mmol, 1,30 eq) Tetrabutylammoniumfluorid versetzt. Es wird 15 Minuten bei RT nachgerührt. Es werden 304 ml Wasser und 152 ml MTBE zugegeben, die Mischung gut gerührt und die Phasen getrennt. Die wässrige Phase wird mit 61 ml MTBE nachextrahiert. Die vereinigten organischen Phasen werden mit 304 ml Wasser gewaschen und anschließend am Rotationsverdampfer einrotiert. Das resultierende Öl wird mit 31 g Isopropanol versetzt, auf 0°C abgekühlt und der ausgefallene Feststoff abgesaugt. Der Rückstand wird mit 10 ml eiskaltem i-PrOH und anschließend mit 10 ml n-Heptan bei RT gewaschen und im Argonstrom für zwei Tage getrocknet.

Ausbeute (V9): 7,1 g (34 %) mit einer HPLC-Reinheit von 91,2 FI-%
¹H-NMR (CDCl₃, zwei Isomere): 7,37-7,03 (m, 4 H, beide Isomere); 4,16-3,98 (m, 2 H, beide Isomere); 3,84 (dd, 1 H, ein Isomer); 3,62 (dd, 1 H, ein Isomer); 3,49-3,27 (m , 1 H, ein Isomer); 3,05-2,89 (m, 1 H, beide Isomere); 2,68-2,20 (m, 3 H, beide Isomere + 1 H, ein Isomer); 1,92-1,19 (m, 5 H beide Isomere); 1,06 (tr, 3 H, ein Isomer); 0,98 (tr, 3 H, ein Isomer) [ppm]

### Herstellungsbeispiel H1-38: Herstellung von Verbindung P18

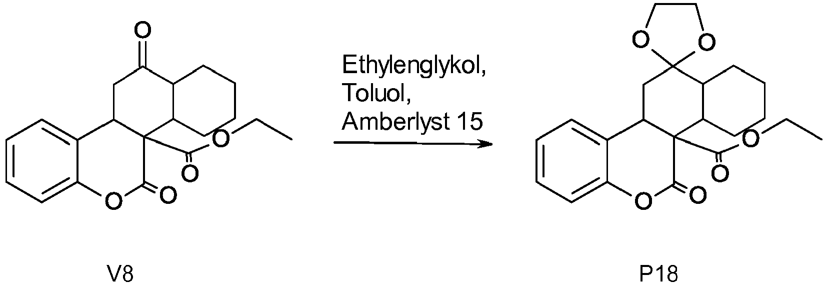

1 g (2,9 mmol) Ketoester V8 werden mit 0,19 g (3,1 mmol, 1,05 eq) Ethylenglykol, 10 ml Toluol und 0,1 g Amberlyst 15 versetzt und über Nacht am Rückfluß gekocht. Es wird filtriert, die resultierende Lösung eingeengt und der Rückstand aus Isopropanol im Eisbad kristallisiert. Es wird filtriert, der Rückstand mit wenig kaltem iPrOH gewaschen und im Trockenschrank bei 40°C getrocknet.

Ausbeute (P18): 0,54 g (48 %) mit einer HPLC-Reinheit von 92,9 FI-%
¹H-NMR (DMSO-d₆): 7,35-7,25 (m, 2 H); 7,17-7,05 (m, 2 H); 4,06-3,83 (m, 6 H); 3,46-3,33 (m, 1 H); 2,23-2,12 (m, 1 H); 2,04 -1,94 (m, 2 H); 1,87-1,75 (m, 2 H); 1,75 -1,65 (m, 2 H); 1,41-1,00 (m, 5 H); 1,80 (tr, 3 H); [ppm]

### Herstellungsbeispiel H1-39: Herstellung von Verbindung P19

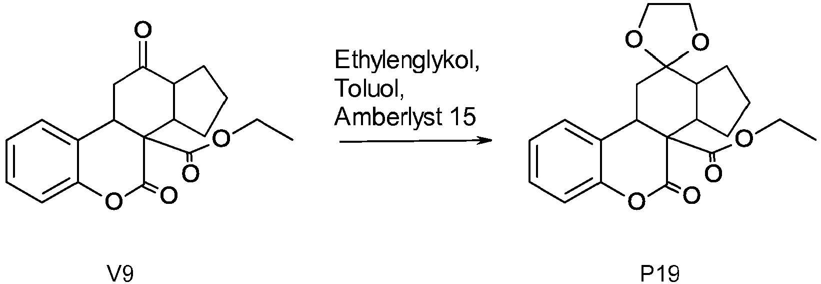

1,2 g (3,65 mmol) Ketoester V9 werden mit 0,45 g (7,3 mmol, 2,0 eq) Ethylenglykol, 12,5 ml Toluol und 0,12 g Amberlyst 15 versetzt und über Nacht am Rückfluß gekocht. Es werden 200 mg 4 Å Molsieb zugegeben und weitere 4 h am Rückfluss erhitzt. Es wird filtriert, die resultierende Lösung zweimal mit je 20 ml Wasser gewaschen und am Rotationsverdampfer eingeengt.

Ausbeute (P19): 1,22 g (90 %) mit einer HPLC-Reinheit von 90,2 FI-%
¹H-NMR (CDCl₃): 7,31-6,96 (m, 4 H, beide Isomere); 4,11-3,84 (m, 6 H, beide Isomere); 3,73 (dd, 1 H, ein Isomer); 3,47 (dd, 1 H, ein Isomer); 3,19-3,09 (m, 1 H, ein Isomer); 2,48-2,15 (m, 2 H, beide Isomere); 1,99-1,32 (m, 6 H, beide Isomere + 1 H ein Isomer); 1,05 (tr, 1 H, ein Isomer); 0,97 (m, 1H, ein Isomer) [ppm]

### Herstellungsbeispiel H1-40: Herstellung von Verbindung P20

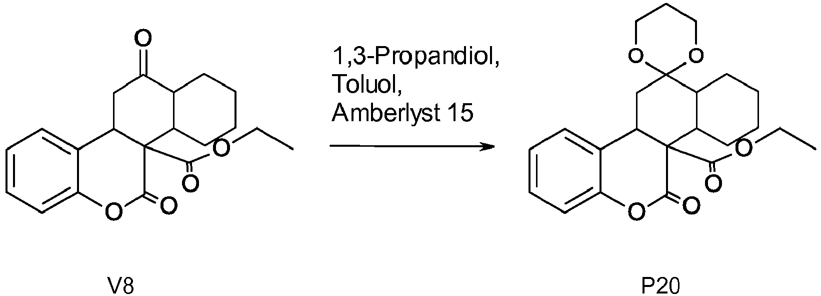

1 g (2,9 mmol) Ketoester V8 werden mit 0,23 g (3,1 mmol, 1,05 eq) 1,3-Propandiol, 10 ml Toluol und 0,1 g Amberlyst 15 versetzt und über Nacht am Rückfluß gekocht. Es wird filtriert, die resultierende Lösung eingeengt und aus Isopropanol im Eisbad kristallisiert. Es wird filtriert, der Rückstand mit wenig kaltem iPrOH gewaschen und im Trockenschrank bei 40°C getrocknet.

Ausbeute (P20): 0,65 g (56 %) mit einer HPLC-Reinheit von 73 FI-%
¹H-NMR (DMSO-d₆): 7,41-7,28 (m, 2 H); 7,20-7,05 (m, 2 H); 4,14-3,82 (m, 5 H); 3,68-3,61 (d, 1 H); 3,24 (d, 1 H); 3,09 (m, 1 H); 2,50-0,91 (m, 13 H); 0,78 (tr, 1H) [ppm]

### Herstellungsbeispiel H1-41: Herstellung von Verbindung P21

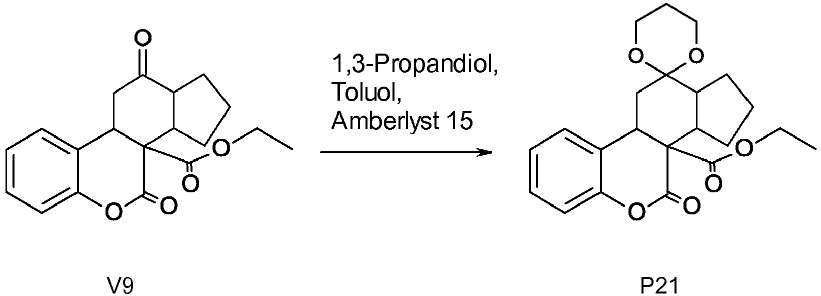

1,2 g (3,65 mmol) Ketoester V9 werden mit 0,56 g (7,3 mmol, 2,0 eq) 1,3-Propandiol, 12,5 ml Toluol und 0,12 g Amberlyst 15 versetzt und über Nacht am Rückfluß gekocht. Es werden 200 mg 4 Å Molsieb und nochmals 0,12 g Amberlyst 15 zugegeben und weitere 88 h am Rückfluss erhitzt. Es wird filtriert und die resultierende Lösung einrotiert. Der Rückstand wird in THF aufgenommen, mit 10 ml 1,3-Propandiol versetzt, aufkonzentriert und nach Zugabe von 0,12 g Amberlyst 15 über Nacht bei 110 °C gerührt. Es wird abgekühlt, mit 250 ml Wasser und 100 ml Toluol versetzt, die organische Phase abgetrennt und noch 2 mal mit je 30 ml Wasser gewaschen. Die organische Phase wird aufkonzentriert und säulenchromatographisch gereinigt

Ausbeute (P21): 0,40 g (28 %) mit einer HPLC-Reinheit von 90,8 FI-%
¹H-NMR (CDCl₃): 7,19-6,98 (m, 4 H, beide Isomere); 4,08-3,87 (m, 5 H, beide Isomere); 3,81-3,72 (m, 1 H, beide Isomere); 3,58 (dd, 1 H, ein Isomer); 3,33 (dd, 1 H, ein Isomer); 2,99-2,83 (m, 1 H, ein Isomer); 2,44-1,20 (m, 11 H, beide Isomere); 1,06 (tr, 1 H, ein Isomer); 0,97 (m, 1H, ein Isomer) [ppm]

### Herstellungsbeispiel H1-40: Herstellung von Verbindung P22

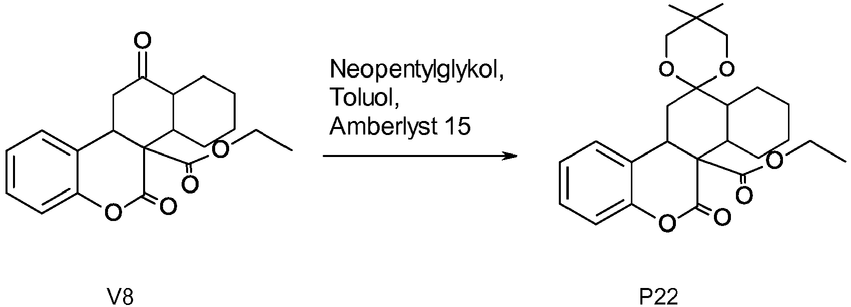

1 g (2,9 mmol) Ketoester V8 werden mit 0,32 g (3,1 mmol, 1,05 eq) Neopentylglykol, 10 ml Toluol und 0,1 g Amberlyst 15 versetzt und über Nacht am Rückfluß gekocht. Es wird filtriert, die resultierende Lösung eingeengt und der Rückstand aus Isopropanol im Eisbad kristallisiert. Es wird filtriert, der Filterkuchen mit wenig kaltem iPrOH gewaschen und im Trockenschrank bei 40°C getrocknet.

Ausbeute (P22): 0,78 g (62 %) mit einer HPLC-Reinheit von 81,7 FI-%
¹H-NMR (CDCl₃): 7,41-7,28 (m, 2 H); 7,20-7,05 (m, 2 H); 4,07-3,87 (m, 3 H); 3,82 (d, 1 H); 3,68 (d, 1 H); 2,24-1,98 (m, 3 H); 1,78-1,64 (m, 3 H); 1,50-1,00 (m, 8 H); 0,97-0,86 (m, 1 H); 0,78 (tr, 3 H); 0,70 (s, 3 H) [ppm]

### Herstellungsbeispiel H1-43: Herstellung von Verbindung P23

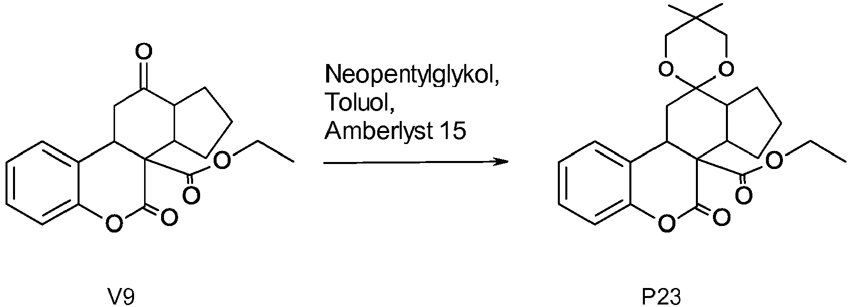

1,2 g (3,65 mmol) Ketoester V9 werden mit 0,56 g (7,3 mmol, 2,0 eq) Neopentylglykol, 12,5 ml Toluol und 0,12 g Amberlyst 15 versetzt und über Nacht am Rückfluß gekocht. Es werden 200 mg 4 Å Molsieb zugegeben und weitere 4 h am Rückfluss erhitzt. Es wird filtriert und die resultierende Lösung einrotiert. Der Rückstand wird aus wenig MTBE/Heptan-Gemisch mit ein paar Tropfen Isopropanol im Eisbad kristallisiert. Es wird filtriert und der Filterrückstand mit wenig Heptan gewaschen und im Stickstoffstrom getrocknet.

Ausbeute (P23): 1,25 g (83 %) mit einer HPLC-Reinheit von 88,2 FI-% (zwei Isomere)
¹H-NMR (CDCl₃): 7,19-6,98 (m, 4 H, beide Isomere); 4,08-3,87 (m, 5 H, beide Isomere); 3,81-3,72 (m, 1 H, beide Isomere); 3,58 (dd, 1 H, ein Isomer); 3,33 (dd, 1 H, ein Isomer); 2,99-2,83 (m, 1 H, ein Isomer); 2,44-1,20 (m, 11 H, beide Isomere); 1,06 (tr, 1 H, ein Isomer); 0,97 (m, 1H, ein Isomer) [ppm]

### Herstellungsbeispiel H1-44: Herstellung von Verbindung P24

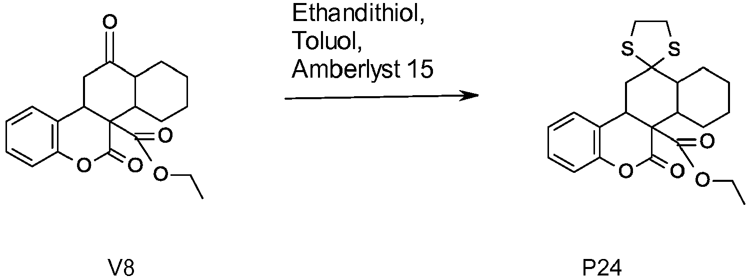

1,0 g (2,9 mmol) Ketoester V8 werden mit 0,55 g (5,8 mmol, 2,0 eq) Ethandithiol 10 ml Toluol und 0,1 g Amberlyst 15 versetzt und 24 h bei 60°C gerührt. Es werden 0,4 g 4 Å Molsieb zugegeben und eine weitere Stunde bei 60°C gerührt.. Es wird filtriert und die resultierende Lösung eingeengt Der Rückstand wird in Dichlormethan aufgenommen, die Lösung mit Wasser und zweimal mit NaHCO₃-Lösung. gewaschen und die organische Phase einrotiert.

Ausbeute (P24): 0,94 g (77 %) mit einer HPLC-Reinheit von 92,9 FI-%
¹H-NMR (CDCl₃);: 7,32-6,95 (m, 4 H); 4,13-3,88 (m, 4 H); 3,48 (d, 1 H); 3,35-3,17 (m, 4 H); 2,43-2,22 (m, 3 H); 2,17-2,04 (m, 2 H); 1,91 (tr, 1 H); 1,76 (d, 2 H); 1,51-1,28 (m, 3 H); 1,24-1,10 (m, 1 H); 0,90 (tr, 3 H) [ppm]

### Herstellungsbeispiel H1-45: Herstellung von Verbindung P25

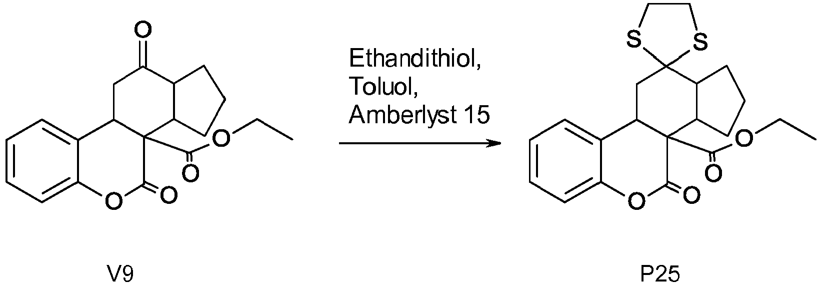

1,2 g (3,65 mmol) Ketoester V9 werden mit 0,69 g (7,3 mmol, 2,0 eq) Ethandithiol 12,5 ml Toluol und 0,12 g Amberlyst 15 versetzt und über Nacht bei 80°C gerührt. Es wird filtriert und die organische Phase zweimal mit je 15 ml ges. Natriumhydrogen-carbonat-Lösung und einmal mit Wasser gewaschen. Die organische Phase wird einrotiert und der Rückstand säulenchromatographisch gereinigt.

Ausbeute (P25): 1,20 g (81 %) mit einer HPLC-Reinheit von 93,7 FI-% (zwei Isomere)
¹H-NMR (CDCl₃): 7,30-6,96 (m, 4 H); 4,10-3,95 (m, 2 H); 3,76 (d, 1 H, ein Isomer); d, 3,43 (d, 1 H, ein Isomer); 3,40-3,06 (m, 5 H); 2,72 -1,38 (m, 9 H); 1,05 (tr, 3 H, ein Isomer); 0,97 (tr, 3 H, ein Isomer) [ppm]

### Herstellungsbeispiel H1-46: Herstellung von Verbindung P26

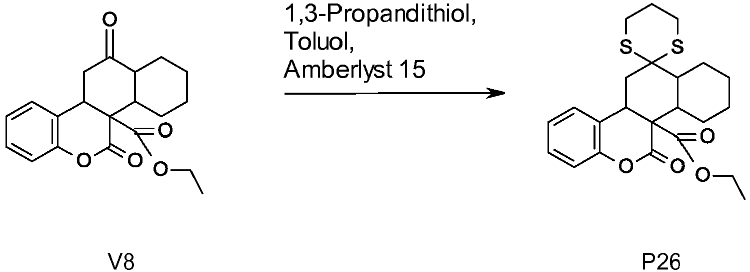

1,0 g (2,9 mmol) Ketoester V8 werden mit 0,63 g (5,8 mmol, 2,0 eq) 1,3-Propandithiol, 10 ml Toluol und 0,1 g Amberlyst 15 versetzt und 24 h bei 60°C gerührt. Es werden 0,4 g 4 Å Molsieb zugegeben und eine weitere Stunde bei 60°C gerührt. Es wird filtriert und die resultierende Lösung eingeengt Der Rückstand wird aus iPrOH kristallisiert, die Suspension im Eisbad abgekühlt und filtriert. Der Filterkuchen wird mit wenig iPrOH gewaschen und im Vakuumtrockenschrank bei 40°C getrocknet.

Ausbeute (P26): 0,85 g (67 %) mit einer HPLC-Reinheit von 98,9 FI-%
¹H-NMR (CDCl₃): 7,32-6,98 (m, 4 H); 4,10-3,89 (m, 2 H); 3,74 (d, 1 H); 3,11-2,94 (m, 2 H); 2,87 (d, 1 H); 2,75 (d, 1 H); 2,70-2,55 (m, 1 H); 2,44-2,22 (m, 3 H); 2,13-1,96 (m, 2 H); 1,89-1,67 (m, 4 H); 1,56 (q, 1 H); 1,46 (d, 1 H); 1,33 (q, 1 H); 1,22-1,09 (m, 1 H); 0,92 (tr, 3 H) [ppm]

### Herstellungsbeispiel H1-47: Herstellung von Verbindung P27

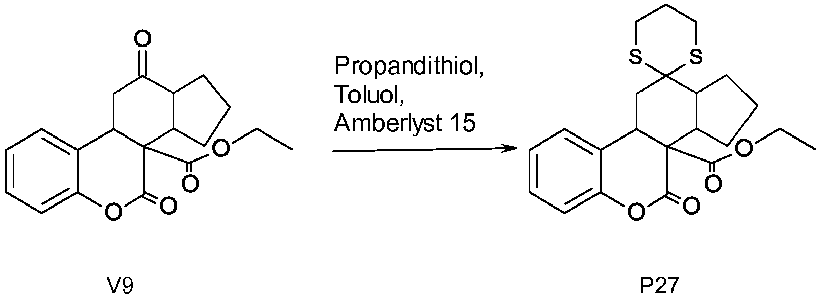

1,2 g (3,65 mmol) Ketoester V9 werden mit 0,79 g (7,3 mmol, 2,0 eq) 1,3-Propandithiol, 12,5 ml Toluol und 0,12 g Amberlyst 15 versetzt und 20 h bei 80°C gerührt. Es wird filtriert und die organische Phase mit 15 ml ges. Natriumhydrogen-carbonat-Lösung und mit 10 ml Wasser gewaschen. Die organische Phase wird einrotiert und der Rückstand aus MTBE/Heptan mit wenigen Tropfen iPrOH kristallisiert.

Ausbeute (P27): 0,85 g (56 %) mit einer HPLC-Reinheit von 100 FI-% (zwei Isomere)
¹H-NMR (CDCl₃): 7,32-6,99 (m, 4 H); 4,09-3,96 (m, 2 H); 3,93 (d, 1 H, ein Isomer); 3,69 (d, 1 H, ein Isomer); 3,15-2,73 (m, 4 H); 2,67-1,40 (m, 12 H); 1,05 (tr, 3 H, ein Isomer); 0,97 (tr, 3 H, ein Isomer) [ppm]

### Herstellungsbeispiel H1-48: Herstellung der Vorstufe V10

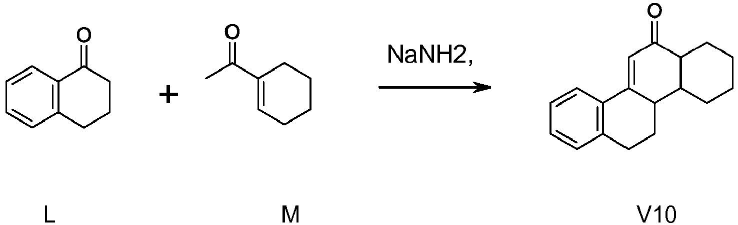

Die Herstellung erfolgt analog: Peak, Robinson, J. Chem. Soc., 1936, 759-762 oder Peak, Robinson, J. Chem. Soc., 1937, 1881-1583.

CAS (Produkt): 99887-26-0

### Herstellungsbeispiel H1-49: Herstellung der Vorstufe V11

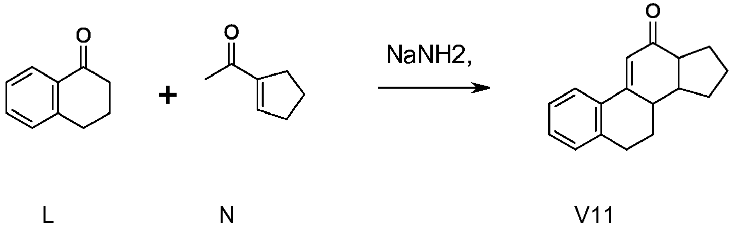

Die Herstellung erfolgt analog: Hawthorne, Robinson, J. Chem. Soc., 1936, 763.

CAS (Produkt): 31301-53-8

### Herstellungsbeispiel H1-50: Herstellung von Verbindung P28

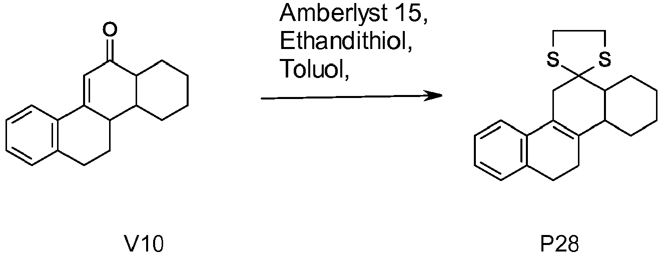

1,5 g (5,94 mmol) Keton V10 werden mit 0,70 g (7,43 mmol, 1,25 eq) Ethandithiol, 10 ml Toluol und 0,3 g Amberlyst 15 versetzt und über Nacht bei 80-100°C gerührt. Es wird filtriert und das Filtrat einrotiert. Der Rückstand wird aus n-Heptan kristallisiert und im Vakuumtrockenschrank bei RT getrocknet.

Ausbeute (P28): 1,35 g (69 %)
¹H-NMR (CDCl₃): 7,14-6,95 (m, 4 H); 3,34-3,05 (m, 5 H); 2,98 (d, 1 H); 2,81-2,60 (m, 2 H); 2,39-2,04 (m, 5 H); 1,92-1,71 (m, 2 H); 1,65 (tr, 1 H); 1,32 (br s, 3 H), 0,93-0,78 (m, 1 H) [ppm]

### Herstellungsbeispiel H1-51: Herstellung von Verbindung P9

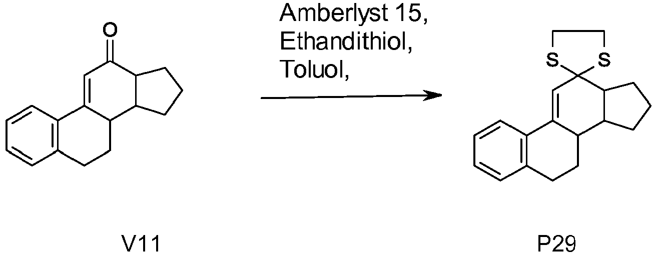

1,2 g (5,04 mmol) Keton V11 werden mit 0,52 g (5,54 mmol, 1,1 eq) Ethandithiol, 15 ml Toluol und 0,2 g Amberlyst 15 versetzt und 2 h bei 80-100°C gerührt. Es wird filtriert und das Filtrat einrotiert. Der Rückstand wird aus n-Heptan kristallisiert und im Vakuumtrockenschrank bei RT getrocknet.

Ausbeute (P29): 0,60 g (38 %) mit einer HPLC-Reinheit von 88,4 FI-%
¹H-NMR (CDCl₃): 7,69 (d, 1 H); 7,20-7,00 (m, 3 H); 6,43 (s, 1H); 3,47-3,20 (m, 3 H); 3,20-3,07 (m, 1 H); 2,91-2,75 (m, 2 H); 2,13-1,23 (m, 11H)[ppm]

### Herstellungsbeispiel H1-52: Herstellung von Verbindung P30

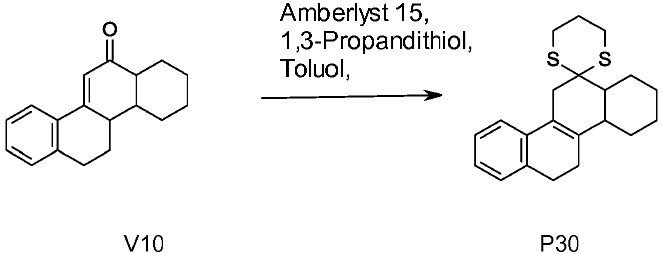

1,5 g (5,94 mmol) Keton V10 werden mit 0,80 g (7,43 mmol, 1,25 eq) 1,3-Propandithiol, 10 ml Toluol und 0,3 g Amberlyst 15 versetzt und 26 h bei 80-100°C gerührt. Es wird filtriert und das Filtrat einrotiert. Der Rückstand wird aus n-Heptan kristallisiert und im Vakuumtrockenschrank bei 30°C getrocknet.

Ausbeute: 1,06 g (52 %) mit einer HPLC-Reinheit von 90,0 FI-%
¹H-NMR (CDCl₃): 7,25-7,01 (m, 4 H); 3,50 (d, 1 H); 3,23 ((tr, 1 H); 3,04-2,63 (m, 5 H); 2,59 (dtr, 1 H); 2,37 (d, 1 H); 2,35-2,01 (m, 5 H); 1,96-1,70 (m, 3 H); 1,70-1,53 (m, 1 H); 1,51-1,22 (m, 3 H); 0,96-0,83 (m, 1 H) [ppm]

### Herstellungsbeispiel H1-53: Herstellung von Verbindung P31

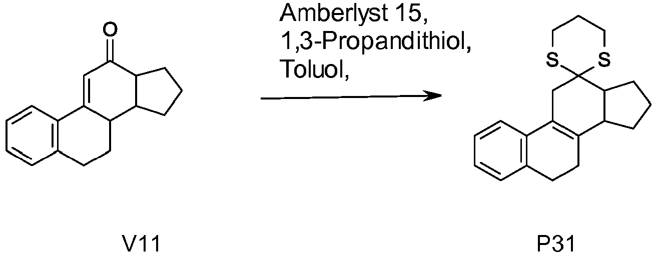

1,2 g (5,04 mmol) Keton V11 werden mit 0,60 g (5,54 mmol, 1,1 eq) 1,3-Propandithiol, 15 ml Toluol und 0,2 g Amberlyst 15 versetzt und 2 h bei 80-100°C gerührt. Es wird filtriert, das Filtrat einrotiert und der Rückstand säulenchromatographisch gereinigt.

Ausbeute: 1,08 g (65 %)
¹H-NMR (CDCl₃): 7,24-7,03 (m, 4 H); 3,03-2,63 (m, 8 H); 2,33-2,21 (m, 1 H); 2,16-1,61 (m, 7 H); 1,60-1,40 (m, 2 H); 1,35-1,18 (m, 2 H); 0,91-0,83 (m, 1 H) [ppm]

### 3. Formulierungsbeispiele

In den folgenden Formulierungsbeispielen werden, ohne darauf beschränkt zu sein, als Wirkstoff ("TRPM8-Agonist") insbesondere Verbindungen gemäß den Tabellen 1 und 2A bis D eingesetzt.

### a) Mundpflege

### Formulierungsbeispiel FM-1: Mundwasser

Geeignete Mundwässer können nach folgender Basisrezeptur hergestellt werden:

| Gew.-% | Inhaltsstoff-Typ | Inhaltsstoff-Beispiele |
|---|---|---|
| 0,01-0,1% | antibakterielle Mittel | beta-Naphthol, Thymol, Chlorthymol und Hexylresorcin |
| 5-25% | Feuchthaltemittel | Glycerin, Sorbit, Propylenglykol und Polyalkylenglykol |
| 0,01-0,2% | etherische Öle | Nelkenöl, Pfefferminzöl und Krauseminzöl |
| 0-30% | Ethanol | |
| 0-5 % | Polymer | Polyoxyalkylenblockcopoymere Mw 5000-30000 |
| 40-80% | Wasser | |
| | | |
| | | |
| 0,001-10% | TRPM8-Agonist | |
| 0-10% | Weitere Zusätze | |

Ein Mundwasser folgender Zusammensetzung wird hergestellt:

| Anteil | Inhaltsstoff |
|---|---|
| 177mL | Ethanol 95% |
| 250 g | Sorbit 70% |
| 50mL | TRPM8 Agonist als 1% Lösung im Ethanol |
| 0.30 g | Pfefferminzöl, |
| 0.64 g | Methylsalicylat |
| 0.922 g | Eucalyptol |
| 0.639 g | Thymol |
| 1.50 g | Benzoesäure |
| 5.00 g | Pluronic ® F127 nichtionisches Tensid |
| 0.60 g | Natrium-Saccharin |
| 0.30 g | Natriumcitrat |
| 0.10 g | Zitronensäure |
| q.s. 1 Liter | Wasser |

Zur Herstellung eines Mundwassers werden die oben beschriebenen Komponenten in den angegebenen Mengen miteinander vermischt.

### Formulierungsbeispiel FM-2: Zahnpasta

Geeignete Zahnpasten können nach folgender Basisrezeptur hergestellt werden:

| Gew.-% | Inhaltsstoff-Typ | In haltsstoff-Beispiele |
|---|---|---|
| 0,05-0,2% | Fluoride | Natriumfluorid, Zinn(II)-fluorid, Natriummonofluorophosphat; |
| 10-55% | Feuchthaltemittel | Glycerin, Sorbit, Propylenglykol, Polyalkylenglykol |
| 0-50% | Polymer | Polyoxyalkylenblockcopoymere Mw 5000-30000 |
| 10-50% | Wasser | |
| 10-55% | Abrasiva | Calciumpyrophosphat, Dicalciumphosphat, Siliziumoxidhydrat; |
| 2-10% | Bindemittel | Karayagummi, Tragant USP, Natriumalginat, irländisches Moos, Methylcellulose |
| 2-8% | Tensid | Natriumlaurylsulphat, Natrium-N-laurylsarcosinat, Dioctlnatriumsulphosuccinat, Natriumlaurylsulphoacetat |
| 0-10% | Persauerstoffverbindung | Wasserstoffperoxid, anorganischen Peroxiden |
| 0,001-10% | TRPM8-Agonist | |
| 0-10% s.o. | Weitere Zusätze | |

### Formulierungsbeispiel FM-3: Kaugummi

Geeignete Kaugummis können nach folgender Basisrezeptur hergestellt werden:

| Gew.-% | Inhaltsstoff |
|---|---|
| 15-25% | Kaugummi-Grundmasse ("gum-base") |
| 20 - 30% | Glucosesirup |
| 50 - 60% | Puderzucker |
| 0,001 -10% | TRPM8-Agonist |
| 1-2% | Weichmacher (z.B. Glycerin) |
| 3-6% | Wasser |

Anstelle des Glucosesirups und des Puderzuckers können für "zuckerfreie" Rezepturen auch die Zuckeralkohole Mannit, Xylit und Sorbit, "Palatinit" und andere sowie künstliche Süßstoffe, wie Saccharin, Cyclamat, Acesulfam-K und Aspartame, eingesetzt werden.

### b) Körperpflege

### Formulierungsbeispiel FK-1: Haarwasser

| | % | Inhaltsstoff (INCI) |
|---|---|---|
| A | q.s. | Parfümöl |
| | 1,00 | PEG-40 Hydrogenated Castor Oil |
| B | 65,0 | Alkohol |
| | 1,0 | Panthenol |
| | 0,5 | Polyquarternium-16 |
| | 0,1 | Menthol |
| | 27,4 | Aqua dem. |
| | 5,00 | wässrige Lösung mit ca. x 0,001-10% % TRPM8 Agonist |

Herstellung: Phase A mischen. Phase B zugeben und rühren bis alles gelöst ist, pH-Wert einstellen auf pH 7,0.

### Formulierungsbeispiel FK-2: Haargel

| | % | Inhaltsstoff (INCI) |
|---|---|---|
| A | 45,00 | Carbopol 940 1% in Wasser |
| | 0,70 | Aminomethyl Propanol |
| B | 7,50 | VP/MethacrylamideNinyl Imidazole Copolymer |
| | 0,10 | Parfümöl |
| | 0,30 | PEG-40 Hydrogenated Castor Oil |
| | 0,30 | Konservierungsmittel |
| | 0,05 | Disodium EDTA |
| | 0,30 | Panthenol |
| | 8,00 | Alcohol |
| | 5,00 | wässrige Lösung mit ca. 0,001-10 % TRPM8 Agonist |
| | 32,75 | Aqua dem. |

Herstellung: Die Komponenten der Phase A einwiegen und homogenisieren. Phase B lösen und in Phase A einrühren. pH Wert einstellen auf pH 6,9.

### Formulierungsbeispiel FK-3: Kosmetische Sonnenschutzzubereitung

In den folgenden Rezepturen wird eine kosmetische Sonnenschutzzubereitung, enthaltend eine Kombination aus mindestens anorganischem Pigment und organische m UV-Filter beschrieben.

Die Herstellung der nachfolgend genannten Formulierungen erfolgt auf übliche, dem Fachmann bekannte Art und Weise.

| | | |
|---|---|---|
| A | 7,50 Uvinul MC 80 | Ethylhexylcinnamat |
| | 2,00 Uvinul M 40 | Benzophenon-3 |
| | 0,80 Rylo PG 11 | Polyglyceryldimersoyat |
| | 1,00 Span 60 | Sorbitanstearat |
| | 0,50 Vitamin E-Acetat | Tocopherylacetat |
| | 3,00 Dracorin 100 SE | Glycerylstearat, PEG-100 Stearat |
| | 1,00 Cremophor CO 410 | PEG-40-hydriertes Rizinusöl |
| B | 3,00 T-Lite SF | Titandioxid, Aluminiumoxidhydrat, Dimethicon-/Methicon Copolymer |
| | 1,00 Cetiol SB 45 | *Butyrospermum parkii* (Shea Butter) |
| | 6,50 Finsolv TN | C₁₂₋₁₅-Alkylbenzoat |
| C | 5,00 Butylenglykol | Butylenglycol |
| | 0,30 Keltrol | Xanthangummi |
| | 0,10 Edeta BD | Dinatrium-EDT A |
| | 0,10 Allantoin | Allantoin |
| | 66,20 Wasser dem. | Aqua dem. |
| D | 1,00 Sepigel 305 | Polyacrylamid, C₁₃₋₁₄-Isoparaffin, Laureth-7 |
| | 0,001-10 % TRPM8 Agonist | |
| | q.s. | Konservierungsmittel |

### Formulierungsbeispiel FK-4: Feuchtigkeitsspendende Körperpflegecreme

| | % | Inhaltsstoff (INCI) |
|---|---|---|
| A | 6,0 | PEG-7-hydriertes Rizinusöl |
| | 10,0 | Cetearylethylhexanoat |
| | 5,0 | Isopropylmyristat |
| | 7,0 | Mineralöl |
| | 0,5 | Shea Butter (*Butyrospermum parkii*) |
| | 0,5 | Aluminumstearat |
| | 0,5 | Magnesiumstearat |
| | 0,2 | Bisabolol |
| | 0,7 | Quaternium-18-Hectorit |
| B | 5,0 | Dipropylenglykol |
| | 0,7 | Magnesiumsulfat |
| | q.s. | Konservieru ngsm ittel |
| | 62,9 | Aqua dem. |
| | q.s. | Parfümöl |
| C | 1,0 | wässrige Lösung mit 0,001-10 % TRPM8 Agonist |

Herstellung: Die Phasen A und B getrennt auf ca. 80 °C erwärmen. Phase B in Phase A einrühren und homogenisieren. Unter Rühren auf ca. 40 °C abkühlen, Phase C zugeben und nochmals homogenisieren. Unter Rühren auf Raumtemperatur abkühlen lassen.

### Formulierungsbeispiel FK-5: Pflegeshampoo

| | % | Inhaltsstoff (INCI) |
|---|---|---|
| A | 30,0 | Natriumlaurethsulfat |
| | 6,0 | Natriumocoamphoacetat |
| | 6,0 | Cocamidopropylbetain |
| | 3,0 | Natriumlaurethsulfat, Glykoldistearat, Cocamid-MEA, Laureth-10 |
| | 1,0 | wässrige Lösung mit 0,001 -10 % TRPM8 Agonist |
| | 7,7 | Polyquaternium-44 |
| | 2,0 | Amodimethicon |
| | q.s. | Parfümöl |
| | q.s. | Konservierungsmittel |
| | 1,0 | Natriumchlorid |
| | 43,3 | Aqua dem. |
| B | q.s. | Zitronensäure |

Herstellung: Die Komponenten der Phase A mischen und lösen. Den pH-Wert mit Zitronensäure auf 6-7 einstellen.

### Formulierungsbeispiel FK-6: Duschgel

| | % | Inhaltsstoff (INCI) |
|---|---|---|
| A | 40,0 | Natriumlaurethsulfat |
| | 5,0 | Decylglukosid |
| | 5,0 | Cocamidopropylbetain |
| | 1,0 | wässrige Lösung mit 0,001 -10 % TRPM8 Agonist |
| | 1,0 | Panthenol |
| | q.s. | Parfümöl |
| | q.s. | Konservieru ngsm ittel |
| | 2,0 | Natriumchlorid |
| | 46,0 | Aqua dem. |
| B | q.s. | Zitronensäure |

Herstellung: Die Komponenten der Phase A mischen und lösen. Den pH-Wert mit Zitronensäure auf 6-7 einstellen.

### Formulierungsbeispiel FK-7: Shampoo

| | % | Inhaltsstoff (INCI) |
|---|---|---|
| A | 40,0 | Natriumlaurethsulfat |
| | 5,0 | Natrium-C₁₂₋₁₅-Pareth-15-sulfonat |
| | 5,0 | Decylglukosid |
| | q.s. | Parfümöl |
| | 0,1 | Phytantriol |
| | 44,6 | Aqua dem. |
| | 1,0 | wässrige Lösung mit 0,001 -10 % TRPM8 Agonist |
| | 0,3 | Polyquaternium-10 |
| | 1,0 | Panthenol |
| | q.s. | Konservierungsmittel |
| | 1,0 | Laureth-3 |
| | 2,0 | Natriumchlorid |

Herstellung: Die Komponenten der Phase A mischen und lösen. Den pH-Wert mit Zitronensäure auf 6-7 einstellen.

### Formulierungsbeispiel FK-8: Fußbalsam

| | % | Inhaltsstoff (INCI) |
|---|---|---|
| A | 2,0 | Ceteareth-6, Stearylalkohol |
| | 2,0 | Ceteareth-25 |
| | 5,0 | Cetearylethylhexanoat |
| | 4,0 | Cetylalkohol |
| | 4,0 | Glycerylstearat |
| | 5,0 | Mineralöl |
| | 0,2 | Menthol |
| | 0,5 | Kampfer |
| B | 69,3 | Aqua dem. |
| | q.s. | Konservieru ngsm ittel |
| C | 1,0 | Bisabolol |
| | 1,0 | Tocopherylacetat |
| D | 1,0 | wässrige Lösung mit 0,001 - 10 % TRPM8 Agonist |
| | 5,0 | Zaubernussextrakt |

Herstellung: Die Komponenten der Phasen A und B getrennt voneinander auf ca. 80 °C erwärmen. Phase B in Phase A unter Homogenisieren einrühren. Unter Rühren abkühlen auf ca. 40 °C, die Phasen C und D hinzugeben und kurz nachhomogenisieren. Unter Rühren auf Raumtemperatur abkühlen.

### Formulierungsbeispiel FK-9: Gesichtsreinigungslotion - Typ O/W

| | % | Inhaltsstoff (INCI) |
|---|---|---|
| A | 10,0 | Cetearylethylhexanoat |
| | 10,0 | Capryl-/Caprintriglycerid |
| | 1,5 | Cyclopentasiloxan, Cyclohexasilosan |
| | 2,0 | PEG-40-hydriertes Rizinusöl |
| B | 3,5 | Capryl-/Caprintriglycerid, Natriumacrylat-Copolymer |
| C | 1,0 | Tocopherylacetat |
| | 0,2 | Bisabolol |
| | q.s. | Konservieru ngsm ittel |
| | q.s. | Parfümöl |
| D | 3,0 | Polyquaternium-44 |
| | 0,5 | Cocotrimoniummethosulfat |
| | 0,5 | Ceteareth-25 |
| | 2,0 | Panthenol, Propylenglykol |
| | 4,0 | Propylen glykol |
| | 0,1 | Dinatrium-EDT A |
| | 1,0 | wässrige Lösung mit 0,001 - 10 % TRPM8 Agonist |
| | 60,7 | Aqua dem. |

Herstellung: Phase A lösen. Phase B in Phase A einrühren, Phase C in die kombinierten Phasen A und B einarbeiten. Phase D lösen, in die kombinierten Phasen A, B und C einrühren und homogenisieren. 15 min nachrühren.

### Formulierungsbeispiel FK-10: Body-Spray

| | % | Inhaltsstoff (INCI) |
|---|---|---|
| A | 3,0 | Ethylhexylmethoxycinnamat |
| | 2,0 | Diethylaminohydroxybenzoylhexylbenzoat |
| | 1,0 | Polyquaternium-44 |
| | 3,0 | Propylenglykol |
| | 2,0 | Panthenol, Propylenglykol |
| | 1,0 | Cyclopentasiloxan, Cyclohexasilosan |
| | 10,0 | Octyldodecanol |
| | 0,5 | PVP |
| | 10,0 | Capryl-/Caprintriglycerid |
| | 3,0 | C₁₂₋₁₅-Alkylbenzoat |
| | 3,0 | Glycerin |
| | 1,0 | Tocopherylacetat |
| | 0,3 | Bisabolol |
| | 1,0 | wässrige Lösung mit 0,001 - 10 % TRPM8 Agonist |
| | 59,2 | Alkohol |

Herstellung: Die Komponenten der Phase A einwiegen und klar lösen.

### Formulierungsbeispiel FK-11: Hautpflegegel

| | % | Inhaltsstoff (INCI) |
|---|---|---|
| A | 3,6 | PEG-40-hydriertes Rizinusöl |
| | 15,0 | Alkohol |
| | 0,1 | Bisabolol |
| | 0,5 | Tocopherylacetat |
| | q.s. | Parfümöl |
| B | 3,0 | Panthenol |
| | 0,6 | Carbomer |
| | 1,0 | wässrige Lösung mit 0,001 - 10 % TRPM8 Agonist |
| | 75,4 | Aqua dem. |
| C | 0,8 | Triethanolamin |

### Formulierungsbeispiel FK-12: After-Shave-Lotion

| | % | Inhaltsstoff (INCI) |
|---|---|---|
| A | 10,0 | Cetearylethylhexanoat |
| | 5,0 | Tocopherylacetat |
| | 1,0 | Bisabolol |
| | 0,1 | Parfümöl |
| | 0,3 | Acrylate/C₁₀₋₃₀ Alkylacrylat-Crosspolymer |
| B | 15,0 | Alkohol |
| | 1,0 | Panthenol |
| | 3,0 | Glycerin |
| | 1,0 | wässrige Lösung mit 0,001 - 10 % TRPM8 Agonist |
| | 0,1 | Triethanolamin |
| | 63,5 | Aqua dem. |

Herstellung: Die Komponenten der Phase A mischen. Phase B lösen, in Phase A einarbeiten und homogenisieren.

### Formulierungsbeispiel FK-13: After-Sun-Lotion

| | % | Inhaltsstoff (INCI) |
|---|---|---|
| A | 0,4 | Acrylate/C10-30-Alkylacrylat-Crosspolymer |
| | 15,0 | Cetearylethylhexanoat |
| | 0,2 | Bisabolol |
| | 1,0 | Tocopherylacetat |
| | q.s. | Parfümöl |
| B | 1,0 | Panthenol |
| | 15,0 | Alkohol |
| | 3,0 | Glycerin |
| | 1,0 | wässrige Lösung mit 0,001 - 10 % TRPM8 Agonist |
| | 63,2 | Aqua dem. |
| C | 0,2 | Triethanolamin |

Herstellung: Die Komponenten der Phase A mischen. Phase B unter Homogenisieren in Phase A einrühren. Mit Phase C neutralisieren und erneut homogenisieren.

### Formulierungsbeispiel FK-14: Sonnenschutzlotion

| | % | Inhaltsstoff (INCI) |
|---|---|---|
| A | 4,5 | Ethylhexylmethoxycinnamat |
| | 2,0 | Diethylaminohydroxybenzoylhexylbenzoat |
| | 3,0 | Octocrylen |
| | 2,5 | Di-C12-13-Alkylmalat |
| | 0,5 | Tocopherylacetat |
| | 4,0 | Polyglyceryl-3-methylglucosedistearat |
| B | 3,5 | Cetearylisononanoat |
| | 1,0 | VP-/EicosenCopolymer |
| | 5,0 | Isohexadecan |
| | 2,5 | Di-C12-13-Alkylmalat |
| | 3,0 | Titaniumdioxid, Trimethoxycaprylylsilan |
| C | 5,0 | Glycerin |
| | 1,0 | Natriumcetearylsulfat |
| | 0,5 | Xanthangummi |
| | 59,7 | Aqua dem. |
| D | 1,0 | wässrige Lösung mit 0,001 - 10 % TRPM8 Agonist |
| | 1,0 | Phenoxyethanol, Methylparaben, Ethylparaben, Butylparaben, Propyl-paraben, Isobutylparaben |
| | 0,3 | Bisabolol |

Herstellung: Die Komponenten der Phasen A und B getrennt voneinander auf ca. 80 °C erwärmen. Phase B in Phase A einrühren und homogenisieren. Phase C auf ca. 80 °C erwärmen und unter Homogenisieren in die kombinierten Phasen A und B einrühren. Unter Rühren auf ca. 40 °C abkühlen, Phase D zugeben und nochmals homogenisieren.

### Formulierungsbeispiel FK-15: Pflaster

50 Teile Wirkstoff gemäß Herstellungsbeispiel H 3-7 wurden in 100 Teilen einer 10 %igen Natriumlaurylsulfatlösung unter starkem Rühren und Erwärmen auf 50°C dispergiert. In die entstandene Emulsion wurden 880 Teile einer 50 %igen Butylacrylatdispersion eingerührt und die erhaltene wirkstoffhaltige Polymerdispersion mittels einer geeigneten Ausstreichrakel auf einer Polyesterfolie mit 15 µm Dicke (Fa. Kalle, D-Wiesbaden) ausgestrichen und bei 35 bis 40°C unter kontrollierter Luftfeuchtigkeit getrocknet. Je nach Rakeleinstellung resultierten Flächengewichte von 5 mg/cm², die durch Mehrfachauftrag weiter erhöht werden konnten. Der so hergestellte selbstklebende Film mit einem Wirkstoffgehalt von 5 % wurde mit einer silikonisierten Abziehfolie aus Polyester (Scotch Pak 75 mu m, 3M) versehen und in die gewünschten Abmessungen geschnitten.

Die Mengenangabe betreffen jeweils Gewichtsteile.

### c) Nahrungsmittel

### Formulierungsbeispiel FN-1: Pudding

Rezept (für 100 ml)

| Inhaltsstoff | Menge |
|---|---|
| Fettfreie Trockenmilch | 10,715 g |
| Saccharose | 5 g |
| Novelose Stärke, National Starch | 7 g |
| Pflanzenölgemisch | 2,2 g |
| Carrageenan | 0,016 g |
| Vanillearoma | 0,5 g |
| Natriumstearoyl-2-lactylat | 0,095 g |
| Gelber Farbstoff | 0,189 g |
| Magnesiumphosphat | 0,165 g |
| Vitaminvormischung | 1,84 g |
| Spurenelementvormischung | 0,015 g |
| TRPM8 Agonist | 0,5 g |
| Wasser | 81,94 g |

### Herstellung:

Neun Zehntel des Wassers auf 43,3 °C erhitzen. Magermilchpulver im Wasser auflösen. Öl auf 60 °C erhitzen und Carrageenan und öllösliche Vitamine zum Öl geben. Öl in das Erzeugnis einmischen. Die übrigen Bestandteile außer der modifizierten Stärke, Vanillearoma und Vitaminvormischung hinzufügen. Das Gemisch homogenisieren. Stärke langsam hinzufügen. Wirkstoff, Vitamine und Aroma hinzufügen. Feststoffgehalt standardisieren. In sterilen Einheiten erhitzen und in Dosen verpacken.

### d) Formulierungsbeispiel FT-1: Textilausrüstung mit erfindungsgemäß anzuwendenden Wirkstoffen

Zunächst stellt man eine wässrige Aufschlämmung amylosehaltiger Stärke her, indem man 570 g entionisiertes Wasser wurden mit 10 g eines handelsüblichen Konservierungsmittels versetzt. Hierin löste man 20 g Carboxymethylcellulose, gab anschließend 400 g einer amylosehaltigen Stärke mit einem Amylosegehalt von 50 Gew.-% zu und stellte unter Rühren eine Aufschlämmung her.

Anschließend erfolgt die Herstellung wässriger Flotten mit amylosehaltiger Stärke nach einer der beiden folgenden Methoden:
Methode 1: Die jeweilige Aufschlämmung wird durch Verdünnen mit Wasser auf einen Stärkegehalt von 5 oder 15 Gew.-% eingestellt.
Methode 2: Die jeweilige Aufschlämmung werden zunächst mit Wasser auf einen Stärke-Gehalt von 5 oder 15 Gew.-% verdünnt und anschließend mit 30 g/l einer 30 Gew.-%igen, wässrigen Polyurethandispersion (nicht-ionogen) versetzt.

Anschießend erfolgt die Ausrüstung eines Gewebes mit amylosehaltiger Stärke und erfindungsgemäß anzuwendendem Wirkstoff:
Baumwollgewebemuster mit einem Flächengewicht von 124 g/m² wird mit einer der obe hergestellten Flotten mittels eines Foulards bis zu einer Flottenaufnahme von 80 Gew.-%, bezogen auf das Gewicht des Gewebes behandelt. Anschließend trocknet man 2 min bei 120°C.

Anschließend werden die so ausgerüsteten Gewebemuster mit einer wässrigen Wirkstoff-Formulierung behandelt, indem man eine wässrige Emulsion/Suspension eines erfindungsgemäß anzuwendenden Wirkstoffs mit einem Wirkstoffgehalt von 1 bis 7 Gew.-% bis zu einer Flottenaufnahme von 79 - 80 Gew.-% auf das Gewebemuster auffoulardiert. Anschließend werden die so behandelten Gewebemuster in einem Haushaltstrockner bis zu einer Restfeuchte von 15 % getrocknet.

Die so hergestellten wirkstoffbeladenen Gewebe können dann weiter untersucht werden, wie z.B. auf deren Kühlende Wirkung bei Hautkontakt oder deren Repellent-Wirkung auf Insekten.

### Weitere Formulierungsbeispiele

### Beispiel S-1 - Herstellung von Aromen mit Kühlwirkung vom Eukalyptus-Menthol-Typ unter Verwendung der erfindungsgemäß anzuwendenden Kühlsubstanzen:

Es wurden vermischt (alle Angaben, soweit nicht anders vermerkt, in Gew-%):

| **Komponente** | |
|---|---|
| Anethol | 10 |
| Pfefferminzöl Mentha piperita Typ Willamette | 20 |

| | |
|---|---|
| Pfefferminzöl Mentha arvensis, rektifiziert | 20 |
| *I*-Menthyllactat | 1 |
| 2-Hydroxyethylmenthylcarbonat | 2 |
| 2-Hydroxypropyl-menthylcarbonat | 2 |
| 1,8-Cineol (Eucalyptol) | 5 |
| *I*-Menthol | 39,4 |
| TRPM8 Agonist | 0,6 |
| Total | 100 |

Die so erhaltenen Aromen wurden in eine Standard-Zahnpasta-Masse auf Kieselsäure-Basis in einer Konzentration von 1,2 Gew.-% eingearbeitet.

### Beispiel S-2 -Herstellung von Aromen mit Kühlwirkung vom Krauseminz-Typ unter Verwendung der erfindungsgemäß anzuwendenden Kühlsubstanzen.

Es wurden vermischt (alle Angaben, soweit nicht anders vermerkt, in Gew-%):

| **Komponente** | |
|---|---|
| Menthol | 29,25 |
| Carvon | 20 |
| Krauseminzöl Typ Native | 20 |
| Anethol | 5 |
| Pfefferminzöl Mentha arvensis rektifiziert | 10 |
| Pfefferminzöl Mentha piperita Typ Willamette | 15 |
| TRPM8 Agonist | 0,75 |
| Total | 100 |

Die erhaltenen Aromen wurden mit einer Konzentration von 1,2 % in eine Zahnpasta-Masse eingearbeitet, die zu einem Anteil von 65% aus Natriumbicarbonat besteht.

### Beispiel S-3 - Herstellung von Aromen mit Kühlwirkung und einem würzigaromatischen Geschmackseindruck unter Verwendung der erfindungsgemäß anzuwendenden Kühlsubstanzen.

Es wurden vermischt (alle Angaben, soweit nicht anders vermerkt, in Gew-%):

| Komponente | |
|---|---|
| /-Menthol | 29,2 |
| Pfefferminzöl Mentha arvensis, rektifiziert | 25 |
| Pfefferminzöl Mentha piperita Typ Willamette | 15 |
| Anethol | 10 |
| Krauseminzöl Typ Native | 10 |
| Zimtaldehyd | 5 |
| Eugenol | 5 |
| TRPM8 Agonist | 0,8 |
| Total | 100 |

Die so erhaltenen Aromen wurden jeweils in eine Standard-Zahnpasta-Masse auf Kieselsäure-Basis in einer Konzentration von 1,2 Gew.-% eingearbeitet.

### Beispiel S-4 - Herstellung von Aromen mit Kühlwirkung und Wintergrün-Geschmack unter Verwendung der erfindungsgemäß anzuwendenden Kühlsubstanzen:

Es wurden vermischt (alle Angaben, soweit nicht anders vermerkt, in Gew-%):

| Komponente | |
|---|---|
| Anethol | 10 |
| Pfefferminzöl Mentha arvensis | 12 |
| Pfefferminzöl Mentha piperita Typ Willamette | 12 |
| Methylsalicylat | 25 |
| I-Menthol | 40,5 |
| Verbindungen der Formel ... | 0,5 |
| Total | 100 |

Die so erhaltenen Aromen wurden in eine Standard-Zahnpasta-Masse auf Kieselsäure-Basis in einer Konzentration von 1,2 Gew.-% eingearbeitet.

### Beispiel S-5 - Herstellung von Aromen mit Kühlwirkung und Pfefferminzgeschmack unter Verwendung von der erfindungsgemäß anzuwendenden Kühlsubstanzen:

Es wurden vermischt (alle Angaben, soweit nicht anders vermerkt, in Gew-%):

| Komponente | |
|---|---|
| Pfefferminzöl Mentha arvensis | 59 |
| I-Menthon | 20 |
| I-Menthol | 20 |
| TRPM8 Agonist | 1 |
| Total | 100 |

Die so erhaltenen Aromen wurden jeweils in eine zuckerfreie Standard-KaugummiMasse in einer Konzentration von 1,5 Gew.-% eingearbeitet.

### Beispiel S-6 - Herstellung von Aromen mit Kühlwirkung und Krauseminzgeschmack unter Verwendung der erfindungsgemäß anzuwendenden Kühlsubstanzen:

Es wurden vermischt (alle Angaben, soweit nicht anders vermerkt, in Gew-%):

| Komponente | |
|---|---|
| Pfefferminzöl Mentha piperita Typ Madras | 50 |
| Eucalyptol | 20 |
| I-Menthol | 13,5 |
| I-Menthon | 10 |
| Krauseminzöl Typ Midwest Scotch | 5 |
| TRPM8 Agonist | 1,5 |
| Total | 100 |

Die so erhaltenen Aromen wurden jeweils in eine zuckerfreie Standard-KaugummiMasse in einer Konzentration von 1,5 Gew.-% eingearbeitet.

### Beispiel S-7 - Herstellung von Aromen mit Kühlwirkung und einem aromatischwürzigen Zimtgeschmack unter Verwendung der erfindungsgemäß anzuwendenden Kühlsubstanzen:

Es wurden vermischt (alle Angaben, soweit nicht anders vermerkt in Gew.-%:

| Komponente | |
|---|---|
| Menthylmethylether | 3 |
| Zimtaldehyd | 10 |
| Anethol | 10 |
| Eugenol | 2 |
| Pfefferminzöl Mentha piperita Typ Madras | 10 |

| | |
|---|---|
| Pfefferminzöl mentha arvensis | 10 |
| Krauseminzöl Typ Midwest Scotch | 10 |
| I-Menthol | 40 |
| 2-Hydroxyethylmenthylcarbonat | 2 |
| 2-Hydroxypropylmenthylcarbonat | 2 |
| TRPM8 Agonist | |
| Total | 100 |

Die so erhaltenen Aromen wurden jeweils in eine zuckerfreie Standard-KaugummiMasse in einer Konzentration von 1,5 Gew.-% eingearbeitet.

### Beispiel S-8 - Herstellung von Mundwasser-Aromen mit Kühlwirkung unter Verwendung der erfindungsgemäß anzuwendenden Kühlsubstanzen:

Es wurden vermischt (alle Angaben, soweit nicht anders vermerkt, in Gew-%):

| Komponente | |
|---|---|
| Anethol | 30 |
| Eucalyptol | 25 |
| I-Menthol | 44,4 |
| TRPM8 Agonist | 0,6 |
| Total | 100 |

Die Aromen wurden jeweils mit einer Konzentration von 0,15 Gew.% in ein gebrauchsfertiges Mundwasser, bzw. mit einer Konzentration von 3 Gew.% in ein Mundwasserkonzentrat eingearbeitet.

### Beispiel S-9 - Zahnpasta ('Silica opaque')

Alle Angaben, soweit nicht anders vermerkt, in Gew-%.

| Bestandteil | |
|---|---|
| | |
| Entionisiertes Wasser | 26,53 |
| Sorbitol 70% | 45 |
| Solbrol M Na-Salz | 0,15 |
| Trinatriumphosphat | 0,1 |
| Saccharin | 0,2 |
| Natriummonofluorphosphat | 1,12 |
| PEG 1500 | 5 |
| Sident 9 (Abrasive Silica) | 10 |
| Sident 22 S (Dickende Silica) | 8 |
| Natriumcarboxymethylcellulose | 0,9 |
| Titan (IV) oxid | 0,5 |
| Natriumlaurylsulfat (SLS) | 1,5 |
| Pellitorin-Lösung PLM (enthaltend 10% Pellitorin) | - |
| Aroma Typ Eucalyptus-Menthol (Beispiel S-1) | 1 |

### Beispiel S-10 - Zahnpasta (Calciumcarbonat-Base)

Alle Angaben, soweit nicht anders vermerkt, in Gew-%.

| Bestandteil | |
|---|---|
| | |
| Entionisiertes Wasser | 27,5 |
| Saccharin | 0,2 |
| Solbrol M Natriumsalz | 0,2 |
| Natriummonofluorphosphat | 0,8 |
| Sorbitol 70% | 29 |
| Calciumcarbonat | 35 |
| Sident 22 S (Verdickende Silica) | 2,5 |
| Natriumcarboxymethylcellulose | 1,3 |
| Titandioxid | 0,5 |
| Natriumlaurylsulfat | 2 |
| Pellitorin-Lösung PLM (enthaltend 10% Pellitorin) | - |
| Aroma Typ Eucalyptus-Menthol (Beispiel S-1) | 1 |

### Beispiel S-20 - Zuckerfreier Kaugummi

Alle Angaben, soweit nicht anders vermerkt, in Gew-%.

| | |
|---|---|
| Bestandteil | 1 |
| | |
| Gum Base (Kaugummibase) | 30 |
| Sorbit gepulvert | 40 |
| Isomalt gepulvert | 9,5 |
| Xylitol | 2 |
| Mannit D | 3 |
| Aspartam | 0,1 |
| Acesulfam K | 0,1 |
| EmulgumTM (Soja-Lecithine mit hohem Gehalt an Phospholipiden) | 0,3 |
| Sorbitol (70% in Wasser) | 13 |
| 1,2-Propylenglyol | - |
| Glycerin | 1 |
| Pellitorin-Lösung PLM (enthaltend 10% Pellitorin) | - |
| Aroma Eucalyptus Typ Pfefferminz (Beispiel S-5) | 1 |

### Beispiel S-22 - Zuckerfreie Kaugummis

Die Kaugummibase K1 bestand aus 2,0% Butyl Rubber (Isobuten-Isopren-Copolymer, MW = 400000, 6,0% Polyisobuten (MW = 43.800), 43,5% Polyvinylacetat (MW = 12.000), 31,5% Polyvinylacetat (MW = 47.000), 6,75% Triacetin und 10,25% Calciumcarbonat. Die Herstellung der Kaugummibase K1 und der Kaugummis kann analog zu US 5,601,858 erfolgen.

Alle Angaben, soweit nicht anders vermerkt, in Gew-%.

| Bestandteil | |
|---|---|
| | |
| Kaugummibase K1 | 26 |
| Triacetin | 0,25 |
| Lecithin | 0,5 |
| Sorbitol, kristallin | Ad 100 |
| Mannitol | 15,3 |
| Glycerin | 12,1 |
| Saccharin-Na | 0,17 |
| Verkapseltes Aspartam | 1,08 |
| Amorphes Silica | 1 |
| Baumwollsamenöl | 0,5 |
| Polyoxyethylen-sorbitan-monolaurat (E-432) | 1 |
| Verkapseltes I-Carvon (Beladung: 30%) | - |
| I-Menthyl-I-Iactat | - |
| Aroma Typ Krauseminz (Beispiel S-6) | 1 |

### Beispiel S-26 - Bonbons ('Hardboiled candy')

Alle Angaben, soweit nicht anders vermerkt, in Gew-%.

| Bestandteil | |
|---|---|
| | |
| Wasser | 2,75 |
| Zucker | 60,1 |
| Glucosesirup | 36,9 |
| Maltose | - |
| Palmkernöl | - |
| Citronensäure | - |
| Ginseng Extrakt | - |
| Blauer Farbstoff | - |
| Aroma Krauseminz-Typ (Beispiel S-2) | 0,25 |

### Beispiel S-27 - Instant-Getränkepulver

Alle Angaben, soweit nicht anders vermerkt, in Gew-%.

| Bestandteil | |
|---|---|
| | |
| Zucker (Saccharose) | Ad 100 |
| Citronensäure | 11,58 |
| Trinatriumcitrat | 0,7 |
| Tricalciumphosphat | 0,6 |
| Vitamin C | 0,66 |
| Grindsted® JU 543 Stabilizer System (Danisco) | 0,9 |
| Saccharin | 0,561 |
| Citronenaroma, sprühgetrocknet | 1,75 |
| Orangenaroma, sprühgetrocknet | |
| Aroma Eucalyptus-Menthol-Typ (Beispiel S-1), sprühgetrocknet auf Maltodextrin (DE 15-19) und Gummi Arabicum, Aromabeladung 40% | 1,75 |

45 g dieser Instant-Getränkepulver wurden jeweils in 1000 mL unter Rühren gelöst. Das erhaltenen Getränke hatten einen erfrischenden, kühlenden Geschmack von Citrus, Zimt und Minze.

### Beispiel S-31 - Herstellung eines Extrudates für die Bereitung von Getränkemischungen mit Kühlwirkung

Alle Angaben, soweit nicht anders vermerkt, in Gew-%.

| Bestandteil | |
|---|---|
| Glukosesirup, sprühgetrocknet (DE-Wert: 31-34) [Glucidex IT33W (Firma Roquette)] | 62,0 |
| Maltodextrin (DE-Wert: 17-20), Firma Cerestar | 28,4 |
| Emulgator Monomuls, Emulgator auf der Basis von gehärtetem Palmöl; Schmelzpunkt: 64°C, (Firma Grünau) | 1,8 |
| Dextrosemonohydrat (DE-Wert: 99,5), Firma Cerestar | 1,8 |
| Wasser | 2,0 |
| Orangen-Vanillearoma | 3,2 |
| Aroma Eucalyptus-Menthol-Typ (Beispiel S-1) | 0,8 |

Herstellungshinweis (siehe auch WO 03/092412):
Alle Bestandteile wurden gemischt und per Einpunktdosierung in einen Doppelschneckenextruder gefördert. Die Extrusionstemperaturen lagen zwischen 100 und 120°C, der spezifische Energieeintrag lag bei 0,2 kWh/kg. Die aus der mit 1 mm Bohrungen versehene Düsenplatte des Extruders austretenden Stränge wurden unmittelbar nach Austritt aus den Düsen durch rotierende Messer auf Partikel mit ca. 1 mm Durchmesser geschnitten.

### Beispiel S-32 - Herstellung von Wirbelschichtgranulaten für die Bereitung von Getränkemischungen mit Kühlwirkung

In einer Granulierapparatur des in EP 163 836 dargestellten Typs (mit den folgenden Merkmalen: Durchmesser Anströmboden: 225 mm, Sprühdüse: Zweistoffdüse; Sichtender Austrag: Zick-Zack-Sichter; Filter: internes Schlauchfilter) wird eine Lösung bestehend aus 44 Gew.-% Wasser, 8 Gew.% Citronenaroma, 3 Gew.-% Aroma Eucalyptus-Menthol-Typ (s. Beispiel S-1), 13 Gew.-% Gummi arabicum und 32 Gew.-% hydrolysierter Stärke (Maltodextrin DE 15-19) sowie etwas grünem Farbstoff granuliert. Die Lösung wird bei einer Temperatur von 32°C in den Wirbelschichtgranulator gesprüht. Zur Fluidisierung des Bettinhaltes wird Stickstoff in einer Menge von 140 kg/h eingeblasen. Die Eintrittstemperatur des Fluidisiergases beträgt 140°C. Die Temperatur des Abgases beträgt 76°C. Als Sichtgas wird ebenfalls Stickstoff in einer Menge von 15 kg/h mit einer Temperatur von 50°C zugeführt. Der Inhalt des Wirbelbettes beträgt ca. 500 g. Die Granulierleistung beträgt ca. 2,5 kg pro Stunde. Man erhält ein frei fließendes Granulat mit einem mittleren Teilchendurchmesser von 360 Mikrometern. Die Granulate sind rund und weisen eine glatte Oberfläche auf. Aufgrund des konstanten Druckverlustes des Filters und des ebenfalls konstant bleibenden Bettinhalts ist von stationären Bedingungen hinsichtlich des Granulationsprozesses auszugehen.

### Beispiel S-33 - Herstellung von Teebeuteln mit Rooibos- bzw. schwarzem Tee und Extrudaten aus Beispiel S-31 bzw. Granulaten aus Beispiel S-32 für die Bereitung von Teegetränken mit kühlernder Wirkung.

Jeweils 800 g Rotbuschtee (Rooibos-Tee) wurden einmal mit 33 g der Extrudate aus Beispiel S-31 und einmal mit 30 g Granulaten aus Anwendungsbeispiel 32 gemischt, portioniert und anschließend in Teebeutel abgefüllt.

Jeweils 800 g schwarzer Tee (Blattgrad Fannings) wurden einmal mit 33 g der Extrudate aus Beispiel S-31 und einmal mit 30 g Granulaten aus Beispiel S-32 gemischt, portioniert und anschließend in Teebeutel abgefüllt.

### Testbeispiel 1 - Evaluierung des zeitlichen Verlaufs der Kühlintensitäten der erfindungsgemäß einzusetzenden Verbindungen.

Die erfindungsgemäß einzusetzenden Kühlsubstanzen wurden in Zahnpasta gemäß folgender Tabelle eingearbeitet.

**Tabelle**

| | |
|---|---|
| Entionisiertes Wasser | 27,52 |
| Sorbitol 700/0 | 45 |
| Solbrol M Na-Salz | 0.15 |
| Trinatriumphosphat | 0,1 |
| Saccharin | 0.2 |
| Natriummonofluorphosphat | 1.12 |
| PEG 1500 | 5 |
| Sident 9 (Abrasive Silica) | 10 |
| Sident 22 S (Dickende Silica) | 8 |
| Natriumcarboxymethylcellul ose | 0.9 |
| Titan (IV) oxid | 0.5 |
| Natriumlaurylsulfat (SLS) | 1,5 |
| jeweilige Kühlsubstanz | 0,01 |
| | |
| Total | 100 |

Alle Angaben in Gew.-%

Die sensorischen Eigenschaften der resultierenden Zahnpasta wurden durch ein trainiertes Panel (von 6 Personen) evaluiert. Dazu wurden die Zähne mit der die hier beschriebenen Verbindungen enthaltenden Zahnpasta zunächst 30 sec geputzt, dann der Zahnpastaschaum ausgespuckt und der Mund einmal mit Wasser ausgespült. Die Testpersonen beurteilten die Stärke des Kältegefühls auf einer Scala von 0 (kein Kältegefühl) bis 9 (extrem starkes Kältegefühl). Die Beurteilung des Kältegefühls erfolgte jeweils nach 30 sec, 1, 5, 10, 20, 30, 45 und 60 min.

Als erfindungsgemäße Verbindungen aus den Strukturklassen 1, 2 und 3 wurden die Verbindungen getestet.

Zum Vergleich wurden Zahnpasten mit gleicher Zusammensetzung getestet, die als konventionelle Kühlsubstanz Menthan-3-carbonsäure-N-ethylamid ("WS 3", s.a. US 4.150.052) enthielten.

## Patentansprüche

1. Verfahren zur in-vitro Modulation des Kälte-Menthol-Rezeptors TRPM8, wobei man den Rezeptor mit wenigstens einem Modulator in Kontakt bringt, der ausgewählt ist unter den folgenden Verbindungen:
| Nr. | Formel | Nr. | Formel |
|---|---|---|---|
| P1 | 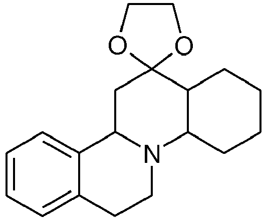 | P2 | 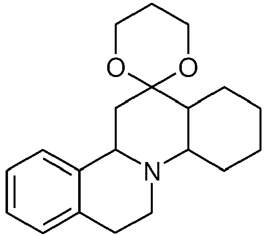 |
| P3 | 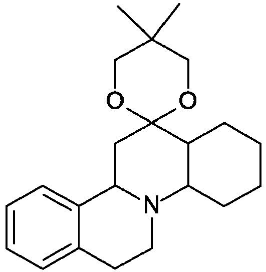 | P4 | 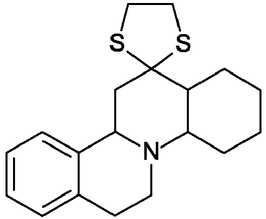 |
| P5 | 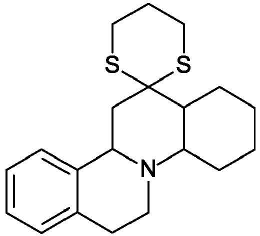 | P6 | 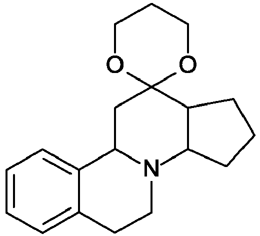 |
| P7 | 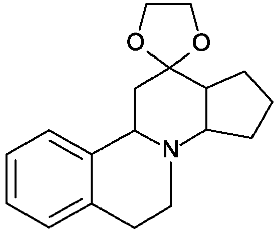 | P8 | 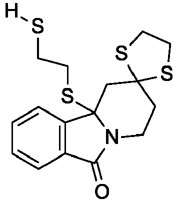 |
| P9 | 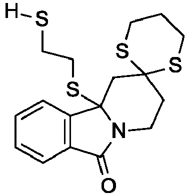 | P10 | 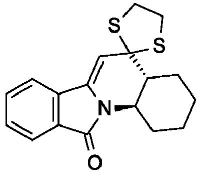 |
| P11 | 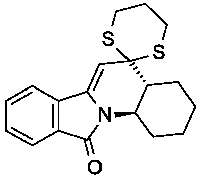 | P12 | 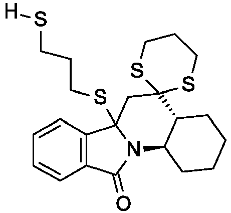 |
| P13 | 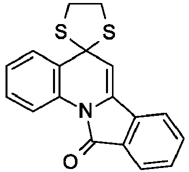 | P14 | 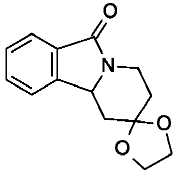 |
| P15 | 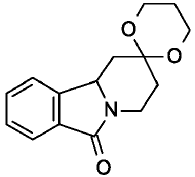 | P16 | 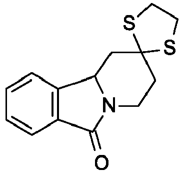 |
| P17 | 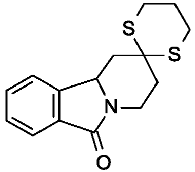 | P18 | 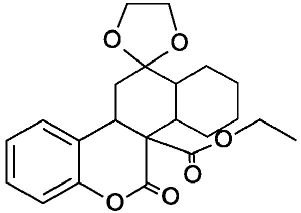 |
| P19 | 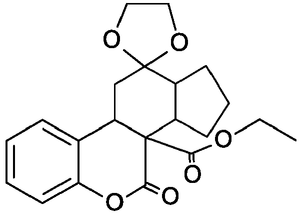 | P20 | 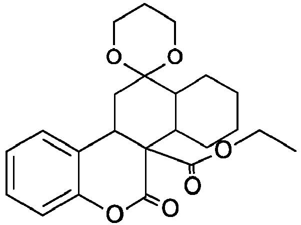 |
| P21 | 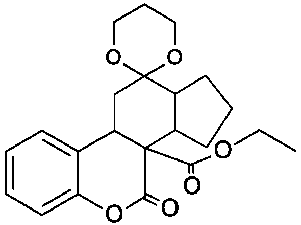 | P22 | 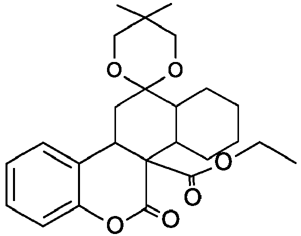 |
| P23 | 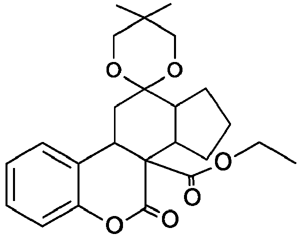 | P24 | 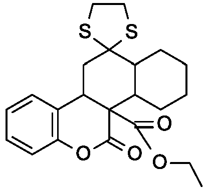 |
| P25 | 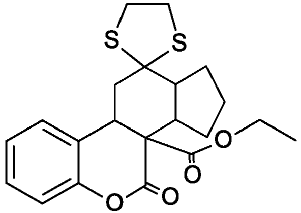 | P26 | 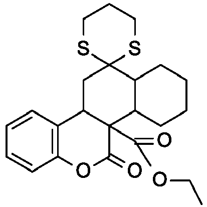 |
| P27 | 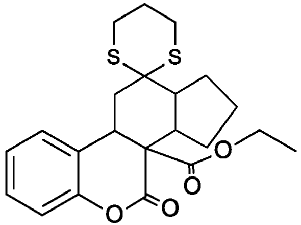 | P28 | 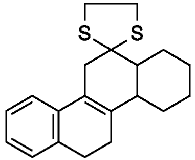 |
| P29 | 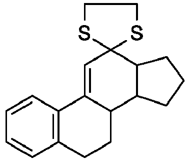 | P30 | 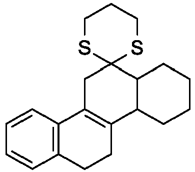 |
| P31 | 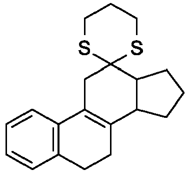 | | |
sowie Salze dieser Verbindungen, insbesondere Säureadditionssalze mit anorganischen oder insbesondere organischen, ein- oder insbesondere mehrwertigen Carbonsäuren;
und gegebenenfalls in Stereoisomeren-reiner Form oder als Gemisch von Stereoisomeren.

2. Verfahren nach Anspruch 1, wobei man den Rezeptor mit wenigstens einer Verbindung in Kontakt bringt, welche in einem zellulären Aktivitätstest unter Verwendung von Zellen, welche den humanen TRPM8-Rezeptor rekombinant exprimieren, die Permeabilität dieser Zellen für Ca²⁺ Ionen modulieren.

3. Verfahren nach einem der vorhergehenden Ansprüche, wobei die modulierende Verbindung auf die zelluläre Ca²⁺-lonen-Permeabilität agonistisch oder antagonistisch wirkt.

4. Verfahren nach einem der vorhergehenden Ansprüche, wobei die modulierende Verbindung ein TRPM8-Rezeptor-Agonist ist.

5. Verwendung einer Verbindung gemäß der Definition in Anspruch 1,
a) zur Induktion von Kältegefühl bei Mensch und/oder Tier zu nichttherapeutischen Zwecken;
d) zur Induktion eines Kältegefühls durch eine Verpackung zu nichttherapeutischen Zwecken; oder
e) zur Induktion eines Kältegefühls durch ein Textil zu nichttherapeutischen Zwecken.

6. Verbindung gemäß der Definition in Anspruch 1,
b) zur Verwendung als aktiven Bestandteil eines pharmazeutischen Mittels; oder
c) zur Verwendung bei der Behandlung von Prostatakarzinomen, zur Behandlung von Blasenschwäche oder in der Schmerztherapie.

7. Verwendung nach Anspruch 5, oder Verbindung zur Verwendung nach Anspruch 6, wobei ein Mittel eingesetzt wird, umfassend eine oder mehrere der Verbindungen gemäß der Definitionen aus einem der Ansprüche 1 bis 4 in einer Konzentration von 0,1 ppm bis 10 Gew-% bezogen auf das Gesamtgewicht des Mittels zur Erzielung einer Kühlwirkung auf Haut oder Schleimhaut, die verglichen mit der Kühlwirkung eines Mittels gleicher Zusammensetzung, bei dem lediglich die Verbindung oder die Verbindungen gemäß der Definitionen aus einem der Ansprüche 1 bis 4 gegen Menthancarbonsäure-N-ethylamid in gleicher Konzentration ausgetauscht sind, um wenigstens 10 Minuten verlängert ist.

8. Mittel enthaltend wenigstens eine Verbindung nach Anspruch 1, wobei das Mittel ausgewählt ist unter
a) pharmazeutischen Mitteln, wie Antitumormittel, Mittel zur Behandlung von Erkrankungen der Blase, Schmerzmittel;
b) Nahrungsmitteln, wie Speiseeis, Mousse, Creme, Getränke, Süßwaren,
c) Mundpflegemitteln, wie Zahnpasta, Mundwasser, Kaugummi,
d) Körperpflegemitteln, wie Haut- oder Haarpflegemitteln, wie Sonnencreme, Sonnenbrandcreme, Lotionen, Shampoos, Pflaster,
e) Schäumen und Gelen,
wobei die Verbindung nach Anspruch 1 ausgewählt ist unter den Verbindungen P1 bis P13, P15 und P17 bis P31; sowie Salzen dieser Verbindungen, insbesondere Säureadditionssalzen mit anorganischen oder insbesondere organischen, ein- oder insbesondere mehrwertigen Carbonsäuren; und gegebenenfalls in Stereoisomeren-reiner Form oder als Gemisch von Stereoisomeren.

9. Mittel nach Anspruch 8, umfassend
a) einen oder mehrere weitere Stoffe mit physiologischer Kühlwirkung, wobei der weitere Stoff bzw. einer, mehrere oder sämtliche der weiteren Stoffe (i) einen geschmacklichen Effekt verursachen oder (ii) keinen geschmacklichen Effekt verursachen,
und/oder
b) einen oder mehrere Aromastoffe ohne physiologische Kühlwirkung und/oder
c) einen oder mehrere trigeminal oder mundwässernd wirksame Stoffe ohne physiologische Kühlwirkung
und/oder
d) (iii) eine oder (iv) mehrere Verbindungen, die im Falle (iv) unabhängig von einander oder gemeinsam zusätzlich einen geschmacksmodulierenden Effekt und/oder einen trigeminalen und/oder einen mundwässernden Reiz verursachen.

10. Produkt enthaltend wenigstens einer Verbindung nach Anspruch 1, ausgewählt unter
a) Textilprodukten,
b) Verpackungsmaterialien,
c) Tabakprodukten;
d) Heilmitteln;
e) Hygieneprodukten, oder
f) Erfrischungstüchern,
wobei die Verbindung nach Anspruch 1 ausgewählt ist unter den Verbindungen P1 bis P13, P15 und P17 bis P31; sowie Salzen dieser Verbindungen, insbesondere Säureadditionssalzen mit anorganischen oder insbesondere organischen, ein- oder insbesondere mehrwertigen Carbonsäuren; und gegebenenfalls in Stereoisomeren-reiner Form oder als Gemisch von Stereoisomeren.

11. Mittel nach einem der Ansprüche 8 oder 9 zur Verwendung bei der Vorbeugung gegen, Bekämpfung oder Linderung von Husten-, Schnupfen-, Entzündungs-, Halsschmerz- oder Heiserkeitssymptomen.

12. Verbindung gemäß der Definition nach Anspruch 1, ausgewählt unter den Verbindungen P1 bis P13, P15 und P17 bis P31; sowie Salze dieser Verbindungen, insbesondere Säureadditionssalze mit anorganischen oder insbesondere organischen, ein- oder insbesondere mehrwertigen Carbonsäuren; und gegebenenfalls in Stereoisomeren-reiner Form oder als Gemisch von Stereoisomeren.

13. Verbindung gemäß der Definition nach Anspruch 12, ausgewählt unter den Verbindungen P4, P5 und P6; sowie Salze dieser Verbindungen, insbesondere Säureadditionssalze mit anorganischen oder insbesondere organischen, ein- oder insbesondere mehrwertigen Carbonsäuren; und gegebenenfalls in Stereoisomeren-reiner Form oder als Gemisch von Stereoisomeren.

## Claims

1. A method for the in vitro modulation of the cold menthol receptor TRPM8, wherein the receptor is brought into contact with at least one modulator selected from the following compounds:
| No. | Formula | No. | Formula |
|---|---|---|---|
| P1 | 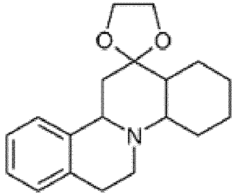 | P2 | 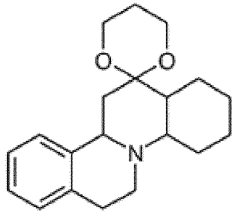 |
| P3 | 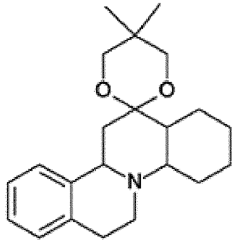 | P4 | 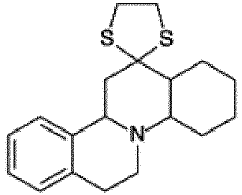 |
| P5 | 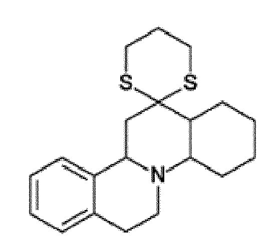 | P6 | 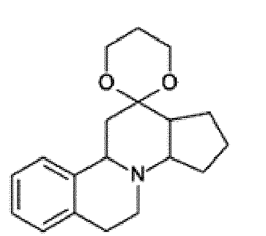 |
| P7 | 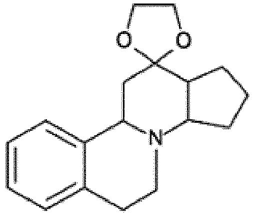 | P8 | 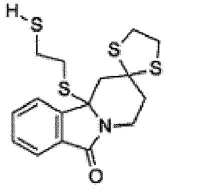 |
| P9 | 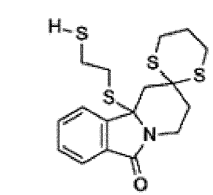 | P10 | 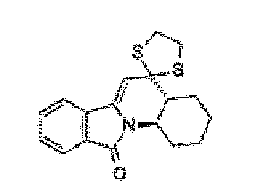 |
| P11 | 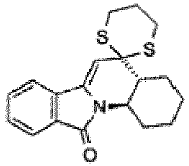 | P12 | 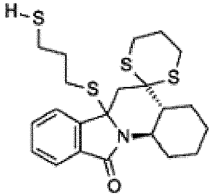 |
| P13 | 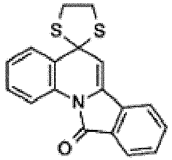 | P1 4 | 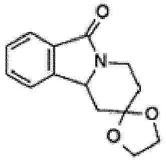 |
| P15 | 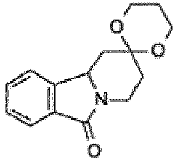 | P16 | 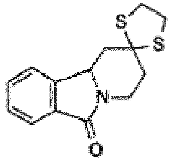 |
| P17 | 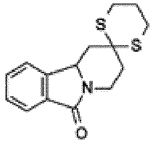 | P18 | 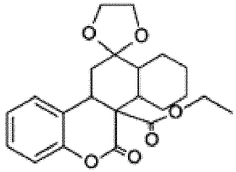 |
| P19 | 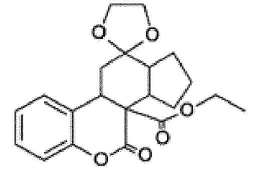 | P20 | 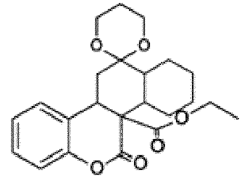 |
| P21 | 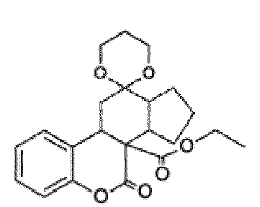 | P22 | 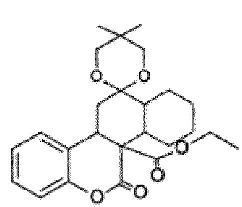 |
| P23 | 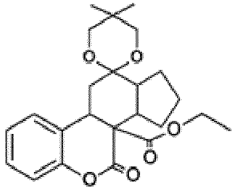 | P24 | 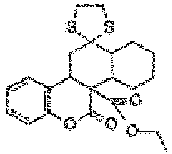 |
| P25 | 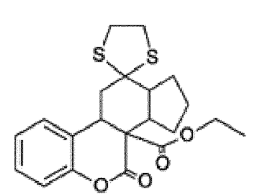 | P26 | 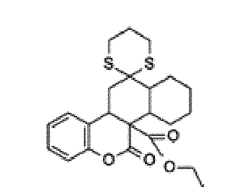 |
| P27 | 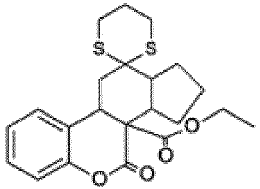 | P28 | 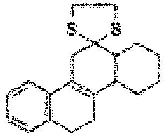 |
| P29 | 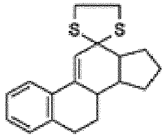 | P30 | 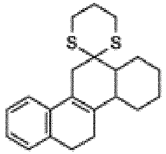 |
| P31 | 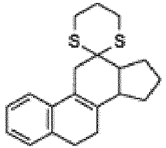 | | |
and salts of these compounds, particularly acid addition salts with inorganic or in particular organic, monobasic or especially polybasic carboxylic acids;
and optionally in stereoisomerically pure form or as a mixture of stereoisomers.

2. The method according to claim 1, wherein the receptor is brought into contact with at least one compound which, in a cellular activity test using cells which recombinantly express the human TRPM8 receptor, modulates the permeability of these cells to Ca²⁺ ions.

3. The method according to either of the preceding claims, wherein the modulating compound has an agonistic or antagonistic effect on the cellular Ca²⁺ ion permeability.

4. The method according to any of the preceding claims, wherein the modulating compound is a TRPM8 receptor agonist.

5. The use of a compound according to the definition in claim 1,
a) for inducing a sensation of coldness in humans and/or animals for non-therapeutic purposes;
d) for inducing a sensation of coldness through a packaging for non-therapeutic purposes; or
e) for inducing a sensation of coldness through a textile for non-therapeutic purposes.

6. The compound according to the definition in claim 1,
b) for use as active constituent of a pharmaceutical composition; or
c) for use in the treatment of prostate carcinomas, for the treatment of bladder weakness or in pain therapy.

7. The use according to claim 5, or compound for use according to claim 6, wherein a composition is used comprising one or more of the compounds according to the definitions from any of claims 1 to 4 at a concentration of from 0.1 ppm to 10% by weight, based on the total weight of the composition, for achieving a cooling effect on skin or mucosa which, compared with the cooling effect of a composition of identical composition in which merely the compound or the compounds according to the definitions from any of claims 1 to 4 are exchanged for menthanecarboxylic acid N-ethylamide in identical concentration, is extended by at least 10 minutes.

8. The composition comprising at least one compound according to claim 1, wherein the composition is selected from
a. pharmaceutical compositions, such as antitumor agents, agents for the treatment of diseases of the bladder, painkillers;
b. foods, such as ice cream, mousse, cream, beverages, confectionery,
c. mouthcare compositions, such as toothpaste, mouthwash, chewing gum,
d. bodycare compositions, such as skincare and haircare compositions, such as suncream, sunburn cream, lotions, shampoos, plasters,
e. foams and gels,
wherein the compound according to claim 1 is selected from compounds P1 to P13, P15 and P17 to P31; and salts of these compounds, particularly acid addition salts with inorganic or in particular organic, monobasic or especially polybasic carboxylic acids; and optionally in stereoisomerically pure form or as a mixture of stereoisomers.

9. The composition according to claim 8, comprising
a) one or more further substances with a physiological cooling effect, where the further substance or one, more than one or all of the further substances (i) cause a gustatory effect or (ii) do not cause a gustatory effect,
and/or
b) one or more aroma substances without a physiological cooling effect
and/or
c) one or more trigeminally or mouth-washing effective substances without a physiological cooling effect
and/or
d) (iii) one or (iv) two or more compounds which, in the case of (iv), independently of one another or together, additionally cause a taste-modulating effect and/or a trigeminal and/or mouth-washing stimulus.

10. The product comprising at least one compound according to claim 1, selected from
a) textile products,
b) packaging materials,
c) tobacco products;
d) remedies;
e) hygiene products, or
f) freshening wipes.
wherein the compound according to claim 1 is selected from compounds P1 to P13, P15 and P17 to P31; and salts of these compounds, particularly acid addition salts with inorganic or in particular organic, monobasic or especially polybasic carboxylic acids; and optionally in stereoisomerically pure form or as a mixture of stereoisomers.

11. The composition according to claims 8 or 9 for use in preventing, controlling or alleviating symptoms of coughing, sneezing, inflammation, throat pain or hoarseness.

12. The compound according to the definition according to claim 1, selected from the compounds P1 to P13, P15 and P17 to P31; and salts of these compounds, particularly acid addition salts with inorganic or in particular organic, monobasic or especially polybasic carboxylic acids; and optionally in stereoisomerically pure form or as a mixture of stereoisomers.

13. The compound according to the definition according to claim 12, selected from the compounds P4, P5 and P6; and salts of these compounds, particularly acid addition salts with inorganic or in particular organic, monobasic or especially polybasic carboxylic acids; and optionally in stereoisomerically pure form or as a mixture of stereoisomers.

## Revendications

1. Procédé pour la modulation in vitro du récepteur au froid et au menthol TRPM8, dans lequel on met en contact le récepteur avec au moins un modulateur qui est choisi parmi les composés suivants :
| N° | Formule | N° | Formule |
|---|---|---|---|
| P1 | 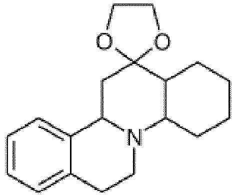 | P2 | 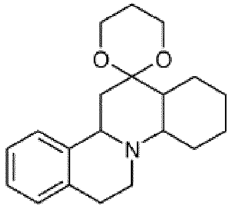 |
| P3 | 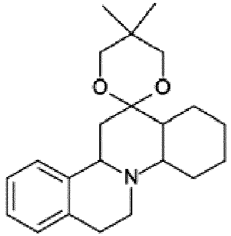 | P4 | 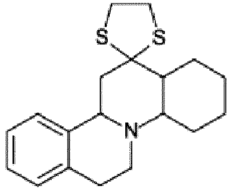 |
| P5 | 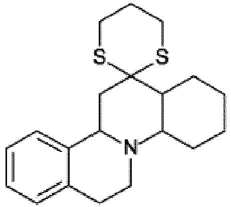 | P6 | 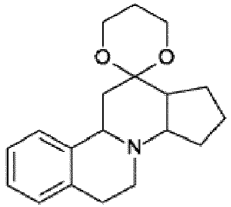 |
| P7 | 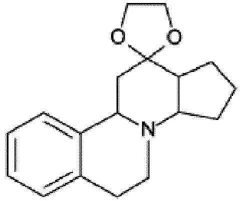 | P8 | 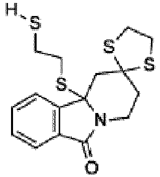 |
| P9 | 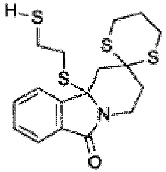 | P10 | 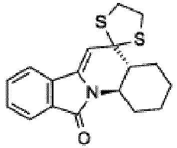 |
| P11 | 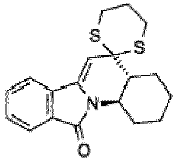 | P12 | 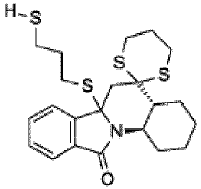 |
| P13 | 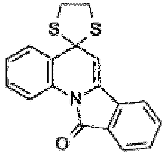 | P14 | 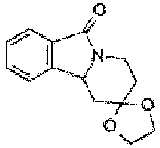 |
| P15 | 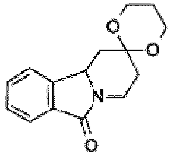 | P16 | 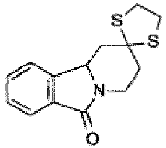 |
| P17 | 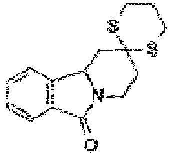 | P18 | 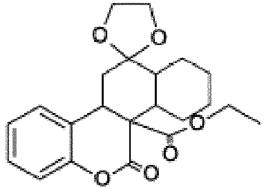 |
| P19 | 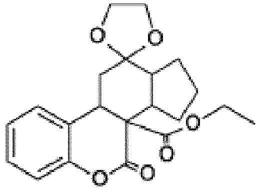 | P20 | 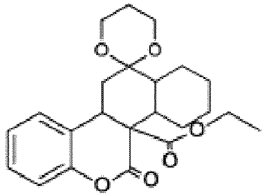 |
| P21 | 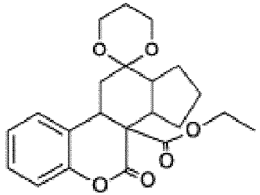 | P22 | 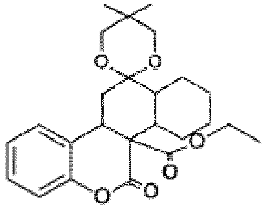 |
| P23 | 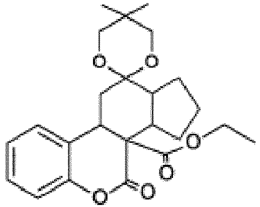 | P24 | 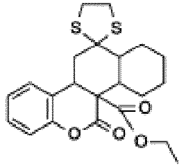 |
| P25 | 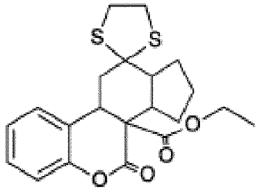 | P26 | 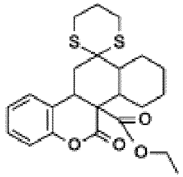 |
| P27 | 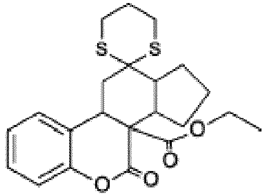 | P28 | 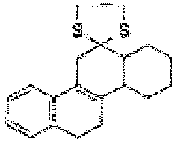 |
| P29 | 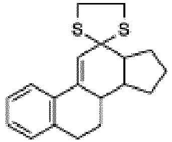 | P30 | 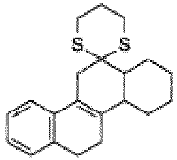 |
| P31 | 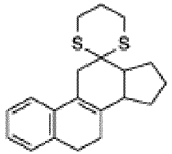 | | |
ainsi que les sels de ces composés, en particulier les sels d'addition acide avec des acides carboxyliques inorganiques ou en particulier organiques, mono- ou en particulier polyvalents ;
et éventuellement sous forme stéréoisomère pure ou sous forme d'un mélange de stéréoisomères.

2. Procédé selon la revendication 1, dans lequel on met en contact le récepteur avec au moins un composé qui, dans un test d'activité cellulaire par utilisation de cellules qui expriment par recombinaison le récepteur TRPM8 humain, modulent la perméabilité de ces cellules pour des ions Ca²⁺.

3. Procédé selon l'une des revendications précédentes, dans lequel le composé modulateur agit d'une manière agoniste ou antagoniste sur la perméabilité cellulaire des ions Ca²⁺.

4. Procédé selon l'une des revendications précédentes, dans lequel le composé modulateur est un agoniste du récepteur TRPM8.

5. Utilisation d'un composé selon la définition de la revendication 1,
a) pour induire une sensation de froid chez l'homme et/ou l'animal à des fins non-thérapeutiques ;
d) pour induire une sensation de froid grâce à un emballage, à des fins non-thérapeutiques ; ou
e) pour induire une sensation de froid grâce à un textile, à des fins non-thérapeutiques.

6. Composé selon la définition de la revendication 1,
b) pour une utilisation en tant que constituant actif d'un produit pharmaceutique ; ou
c) pour une utilisation lors du traitement de carcinomes de la prostate, pour le traitement d'une faiblesse de la vessie ou dans le cadre d'une thérapie de la douleur.

7. Utilisation selon la revendication 5, ou composé pour une utilisation selon la revendication 6, dans lequel on utilise un produit comprenant un ou plusieurs des composés selon les définitions de l'une des revendications 1 à 4 à une concentration de 0,1 ppm à 10 % en poids par rapport au poids total du produit, pour obtenir un effet de froid sur la peau ou la muqueuse, qui, par comparaison avec l'effet de froid d'un produit ayant la même composition, dans lequel seul le composé ou les composés selon les définitions de l'une des revendications 1 à 4 sont remplacés par le N-éthylamide de l'acide méthanecarboxylique à une même concentration, est prolongé d'au moins 10 minutes.

8. Agent contenant au moins un composé selon la revendication 1, le produit étant choisi parmi :
a) les produits pharmaceutiques tels que les agents antitumoraux, les agents pour le traitement de maladies de la vessie, les antalgiques ;
b) les produits alimentaires tels que la crème glacée, la mousse, les crèmes, les boissons, les confiseries,
c) les produits pour les soins de la bouche tels qu'une pâte dentaire, un bain de bouche, une gomme à mâcher,
d) les produits pour les soins corporels tels que les produits pour les soins de la peau ou des cheveux, tels qu'une crème solaire, une crème bronzante, des lotions, des shampoings, un pansement,
e) les mousses et les gels,
le composé selon la revendication 1 étant choisi parmi les composés P1 à P13, P15 et P17 à P31 ; ainsi que les sels de ces composés, en particulier les sels d'addition acide avec des acides carboxyliques inorganiques ou en particulier organiques, mono- ou en particulier polyvalents ; et éventuellement sous forme stéréoisomère pure ou sous forme d'un mélange de stéréoisomères.

9. Agent selon la revendication 8, comprenant
a) une ou plusieurs substances supplémentaires ayant un effet de froid physiologique, la substance supplémentaire, ou une, plusieurs ou la totalité des substances supplémentaires (i) provoquant un effet de saveur ou (ii) ne provoquant aucun effet de saveur,
et/ou
b) une ou plusieurs substances aromatiques sans effet de froid physiologique,
et/ou
c) une ou plusieurs substances à effet trigéminal ou salivaire sans effet de froid physiologique,
et/ou
d) (iii) un ou (iv) plusieurs composés, qui dans le cas (iv) provoquent indépendamment les uns des autres, ou ensemble, en outre un effet modulateur de goût et/ou une irritation trigéminale et/ou salivaire.

10. Produit contenant au moins un composé selon la revendication 1, choisi parmi
a) les produits textiles,
b) les matériaux d'emballage,
c) les produits de tabac ;
d) les produits thérapeutiques ;
e) les produits d'hygiène, ou
f) les lingettes rafraîchissantes,
le composé selon la revendication 1 étant choisi parmi les composés P1 à P13, P15 et P17 à P31 ; ainsi que les sels de ces composés, en particulier les sels d'addition acide avec des acides carboxyliques inorganiques ou en particulier organiques, mono- ou en particulier polyvalents ; et éventuellement sous forme stéréoisomère pure ou sous forme d'un mélange de stéréoisomères.

11. Agent selon l'une des revendications 8 ou 9 pour une utilisation pour la prévention ou le soulagement des symptômes de toux, d'éternuement, d'inflammation, de douleurs du cou ou d'enrouement, ou pour la lutte contre ces symptômes.

12. Composé selon la revendication 1, choisi parmi les composés P1 à P13, P15 et P17 à P31 ; ainsi que les sels de ces composés, en particulier les sels d'addition acide avec des acides carboxyliques inorganiques ou en particulier organiques, mono- ou en particulier polyvalents ; et éventuellement sous forme stéréoisomère pure ou sous forme d'un mélange de stéréoisomères.

13. Composé selon la revendication 12, choisi parmi les composés P4, P5 et P6 ; ainsi que les sels de ces composés, en particulier les sels d'addition acide avec des acides carboxyliques inorganiques ou en particulier organiques, mono- ou en particulier polyvalents ; et éventuellement sous forme stéréoisomère pure ou sous forme d'un mélange de stéréoisomères.
